(19) Europäisches Patentamt
European Patent Office
Office européen des brevets

(11) EP 3 797 102 B1

(12) # EUROPEAN PATENT SPECIFICATION

(45) Date of publication and mention of the grant of the patent:
**16.11.2022 Bulletin 2022/46**

(21) Application number: **19728875.6**

(22) Date of filing: **08.05.2019**

(51) International Patent Classification (IPC):
***C07D 401/14*** (2006.01) ***C09K 11/06*** (2006.01)
***H01L 51/50*** (2006.01) ***H05B 33/10*** (2006.01)
***H01L 51/00*** (2006.01) ***H05B 33/14*** (2006.01)

(52) Cooperative Patent Classification (CPC):
**C07D 401/14; C09K 11/06; H01L 51/0067; H01L 51/0072; H05B 33/14;** H01L 51/5072; Y02E 10/549; Y02P 70/50

(86) International application number:
**PCT/EP2019/061835**

(87) International publication number:
**WO 2019/224006 (28.11.2019 Gazette 2019/48)**

(54) **CARBAZOLE-PYRIDINE-CYANOPHENYL DERIVATIVES AND RELATED COMPOUNDS FOR USE AS LUMINESCENT EMITTERS IN OLEDS**

CARBAZOLE-PYRIDINE-CYANOPHENYL DERIVATE UND ÄHNLICHE VERBINDUNGEN ZUR VERWENDUNG ALS LUMINESZENZEMITTER IN OLEDS

DÉRIVÉS CARBAZOLE-PYRIDINE-CYANOPHENYL ET COMPOSÉS SIMILAIRES À ÊTRE UTILISÉS COMME ÉMETTEURS LUMINESCENTS DANS D'OLEDS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.05.2018 DE 102018112229**

(43) Date of publication of application:
**31.03.2021 Bulletin 2021/13**

(73) Proprietor: **Samsung Display Co., Ltd.**
**Gyeonggi-do 17113 (KR)**

(72) Inventors:
- **ESTEBAN, Alhama Arjona**
  **76131 Karlsruhe (DE)**
- **BERGMANN, Larissa**
  **76199 Karlsruhe (DE)**

(74) Representative: **Dr. Weitzel & Partner**
**Patent- und Rechtsanwälte mbB**
**Friedenstrasse 10**
**89522 Heidenheim (DE)**

(56) References cited:
**EP-A1- 3 287 451**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 797 102 B1

**Description**

The invention relates to light-emitting organic molecules and their use in organic light-emitting diodes (OLEDs) and in other optoelectronic devices. Organic molecules are known from EP 3 287 451 A.

**Description**

The object of the present invention is to provide molecules which are suitable for use in optoelectronic devices.

This object is achieved by the invention which provides a new class of organic molecules.

The organic molecules of the invention are preferably purely organic molecules, i.e. they do not contain any metal ions, which is in contrast to metal complexes known for use in optoelectronic devices. Therefore, according to the present invention, it is preferred that the organic molecules are free of metal atoms or metal ions. The pure organic molecules may, however, include metalloids, in particular, B and Si or exceptionally Sn, Se, and Ge.

According to the present invention, the organic molecules exhibit emission maxima in the blue, sky-blue or green spectral range. The organic molecules exhibit in particular emission maxima between 420 nm and 520 nm, preferably between 440 nm and 495 nm, more preferably between 450 nm and 470 nm. The photoluminescence quantum yields of the organic molecules according to the invention are, in particular, 20% or more. The molecules according to the invention exhibit in particular thermally activated delayed fluorescence (TADF). The use of the molecules according to the invention in an optoelectronic device, for example an organic light-emitting diode (OLED), leads to higher efficiencies of the device. Corresponding OLEDs have a higher stability than OLEDs with known emitter materials and comparable color.

The organic light-emitting molecules of the invention comprise or consist of one first chemical moiety comprising or consisting of a structure of Formula I,

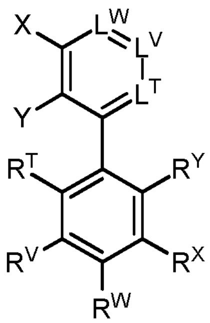

Formula I

and

- two second chemical moieties, each independently from another comprising or consisting of a structure of Formula II,

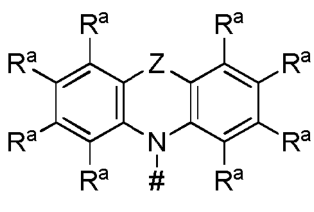

Formula II

wherein the first chemical moiety is linked to each of the two second chemical moieties via a single bond.

$L^T$ is N or C-$R^1$.
$L^V$ is N or C-$R^1$.
$L^W$ is N or C-W.
X is the binding site of a single bond linking the first chemical moiety to one of the two second chemical moieties or is $R^2$.
Y is the binding site of a single bond linking the first chemical moiety to one of the two second chemical moieties or is $R^2$.
W is the binding site of a single bond linking the first chemical moiety to one of the two second chemical moieties

or is $R^2$.
$R^T$ is selected from the group consisting of $R^B$ and $R^I$.
$R^V$ is selected from the group consisting of $R^B$ and $R^I$:
$R^W$ is the binding site of a single bond linking the first chemical moiety to one of the two second chemical moieties or is selected from the group consisting of $R^B$ and $R^I$.
$R^X$ is the binding site of a single bond linking the first chemical moiety to one of the two second chemical moieties or is selected from the group consisting of $R^B$ and $R^I$.
$R^Y$ is the binding site of a single bond linking the first chemical moiety to one of the two second chemical moieties or is selected from the group consisting of $R^B$ and $R^I$.
$R^B$ comprises or consists of a structure of Formula BZN (an aromatic 6-ring with five substituents R"):

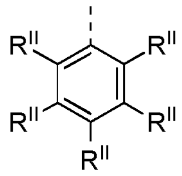

Formula BZN

wherein the dotted line represents the single bond linking the structure of formula BZN with the lower benzene ring of the structure shown in formula I,

# represents the binding site of a single bond linking the second chemical moieties to the first chemical moiety;
Z is at each occurrence independently from another selected from the group consisting of a direct bond, $CR^3R^4$, $C=CR^3R^4$, C=O, $C=NR^3$, $NR^3$, O, $SiR^3R^4$, S, S(O) and $S(O)_2$;
$R^1$ is at each occurrence independently from another selected from the group consisting of hydrogen, deuterium,
$C_1$-$C_5$-alkyl,

wherein one or more hydrogen atoms are optionally substituted by deuterium; C2-C8-alkenyl, wherein one or more hydrogen atoms are optionally substituted by deuterium; C2-C8-alkynyl, wherein one or more hydrogen atoms are optionally substituted by deuterium; and $C_6$-$C_{18}$-aryl, which is optionally substituted with one or more substituents $R^6$.

$R^2$ is at each occurrence independently from another selected from the group consisting of hydrogen, deuterium,
$C_1$-$C_5$-alkyl,

wherein one or more hydrogen atoms are optionally substituted by deuterium; $C_2$-$C_8$-alkenyl, wherein one or more hydrogen atoms are optionally substituted by deuterium; $C_2$-$C_8$-alkynyl, wherein one or more hydrogen atoms are optionally substituted by deuterium; and $C_6$-$C_{18}$-aryl, which is optionally substituted with one or more substituents $R^6$.

$R^I$ is at each occurrence independently from another selected from the group consisting of hydrogen, deuterium,
$C_1$-$C_5$-alkyl,

wherein one or more hydrogen atoms are optionally substituted by deuterium; C2-C8-alkenyl, wherein one or more hydrogen atoms are optionally substituted by deuterium; C2-C8-alkynyl, wherein one or more hydrogen atoms are optionally substituted by deuterium; and $C_6$-$C_{18}$-aryl, which is optionally substituted with one or more substituents $R^6$.

$R^{II}$ is at each occurrence independently from another selected from the group consisting of $R^{III}$ and $R^{IV}$.
$R^{III}$ is selected from the group consisting of CN and $CF_3$.
$R^{IV}$ is at each occurrence independently from another selected from the group consisting of hydrogen, deuterium,

$C_1$-$C_5$-alkyl,

wherein one or more hydrogen atoms are optionally substituted by deuterium; C2-C8-alkenyl, wherein one or more hydrogen atoms are optionally substituted by deuterium; C2-C8-alkynyl, wherein one or more hydrogen atoms are optionally substituted by deuterium; and $C_6$-$C_{18}$-aryl, which is optionally substituted with one or more substituents $R^6$.

$R^a$, $R^3$ and $R^4$ are at each occurrence independently from another selected from the group consisting of hydrogen, deuterium, $N(R^5)_2$, $OR^5$, $Si(R^5)_3$, $B(OR^5)_2$, $OSO_2R^5$, $CF_3$, CN, F, Br, I, $C_1$-$C_{40}$-alkyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$;
$C_3$-$C_{57}$-heteroaryl,
which is optionally substituted with one or more substituents $R^5$; and
a mono- or polycyclic, aliphatic, aromatic and/or benzo-fused ring system formed by ring-closure with one or more of the other substituents selected from the group consisting of $R^a$, $R^3$, $R^4$ and $R^5$.
$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$; and
a mono- or polycyclic, aliphatic, aromatic and/or benzo-fused ring system formed by ring-closure with one or more of the other substituents selected from the group consisting of $R^a$, $R^3$, $R^4$ and $R^5$.
$R^6$ is at each occurrence independently from another selected from the group consisting of hydrogen, deuterium, OPh, $CF_3$, CN, F,
$C_1$-$C_5$-alkyl,
wherein optionally one or more hydrogen atoms are independently from each other substituted by deuterium, CN, $CF_3$, or F;
$C_1$-$C_5$-alkoxy,
wherein optionally one or more hydrogen atoms are independently from each other substituted by deuterium, CN, $CF_3$, or F;
$C_1$-$C_5$-thioalkoxy,
wherein optionally one or more hydrogen atoms are independently from each other substituted by deuterium, CN, $CF_3$, or F;
C2-C5-alkenyl,
wherein optionally one or more hydrogen atoms are independently from each other substituted by deuterium, CN, $CF_3$, or F;
C2-C5-alkynyl,
wherein optionally one or more hydrogen atoms are independently from each other substituted by deuterium, CN, $CF_3$, or F;
$C_6$-$C_{18}$-aryl,

which is optionally substituted with one or more $C_1$-$C_5$-alkyl substituents; $C_3$-$C_{17}$-heteroaryl, which is optionally substituted with one or more $C_1$-$C_5$-alkyl substituents; $N(C_6$-$C_{18}$-aryl$)_2$,

$N(C_3$-$C_{17}$-heteroaryl$)_2$; and
$N(C_3$-$C_{17}$-heteroaryl$)(C_6$-$C_{18}$-aryl$)$.

Optionally, the substituents from the group consisting of $R^a$, $R^3$, $R^4$ or $R^5$ independently from each other form a mono- or polycyclic, aliphatic, aromatic and/or benzo-fused ring system with one or more of the other (remaining) substituents selected from the group consisting of $R^a$, $R^3$, $R^4$ or $R^5$.

If the substituents $R^a$, $R^3$ and $R^4$ are at each occurrence independently from another selected to be a mono- or polycyclic, aliphatic, aromatic and/or benzo-fused ring system together with one or more of the other substituents $R^a$, $R^3$, $R^4$ and/or $R^5$ within the organic molecule, it is preferred for $R^a$, $R^3$ and $R^4$ to form the mono- or polycyclic, aliphatic, aromatic and/or benzo-fused ring system with a substituent $R^a$, $R^3$, $R^4$ or $R^5$ that is positioned adjacent to it in the ring.

Furthermore, it is more preferred in certain embodiments for $R^a$, $R^3$ and $R^4$ to form the mono- or polycyclic, aliphatic, aromatic and/or benzo-fused ring system including at least one or more rings with a ring size of 5 to 8 atoms.

Moreover, it is preferred for the mono- or polycyclic, aliphatic, aromatic and/or benzo-fused ring system described above to include at least one or more aromatic rings.

In certain embodiments, the mono- or polycyclic, aliphatic, aromatic and/or benzo-fused ring system mentioned above and herein may itself comprise at least one substituent $R^5$.

If the substituent $R^5$ is at each occurrence and independently from another selected to form a mono- or polycyclic, aliphatic, aromatic and/or benzo-fused ring system with one or more of the other substituents $R^a$, $R^3$, $R^4$ or $R^5$ within the organic molecule; it is preferred for $R^5$ to form the mono- or polycyclic, aliphatic, aromatic and/or benzo-fused ring system with an immediately ring-adjacent substituent selected from the group consisting of $R^a$, $R^3$, $R^4$ and $R^5$. Furthermore, in certain embodiments, $R^5$ forms the mono- or polycyclic, aliphatic, aromatic and/or benzo-fused ring system including at least one or more rings with a ring of a size of 5 to 8 atoms.

Additionally, in certain embodiments of the organic molecule, the mono- or polycyclic, aliphatic, aromatic and/or benzo-fused ring system includes at least one (one, two, etc.) aromatic rings.

In further embodiments, the mono- or polycyclic, aliphatic, aromatic and/or benzo-fused ring system itself comprises at least one substituent $R^5$.

Moreover, exactly one ring member selected from the group consisting of $L^T$, $L^V$ and $L^W$ is N, exactly one substituent selected from the group consisting of $R^T$, $R^V$, $R^X$, $R^Y$ and $R^W$ is $R^B$; exactly one substituent $R^{II}$ is $R^{III}$ (i.e. the other substituents $R^{II}$ of the aromatic 6-ring comprising of consisting of a structure of formula BZN are $R^{IV}$);

exactly one substituent selected from the group consisting of W, Y and X represents the binding site of a single bond linking the first chemical moiety and one of the two second chemical moieties, and
exactly one substituent selected from the group consisting of $R^W$, $R^Y$ and $R^X$ represents the binding site of a single bond linking the first chemical moiety and one of the two second chemical moieties.

In one embodiment of the invention, exactly one substituent selected from the group consisting of $L^T$, $L^V$ and $L^W$ is N;

exactly one substituent selected from the group consisting of $R^T$, $R^V$ and $R^W$ is $R^B$;
exactly one substituent $R^{II}$ is $R^{III}$;
exactly one substituent selected from the group consisting of W, Y and X represents the binding site of a single bond linking the first chemical moiety and one of the two second chemical moieties;
and exactly one substituent selected from the group consisting of $R^W$, $R^X$ and $R^Y$ represents the binding site of a single bond linking the first chemical moiety and one of the two second chemical moieties;
and apart from that the aforementioned definitions apply.

In one embodiment of the invention, first chemical moiety comprises or consists of a structure of Formula Ia:

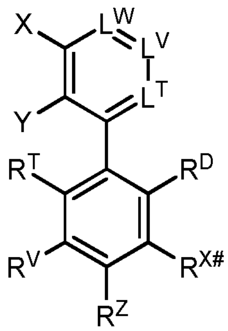

Formula Ia

wherein $R^T$, $R^V$, $L^T$, $L^V$, $L^W$, X, and Y are defined as above,

$R^Z$ is selected from the group consisting of $R^1$ and $R^B$,
$R^{X\#}$ is selected from the group consisting of $R^1$ and $R^B$,
$R^D$ is the binding site of a single bond linking the first chemical moiety to one of the two second chemical moieties, wherein exactly one substituent selected from the group consisting of $L^T$, $L^V$ and $L^W$ is N; wherein exactly one substituent selected from the group consisting of W, Y and X represents the binding site of a single bond linking the first chemical moiety and one of the two second chemical moieties;

and wherein exactly one substituent selected from the group consisting of $R^V$, $R^T$, $R^{X\#}$ and $R^Z$ is $R^B$; and apart from that the aforementioned definitions apply.

In one embodiment of the invention, first chemical moiety comprises or consists of a structure of Formula Iaa:

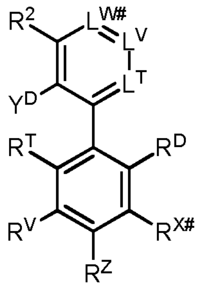

Formula Iaa

wherein $L^V$, $L^T$, $R^2$, $R^T$, and $R^V$ are defined as above,

$R^Z$ is selected from the group consisting of $R^I$ and $R^B$,
$R^{X\#}$ is selected from the group consisting of $R^1$ and $R^B$,
$R^D$ is the binding site of a single bond linking the first chemical moiety to one of the two second chemical moieties,
$L^{W\#}$ is N or C-$R^2$;
$Y^D$ is the binding site of a single bond linking the first chemical moiety to one of the two second chemical moieties or is $R^2$,
wherein exactly one substituent selected from the group consisting of $L^T$, $L^V$ and $L^{W\#}$ is N;
wherein exactly one substituent selected from the group consisting of $R^V$, $R^T$, $R^{X\#}$ and $R^Z$ is $R^B$; and apart from that the aforementioned definitions apply.

In one embodiment, $R^1$, $R^2$, $R^I$ and $R^{IV}$ is at each occurrence independently from another selected from the group consisting of hydrogen (H), methyl, mesityl, tolyl and phenyl (Ph). The term "tolyl" refers to 2-tolyl, 3-tolyl, and 4-tolyl.
In one embodiment, $R^1$, $R^2$, $R^I$ and $R^{IV}$ is at each occurrence independently from another selected from the group consisting of hydrogen (H), methyl, and phenyl.
In one embodiment, $R^1$, $R^2$, $R^I$ and $R^{IV}$ is at each occurrence hydrogen (H).
In one embodiment, $R^V$ is $R^B$.
In one embodiment, $R^T$ is $R^B$.
In one embodiment, $R^W$ is $R^B$.
In one embodiment, $R^X$ is $R^B$.
In one embodiment, $R^Y$ is $R^B$.
In one embodiment, $L^W$ is N.
In one embodiment, $L^T$ is N.
In one embodiment, $L^V$ is N.
In one embodiment, $R^V$ is $R^B$ and $L^W$ is N.
In one embodiment, $R^V$ is $R^B$ and $L^V$ is N.
In one embodiment, $R^V$ is $R^B$ and $L^T$ is N.
In one embodiment, $R^W$ is $R^B$ and $L^W$ is N.
In one embodiment, $R^W$ is $R^B$ and $L^V$ is N.
In one embodiment, $R^W$ is $R^B$ and $L^T$ is N.
In one embodiment, $R^B$ consists or contains of a structure selected from the group consisting of:

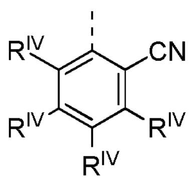
,
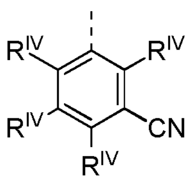
,
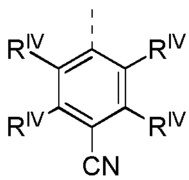
,

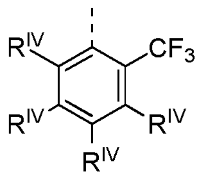, 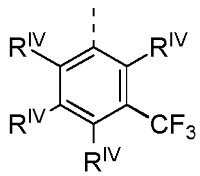, 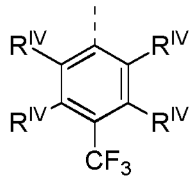.

In one embodiment, $R^B$ consists or contains of a structure selected from the group consisting of:

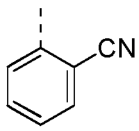, 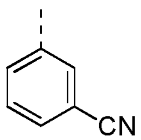, 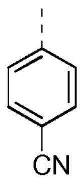, 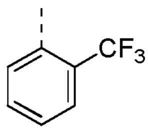, 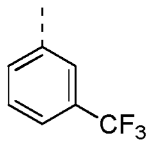, 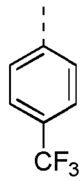.

In one embodiment, $R^B$ consists or contains of a structure selected from the group consisting of:

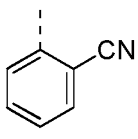, 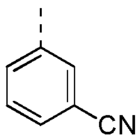, 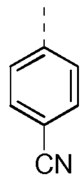.

In one embodiment, $R^B$ is

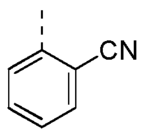.

In one embodiment $R^{III}$ is CN.

In a further embodiment of the invention, the two second chemical moieties each at each occurrence independently from another comprise or consist of a structure of Formula IIa:

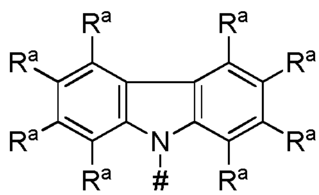

Formula IIa

wherein # and $R^a$ are defined as above.

In a further embodiment of the invention, $R^a$ is at each occurrence independently from another selected from the group consisting of: H,

Me,
$^iPr$,
$^tBu$,
CN,
$CF_3$,
Ph, which is optionally substituted with one or more substituents independently from each other selected from the

group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph,
carbazolyl, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph,
and $N(Ph)_2$.

In a further embodiment of the invention, $R^a$ is at each occurrence independently from another selected from the group consisting of H,

Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph, and
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^a$ is at each occurrence independently from another selected from the group consisting of: H,

Me,
$^{t}Bu$,
Ph, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^a$ is H at each occurrence.

In a further embodiment of the invention, the two second chemical moieties each at each occurrence independently from another comprise or consist of a structure selected from the group consisting of: Formula IIb, Formula IIb-2, Formula IIb-3, and Formula IIb-4:

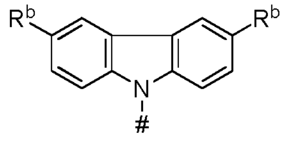
Formula IIb

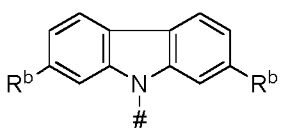
Formula IIb-2

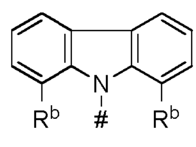
Formula IIb-3

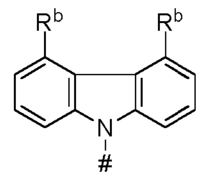
Formula IIb-4

wherein

$R^b$ is at each occurrence independently from another selected from the group consisting of: deuterium,
$N(R^5)_2$,
$OR^5$,
$Si(R^5)_3$,

$B(OR^5)_2$,
$OSO_2R^5$,
$CF_3$,
CN,
F,
Br,
I,
$C_1$-$C_{40}$-alkyl,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.

For the other variables, the aforementioned definitions apply.

In one additional embodiment of the invention, the two second chemical moieties at each occurrence independently from another comprise or consist of a structure selected from the group consisting of: Formula IIc Formula IIc-2, Formula IIc-3 and Formula IIc-4:

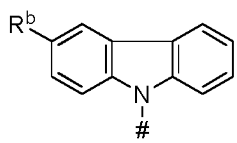
Formula IIc

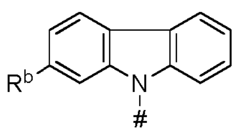
Formula IIc-2

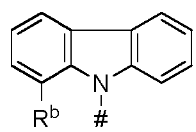
Formula IIc-3

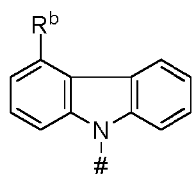
Formula IIc-4

wherein the aforementioned definitions apply.

In a further embodiment of the invention, $R^b$ is at each occurrence independently from another selected from

the group consisting of:

Me,
$^{i}$Pr,
$^{t}$Bu,
CN,
$CF_3$,
Ph, which is optionally substituted with one or more substituents independently from each other selected from the group consisting of Me, $^{i}$Pr, $^{t}$Bu, CN, $CF_3$, and Ph,
pyridinyl, which is optionally substituted with one or more substituents independently from each other selected from the group consisting of Me, $^{i}$Pr, $^{t}$Bu, CN, $CF_3$, and Ph,
pyrimidyl, which is optionally substituted with one or more substituents independently from each other selected from the group consisting of Me, $^{i}$Pr, $^{t}$Bu, CN, $CF_3$, and Ph,
carbazolyl, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph,
and $N(Ph)_2$.

In a further embodiment of the invention, $R^b$ is at each occurrence independently from another selected from the group consisting of:

Me,
$^{i}$Pr,
$^{t}$Bu,
CN,
$CF_3$,
Ph, which is optionally substituted with one or more substituents independently from each other selected from the group consisting of Me, $^{i}$Pr, $^{t}$Bu, CN, $CF_3$, and Ph,
pyridinyl, which is optionally substituted with one or more substituents independently from each other selected from the group consisting of Me, $^{i}$Pr, $^{t}$Bu, CN, $CF_3$, and Ph,
pyrimidinyl, which is optionally substituted with one or more substituents independently from each other selected from the group consisting of Me, $^{i}$Pr, $^{t}$Bu, CN, $CF_3$, and Ph, and
triazinyl, which is optionally substituted with one or more substituents independently from each other selected from the group consisting of Me, $^{i}$Pr, $^{t}$Bu, CN, $CF_3$, and Ph.

In a further embodiment of the invention, $R^b$ is at each occurrence independently from another selected from the group consisting of:

Me,
$^{t}$Bu,
Ph, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph.

In the following, exemplary embodiments of the second chemical moiety are shown:

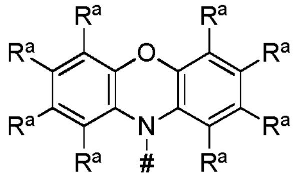
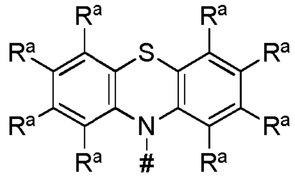
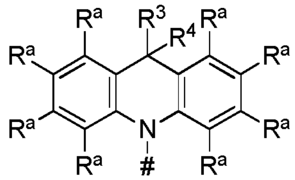
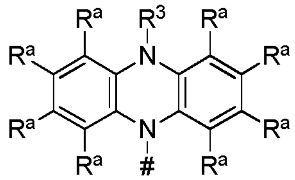

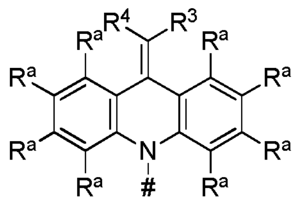
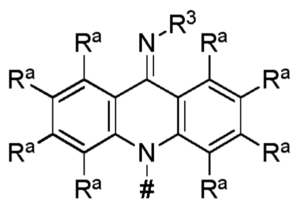
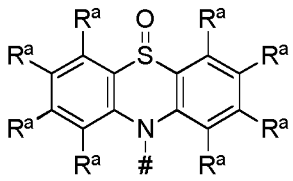

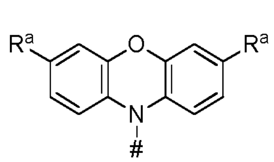
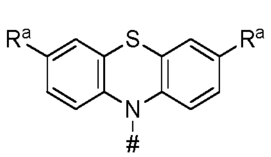
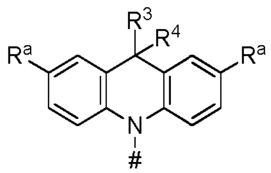
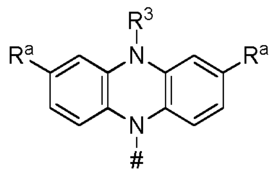

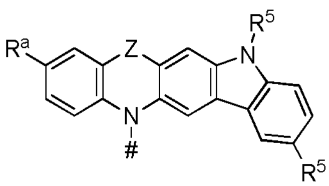
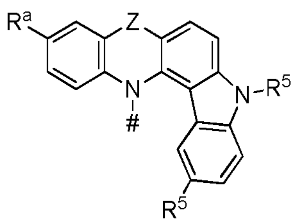
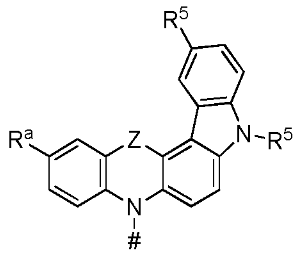

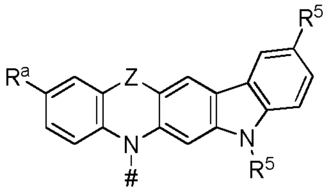
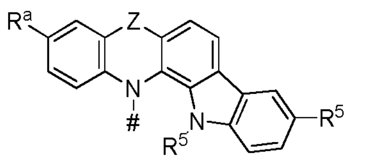
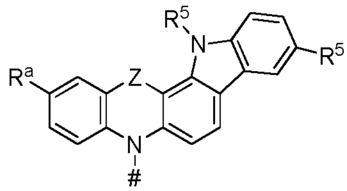

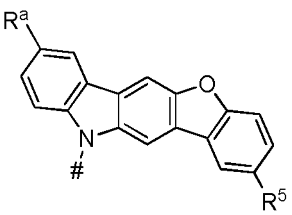
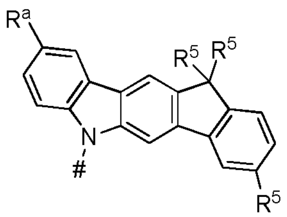
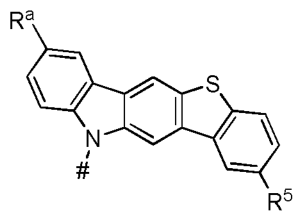

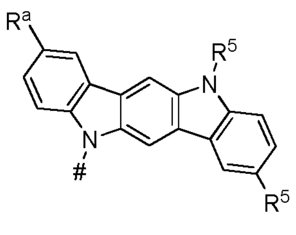

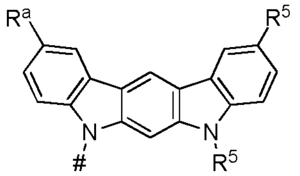 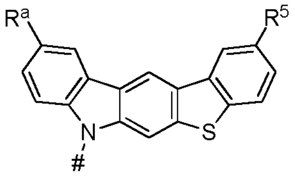 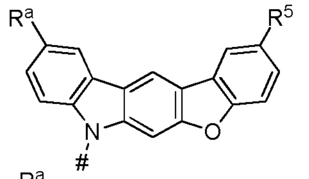

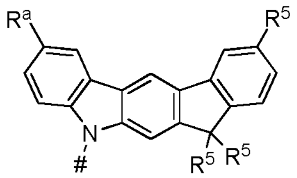 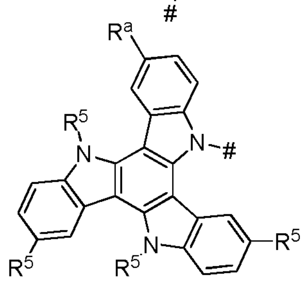

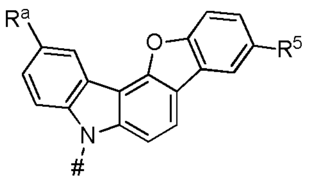 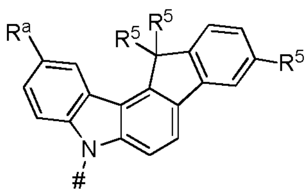 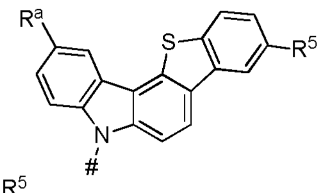

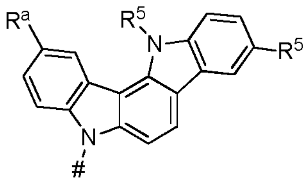 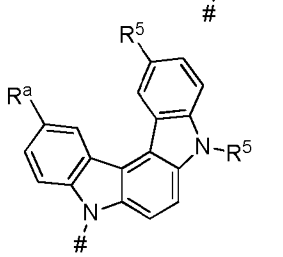

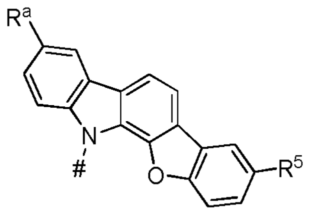 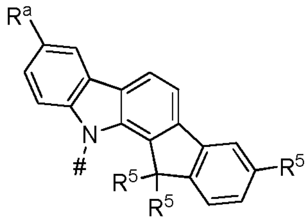 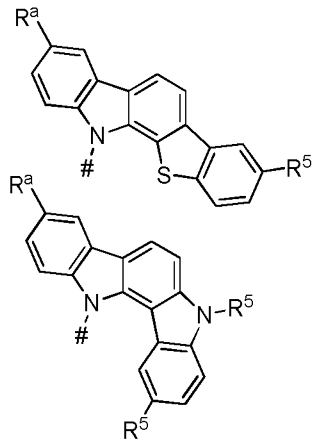

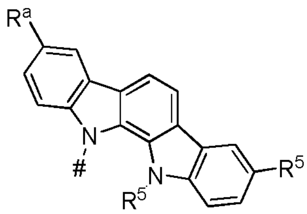

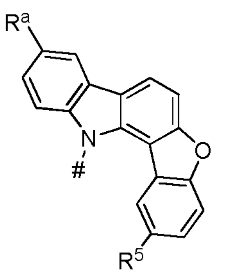 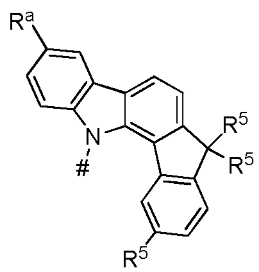 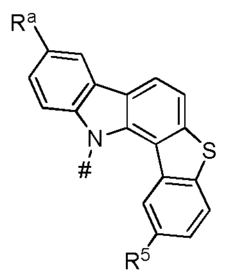

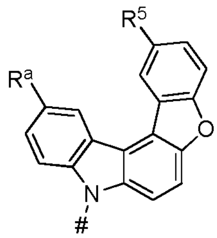

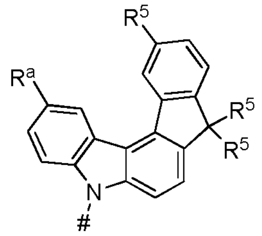

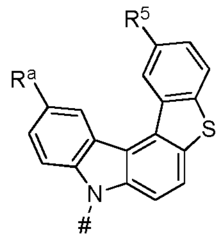

wherein for #, Z, $R^a$, $R^3$, $R^4$ and $R^5$ the aforementioned definitions apply.

In one embodiment, $R^a$ and $R^5$ is at each occurrence independently from another selected from the group consisting of: hydrogen (H), deuterium (D), methyl (Me), i-propyl ($CH(CH_3)_2$) ($^iPr$), t-butyl ($^tBu$), phenyl (Ph), CN, $CF_3$, and diphenylamine ($NPh_2$).

In one embodiment of the invention, the organic molecules comprise or consist of a structure of Formula III:

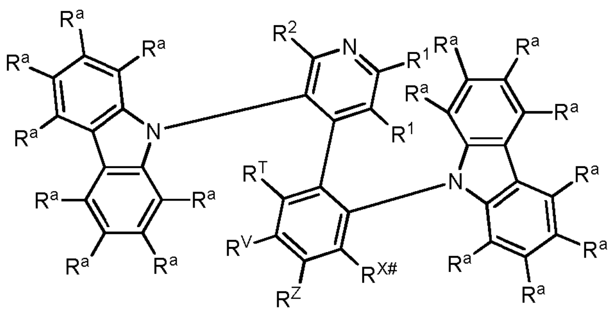

Formula III

$R^{X\#}$ is selected from the group consisting of $R^I$ and $R^B$,
wherein exactly one substituent selected from the group consisting of $R^V$, $R^T$, $R^{X\#}$ and $R^Z$ is $R^B$, and apart from that for the substituents any one of the aforementioned definition applies.

In another embodiment of the invention, the organic molecules comprise or consist of a structure of Formula III and $R^V$ is $R^B$.

In a further embodiment of the invention, the organic molecules comprise or consist of a structure selected from the group of Formula III-1 and Formula III-2:

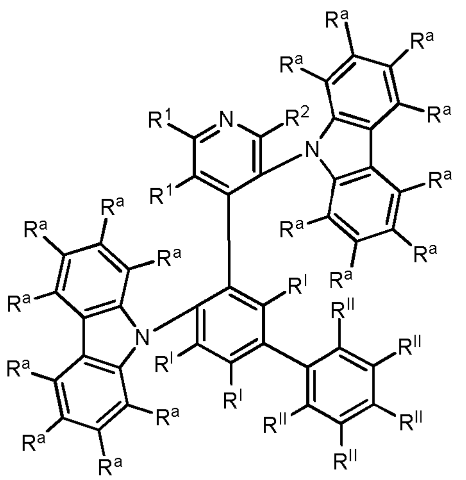

Formula III-1

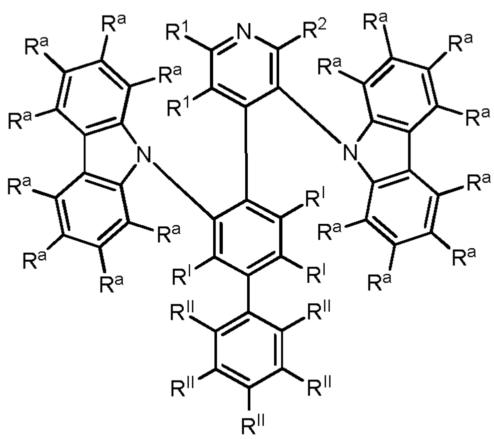

Formula III-2

wherein is $R^{III}$ for the substituents any one of the aforementioned definition applies.

In a further embodiment of the invention, the organic molecules comprise or consist of a structure selected from the group consisting of Formula IIIa-1, Formula IIIa-2, Formula IIIa-3, Formula IIIa-4, Formula IIIa-5, Formula IIIa-6, Formula IIIa-7, Formula IIIa-8, Formula IIIa-9, Formula IIIa-10, Formula IIIa-11 and Formula IIIa-12:

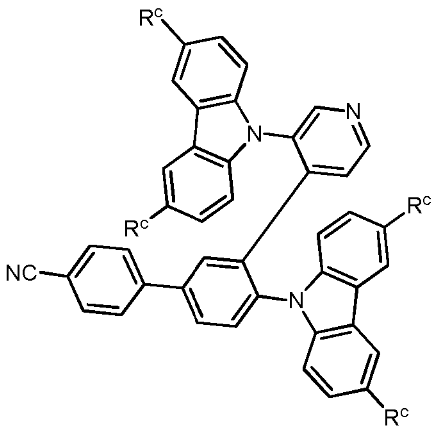

Formula IIIa-1

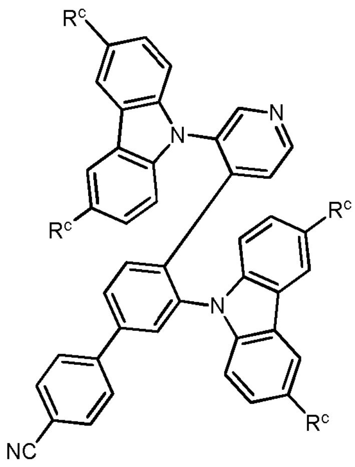

Formula IIIa-2

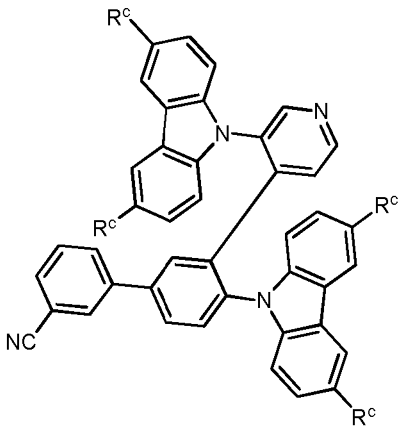

Formula IIIa-3

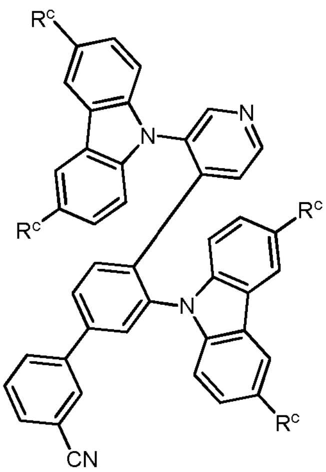

Formula IIIa-4

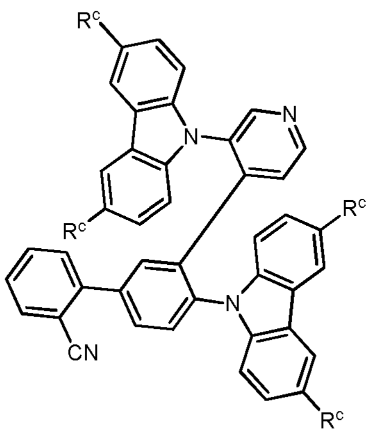

Formula IIIa-5

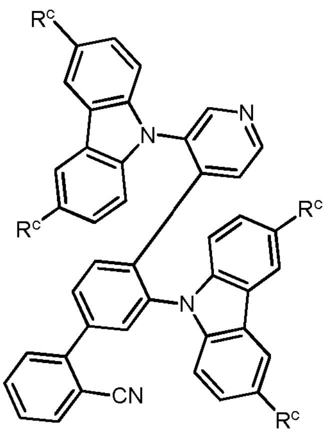

Formula IIIa-6

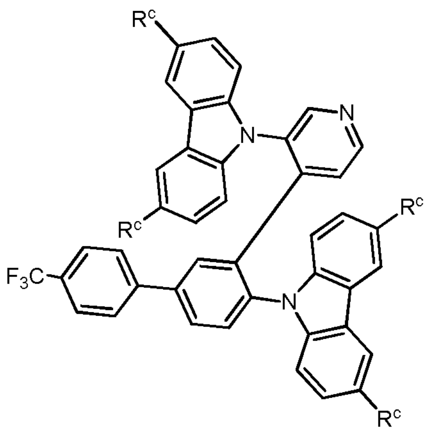

Formula IIIa-7

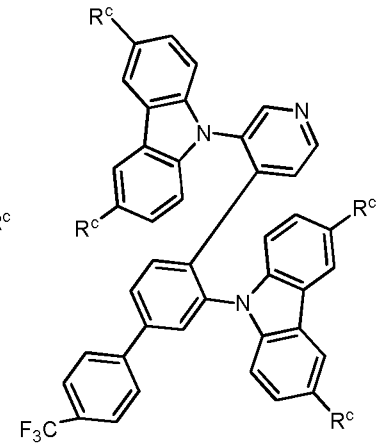

Formula IIIa-8

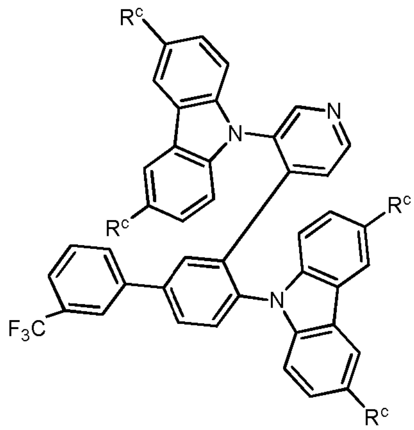

Formula IIIa-9

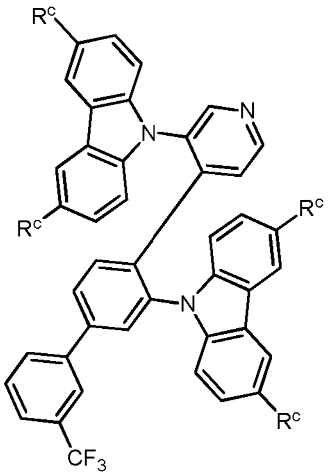

Formula IIIa-10

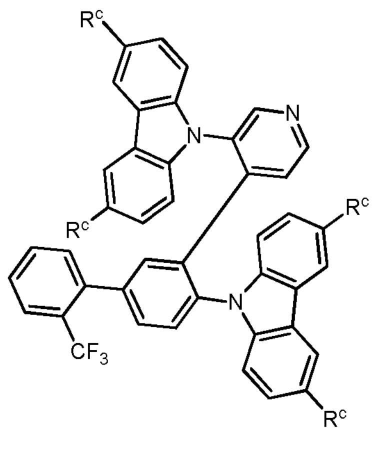

Formula IIIa-11

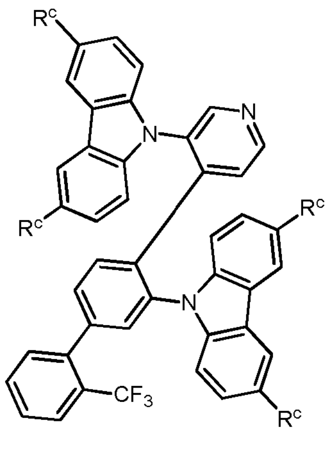

Formula IIIa-12

wherein

$R^c$ is at each occurrence independently from another selected from the group consisting of: deuterium,
Me,
$^iPr$,
$^tBu$,

Ph, which is optionally substituted with one or more substituents independently from each other selected from the group consisting of Me, $^{i}$Pr, $^{t}$Bu, CN, $CF_3$, and Ph;
pyridinyl, which is optionally substituted with one or more substituents independently from each other selected from the group consisting of Me, $^{i}$Pr, $^{t}$Bu, CN, $CF_3$, and Ph;
pyrimidinyl, which is optionally substituted with one or more substituents independently from each other selected from the group consisting of Me, $^{i}$Pr, $^{t}$Bu, CN, $CF_3$, and Ph;
carbazolyl, which is optionally substituted with one or more substituents independently from each other selected from the group consisting of Me, $^{i}$Pr, $^{t}$Bu, CN, $CF_3$, and Ph;
triazinyl, which is optionally substituted with one or more substituents independently from each other selected from the group consisting of Me, $^{i}$Pr, $^{t}$Bu, CN, $CF_3$, and Ph;
and $N(Ph)_2$.

In a preferred embodiment of the invention, the organic molecules comprise or consist of a structure selected from the group consisting of Formula IIIa-5 and Formula IIIa-6.

In a further embodiment of the invention, the organic molecules comprise or consist of a structure selected from the group of Formula IIIb-1, Formula IIIb-2, Formula IIIb-3, Formula IIIb-4, Formula IIIb-5, Formula IIIb-6, Formula IIIb-7, Formula IIIb-8, Formula IIIb-9, Formula IIIb-10, Formula IIIb-11 and Formula IIIb-12:

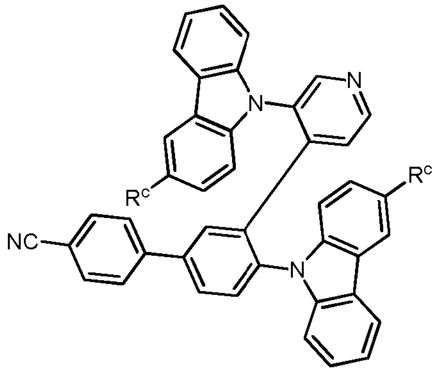

Formula IIIb-1

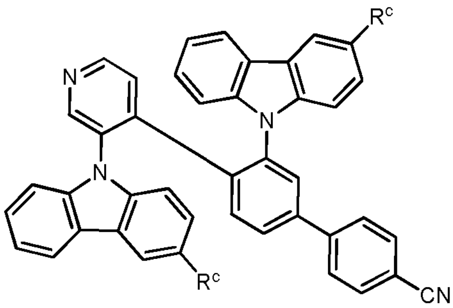

Formula IIIb-2

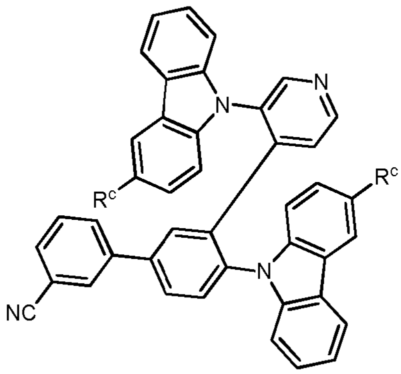

Formula IIIb-3

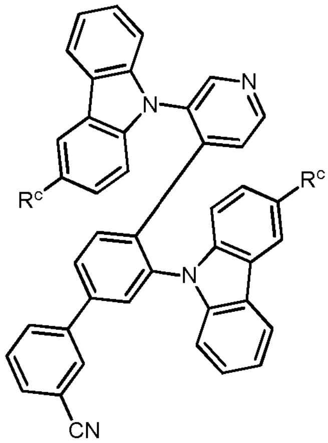

Formula IIIb-4

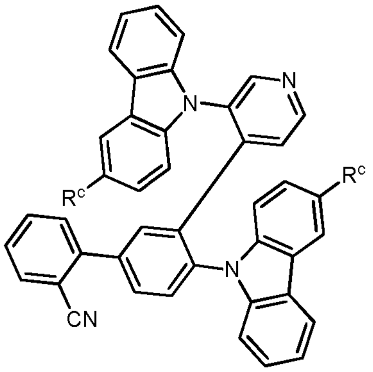

Formula IIIb-5

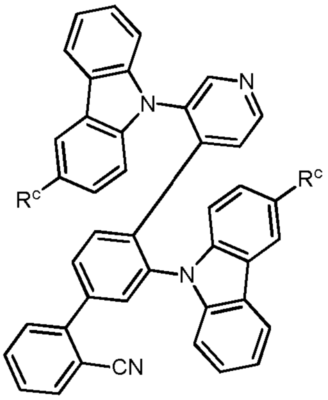

Formula IIIb-6

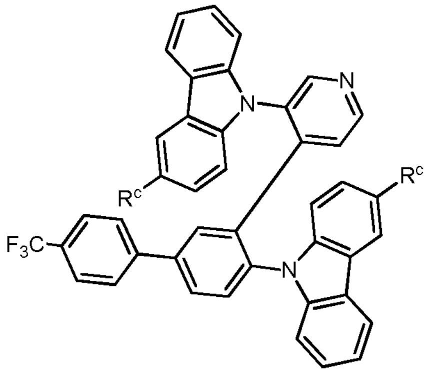

Formula IIIb-7

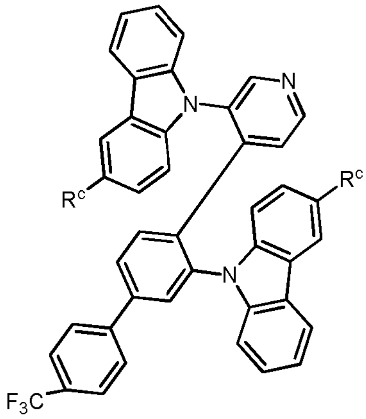

Formula IIIb-8

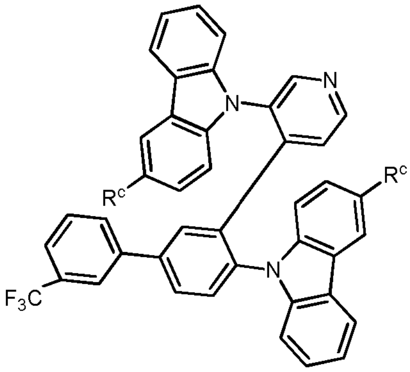

Formula IIIb-9

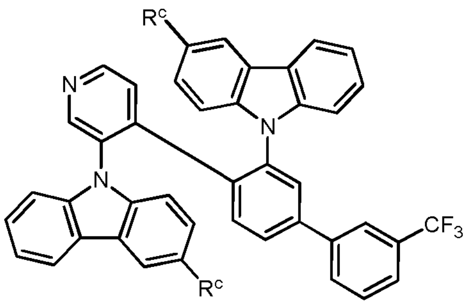

Formula IIIb-10

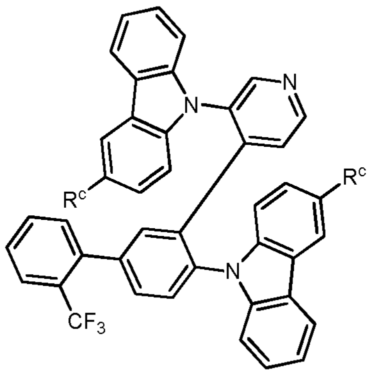

Formula IIIb-11

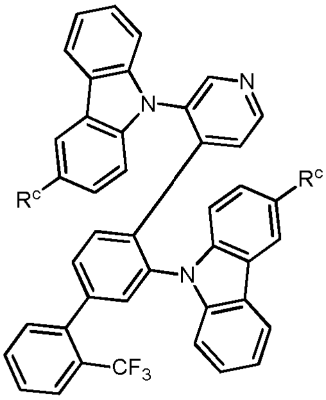

Formula IIIb-12

wherein for $R^c$ any one of the aforementioned definitions apply.

In a preferred embodiment of the invention, the organic molecules comprise or consist of a structure selected from the group consisting of Formula IIIb-5 and Formula IIIb-6.

In one embodiment of the invention, the organic molecules comprise or consist of a structure of Formula IV:

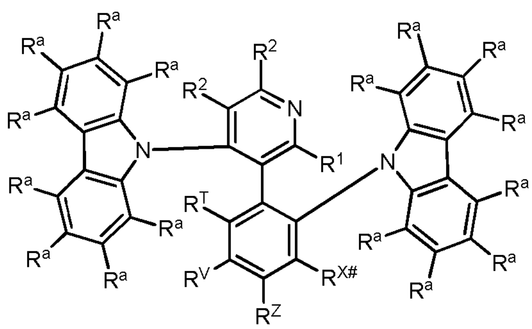

Formula IV

wherein exactly one substituent selected from the group consisting of $R^V$, $R^T$, $R^{X\#}$ and $R^Z$ is $R^B$ and apart from that any one of the aforementioned definitions apply.

In another embodiment of the invention, the organic molecules comprise or consist of a structure of Formula IV and $R^V$ is $R^B$.

In a further embodiment of the invention, the organic molecules comprise or consist of a structure selected from the group of Formula IV-1 and Formula IV-2:

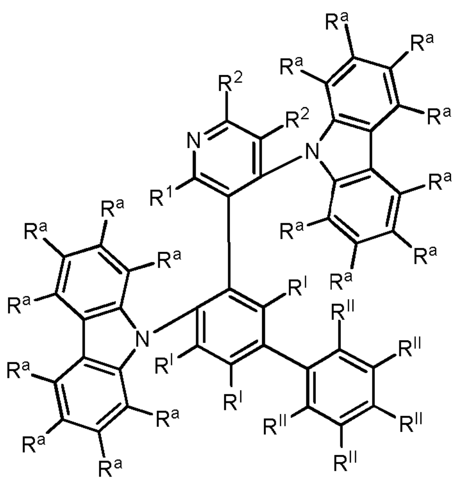

Formula IV-1

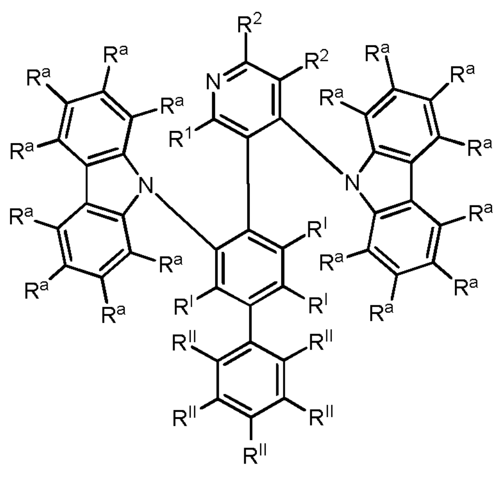

Formula IV-2

wherein for the substituents any one of the aforementioned definitions apply.

In a further embodiment of the invention, the organic molecules comprise or consist of a structure selected from the group of Formula IVa-1, Formula IVa-2, Formula IVa-3, Formula IVa-4, Formula IVa-5, Formula IVa-6, Formula IVa-7, Formula IVa-8, Formula IVa-9, Formula IVa-10, Formula IVa-11 and Formula IVa-12:

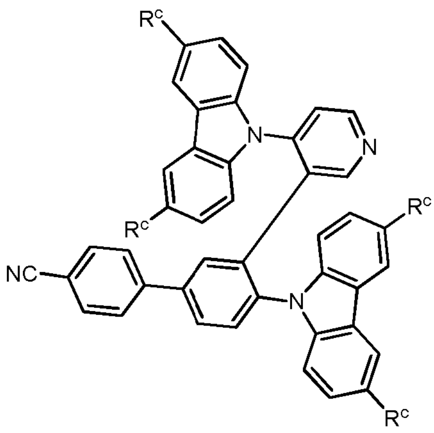

Formula IVa-1

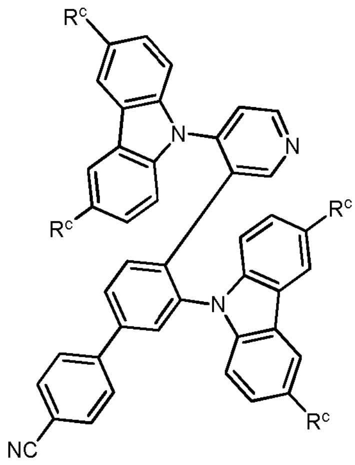

Formula IVa-2

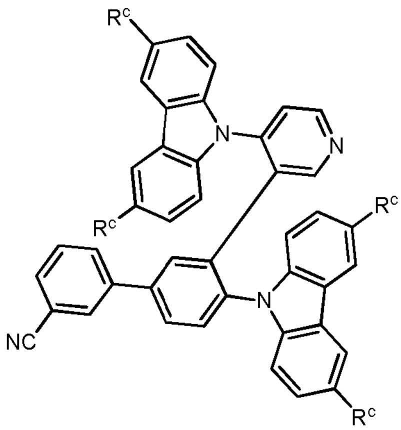

Formula IVa-3

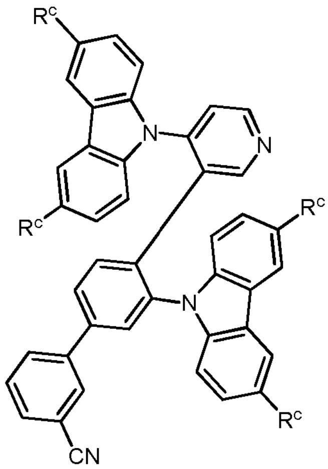

Formula IVa-4

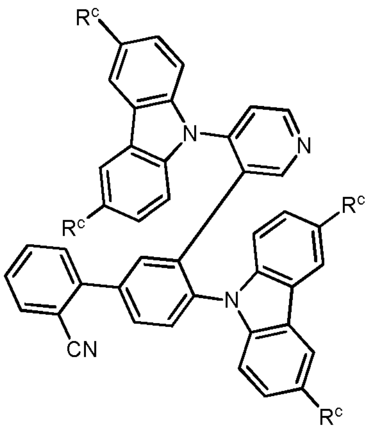

Formula IVa-5

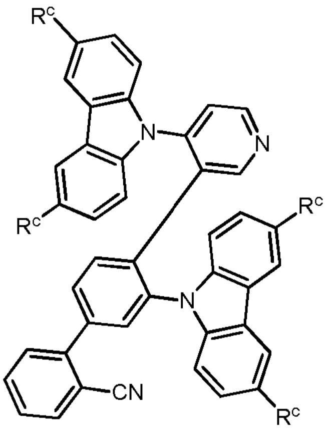

Formula IVa-6

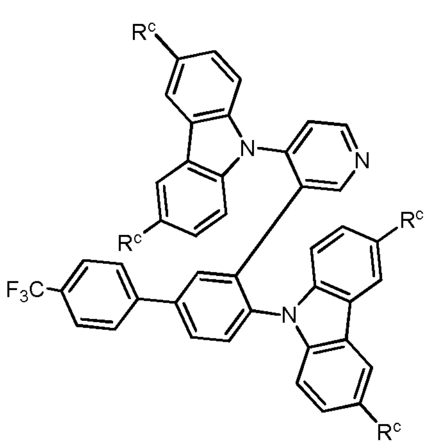
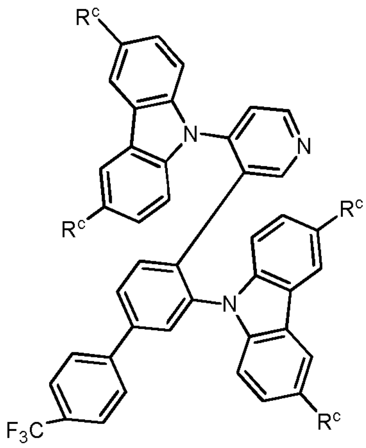

Formula IVa-7 Formula IVa-8

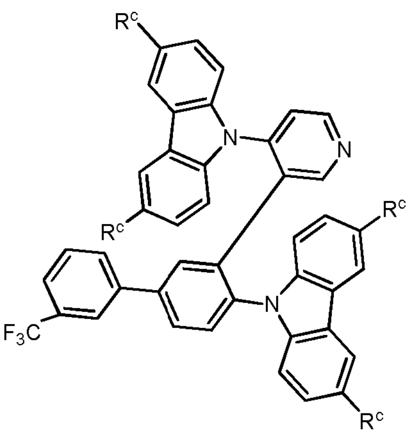
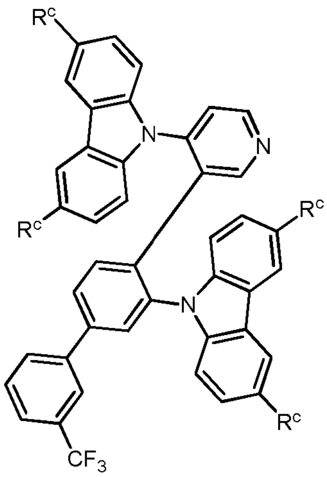

Formula IVa-9 Formula IVa-10

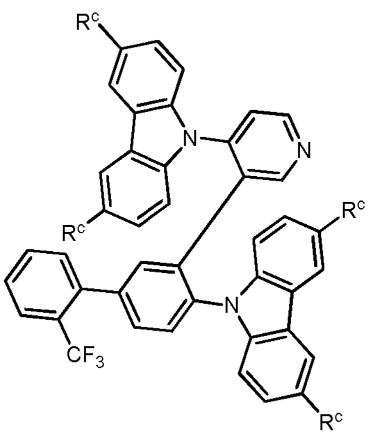
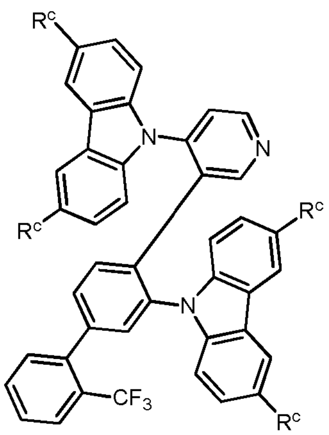

Formula IVa-11 Formula IVa-12

wherein for $R^c$ any one of the aforementioned definitions apply.

In a preferred embodiment of the invention, the organic molecules comprise or consist of a structure selected from the group consisting of Formula IVa-5 and Formula IVa-6.

In a further embodiment of the invention, the organic molecules comprise or consist of a structure selected from the group of Formula IVb-1, Formula IVb-2, Formula IVb-3, Formula IVb-4, Formula IVb-5, Formula IVb-6, Formula IVb-7, Formula IVb-8, Formula IVb-9, Formula IVb-10, Formula IVb-11 and Formula IVb-12:

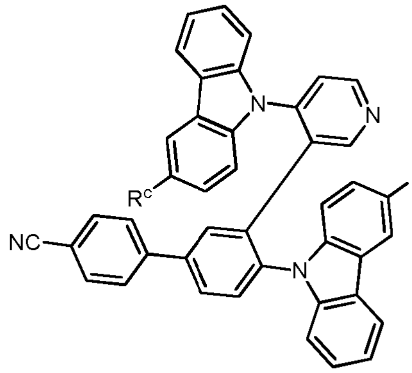
Formula IVb-1

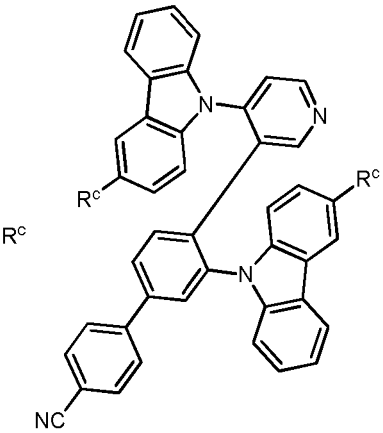
Formula IVb-2

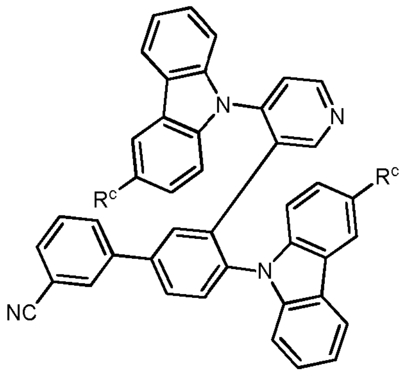
Formula IVb-3

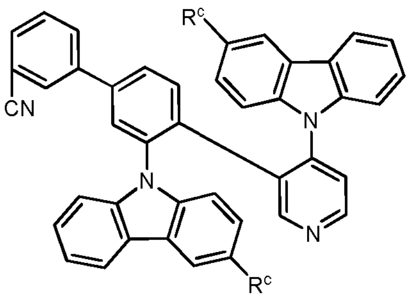
Formula IVb-4

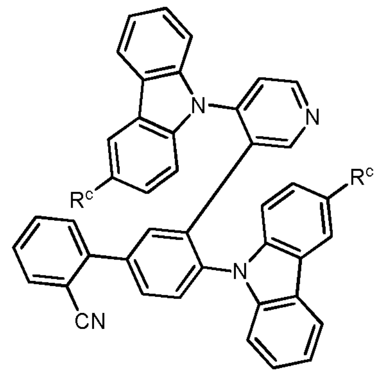
Formula IVb-5

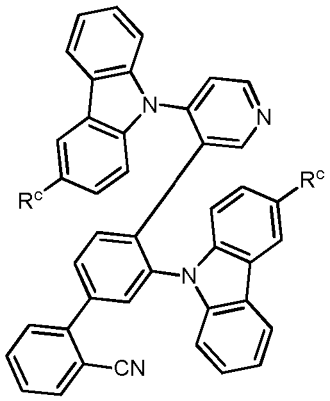
Formula IVb-6

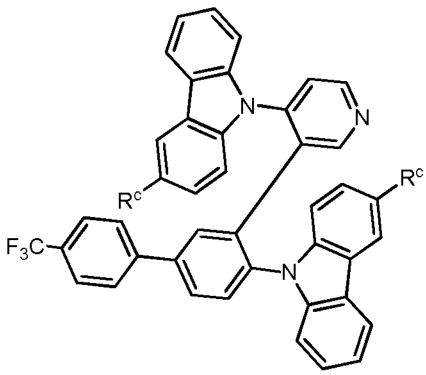

Formula IVb-7

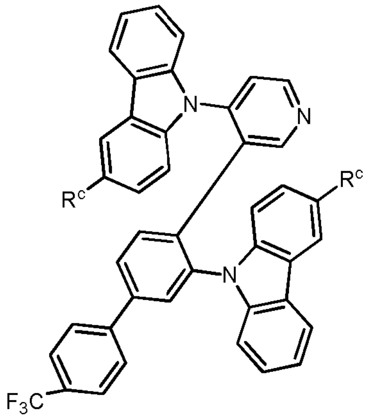

Formula IVb-8

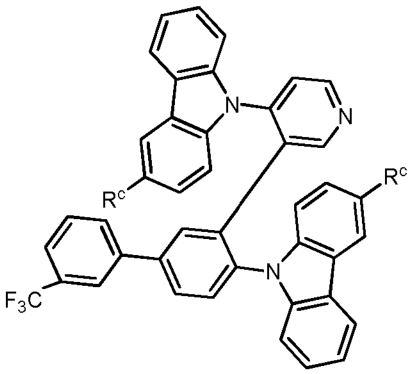

Formula IVb-9

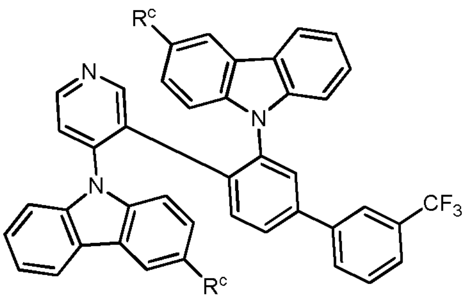

Formula IVb-10

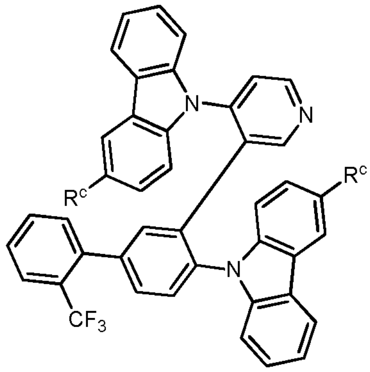

Formula IVb-11

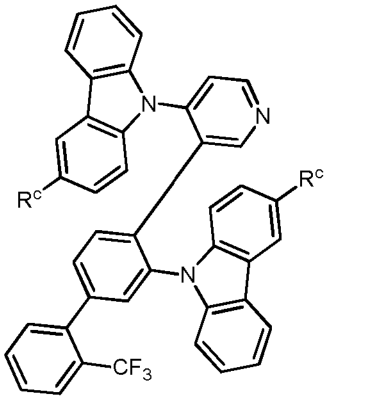

Formula IVb-12

wherein for $R^c$ any one of the aforementioned definitions applies.

In a preferred embodiment of the invention, the organic molecules comprise or consist of a structure selected from the group consisting of Formula IVb-5 and Formula IVb-6.

In one embodiment of the invention, the organic molecules comprise or consist of a structure of Formula V:

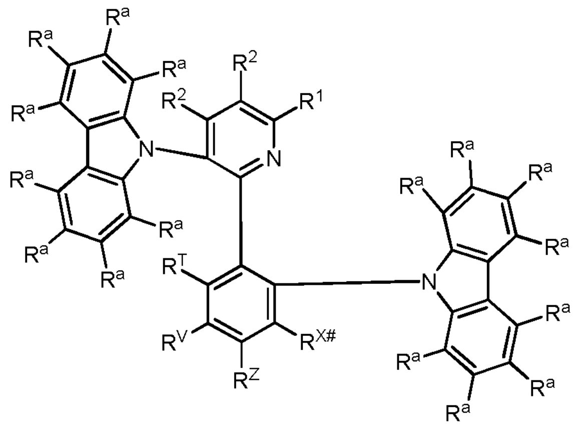

Formula V

wherein for the substituents any one of the aforementioned definitions applies except that exactly one substituent selected from the group consisting of $R^V$, $R^T$, $R^{X\#}$ and $R^Z$ is $R^B$.

In another embodiment of the invention, the organic molecules comprise or consist of a structure of Formula V and $R^V$ is $R^B$.

In one embodiment of the invention, the organic molecules comprise or consist of a structure of Formula VI:

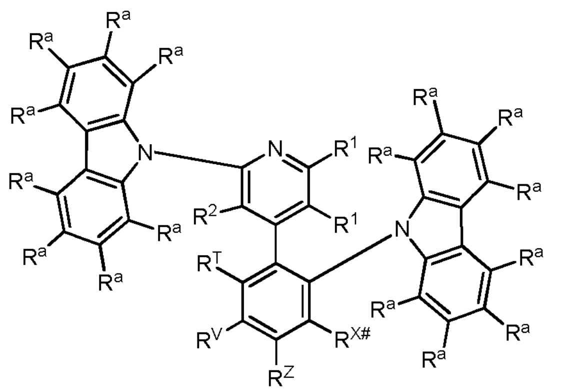

Formula VI

wherein for the substituents any one of the aforementioned definitions applies, except that exactly one substituent selected from the group consisting of $R^V$, $R^T$, $R^{X\#}$ and $R^Z$ is $R^B$.

In another embodiment of the invention, the organic molecules comprise or consist of a structure of Formula VI and $R^V$ is $R^B$.

In one embodiment of the invention, the organic molecules comprise or consist of a structure of Formula VII:

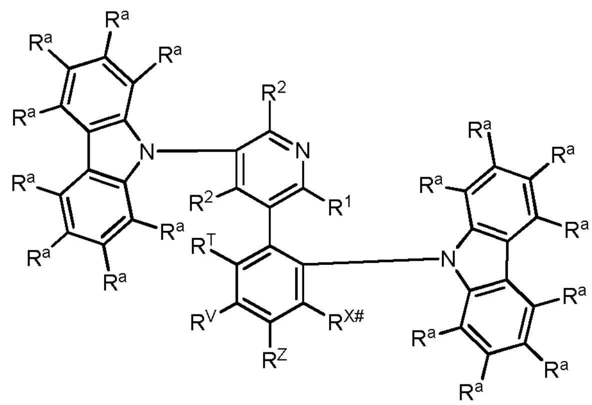

Formula VII

wherein for the substituents any one of the aforementioned definitions applies, except that exactly one substituent selected from the group consisting of $R^V$, $R^T$, $R^{X\#}$ and $R^Z$ is $R^B$.

In another embodiment of the invention, the organic molecules comprise or consist of a structure of Formula VII and $R^V$ is $R^B$.

In one embodiment of the invention, the organic molecules comprise or consist of a structure of Formula VIII:

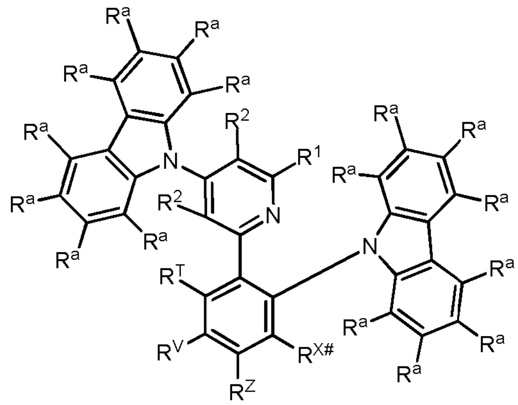

Formula VIII

wherein for the substituents any one of the aforementioned definitions applies, except that exactly one substituent selected from the group consisting of $R^V$, $R^T$, $R^{X\#}$ and $R^Z$ is $R^B$.

In another embodiment of the invention, the organic molecules comprise or consist of a structure of Formula VIII and $R^V$ is $R^B$.

In one embodiment of the invention, the organic molecules comprise or consist of a structure of Formula IX:

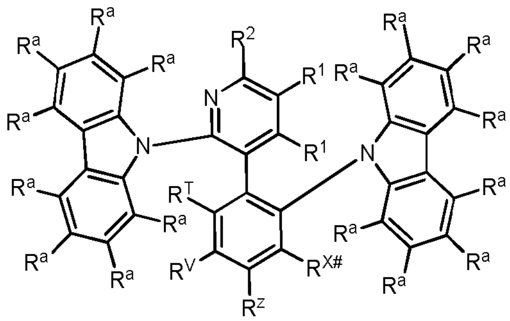

Formula IX

wherein for the substituents any one of the aforementioned definitions applies,
except that exactly one substituent selected from the group consisting of $R^V$ and $R^T$ is $R^B$.

In another embodiment of the invention, the organic molecules comprise or consist of a structure of Formula IX and $R^V$ is $R^B$.

In one embodiment of the invention, the organic molecules comprise or consist of a structure of Formula X:

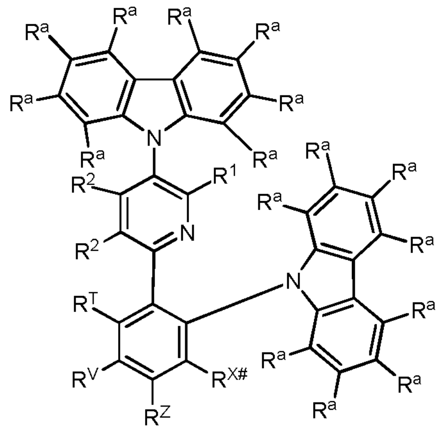

Formula X

wherein for the substituents any one of the aforementioned definitions apply, except that exactly one substituent selected from the group consisting of $R^V$, $R^T$, $R^{X\#}$ and $R^Z$ is $R^B$.

In another embodiment of the invention, the organic molecules comprise or consist of a structure of Formula X and $R^V$ is $R^B$.

In one embodiment of the invention, the organic molecules comprise or consist of a structure of Formula XI:

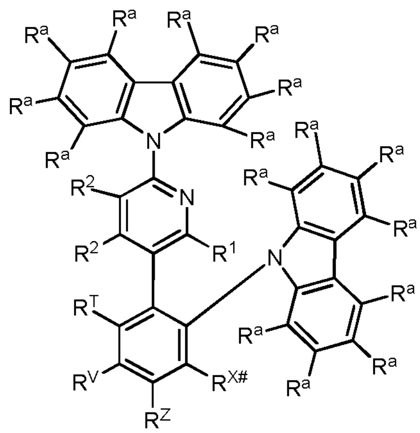

Formula XI

wherein $R^{X\#}$ is selected from the group consisting of $R^I$ and $R^B$,

wherein exactly one substituent selected from the group consisting of $R^V$, $R^T$, $R^{X\#}$ and $R^Z$ is $R^B$, and wherein apart from that any one of the aforementioned definitions applies.

In another embodiment of the invention, the organic molecules comprise or consist of a structure of Formula XI and $R^V$ is $R^B$.

In one embodiment of the invention, the organic molecules comprise or consist of a structure of Formula XII:

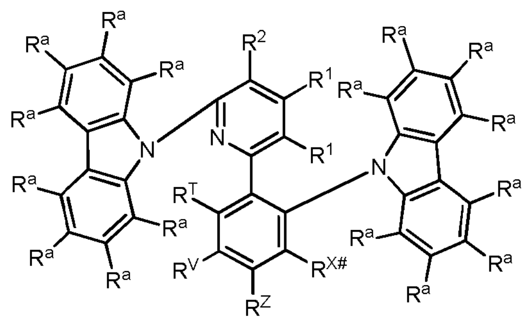

Formula XII

wherein for the substituents any one of the aforementioned definitions apply, except that exactly one substituent selected from the group consisting of $R^V$, $R^T$, $R^{X\#}$ and $R^Z$ is $R^B$.

In another embodiment of the invention, the organic molecules comprise or consist of a structure of Formula XII and $R^V$ is $R^B$.

In one embodiment of the invention, the organic molecules comprise or consist of a structure of Formula XIII:

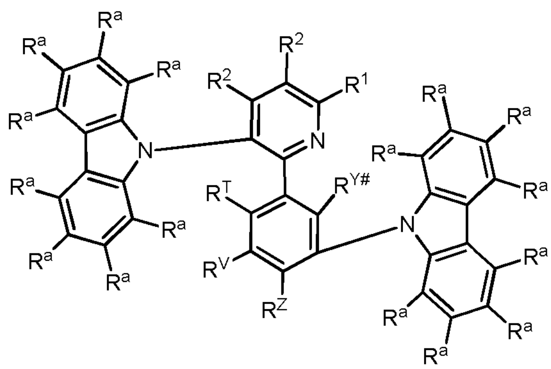

Formula XIII

wherein $R^{Y\#}$ is selected from the group consisting of $R^I$ and $R^B$,

wherein exactly one substituent selected from the group consisting of $R^V$, $R^T$, $R^{Y\#}$ and $R^Z$ is $R^B$, and wherein apart from that any one of the aforementioned definitions applies.

In another embodiment of the invention, the organic molecules comprise or consist of a structure of Formula XIII and $R^T$ is $R^B$.

In one embodiment of the invention, the organic molecules comprise or consist of a structure of Formula XIV:

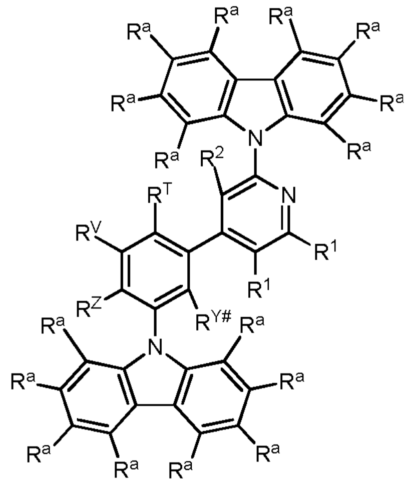

Formula XIV

wherein for the substituents any of the aforementioned definitions apply,
except that exactly one substituent selected from the group consisting of $R^V$, $R^T$, $R^{Y\#}$ and $R^Z$ is $R^B$.

In another embodiment of the invention, the organic molecules comprise or consist of a structure of Formula XIV and $R^T$ is $R^B$.

In one embodiment of the invention, the organic molecules comprise or consist of a structure of Formula XV:

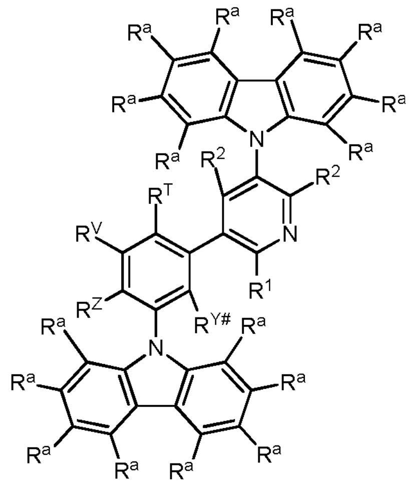

Formula XV

wherein the for the substituents any one of the aforementioned definitions apply,
except that exactly one substituent selected from the group consisting of $R^V$, $R^T$, $R^{Y\#}$ and $R^Z$ is $R^B$.

In another embodiment of the invention, the organic molecules comprise or consist of a structure of Formula XV and $R^T$ is $R^B$.

In one embodiment of the invention, the organic molecules comprise or consist of a structure of Formula XVI:

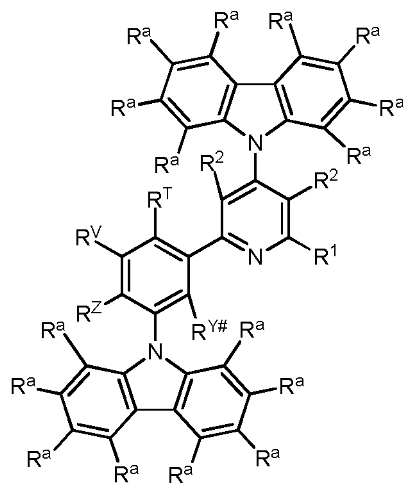

Formula XVI

wherein for the substituents any one of the aforementioned definitions apply, except that exactly one substituent selected from the group consisting of $R^V$, $R^T$, $R^{Y\#}$ and $R^Z$ is $R^B$.

In another embodiment of the invention, the organic molecules comprise or consist of a structure of Formula XVI and $R^T$ is $R^B$.

In one embodiment of the invention, the organic molecules comprise or consist of a structure of Formula XVII:

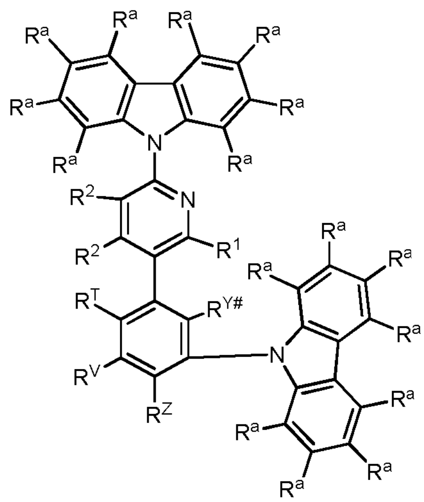

Formula XVII

wherein for the substituents any one of the aforementioned definitions apply, except that exactly one substituent selected from the group consisting of $R^V$, $R^T$, $R^{Y\#}$ and $R^Z$ is $R^B$.

In another embodiment of the invention, the organic molecules comprise or consist of a structure of Formula XVII and $R^T$ is $R^B$.

In one embodiment of the invention, the organic molecules comprise or consist of a structure of Formula XVIII:

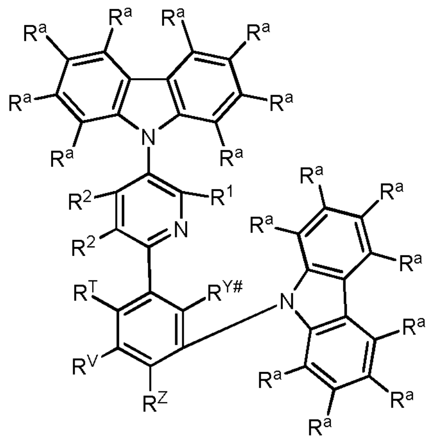

Formula XVIII

wherein for the substituents any one of the aforementioned definitions apply,
except that exactly one substituent selected from the group consisting of $R^V$, $R^T$, $R^{Y\#}$ and $R^Z$ is $R^B$.

In another embodiment of the invention, the organic molecules comprise or consist of a structure of Formula XVIII and $R^T$ is $R^B$.

In one embodiment of the invention, the organic molecules comprise or consist of a structure of Formula XIX:

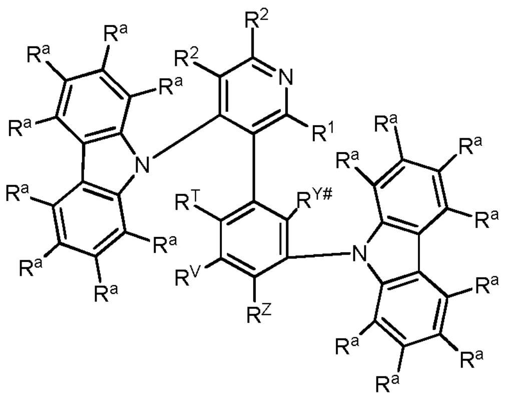

Formula XIX

wherein for the substituents any one of the aforementioned definitions apply,
except that exactly one substituent selected from the group consisting of $R^V$, $R^T$, $R^{Y\#}$ and $R^Z$ is $R^B$.

In another embodiment of the invention, the organic molecules comprise or consist of a structure of Formula XIX and $R^T$ is $R^B$.

In one embodiment of the invention, the organic molecules comprise or consist of a structure of Formula XX:

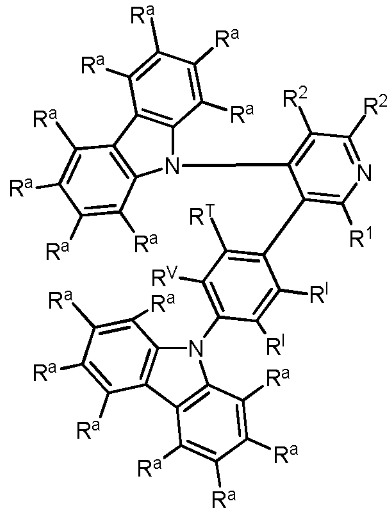

Formula XX

wherein for the substituents any one of the aforementioned definitions apply, except that exactly one substituent selected from the group consisting of $R^V$ and $R^T$ is $R^B$.

In another embodiment of the invention, the organic molecules comprise or consist of a structure of Formula XX and $R^T$ is $R^B$.

In one embodiment of the invention, the organic molecules comprise or consist of a structure of Formula XXI:

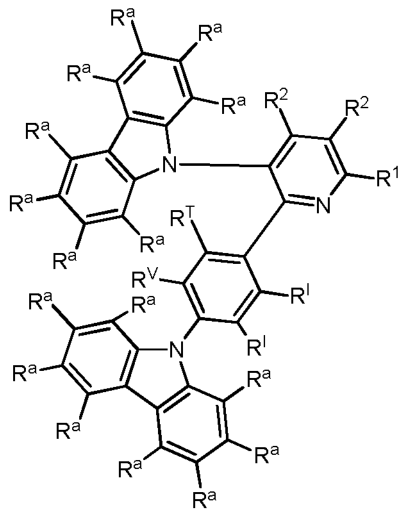

Formula XXI

wherein for the substituents any one of the aforementioned definitions apply, except that exactly one substituent selected from the group consisting of $R^V$ and $R^T$ is $R^B$.

In another embodiment of the invention, the organic molecules comprise or consist of a structure of Formula XXI and $R^T$ is $R^B$.

In one embodiment of the invention, the organic molecules comprise or consist of a structure of Formula XXII:

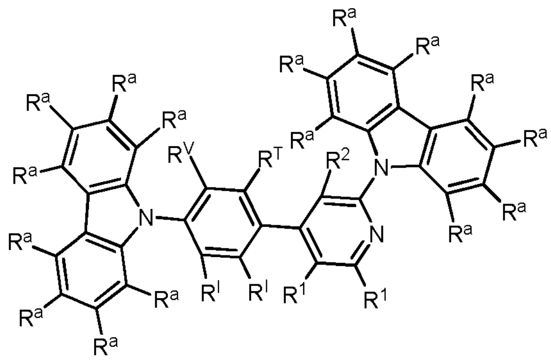

Formula XXII

wherein for the substituents any one of the aforementioned definitions apply,
except that exactly one substituent selected from the group consisting of $R^V$ and $R^T$ is $R^B$.

In another embodiment of the invention, the organic molecules comprise or consist of a structure of Formula XXII and $R^T$ is $R^B$.

In one embodiment of the invention, the organic molecules comprise or consist of a structure of Formula XXIII:

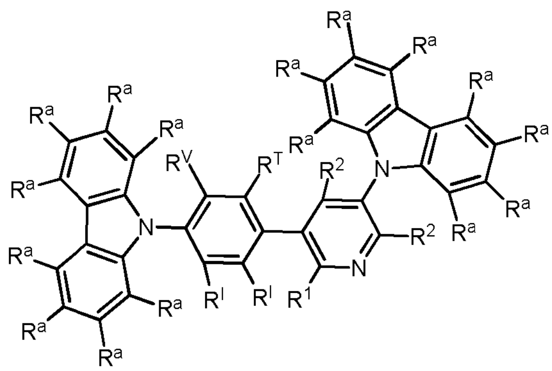

Formula XXIII

wherein for the substituents any one of the aforementioned definitions apply,
except that exactly one substituent selected from the group consisting of $R^V$ and $R^T$ is $R^B$.

In another embodiment of the invention, the organic molecules comprise or consist of a structure of Formula XXIII and $R^T$ is $R^B$.

In one embodiment of the invention, the organic molecules comprise or consist of a structure of Formula XXIV:

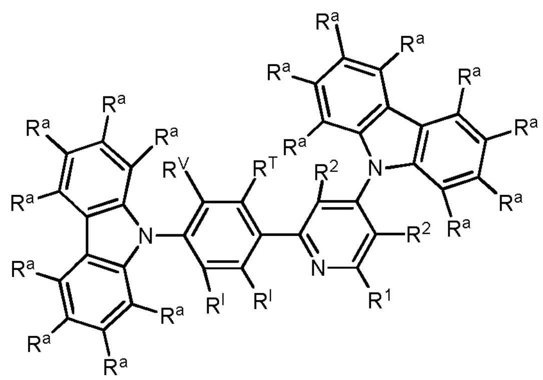

Formula XXIV

wherein for the substituents any one of the aforementioned definitions apply,
except that exactly one substituent selected from the group consisting of $R^V$ and $R^T$ is $R^B$.

In another embodiment of the invention, the organic molecules comprise or consist of a structure of Formula XXIV and $R^T$ is $R^B$.

In one embodiment of the invention, the organic molecules comprise or consist of a structure of Formula XXV:

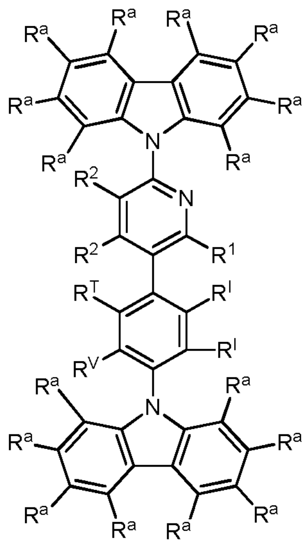

Formula XXV

wherein for the substituents any one of the aforementioned definitions apply,
except that exactly one substituent selected from the group consisting of $R^V$ and $R^T$ is $R^B$.

In another embodiment of the invention, the organic molecules comprise or consist of a structure of Formula XXV and $R^T$ is $R^B$.

In one embodiment of the invention, the organic molecules comprise or consist of a structure of Formula XXVI:

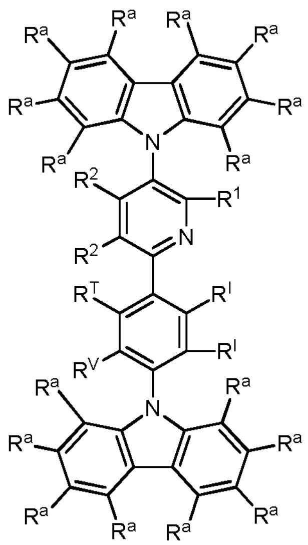

Formula XXVI

wherein for the substituents any one of the aforementioned definitions apply,
except that exactly one substituent selected from the group consisting of $R^V$ and $R^T$ is $R^B$.

In another embodiment of the invention, the organic molecules comprise or consist of a structure of Formula

XXVI and $R^T$ is $R^B$.

In one embodiment of the invention, the organic molecules comprise or consist of a structure of Formula XXVII:

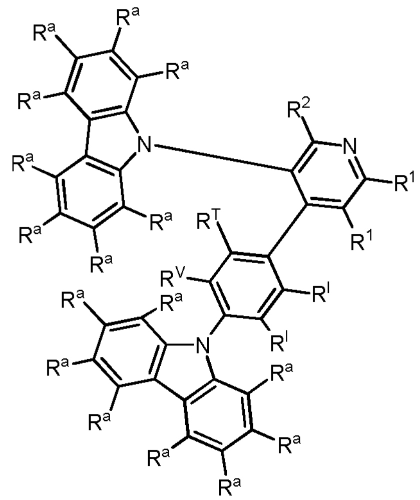

Formula XXVII

wherein for the substituents any one of the aforementioned definitions apply,
except that exactly one substituent selected from the group consisting of $R^V$ and $R^T$ is $R^B$.

In another embodiment of the invention, the organic molecules comprise or consist of a structure of Formula XXVII and $R^T$ is $R^B$.

In one embodiment of the invention, the organic molecules comprise or consist of a structure of Formula XXVIII:

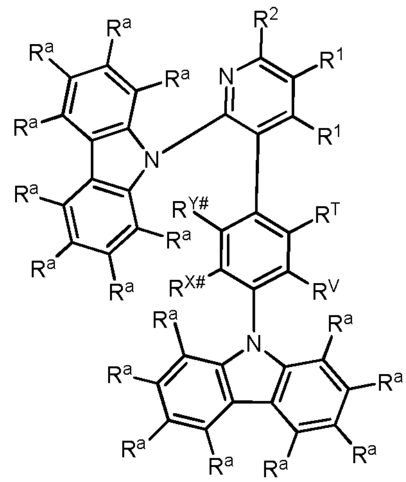

Formula XXVIII

wherein for the substituents any one of the aforementioned definitions apply,
except that exactly one substituent selected from the group consisting of $R^V$, $R^{X\#}$, $R^{Y\#}$, $R^T$ and $R^Z$ is $R^B$.

In another embodiment of the invention, the organic molecules comprise or consist of a structure of Formula XXVIII and $R^T$ is $R^B$.

In one embodiment of the invention, the organic molecules comprise or consist of a structure of Formula XXIX:

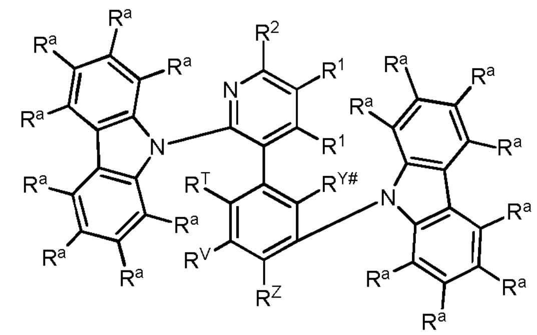

Formula XXIX

wherein for the substituents any one of the aforementioned definitions apply, except that exactly one substituent selected from the group consisting of $R^V$, $R^{Y\#}$, $R^T$ and $R^Z$ is $R^B$.

In another embodiment of the invention, the organic molecules comprise or consist of a structure of Formula XXIX and $R^T$ is $R^B$.

In one embodiment of the invention, the organic molecules comprise or consist of a structure of Formula XXX:

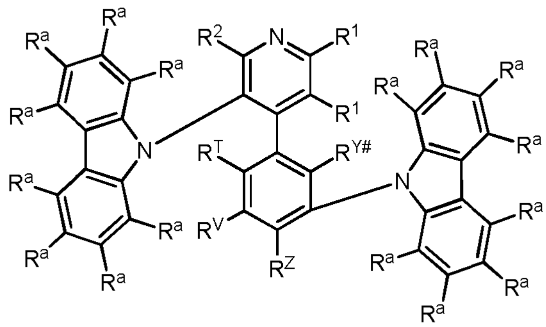

Formula XXX

wherein for the substituents any one of the aforementioned definitions apply, except that exactly one substituent selected from the group consisting of $R^V$, $R^{Y\#}$, $R^T$ and $R^Z$ is $R^B$.

In another embodiment of the invention, the organic molecules comprise or consist of a structure of Formula XXX and $R^T$ is $R^B$.

In one embodiment of the invention, the organic molecules comprise or consist of a structure of Formula XXXI:

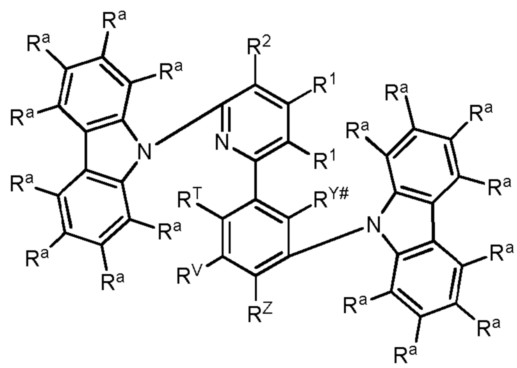

Formula XXXI

wherein for the substituents any one of the aforementioned definitions apply, except that exactly one substituent selected from the group consisting of $R^V$, $R^{Y\#}$, $R^T$ and $R^Z$ is $R^B$.

In another embodiment of the invention, the organic molecules comprise or consist of a structure of Formula XXXI and $R^T$ is $R^B$.

In one embodiment of the invention $R^c$ is at each occurrence independently from another selected from the group consisting of:

Me,
$^iPr$,
$^tBu$,
Ph, which is optionally substituted with one or more substituents independently from each other selected from the group consisting of Me, $^iPr$, $^tBu$, CN, $CF_3$ and Ph; and
triazinyl, which is optionally substituted with one or more substituents independently from each other selected from the group consisting of Me, $^iPr$, $^tBu$, CN, $CF_3$ and Ph.

As used throughout the present application, the terms "aryl" and "aromatic" may be understood in the broadest sense as any mono-, bi- or polycyclic aromatic moieties. Accordingly, an aryl group contains 6 to 60 aromatic ring atoms, and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used throughout the present application the term aryl group or heteroaryl group comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used throughout the present application the term cyclic group may be understood in the broadest sense as any mono-, bi- or polycyclic moieties.

As used throughout the present application the term biphenyl as a substituent may be understood in the broadest sense as ortho-biphenyl, meta-biphenyl, or para-biphenyl, wherein ortho, meta and para is defined in regard to the binding site to another chemical moiety.

As used throughout the present application the term alkyl group may be understood in the broadest sense as any linear, branched, or cyclic alkyl substituent. In particular, the term alkyl comprises the substituents methyl (Me), ethyl (Et), n-propyl ($^nPr$), i-propyl ($^iPr$), cyclopropyl, n-butyl ($^nBu$), i-butyl ($^tBu$), s-butyl ($^sBu$), t-butyl ($^tBu$), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neohexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used throughout the present application the term alkenyl comprises linear, branched, and cyclic alkenyl substituents. The term alkenyl group exemplarily comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout the present application the term alkynyl comprises linear, branched, and cyclic alkynyl

substituents. The term alkynyl group exemplarily comprises ethynyl, propynyl, butynyl, pentynyl, hexynyl, heptynyl or octynyl.

As used throughout the present application the term alkoxy comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy and 2-methylbutoxy.

As used throughout the present application the term thioalkoxy comprises linear, branched, and cyclic thioalkoxy substituents, in which the O of the exemplarily alkoxy groups is replaced by S.

As used throughout the present application, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphtyl, dibenzofuryl) or as if it were the whole molecule (e.g. naphthalene, dibenzofuran). As used herein, these different ways of designating a substituent or attached fragment are considered to be equivalent.

In one embodiment, the organic molecules according to the invention have an excited state lifetime of not more than 150 μs, of not more than 100 μs, in particular of not more than 50 μs, more preferably of not more than 10 μs or not more than 7 μs in a film of poly(methyl methacrylate) (PMMA) with 10 % by weight of organic molecule at room temperature.

In one embodiment of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In a further embodiment of the invention, the energy level of the highest occupied molecular orbital HOMO(E) of the organic molecule according to the invention E ($E^{HOMO}(E)$) is larger than -6.2 eV, preferably larger than -6.0 eV, more preferably larger than -5.9 eV, even more preferably larger than -5.8 eV, wherein $E^{HOMO}(E)$ is determined by cyclic voltammetry.

In a further embodiment of the invention, the energy level of the highest occupied molecular orbital HOMO(E) of the organic molecule according to the invention E ($E^{HOMO}(E)$) is larger than -5.8 eV, wherein $E^{HOMO}(E)$ is determined by cyclic voltammetry.

In a further embodiment of the invention, the organic molecules according to the invention have an emission peak in the visible or nearest ultraviolet range, i.e., in the range of a wavelength of from 380 to 800 nm, with a full width at half maximum of less than 0.50 eV, preferably less than 0.48 eV, more preferably less than 0.45 eV, even more preferably less than 0.43 eV or even less than 0.40 eV in a film of poly(methyl methacrylate) (PMMA) with 10 % by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500. The photoluminescence quantum yield determination method applied is described in the experimental section.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The cyclic voltammetry measurement methods are described in the experimental section. The energy of the lowest unoccupied molecular orbital $E^{LUMO}$ is calculated as $E^{HOMO} + E^{gap}$, wherein $E^{gap}$ is determined as follows: For host compounds, the onset of the emission spectrum of a film with 10 % by weight of host in poly(methyl methacrylate) (PMMA) is used as $E^{gap}$, unless stated otherwise. For emitter molecules, $E^{gap}$ is determined as the energy at which the excitation and emission spectra of a film with 10% by weight of emitter in PMMA cross.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF. The triplet energy can thus be determined as the onset of the phosphorescence spectrum. For TADF emitter molecules, the energy of the first excited triplet state T1 is determined from the onset of the delayed emission spectrum at 77 K, if not otherwise stated measured in a film of PMMA with 10% by weight of emitter. Both for host and emitter compounds, the energy of the first excited singlet state S1 is determined from the onset of the emission spectrum, if not otherwise stated measured in a film of PMMA with 10% by weight of host or emitter compound.

The onset of an emission spectrum is determined by computing the intersection of the tangent to the emission spectrum with the x-axis. The tangent to the emission spectrum is set at the high-energy side of the emission band and

at the point at half maximum of the maximum intensity of the emission spectrum.

A further aspect of the invention relates to a process for preparing organic molecules (with an optional subsequent reaction) according to the invention, wherein a $R^1$-, $R^2$-substituted $Hal^a$-fluoropyridine is used as a reactant:

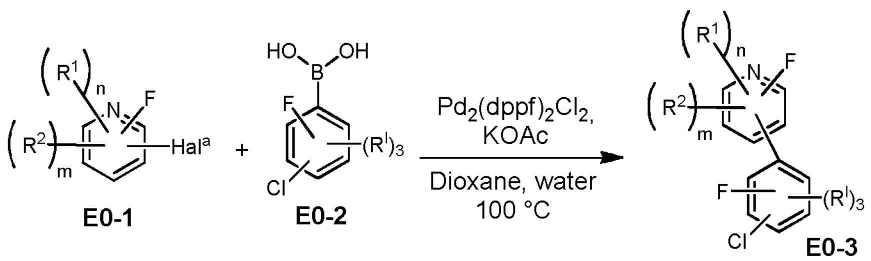


**n = 1 or 2**
**m = 1 or 2**
**n + m = 3**

**$Hal^a$ = Cl, Br, I**

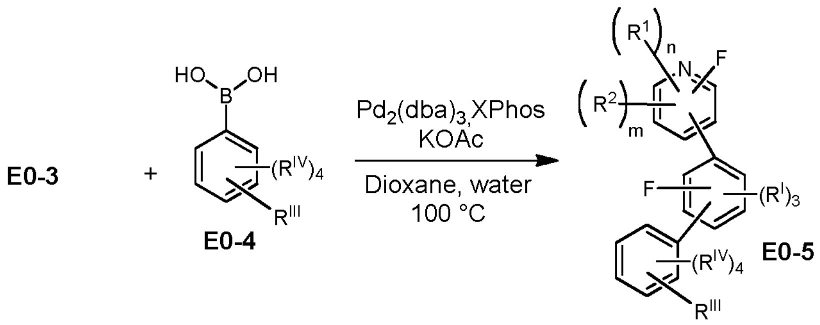


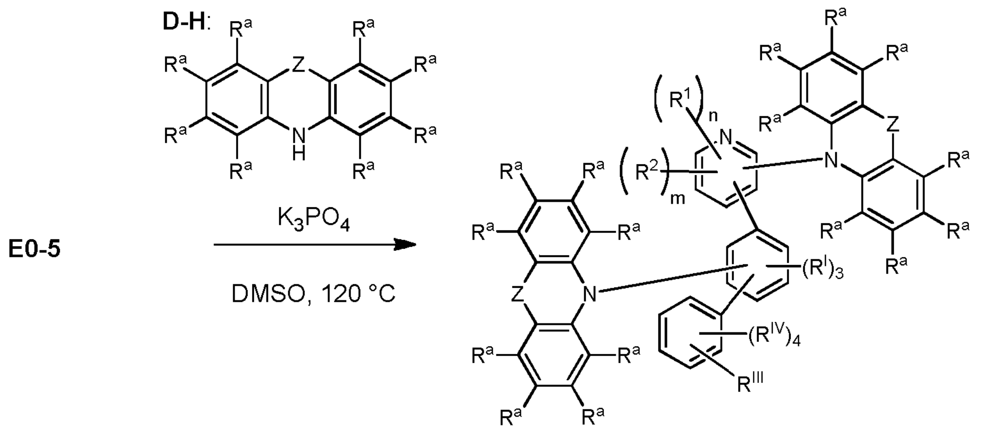


,

wherein $Hal^a$ is selected from the group consisting of Cl, Br and I.

Typically, $Pd_2(dba)_3$ (tris(dibenzylideneacetone)dipaladium(0)) is used as a Pd catalyst, but alternatives are known in the art. For example, the ligand may be selected from the group consisting of S-Phos ([2-dicyclohexylphoshino-2',6'-dimethoxy-1,1'-biphenyl]), X-Phos (2-(dicyclohexylphosphino)-2",4",6"-triisopropylbiphenyl), and $P(Cy)_3$ (tricyclohexylphosphine). The salt is, for example, selected from tribasic potassium phosphate and potassium acetate and the solvent can be a pure solvent, such as toluene or dioxane, or a mixture, such as toluene/dioxane/water or dioxane/toluene. A person of skill in the art can determine which Pd catalyst, ligand, salt and solvent combination will result in high reaction yields.

For the reaction of a nitrogen heterocycle in a nucleophilic aromatic substitution with an aryl halide, preferably an aryl fluoride, typical conditions include the use of a base, such as tribasic potassium phosphate or sodium hydride, for example, in an aprotic polar solvent, such as dimethyl sulfoxide (DMSO) or N,N-dimethylformamide (DMF), for example.

An alternative synthesis route comprises the introduction of a nitrogen heterocycle via copper- or palladium-catalyzed coupling to an aryl halide or aryl pseudohalide, preferably an aryl bromide, an aryl iodide, aryl triflate or an aryl tosylate.

A further aspect of the invention relates to the use of an organic molecule according to the invention as a luminescent emitter or as an absorber, and/or as host material and/or as electron transport material, and/or as hole

injection material, and/or as hole blocking material in an optoelectronic device, in particular as a luminescent emitter.

The optoelectronic device may be understood in the broadest sense as any device based on organic materials that is suitable for emitting light in the visible or nearest ultraviolet (UV) range, i.e., in the range of a wavelength of from 380 to 800 nm. More preferably, the optoelectronic device may be able to emit light in the visible range, i.e., of from 400 to 800 nm.

In the context of such use, the optoelectronic device is more particularly selected from the group consisting of:

- organic light-emitting diodes (OLEDs),
- light-emitting electrochemical cells,
- OLED sensors, especially in gas and vapor sensors not hermetically externally shielded,
- organic diodes,
- organic solar cells,
- organic transistors,
- organic field-effect transistors,
- organic lasers, and
- down-conversion elements.

In a preferred embodiment in the context of such use, the optoelectronic device is a device selected from the group consisting of an organic light emitting diode (OLED), a light emitting electrochemical cell (LEC), and a light-emitting transistor.

In the case of the use, the fraction of the organic molecule according to the invention in the emission layer in an optoelectronic device, more particularly in OLEDs, is 1 % to 99 % by weight, more particularly 5 % to 80 % by weight. In an alternative embodiment, the proportion of the organic molecule in the emission layer is 100 % by weight.

In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

In one embodiment, the light-emitting layer EML comprises or (essentially) consists of a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

Particularly preferably the light-emitting layer EML comprises or (essentially) consists of a composition comprising or consisting of:

(i) 1-50 % by weight, preferably 5-40 % by weight, in particular 10-30 % by weight, of one or more organic molecules according to the invention E;
(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention E, in particular transferred from the first excited triplet state T1(H) of the host compound H to the first excited triplet state T1(E) of the one or more organic molecules according to the invention E and/ or from the first excited singlet state S1(H) of the host compound H to the first excited singlet state S1(E) of the one or more organic molecules according to the invention E.

In a further embodiment, the light-emitting layer EML comprises or (essentially) consists of a composition

comprising or consisting of:

(i) 1-50 % by weight, preferably 5-40 % by weight, in particular 10-30 % by weight, of one organic molecule according to the invention E;
(ii) 5-99 % by weight, preferably 30-94.9 % by weight, in particular 40-89% by weight, of one host compound H; and
(iii) optionally, 0-94 % by weight, preferably 0.1-65 % by weight, in particular 1-50 % by weight, of at least one further host compound D with a structure differing from the structure of the molecules according to the invention; and
(iv) optionally, 0-94 % by weight, preferably 0-65 % by weight, in particular 0-50 % by weight, of a solvent; and
(v) optionally, 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy $E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.
In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.
In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention E has a highest occupied molecular orbital HOMO(E) having an energy $E^{HOMO}(E)$ and a lowest unoccupied molecular orbital LUMO(E) having an energy $E^{LUMO}(E)$,
wherein
$E^{HOMO}(H) > E^{HOMO}(D)$ and the difference between the energy level of the highest occupied molecular orbital HOMO(E) of the organic molecule according to the invention E ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and $E^{LUMO}(H) > E^{LUMO}(D)$ and the difference between the energy level of the lowest unoccupied molecular orbital LUMO(E) of the organic molecule according to the invention E ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.

In a further aspect, the invention relates to an optoelectronic device comprising an organic molecule or a composition of the type described here, more particularly in the form of a device selected from the group consisting of organic light-emitting diode (OLED), light-emitting electrochemical cell, OLED sensor, more particularly gas and vapour sensors not hermetically externally shielded, organic diode, organic solar cell, organic transistor, organic field-effect transistor, organic laser and down-conversion element.
In a preferred embodiment, the optoelectronic device is a device selected from the group consisting of an organic light emitting diode (OLED), a light emitting electrochemical cell (LEC), and a light-emitting transistor.
In one embodiment of the optoelectronic device of the invention, the organic molecule according to the invention E is used as emission material in a light-emitting layer EML.
In one embodiment of the optoelectronic device of the invention the light-emitting layer EML consists of the composition according to the invention described here.
For example, when the optoelectronic device is an OLED, it may exhibit the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL
10. cathode layer,

wherein the OLED comprises each layer only optionally, except for the EML, which is mandatory. In some embodiments, different layers may be merged and the OLED may comprise more than one layer of each layer type as defined above.

Furthermore, the optoelectronic device may optionally comprise one or more protective layers protecting the device from damaging exposure to harmful species in the environment including, exemplarily moisture, vapor and/or gases.

In one embodiment of the invention, the optoelectronic device is an OLED, which exhibits the following inverted layer structure:

1. substrate
2. cathode layer
3. electron injection layer, EIL
4. electron transport layer, ETL
5. hole blocking layer, HBL
6. emitting layer, B
7. electron blocking layer, EBL
8. hole transport layer, HTL
9. hole injection layer, HIL
10. anode layer A

wherein the OLED with an inverted layer structure comprises each layer other than the emitting layer B only optionally. In some embodiments, different layers may be merged and the OLED may comprise more than one layer of each layer types defined above.

In one embodiment of the invention, the optoelectronic device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may exemplarily comprise indium tin oxide, aluminum zinc oxide, fluorine doped tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene.

Particularly preferably, the anode layer A (essentially) consists of indium tin oxide (ITO) (e.g., $(InO_3)0.9(SnO_2)0.1$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may exemplarily comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bisphenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may exemplarily be used as inorganic dopant. Tetrafluorotetracyanoquinodimethane ($F_4$-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particularly, the EML comprises at least one light emitting molecule according to the invention E. In one embodiment, the light-emitting layer comprises only the organic molecules according to the invention E. Typically, the EML additionally comprises one or more host materials H. Exemplarily, the host material H is selected from CBP (4,4'-Bis-(N-carbazolyl)-biphenyl), mCP, mCBP Sif87 (dibenzo[b,d]thiophen-2-yltriphenylsilane), CzSi, SiMCP (3,5-Di(9H-carbazol-9-yl)phenyl]triphenylsilane), Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material H typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule according to the invention E and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. Exemplarily, electron-poor compounds such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may, for example, comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. Exemplarily, the cathode layer C may comprise or may consist of a metal (e.g., Al, Au, Ag, Pt, Cu, Zn, Ni, Fe, Pb, LiF, Ca, Ba, Mg, In, W, or Pd)

or a metal alloy. For practical reasons, the cathode layer may also consist of (essentially) non-transparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may further, optionally, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds H.

In order to modify the emission spectrum and/or the absorption spectrum of the light-emitting layer EML further, the light-emitting layer EML may further comprise one or more further emitter molecules F. Such an emitter molecule F may be any emitter molecule known in the art. Preferably such an emitter molecule F is a molecule with a structure differing from the structure of the molecules according to the invention E. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. Exemplarily, the triplet and/or singlet excitons may be transferred from the emitter molecule according to the invention E to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an optoelectronic device (e.g., an OLED) may exemplarily be an essentially white optoelectronic device. Exemplarily such white optoelectronic device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm. |

With respect to emitter molecules, such colors refer to the emission maximum. Therefore, exemplarily, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 cd/$m^2$ of more than 8 %, more preferably of more than 10 %, more preferably of more than 13 %, even more preferably of more than 15 % or even more than 20 % and/or exhibits an emission maximum between 420 nm and 500 nm, preferably between 430 nm and 490 nm, more preferably between 440 nm and 480 nm, even more preferably between 450 nm and 470 nm and/or exhibits a LT80 value at 500 cd/$m^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.50 eV, preferably less than 0.48 eV, more preferably less than 0.45 eV, even more preferably less than 0.43 eV or even less than 0.40 eV.

A further aspect of the present invention relates to an OLED, which emits light with CIEx and CIEy color coordinates close to the CIEx and CIEy color coordinates of the primary color blue (CIEx = 0.131 and CIEy = 0.046) as

defined by ITU-R Recommendation BT.2020 (Rec. 2020) and thus is suited for the use in Ultra High Definition (UHD) displays, e.g. UHD-TVs. Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The optoelectronic device, in particular the OLED according to the present invention can be produced by any means of vapor deposition and/or liquid processing. Accordingly, at least one layer is

- prepared by means of a sublimation process,
- prepared by means of an organic vapor phase deposition process,
- prepared by means of a carrier gas sublimation process,
- solution processed, or
- printed.

Another aspect of the present invention relates to a process for producing an optoelectronic device, wherein an organic molecule or composition according to the invention is used, in particular comprising the processing of the organic molecule or of the composition by a vacuum evaporation method or from a solution.

Further steps used to produce the optoelectronic device, in particular the OLED according to the present invention, comprise depositing different layers individually and successively on a suitable substrate by means of subsequent deposition processes. The individual layers may be deposited using the same or differing deposition methods.

Vapor deposition processes exemplarily comprise thermal (co)evaporation, chemical vapor deposition and physical vapor deposition. For active matrix OLED display, an AMOLED backplane is used as substrate. The individual layer may be processed from solutions or dispersions employing adequate solvents. Solution deposition process exemplarily comprise spin coating, dip coating and jet printing. Liquid processing may optionally be carried out in an inert atmosphere (e.g., in a nitrogen atmosphere) and the solvent may optionally be completely or partially removed by means known in the state of the art.

## Examples

General synthesis scheme I

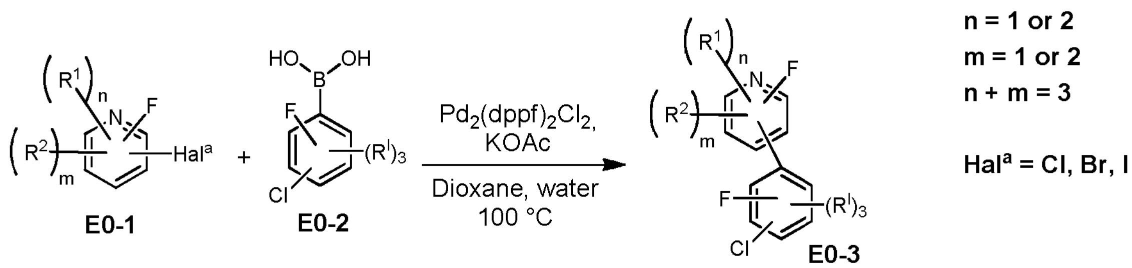


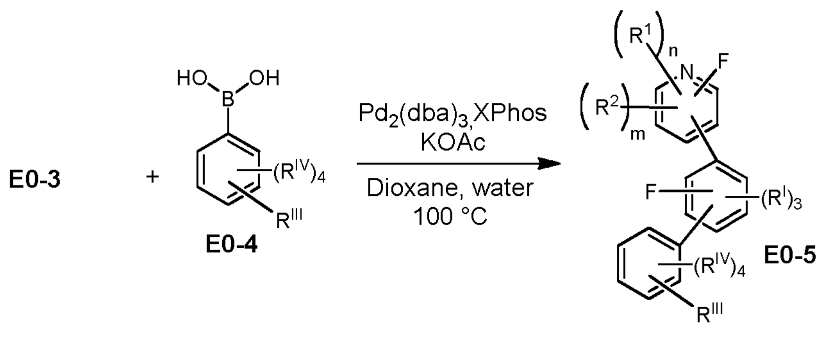

E0-5

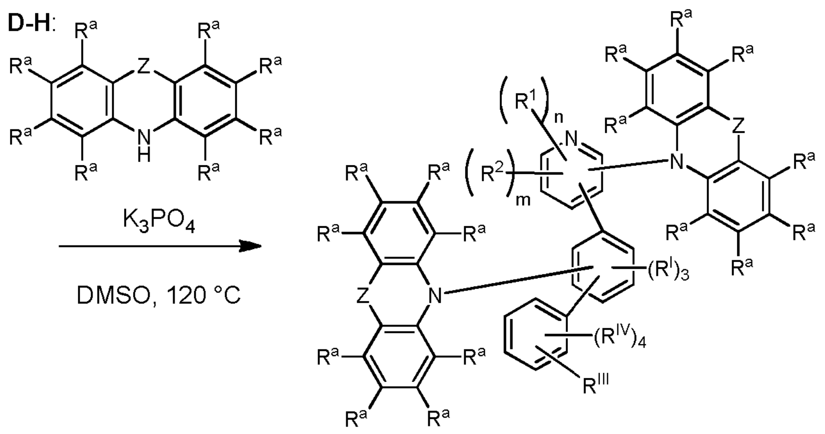


*General procedure for synthesis AAV1*

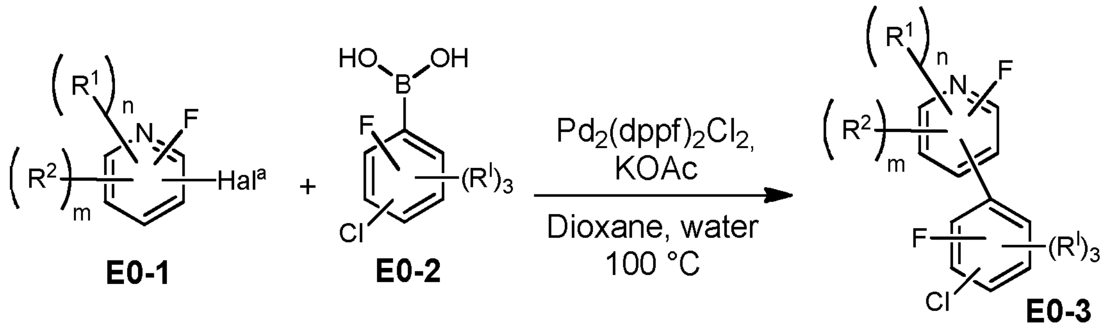


E0-1 + E0-2 → E0-3

**E0-1** (1.00 equivalents), **E0-2** (1.2 equivalent), $Pd(dppf)_2Cl_2$ (0.05 equivalents), and potassium acetate (2.50 equivalents) are stirred under nitrogen atmosphere in a dioxane/water mixture (ratio of 5:1) at 100 °C for 20 h. Active carbon and Celite® (diatomaceous silica) are added to the reaction mixture and stirred at 90 °C for 10 min, then the mixture is hot filtered and the organic phase is concentrated under reduced pressure. The residue is purified by chromatography and the product **E0-3** is obtained as a solid.

*General procedure for synthesis AAV2*

E0-3 +

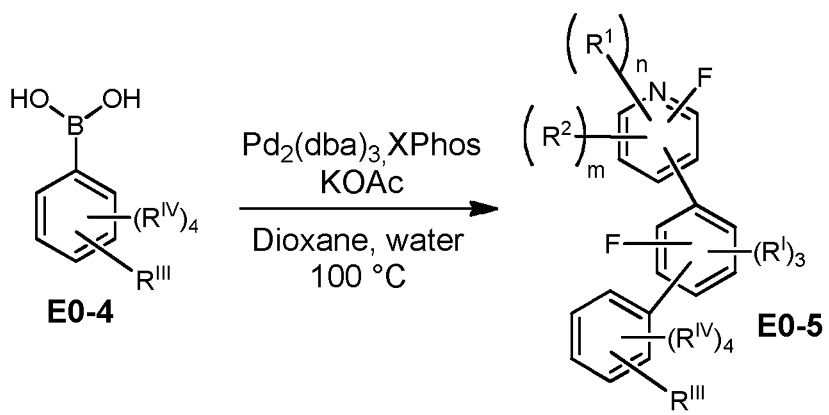


E0-4 → E0-5

E0-3 (1.00 equivalent), the corresponding phenyl boronic acid **E0-4** (1.1 equivalents), $Pd(dba)_3Cl_2$ (0.02 equivalent), XPhos (0.08 equivalents) and potassium acetate (2.50 equivalents) are stirred under nitrogen atmosphere in a

dioxane/water mixture (ratio of 10:1) at 100 °C for 20 h. Active carbon and Celite® are added to the reaction mixture and stirred at 90 °C for 10 min, then the mixture is hot filtered and the organic phase is concentrated under reduced pressure. The residue is purified by chromatography and the product **E0-5** is obtained as a solid.

*General procedure for synthesis **AAV3***

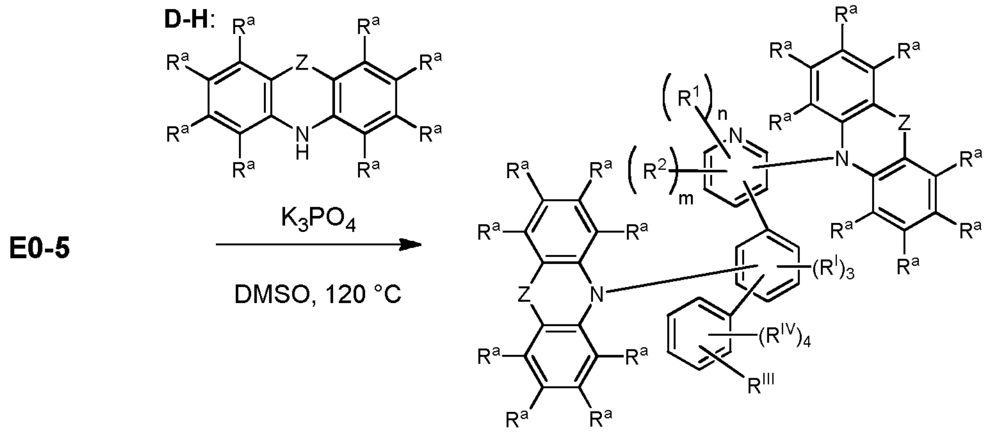


E0-5 (1 equivalent), the corresponding donor molecule D-H (2.00 equivalents) and tribasic potassium phosphate (4.0 equivalents) are suspended under nitrogen atmosphere in DMSO and stirred at 120 °C for 20 h. The reaction mixture is poured into a saturated sodium chloride solution and the resulting precipitate is filtered. Subsequently the residue is dissolved in dichloromethane and washed with saturated sodium chloride solution, dried over $MgSO_4$ and the solvent evaporated under reduced pressure. The crude product is purified by recrystallization out of ethanol or by flash chromatography. The product is obtained as a solid.

In particular, the donor molecule D-H is a 3,6-substituted carbazole (e.g., 3,6-dimethylcarbazole, 3,6-diphenylcarbazole, 3,6-di-tert-butylcarbazole), a 2,7-substituted carbazole (e.g., 2,7-dimethylcarbazole, 2,7-diphenylcarbazole, 2,7-di-tert-butylcarbazole), a 1,8-substituted carbazole (e.g., 1,8-dimethylcarbazole, 1,8-diphenylcarbazole, 1,8-di-tert-butylcarbazole), a 1-substituted carbazole (e.g., 1-methylcarbazole, 1-phenylcarbazole, 1-tert-butylcarbazole), a 2-substituted carbazole (e.g., 2-methylcarbazole, 2-phenylcarbazole, 2-tert-butylcarbazole), or a 3-substituted carbazole (e.g., 3-methylcarbazole, 3-phenylcarbazole, 3-tert-butylcarbazole).

Exemplarily a halogen-substituted carbazole, particularly 3-bromocarbazole, can be used as D-H.

In a subsequent reaction, a boronic acid ester functional group or boronic acid functional group may be, for example, introduced at the position of the one or more halogen substituents, which was introduced via D-H, to yield the corresponding carbazol-3-ylboronic acid ester or carbazol-3-ylboronic acid, e.g., via the reaction with bis(pinacolato)diboron (CAS No. 73183-34-3). Subsequently, one or more substituents $R^a$ may be introduced in place of the boronic acid ester group or the boronic acid group via a coupling reaction with the corresponding halogenated reactant $R^a$-Hal, preferably $R^a$-Cl and $R^a$-Br.

Alternatively, one or more substituents $R^a$ may be introduced at the position of the one or more halogen substituents, which was introduced via D-H, via the reaction with a boronic acid of the substituent $R^a$ [$R^a$-$B(OH)_2$] or a corresponding boronic acid ester.

In particular, **E0-1** is a fluoro-substituted, $Hal^a$-substituted pyridine (e.g., 2-chloro-3-fluoropyridine, 2-chloro-4-fluoropyridine, 2-chloro-5-fluoropyridine, 2-chloro-6-fluoropyridine, 3-chloro-2-fluoropyridine, 3-chloro-4-fluoropyridine, 3-chloro-5-fluoropyridine, 3-chloro-6-fluoropyridine, 4-chloro-2-fluoropyridine, 4-chloro-3-fluoropyridine, 2-bromo-3-fluoropyridine, 2-bromo-4-fluoropyridine, 2-bromo-5-fluoropyridine, 2-bromo-6-fluoropyridine, 3-bromo-2-fluoropyridine, 3-bromo-4-fluoropyridine, 3-bromo-5-fluoropyridine, 3-bromo-6-fluoropyridine, 4-bromo-2-fluoropyridine, 4-bromo-3-fluoropyridine).

In particular, **E0-2** is a chloro-fluoro-phenylboronic acid (e.g., 2-chloro-3-fluoro-phenylboronic acid, 2-chloro-4-fluoro-phenylboronic acid, 2-chloro-5-fluoro-phenylboronic acid, 2-chloro-6-fluoro-phenylboronic acid, 3-chloro-2-fluoro-phenylboronic acid, 3-chloro-4-fluoro-phenylboronic acid, 3-chloro-5-fluoro-phenylboronic acid, 3-chloro-6-fluoro-phenylboronic acid, 4-chloro-2-fluoro-phenylboronic acid, 2-chloro-4-fluoro-phenylboronic acid).

In particular, **E0-4** is a cynanophenylboronic acid (e.g., 2-cyanophenylboronic acid, 3-cyanophenylboronic acid, 4-cyanophenylboronic acid).

*Cyclic voltammetry*

Cyclic voltammograms are measured from solutions having concentration of $10^{-3}$ mol/L of the organic molecules in dichloromethane or a suitable solvent and a suitable supporting electrolyte (e.g. 0.1 mol/L of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against a saturated calomel electrode (SCE).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.
The sample concentration is 10 mg/ml, dissolved in a suitable solvent.
Program: 1) 3 s at 400 U/min; 20 s at 1000 U/min at 1000 Upm/s. 3) 10 s at 4000 U/min at 1000 Upm/s. After coating, the films are tried at 70 °C for 1 min.

Photoluminescence spectroscopy and TCSPC *(Time-correlated single-photon counting)* Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.

Excited state lifetimes are determined employing the same system using the TCSPC method with FM-2013 equipment and a Horiba Y von TCSPC hub.

Excitation sources:

NanoLED 370 (wavelength: 371 nm, puls duration: 1,1 ns)
NanoLED 290 (wavelength: 294 nm, puls duration: <1 ns)
SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Photoluminescence quantum yield measurements

For photoluminescence quantum yield (PLQY) measurements an *Absolute PL Quantum Yield Measurement C9920-03G* system *(Hamamatsu Photonics)* is used. Quantum yields and CIE coordinates are determined using the software U6039-05 version 3.6.0.

Emission maxima are given in nm, quantum yields $\Phi$ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement

Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

**Production and characterization of optoelectronic devices**

OLED devices comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100 %.

The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc.

Accelerated lifetime measurements are performed (e.g. applying increased current densities). Exemplarily LT80 values at 500 cd/m$^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.

The values correspond to the average of several pixels (typically two to eight), the standard deviation between these pixels is given.

**HPLC-MS:**

HPLC-MS spectroscopy is performed on a HPLC by Agilent (1100 series) with MS-detector (Thermo LTQ XL). A reverse phase column 4,6mm x 150mm, particle size 5,0 μm from Waters (without pre-column) is used in the HPLC. The HPLC-MS measurements are performed at room temperature (rt) with the solvents acetonitrile, water and THF in the following concentrations:

| | | |
|---|---|---|
| solvent A: | $H_2O$ (90%) | MeCN (10%) |
| solvent B: | $H_2O$ (10%) | MeCN (90%) |
| solvent C: | THF (100%) | |

From a solution with a concentration of 0.5mg/ml an injection volume of 15 μL is taken for the measurements. The following gradient is used:

| Flow rate [ml/min] | time [min] | A[%] | B[%] | D[%] |
|---|---|---|---|---|
| 3 | 0 | 40 | 50 | 10 |
| 3 | 10 | 10 | 15 | 75 |
| 3 | 16 | 10 | 15 | 75 |
| 3 | 16.01 | 40 | 50 | 10 |
| 3 | 20 | 40 | 50 | 10 |

Ionisation of the probe is performed by atmospheric pressure chemical ionization (APCI).

**Example 1**

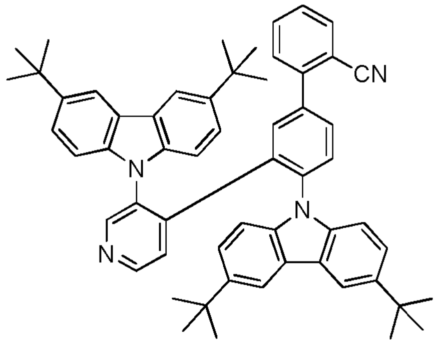

Example 1 was synthesized according **to *AAV1*** (yield 82 %), wherein 4-chloro-3-fluoropyridine (CAS 2546-56-7) was used as reactant **E0-1** and 5-chloro-2-fluorophenylboronic acid (CAS 352535-83-2) was used as reactant **E0-2,**

***AAV2*** (yield 88 %), wherein 2-cyanophenylboronic acid (CAS 138642-62-3) was used as reactant **E0-4,** and ***AAV3*** (yield 81 %).

HPLC-LCMS:

| Molecular Formula | Retention Time | m/z calculated | m/z found |
|---|---|---|---|
| $C_{58}H_{58}N_4$ | 17.75 min | 810.47 | 809.90 |

Figure 1 depicts the emission spectrum of example 1 (10 % by weight in PMMA). The emission maximum ($\lambda_{max}$) is at 446 nm. The photoluminescence quantum yield (PLQY) is 62 %, the full width at half maximum (FWHM) is 0.41 eV. The resulting $CIE_x$ coordinate is determined at 0.17 and the $CIE_y$ coordinate at 0.13. The HOMO(E) is -5.70 eV.

**Example 2**

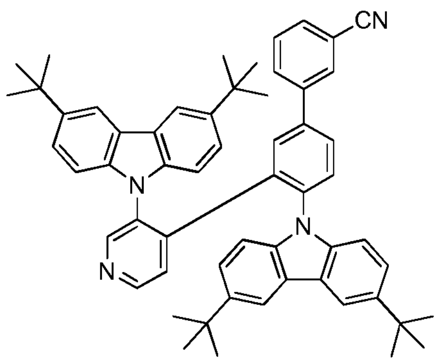

Example 2 was synthesized according to ***AAV1*** (yield 82 %), wherein 4-chloro-3-fluoropyridine (CAS 2546-56-7) was used as reactant **E0-1** and 5-chloro-2-fluorophenylboronic acid (CAS 352535-83-2) was used as reactant **E0-2,**

***AAV2*** (yield 89 %), wherein 3-cyanophenylboronic acid (CAS 150255-96-2) was used as reactant **E0-4,** and ***AAV3*** (yield 56 %).

HPLC-LCMS:

| Molecular Formula | Retention Time | m/z calculated | m/z found |
|---|---|---|---|
| $C_{58}H_{58}N_4$ | 17.97 min | 810.47 | 810.81 |

Figure 2 depicts the emission spectrum of example 2 (10 % by weight in PMMA). The emission maximum ($\lambda_{max}$) is at 449 nm. The photoluminescence quantum yield (PLQY) is 59 %, the full width at half maximum (FWHM) is 0.41 eV. The resulting $CIE_x$ coordinate is determined at 0.17 and the $CIE_y$ coordinate at 0.14 and HOMO(E) is -5.72 eV.

**Example 3**

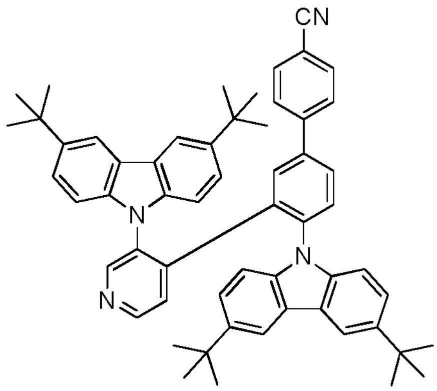

Example 3 was synthesized according to ***AAV1*** (yield 82 %), wherein 4-chloro-3-fluoropyridine (CAS 2546-56-7) was used as reactant **E0-1** and 5-chloro-2-fluorophenylboronic acid (CAS 352535-83-2) was used as reactant **E0-2,**
***AAV2*** (yield 83 %), wherein 4-cyanophenylboronic acid (CAS 126747-14-6) was used as reactant **E0-4,** and ***AAV3*** (yield 77 %).

HPLC-LCMS:

| Molecular Formula | Retention Time | m/z calculated | m/z found |
|---|---|---|---|
| $C_{58}H_{58}N_4$ | 17.95 min | 810.47 | 809.80 |

Figure 3 depicts the emission spectrum of example **3 (10** % by weight in PMMA). The emission maximum ($\lambda_{max}$) is at 451 nm. The photoluminescence quantum yield (PLQY) is 57 %, the full width at half maximum (FWHM) is 0.39 eV. The resulting $CIE_x$ coordinate is determined at 0.16 and the $CIE_y$ coordinate at 0.14. The energy level of the highest occupied molecular orbital HOMO(E) is -5.72 eV.

**Example 4**

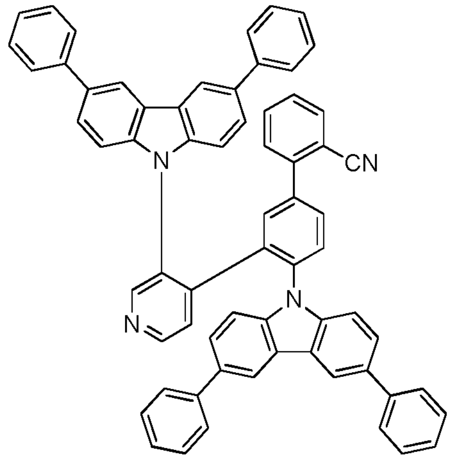

Example 4 was synthesized according to

***AAV1*** (yield 82 %), wherein 4-chloro-3-fluoropyridine (CAS 2546-56-7) was used as reactant **E0-1** and 5-chloro-2-fluorophenylboronic acid (CAS 352535-83-2) was used as reactant **E0-2, *AAV2*** (yield 88 %), wherein 2-cyanophenylboronic acid (CAS 138642-62-3) was used as reactant **E0-4,**
and ***AAV3*** (yield 74 %).

HPLC-LCMS:

| Molecular Formula | Retention Time | m/z calculated | m/z found |
|---|---|---|---|
| $C_{66}H_{42}N_4$ | 13.74 min | 890.34 | 889.74 |

Figure 4 depicts the emission spectrum of example 4 (10 % by weight in PMMA). The emission maximum ($\lambda_{max}$) is at 449 nm. The photoluminescence quantum yield (PLQY) is 47 %, the full width at half maximum (FWHM) is 0.41 eV. The resulting $CIE_x$ coordinate is determined at 0.17 and the $CIE_y$ coordinate at 0.14. The energy level of the highest occupied molecular orbital HOMO(E) is -5.75 eV.

Example 5

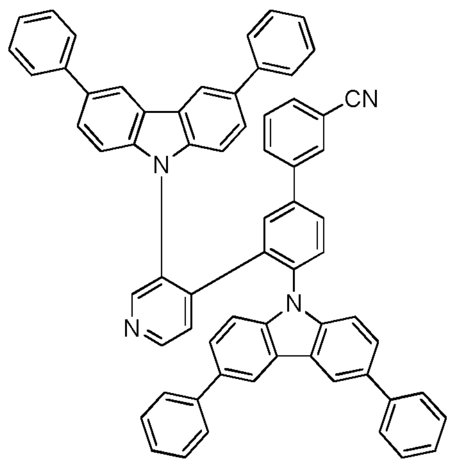

Example 5 was synthesized according to

***AAV1*** (yield 82 %), wherein 4-chloro-3-fluoropyridine (CAS 2546-56-7) was used as reactant **E0-1** and 5-chloro-2-fluorophenylboronic acid (CAS 352535-83-2) was used as reactant **E0-2,** ***AAV2*** (yield 89 %), wherein 3-cyanophenylboronic acid (CAS 150255-96-2) was used as reactant **E0-4,**
and ***AAV3*** (yield 81 %).

HPLC-LCMS:

| Molecular Formula | Retention Time | m/z calculated | m/z found |
|---|---|---|---|
| $C_{66}H_{42}N_4$ | 13.79 min | 890.34 | 889.64 |

Figure 5 depicts the emission spectrum of example 5 (10 % by weight in PMMA). The emission maximum ($\lambda_{max}$) is at 444 nm. The photoluminescence quantum yield (PLQY) is 36 %, the full width at half maximum (FWHM) is 0.42 eV. The resulting $CIE_x$ coordinate is determined at 0.16 and the $CIE_y$ coordinate at 0.12. The energy level of the highest occupied molecular orbital HOMO(E) is -5.77 eV.

Example 6

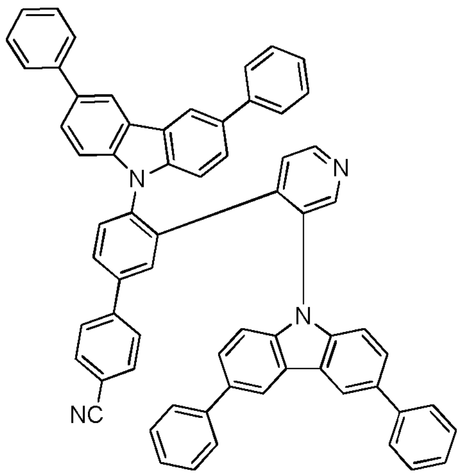

Example 6 was synthesized according to ***AAV1*** (yield 82 %), wherein 4-chloro-3-fluoropyridine (CAS 2546-56-7) was used as reactant **E0-1** and 5-chloro-2-fluorophenylboronic acid (CAS 352535-83-2) was used as reactant **E0-2,**
***AAV2*** (yield 83 %), wherein 4-cyanophenylboronic acid (CAS 126747-14-6) was used as reactant **E0-4,**
and ***AAV3*** (yield 70 %).

HPLC-LCMS:

| Molecular Formula | Retention Time | m/z calculated | m/z found |
|---|---|---|---|
| $C_{66}H_{42}N_4$ | 13.78 min | 890.34 | 890.34 |

Figure 6 depicts the emission spectrum of example 6 (10 % by weight in PMMA). The emission maximum ($\lambda_{max}$) is at 448 nm. The photoluminescence quantum yield (PLQY) is 44 %, the full width at half maximum (FWHM) is 0.40 eV. The resulting $CIE_x$ coordinate is determined at 0.16 and the $CIE_y$ coordinate at 0.13.

Example 7

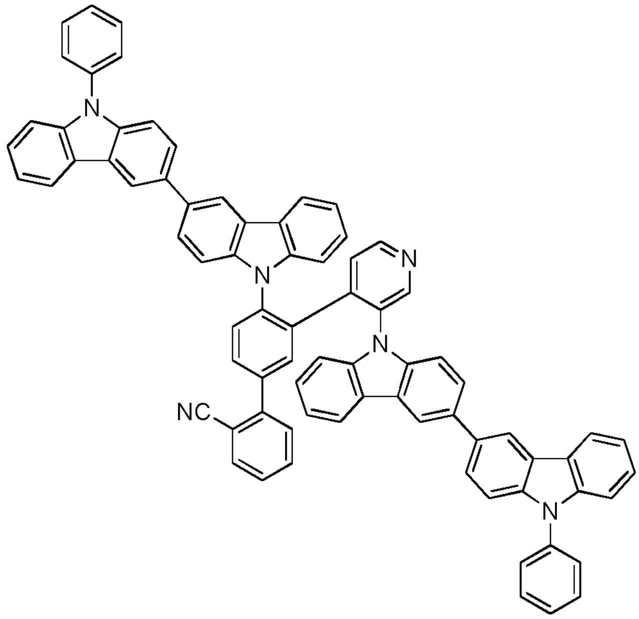

Example 7 was synthesized according to ***AAV1*** (yield 82 %), wherein 4-chloro-3-fluoropyridine (CAS 2546-56-7) was used as reactant **E0-1** and 5-chloro-2-fluorophenylboronic acid (CAS 352535-83-2) was used as reactant **E0-2,**

***AAV2*** (yield 88 %), wherein 2-cyanophenylboronic acid (CAS 138642-62-3) was used as reactant **E0-4,** and ***AAV3*** (yield 67 %), wherein 9-phenyl-3,3'-bicarbazolyl (CAS 1060735-14-9) was used as reactant D-H.

HPLC-LCMS:

| Molecular Formula | Retention Time | m/z calculated | m/z found |
|---|---|---|---|
| $C_{78}H_{48}N_6$ | 22.25 min, 23.11 min (rotational isomers) | 1069.28 | 1069.54 |

Figure 7 depicts the emission spectrum of example 7 (10 % by weight in PMMA). The emission maximum ($\lambda_{max}$) is at 457 nm. The photoluminescence quantum yield (PLQY) is 51 %, the full width at half maximum (FWHM) is 0.43 eV. The resulting $CIE_x$ coordinate is determined at 0.16 and the $CIE_y$ coordinate at 0.17. The energy level of the highest occupied molecular orbital HOMO(E) is -5.59 eV.

**Additional Examples of Organic Molecules of the Invention**

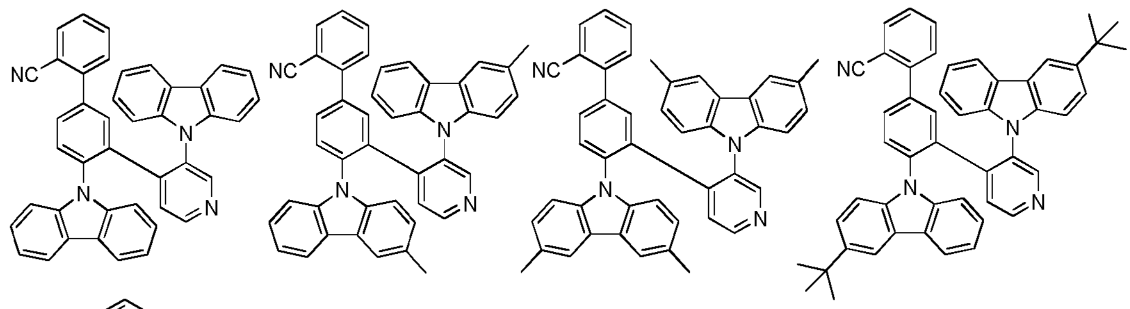

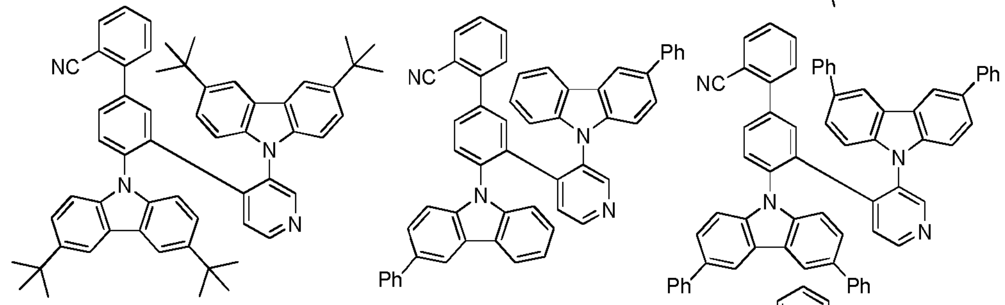

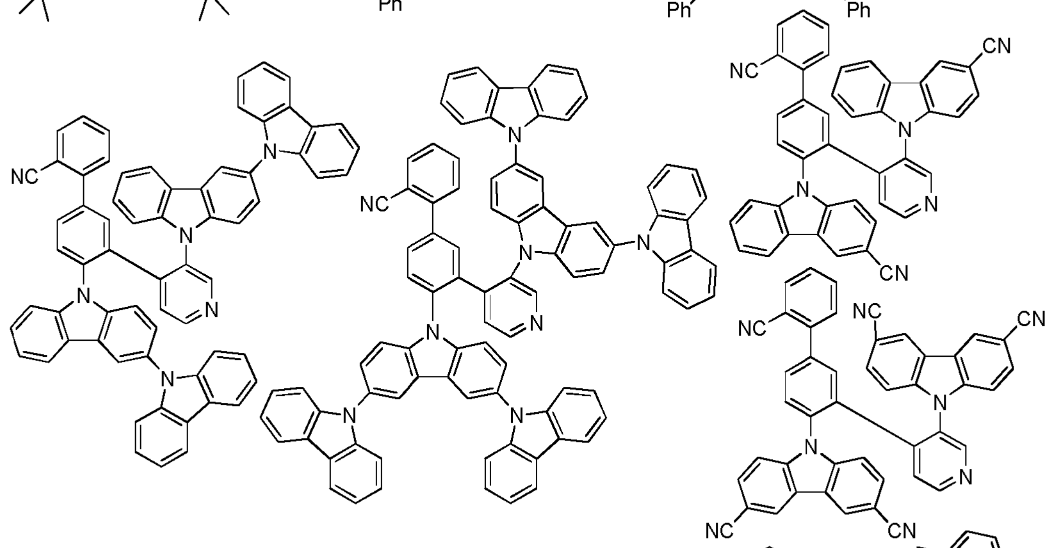

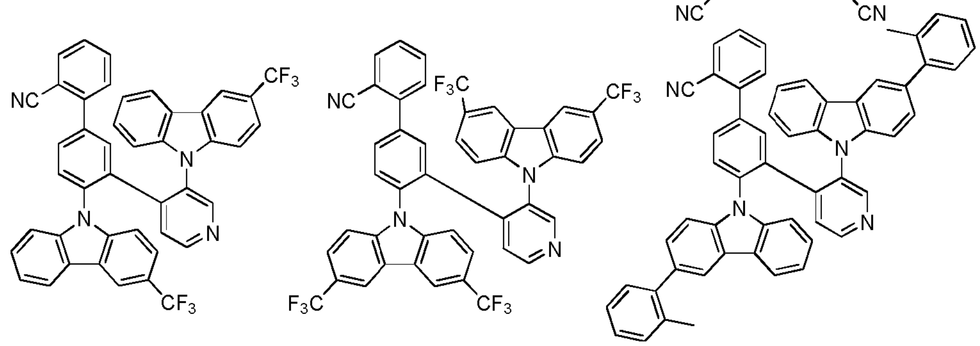

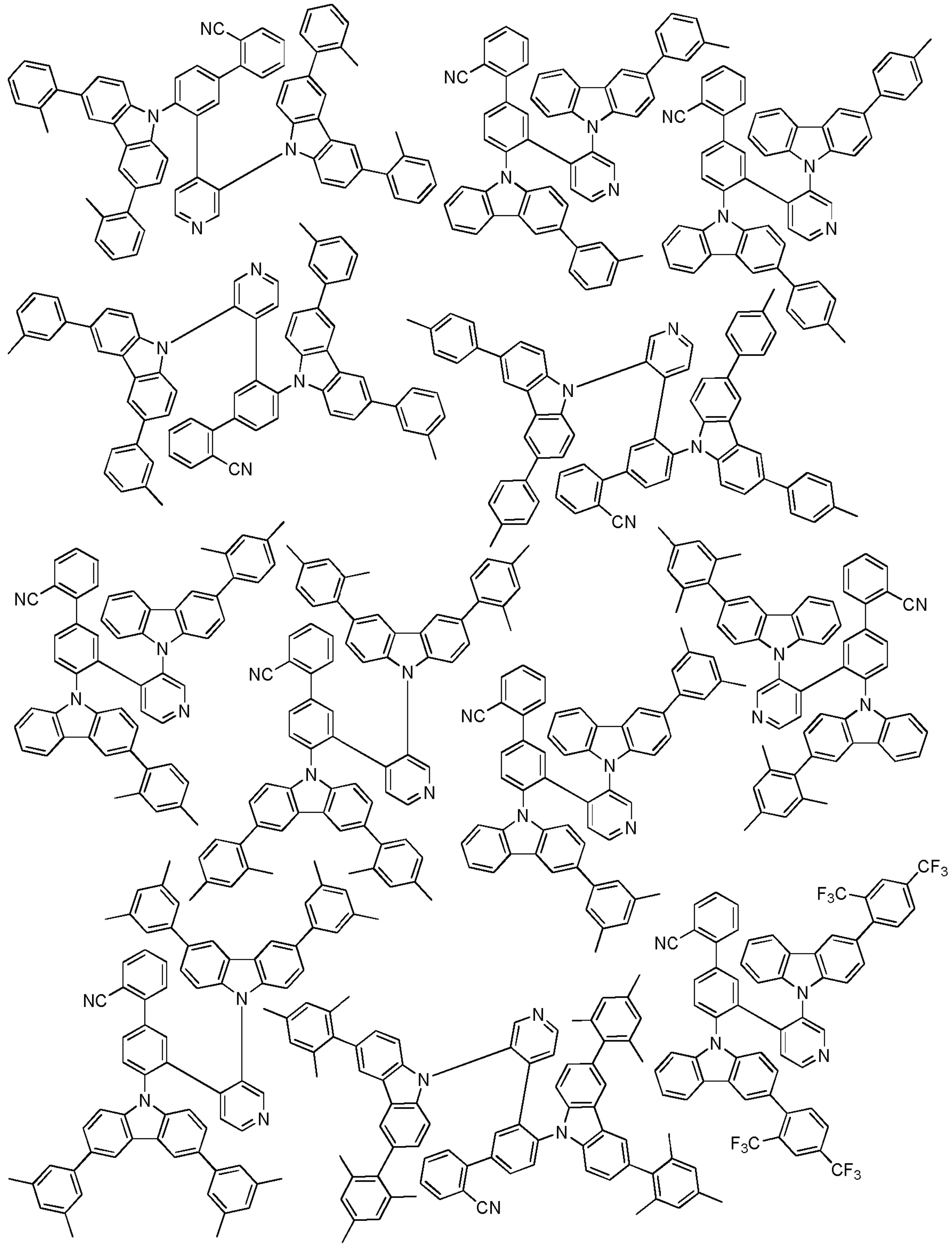

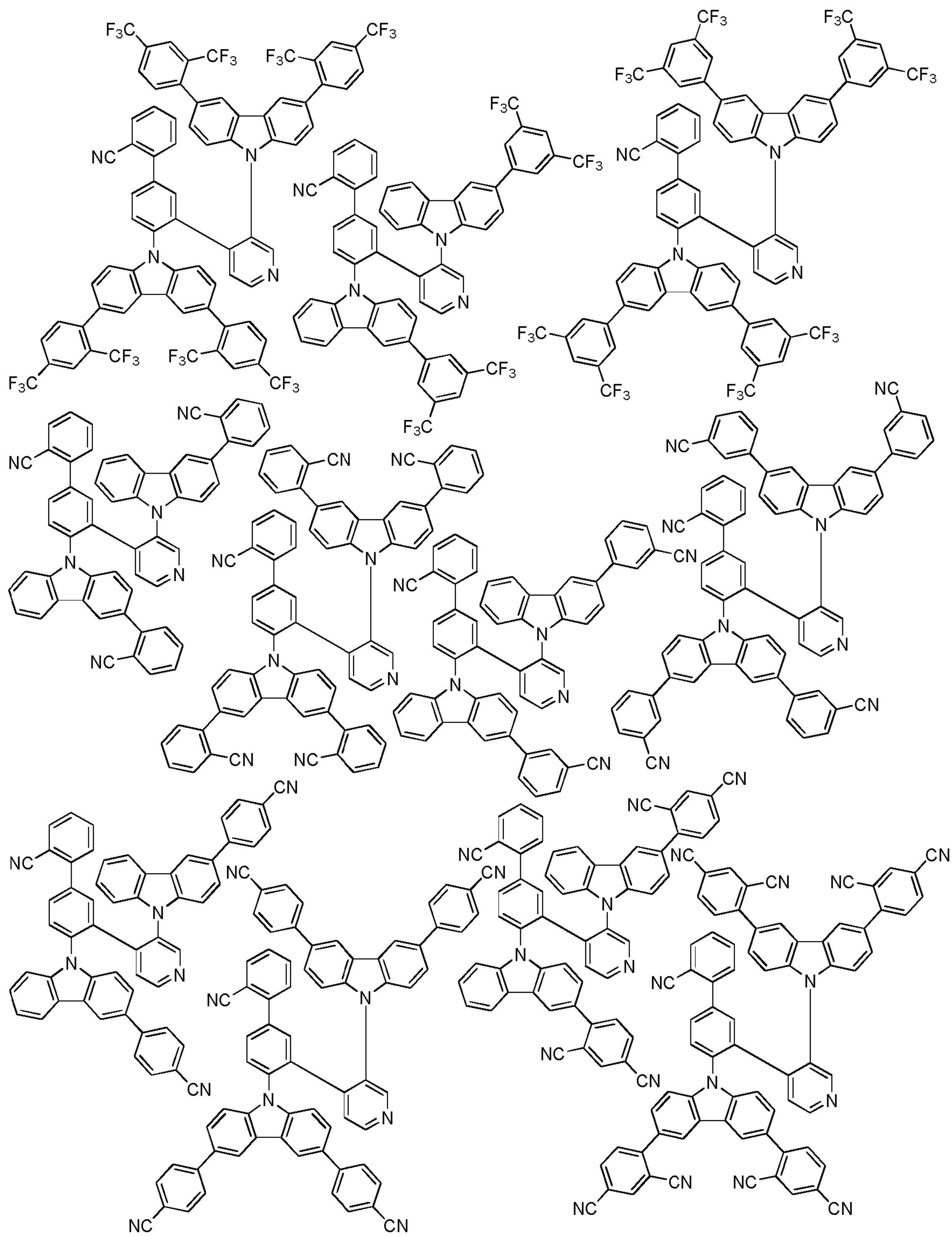

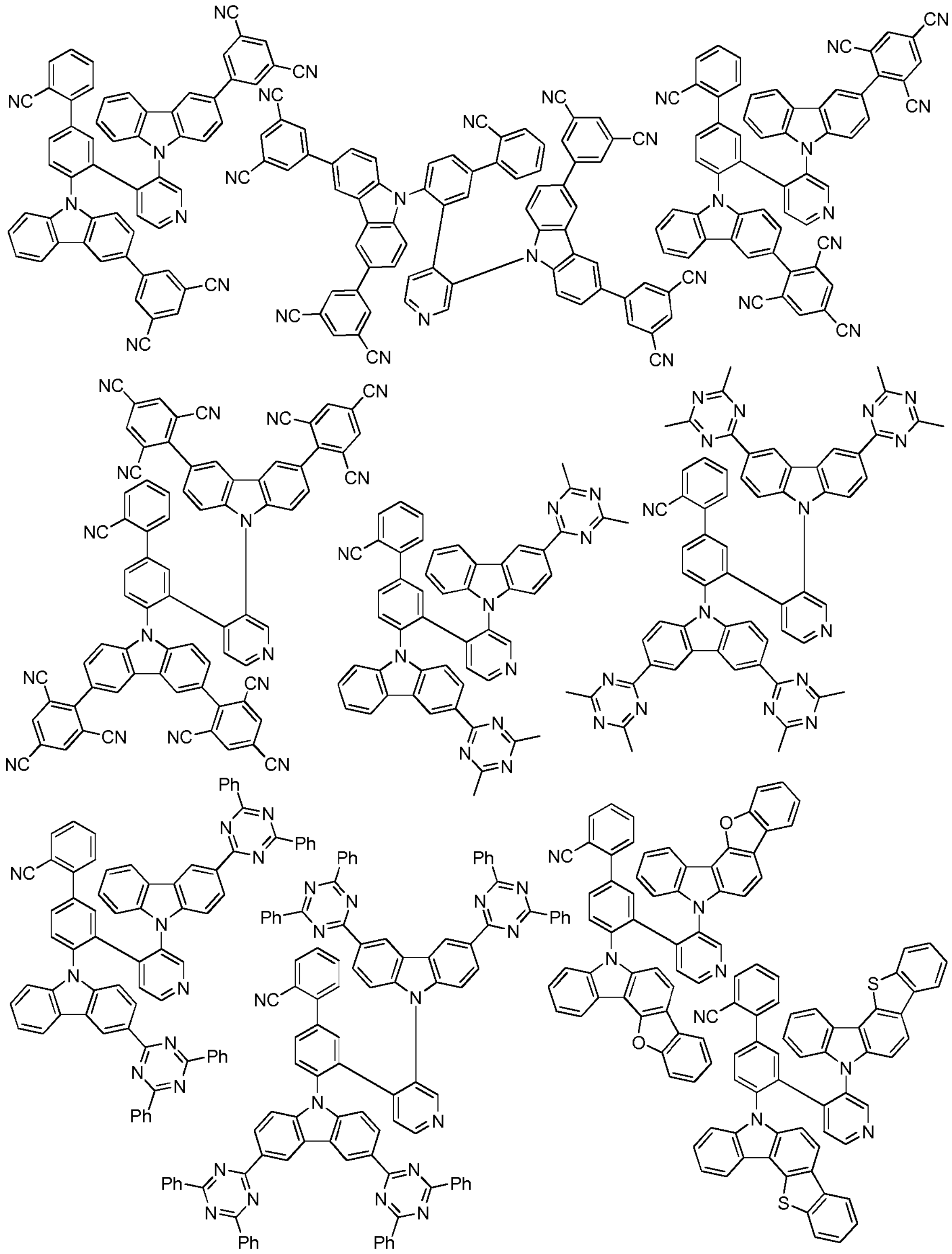

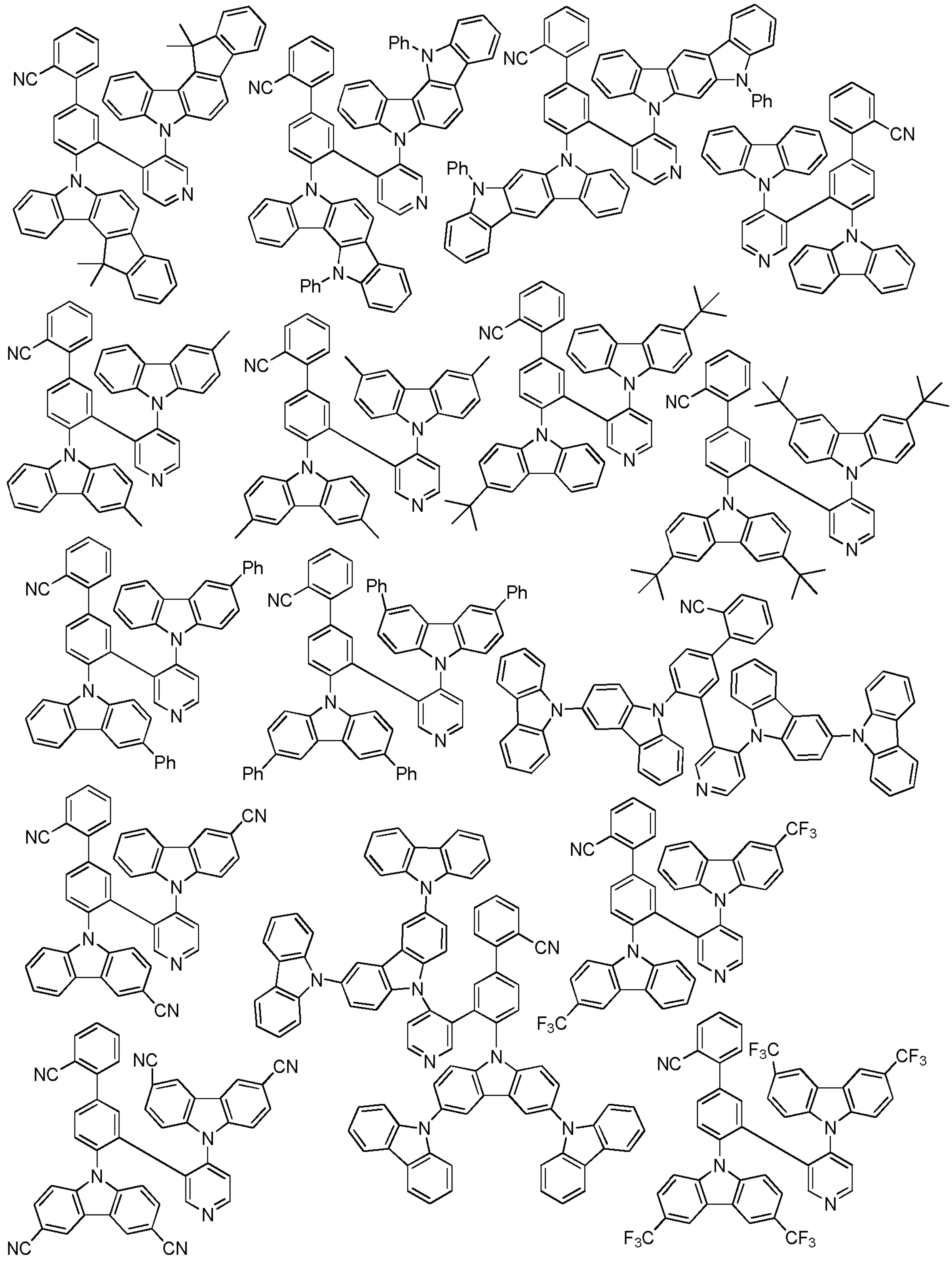

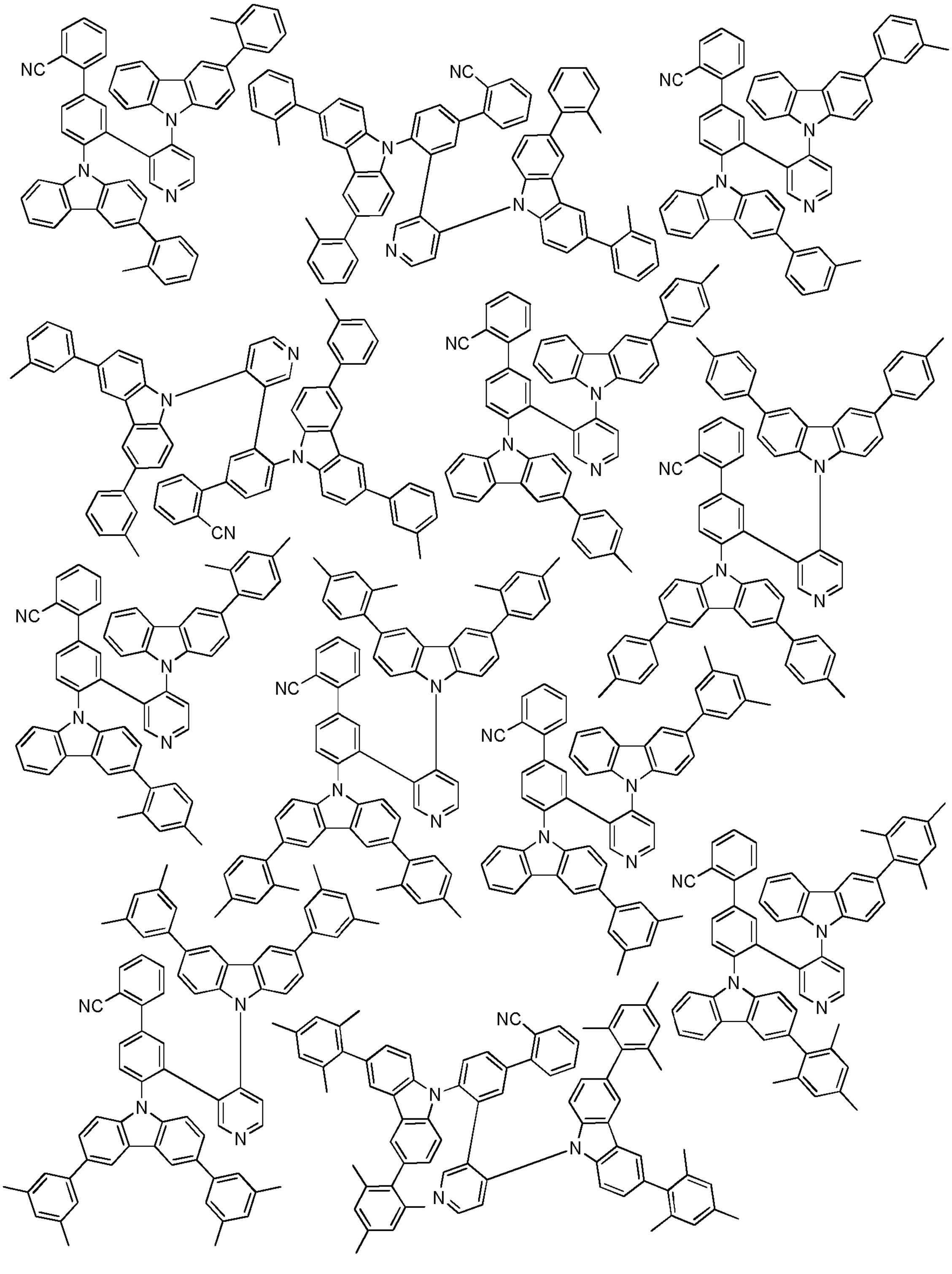

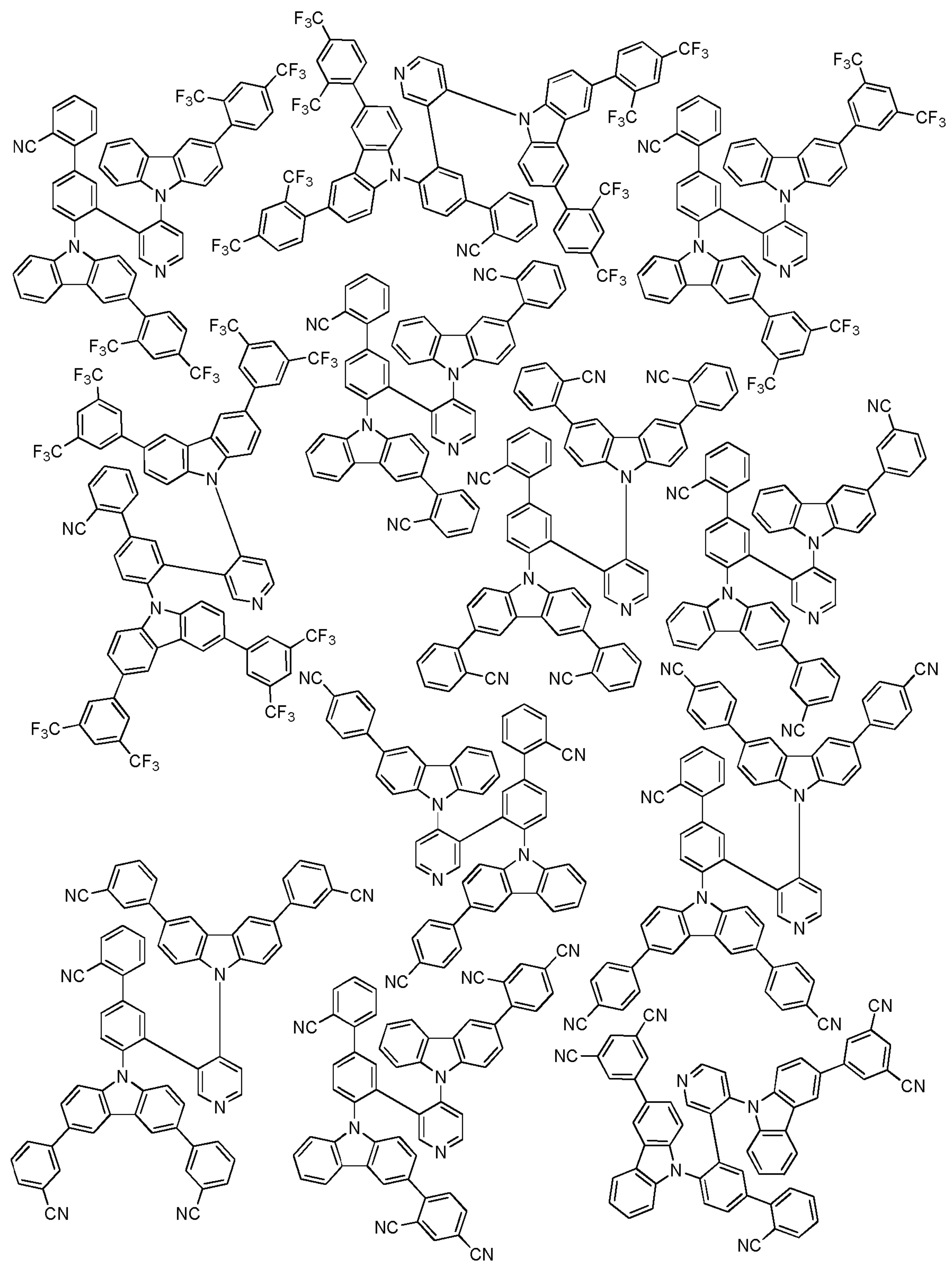

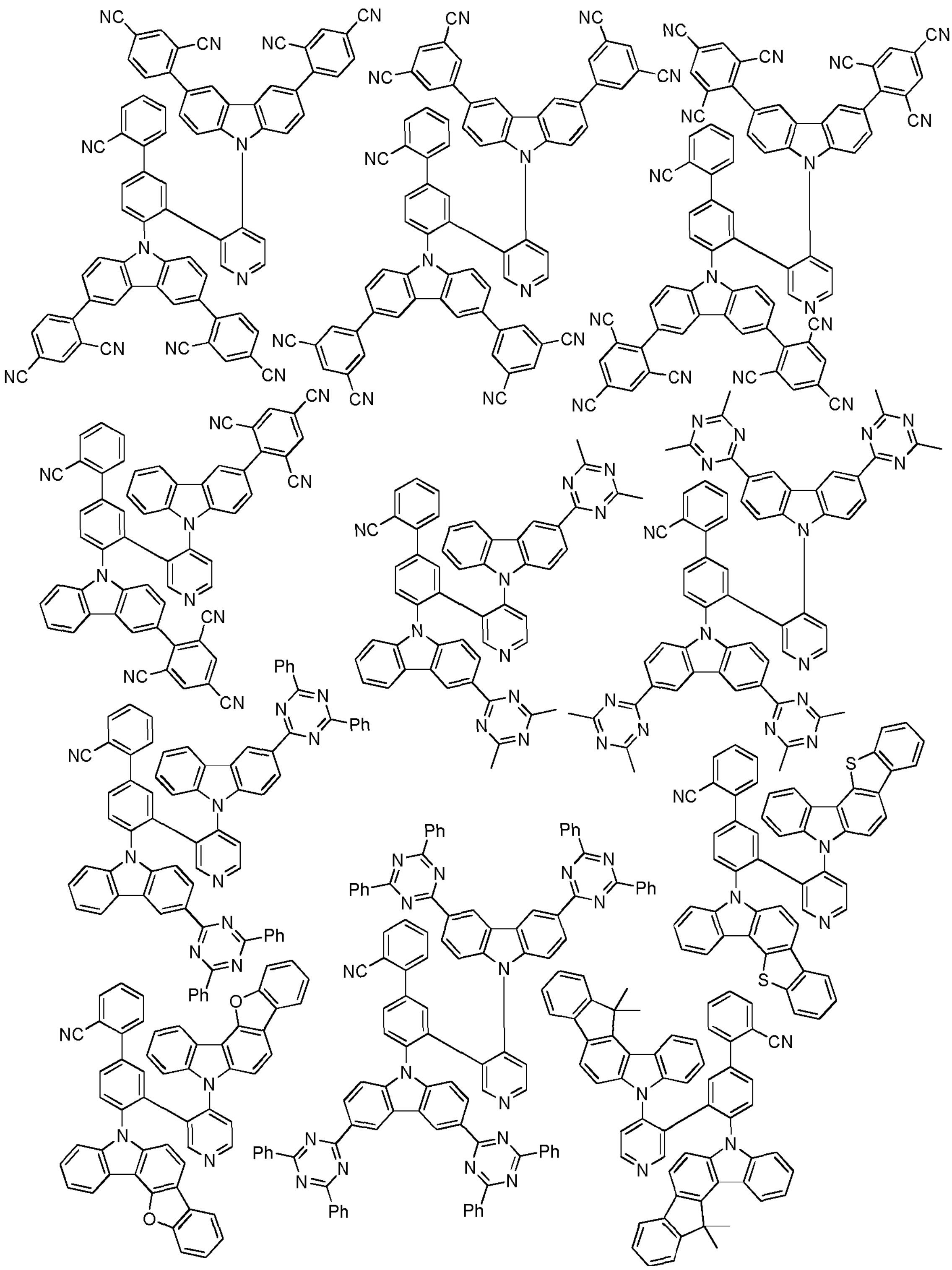

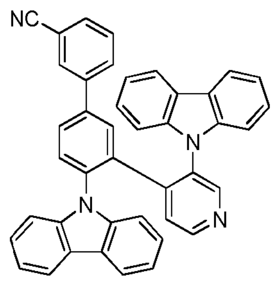
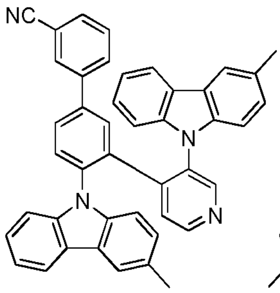
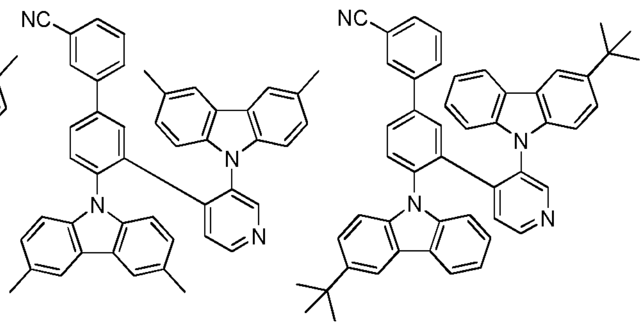
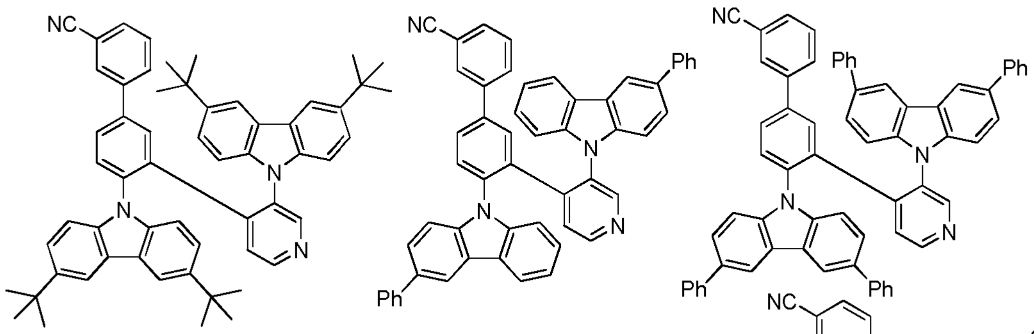
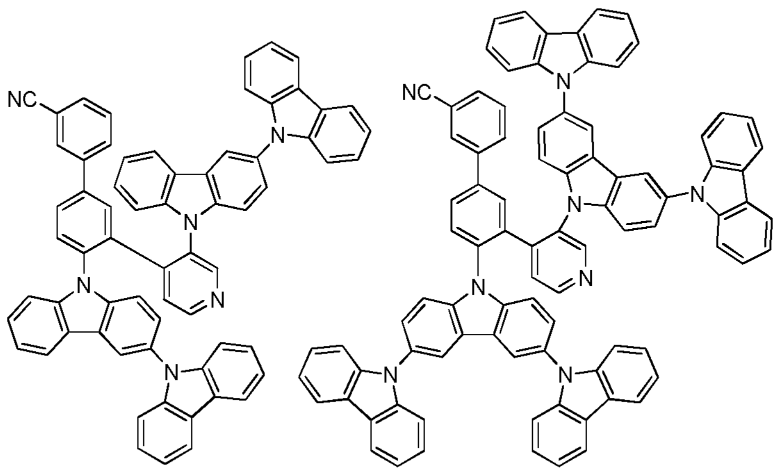
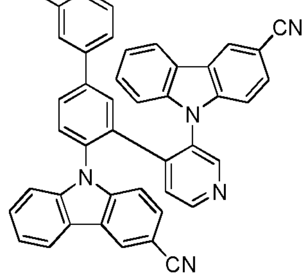
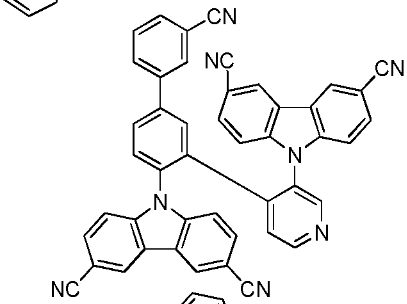
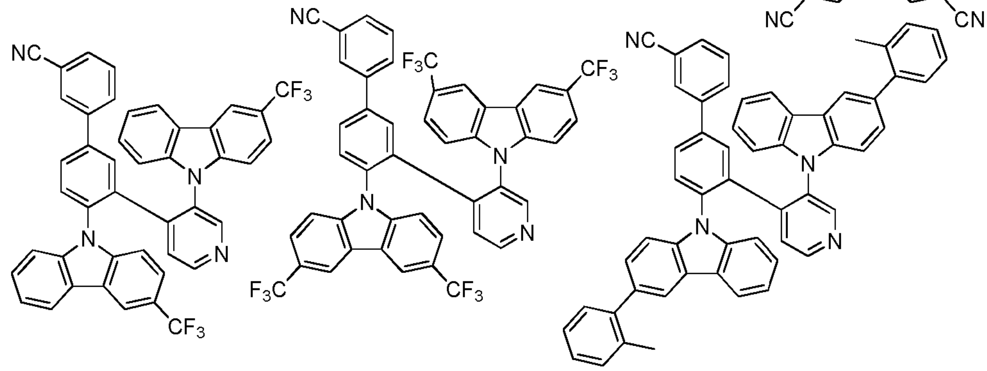

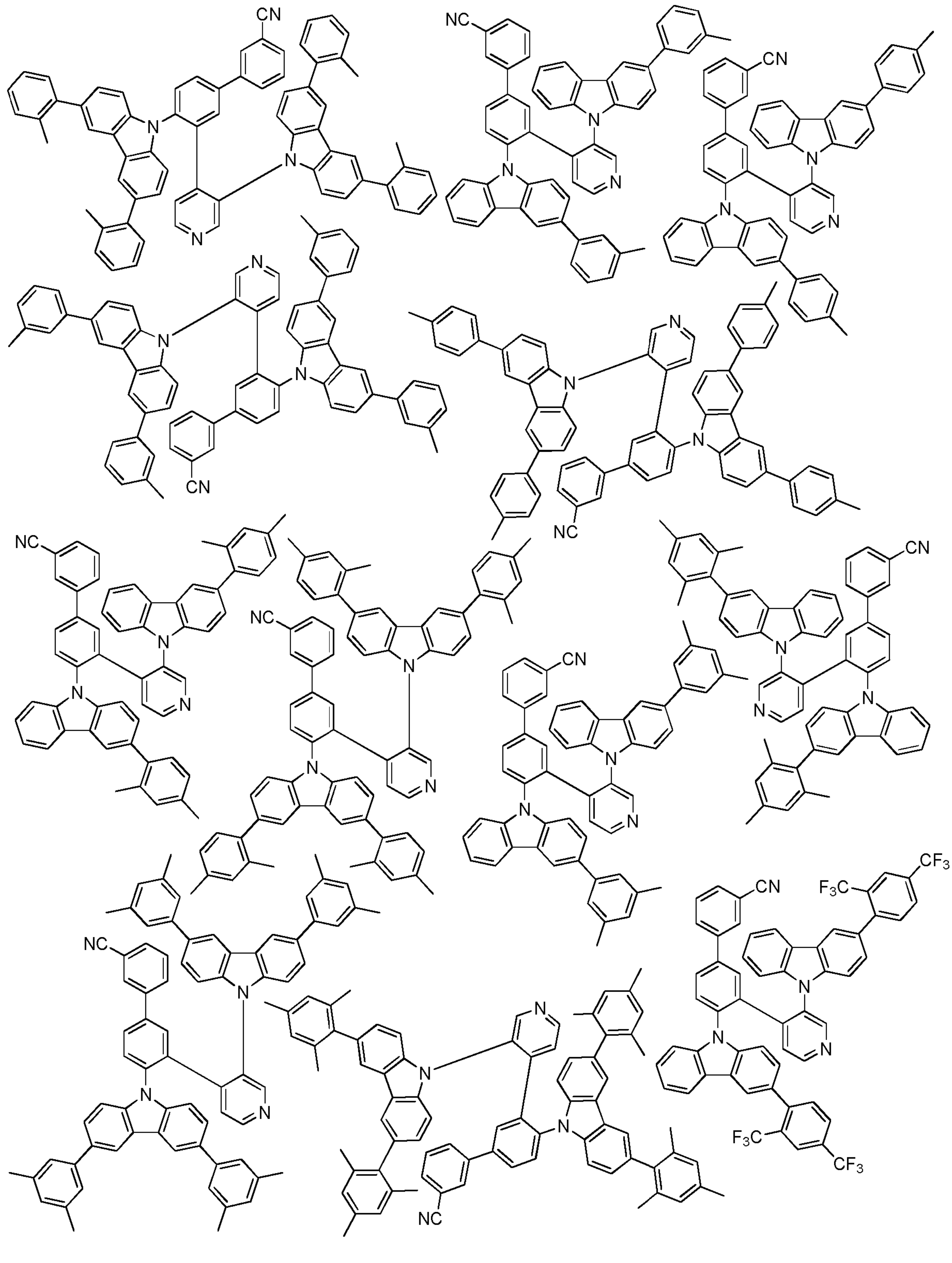

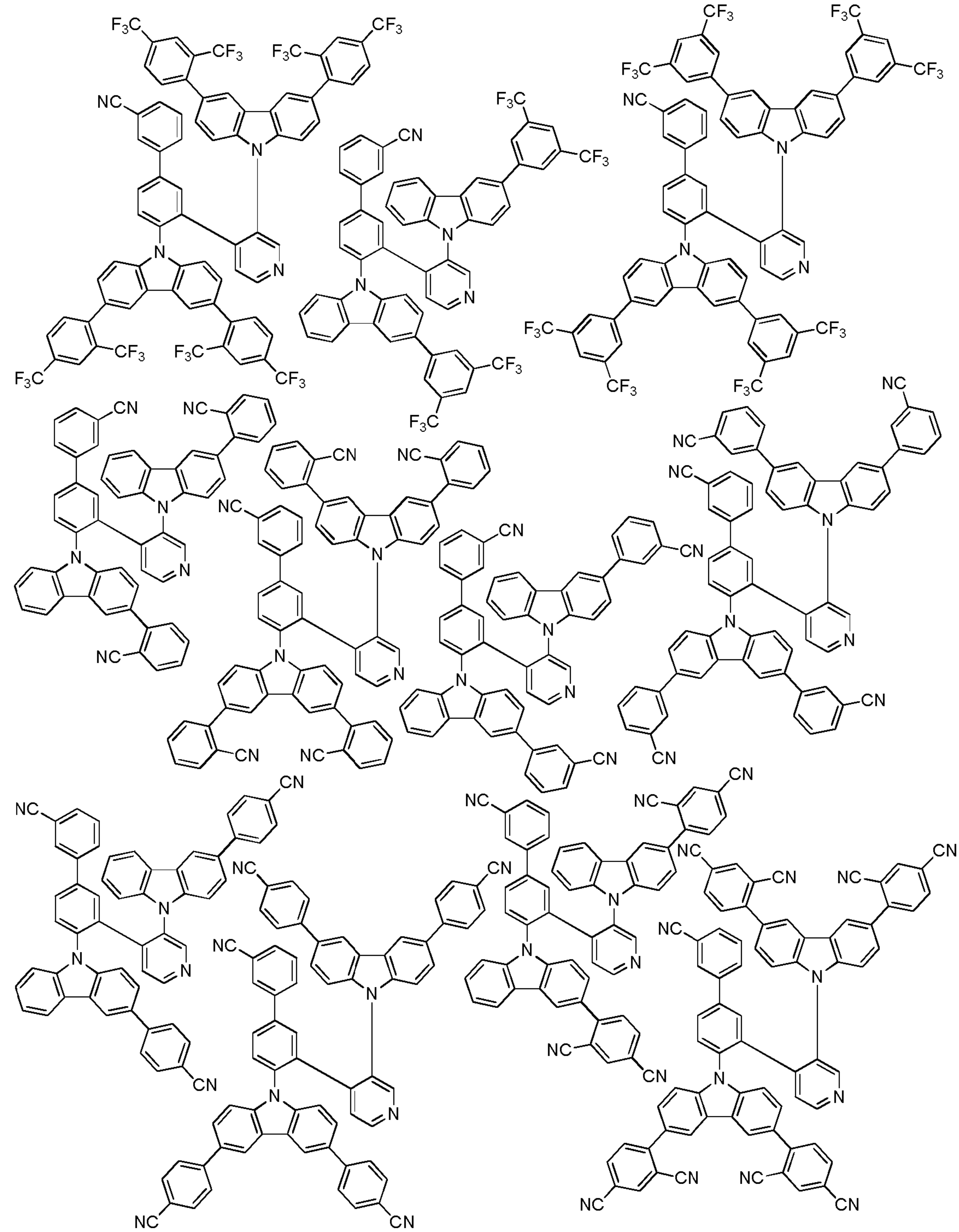

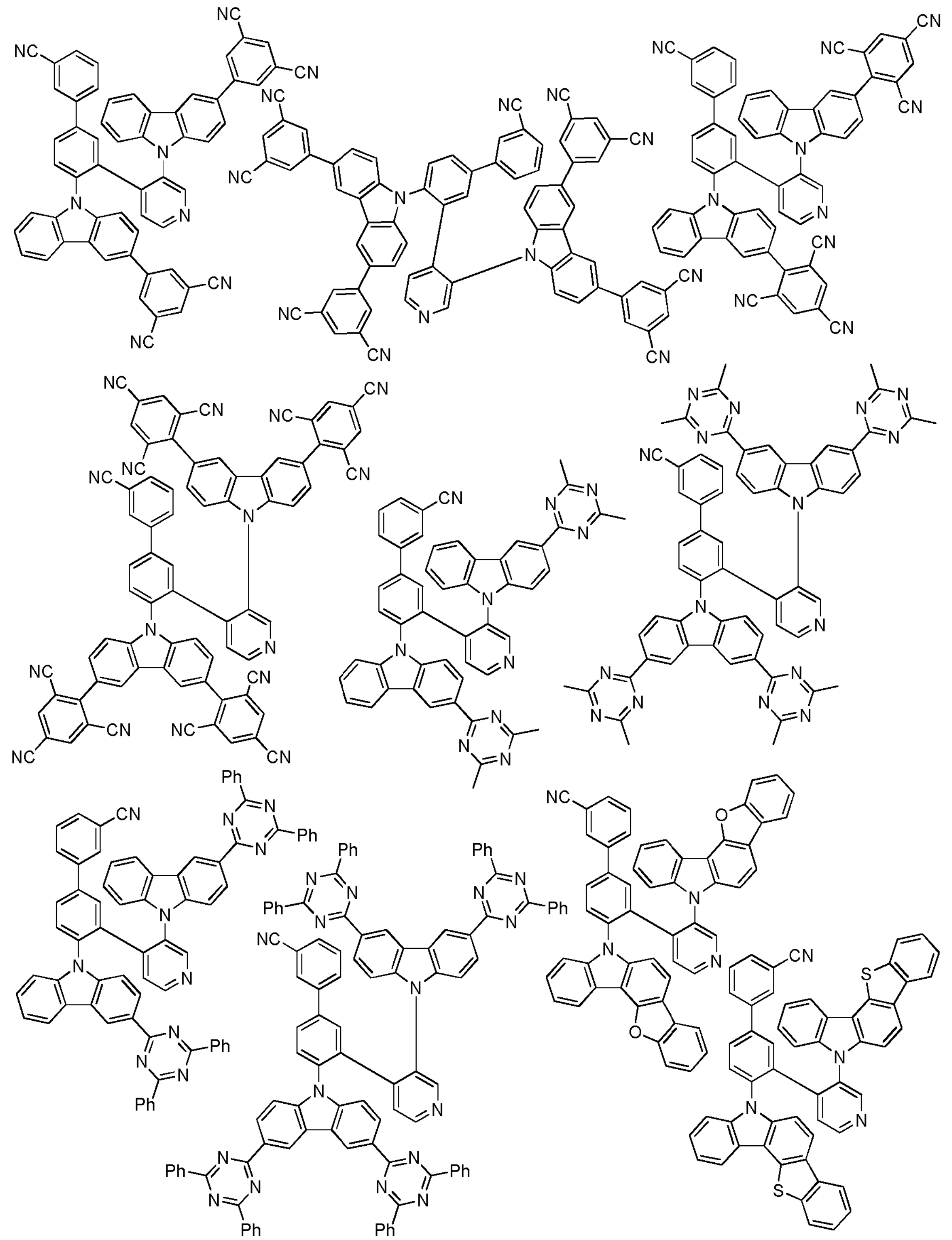

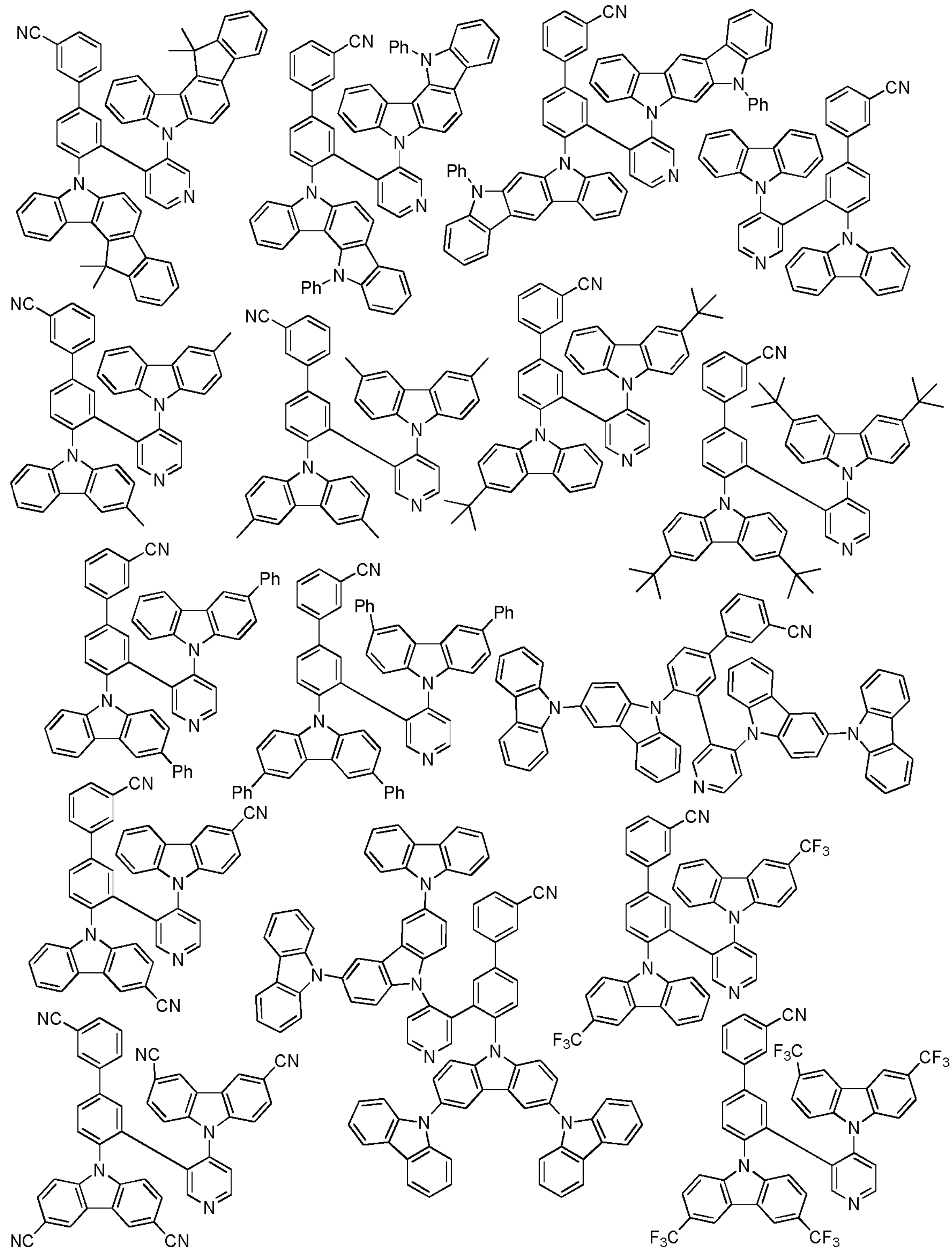

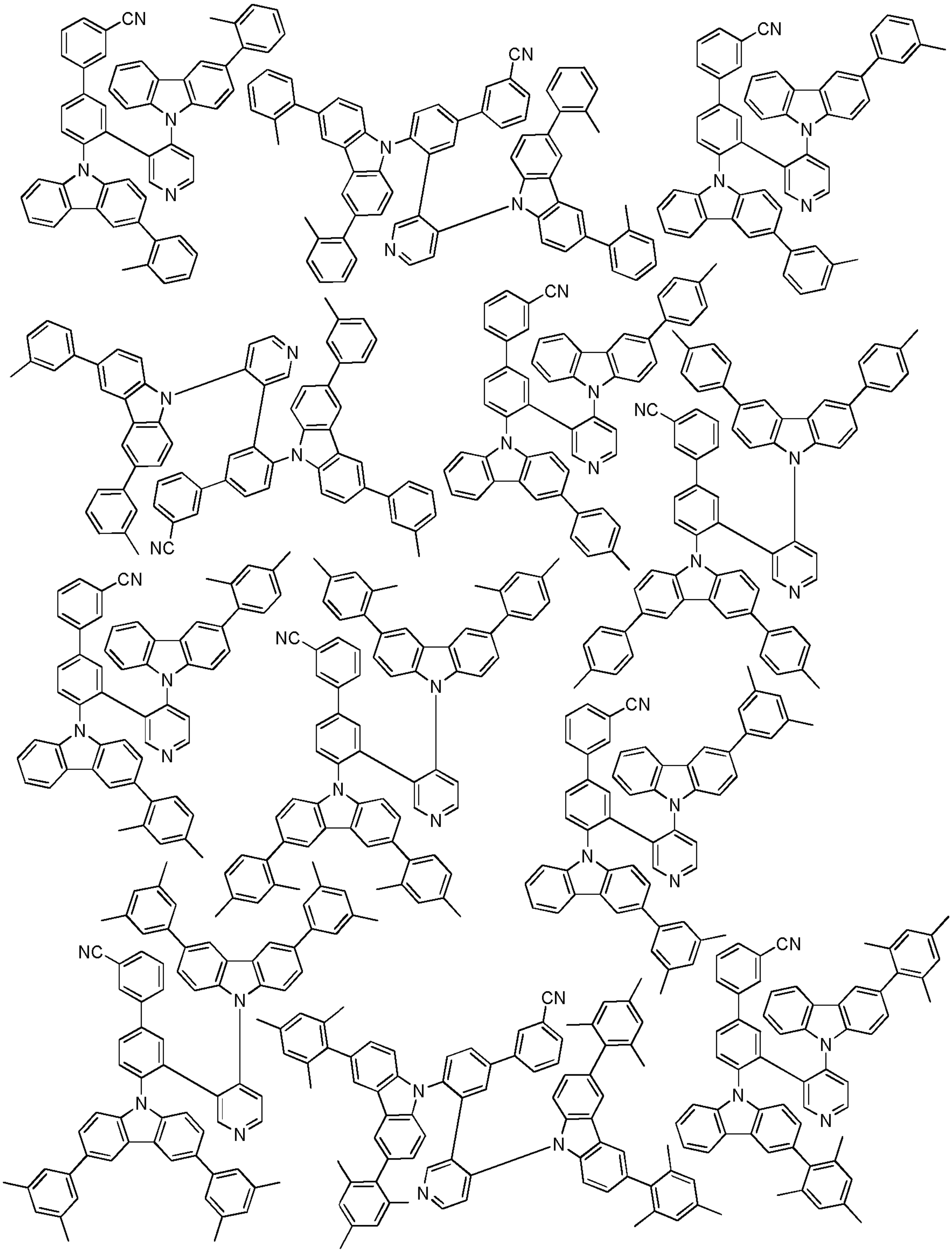

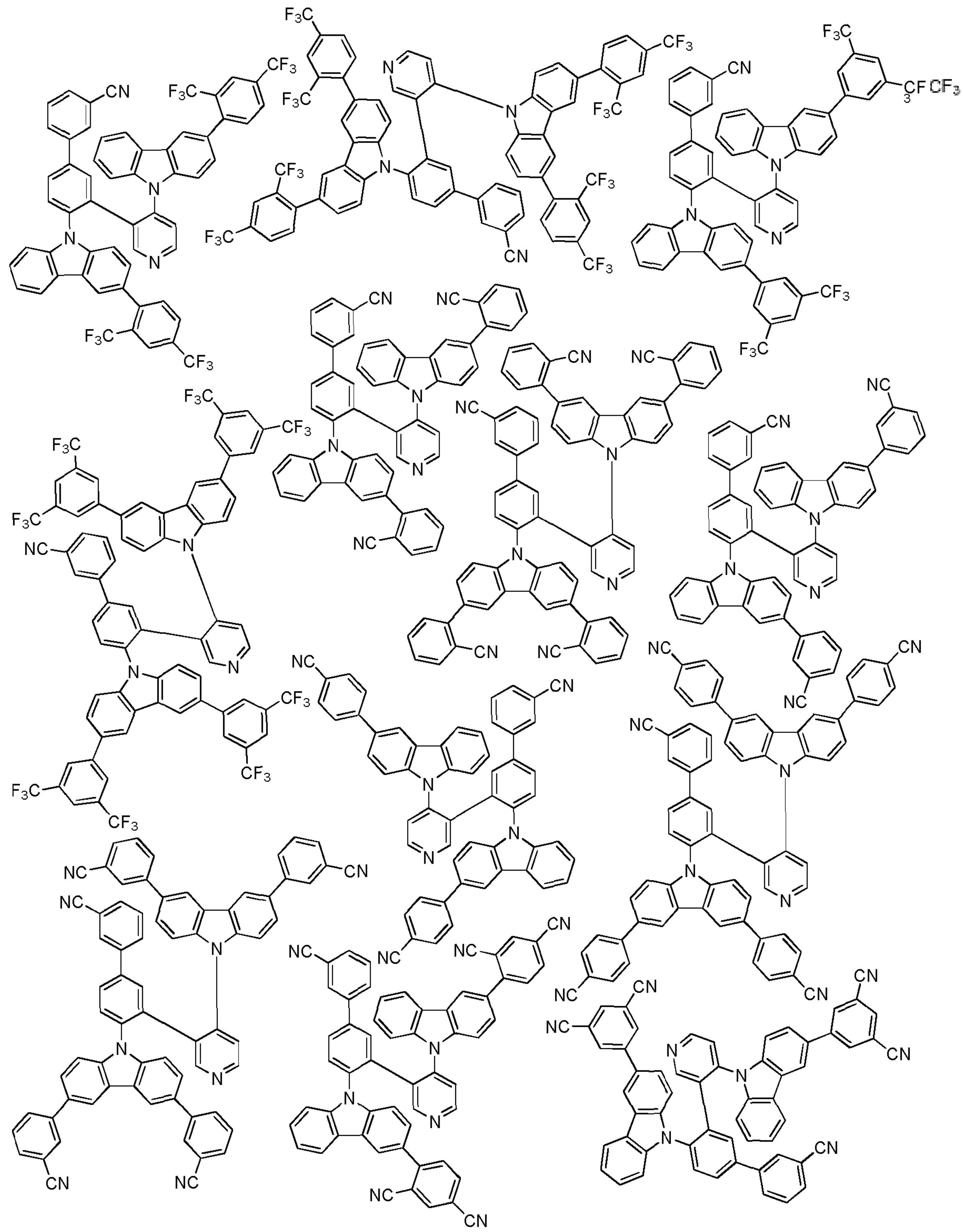

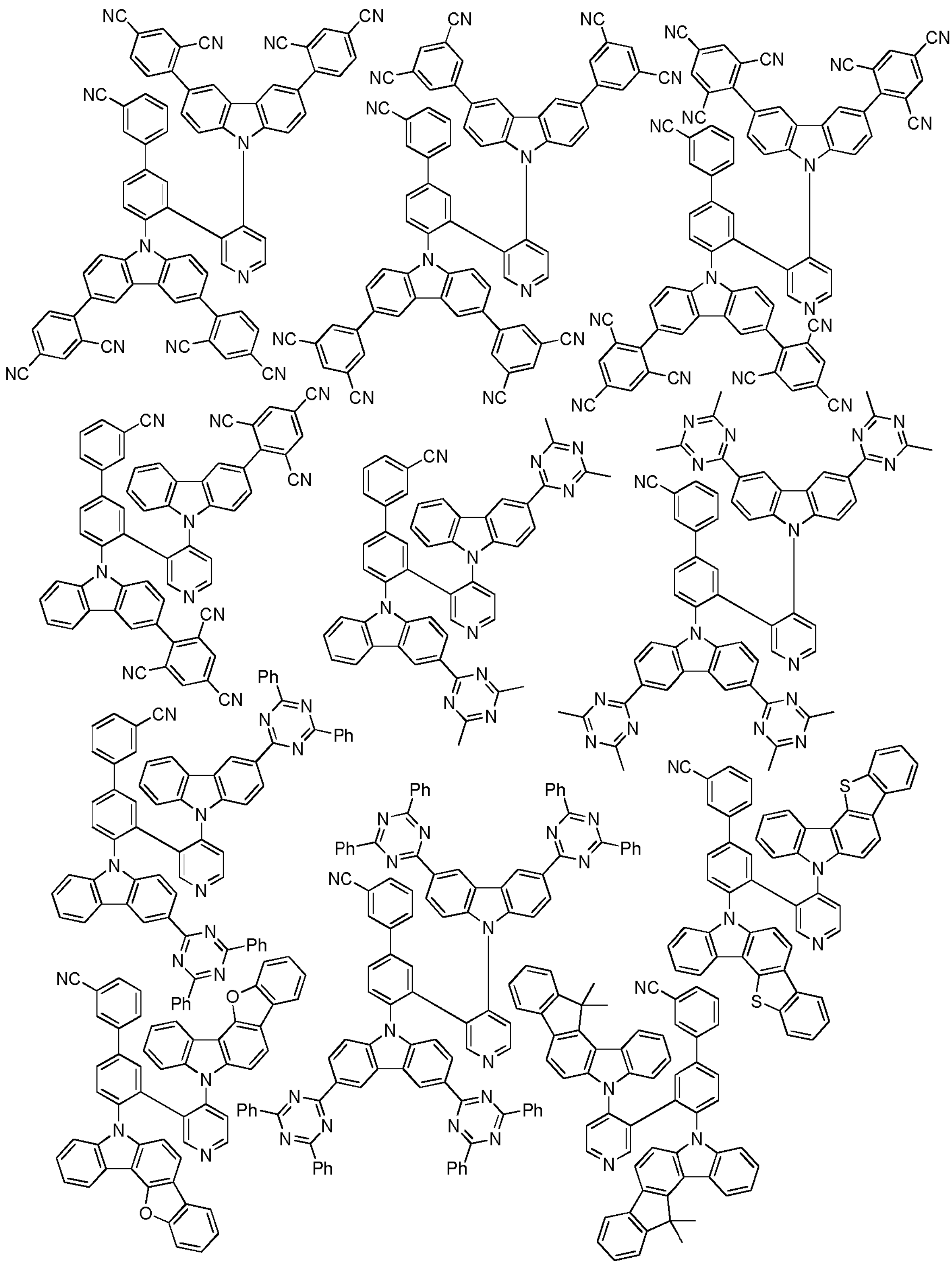

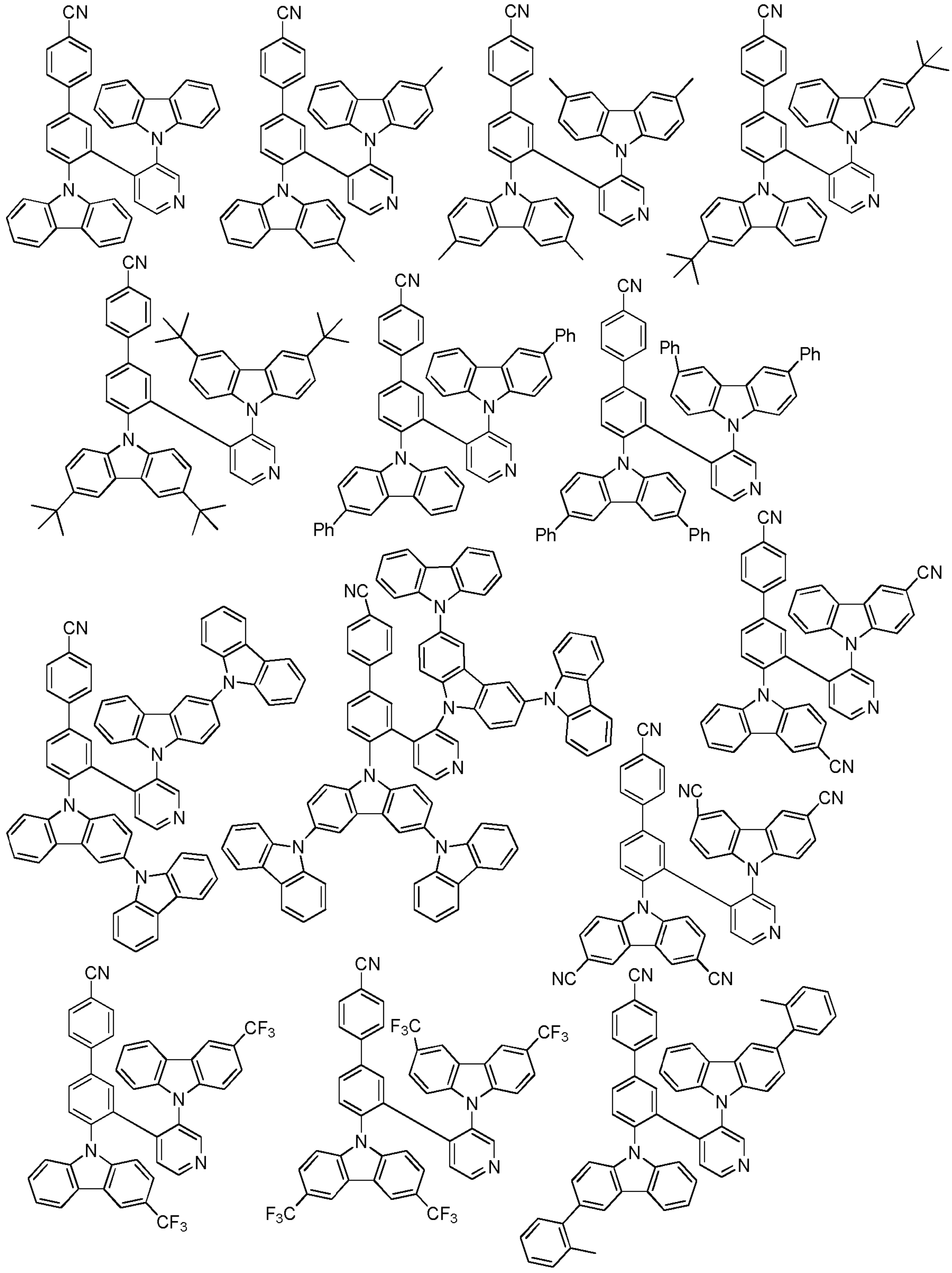

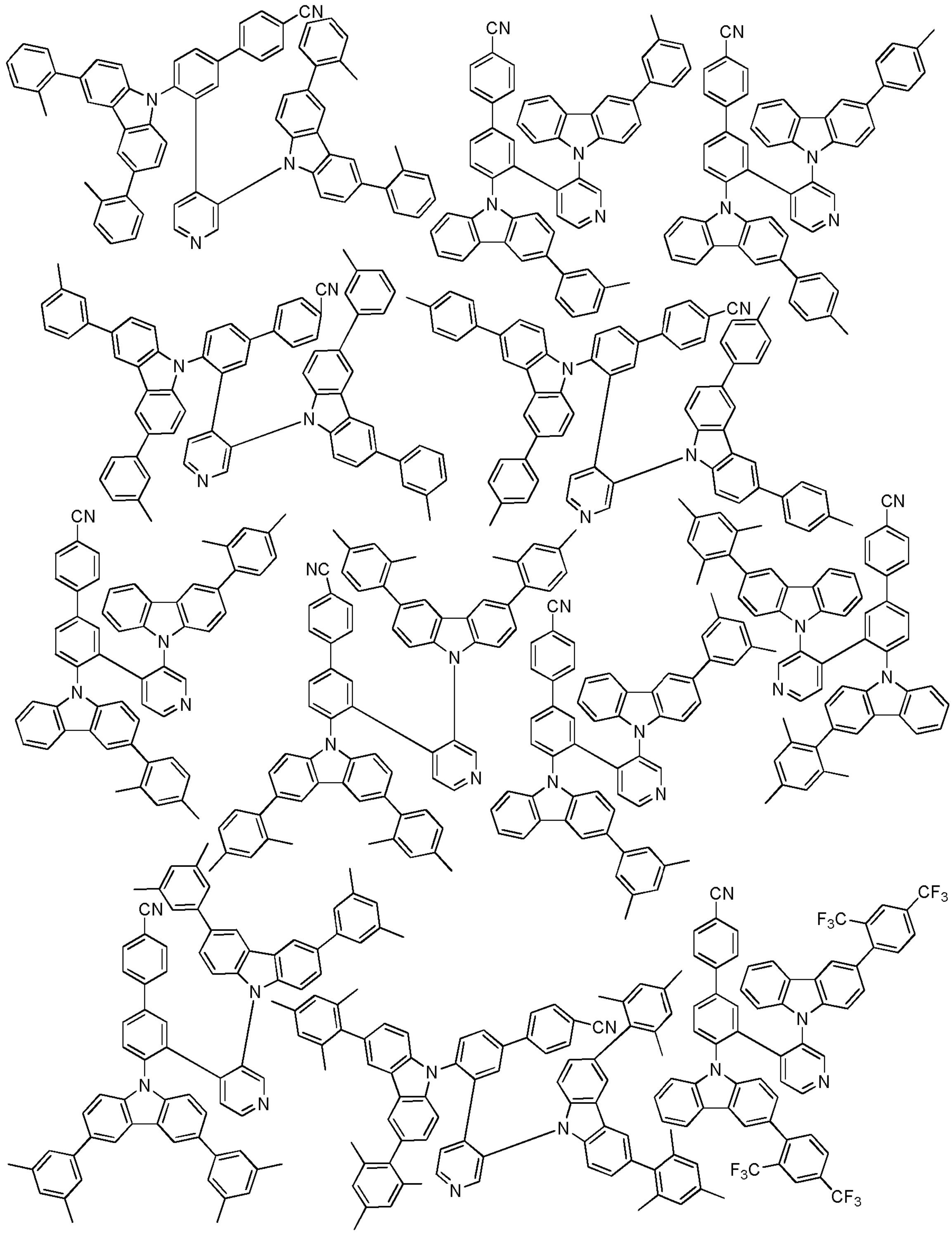

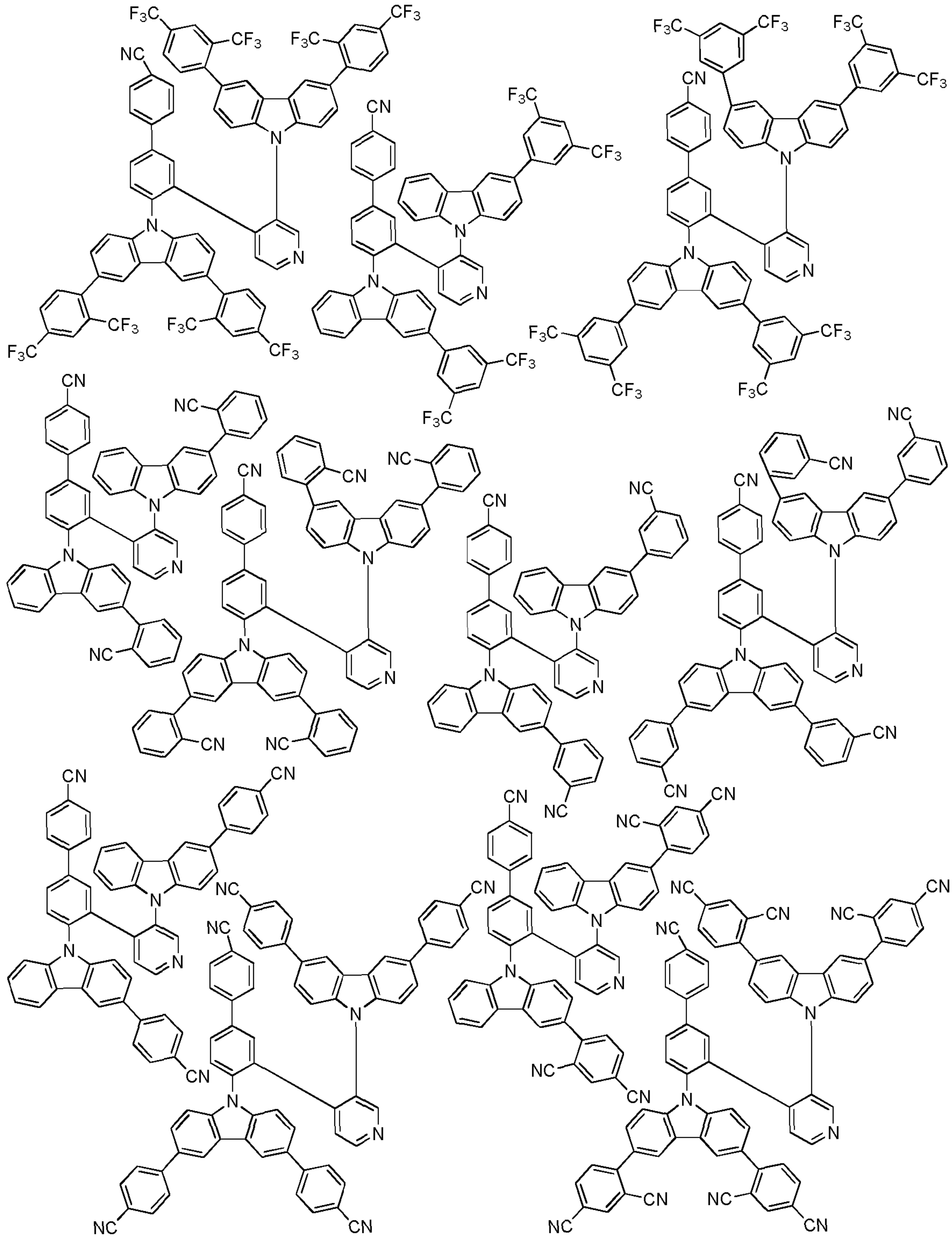

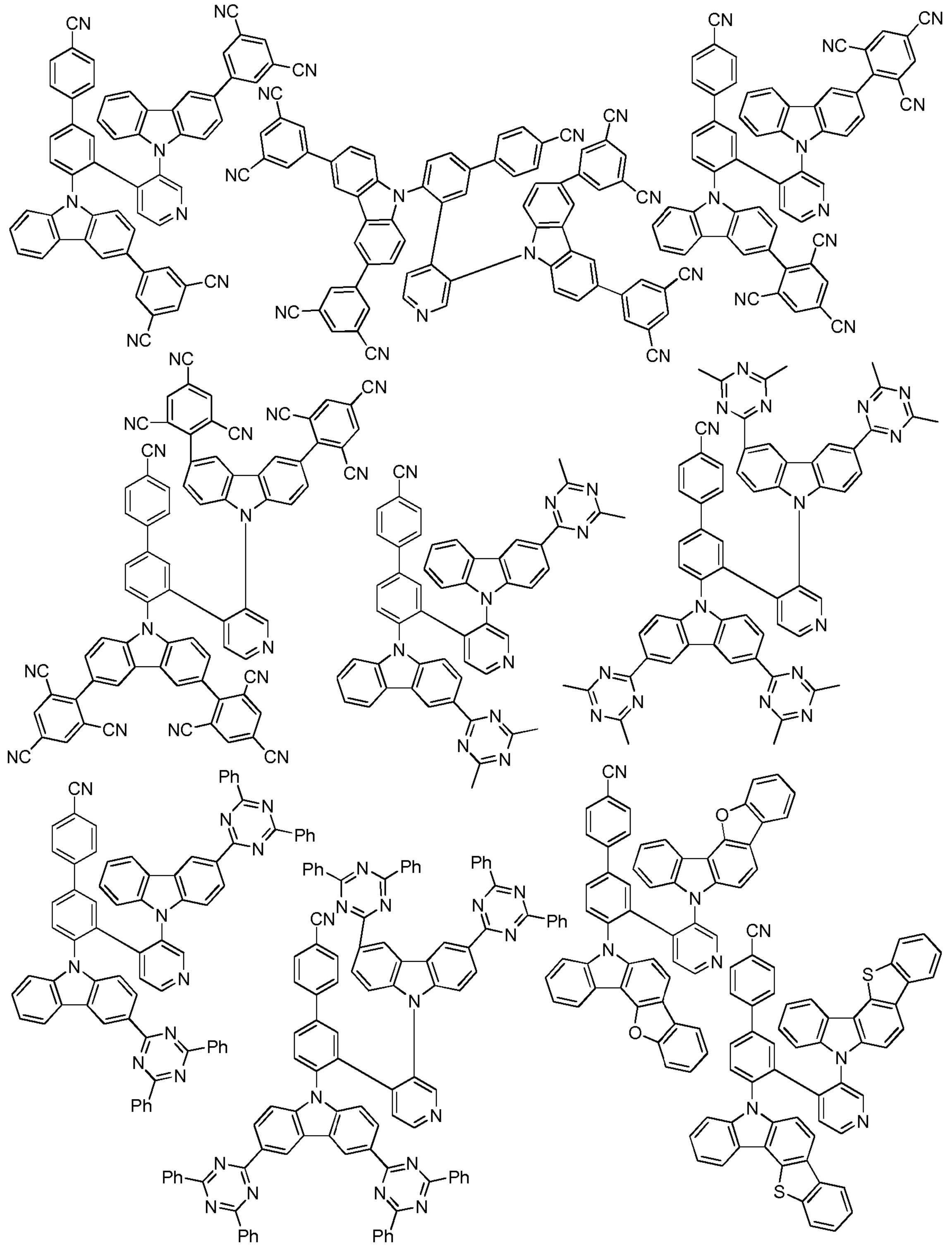

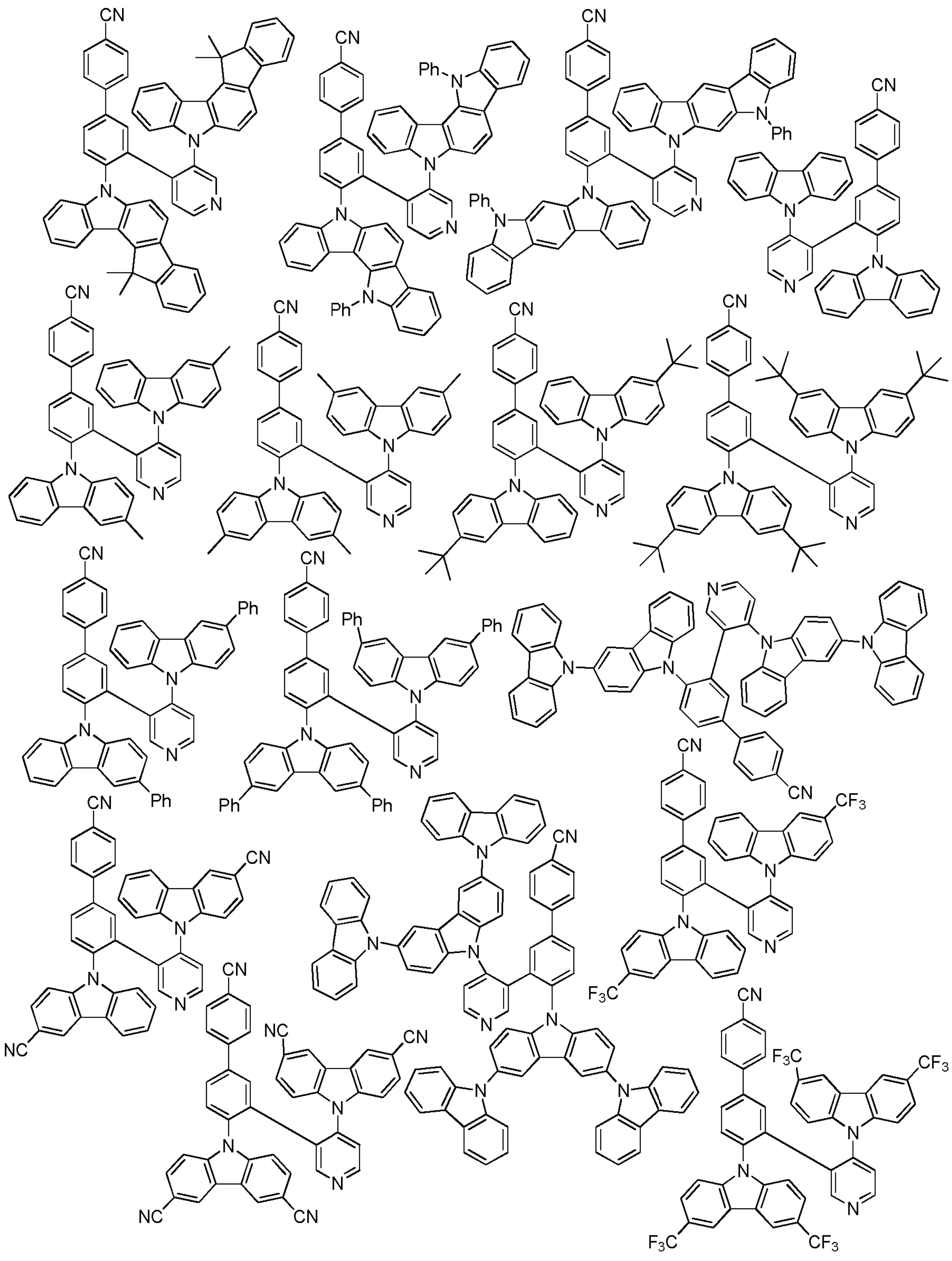

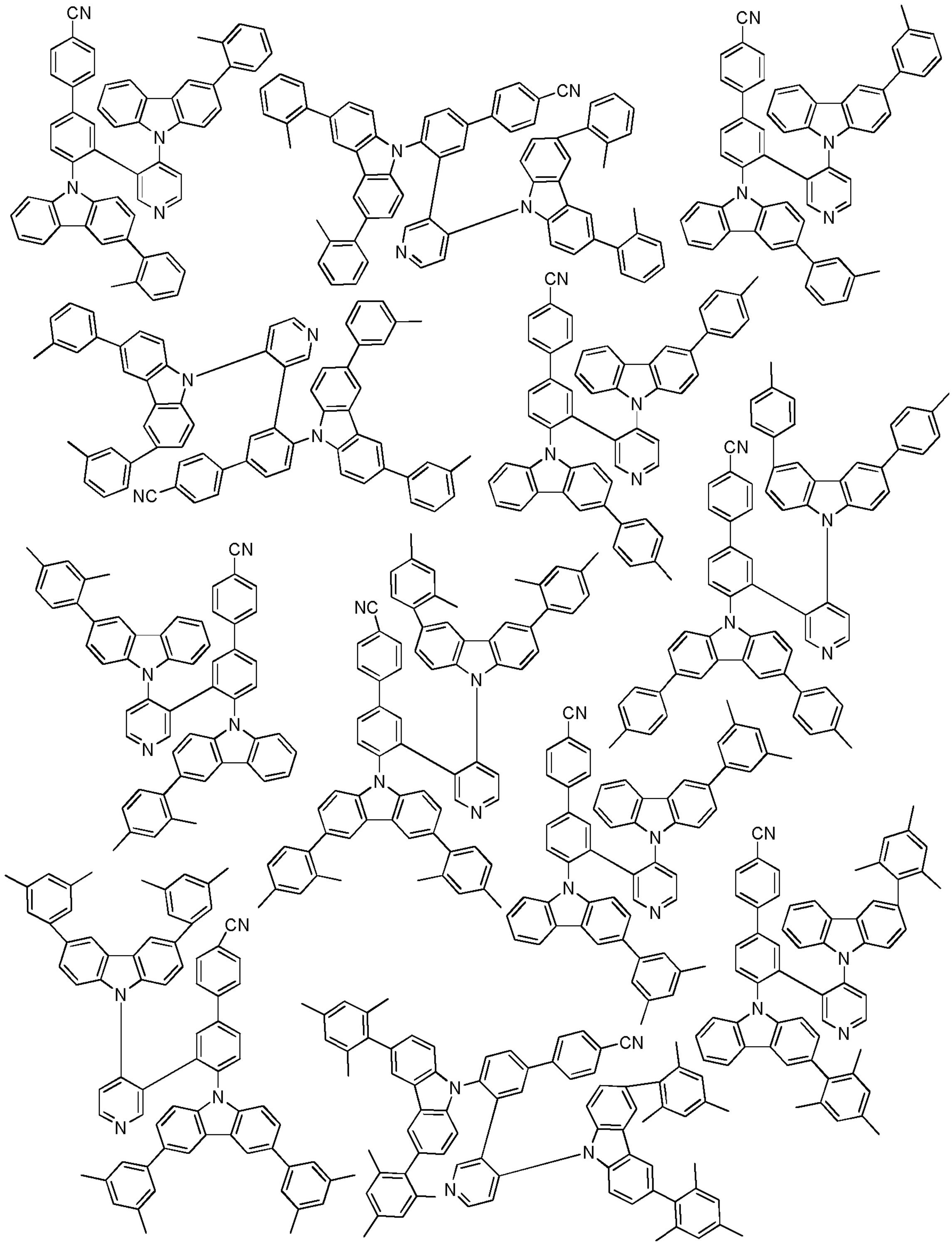

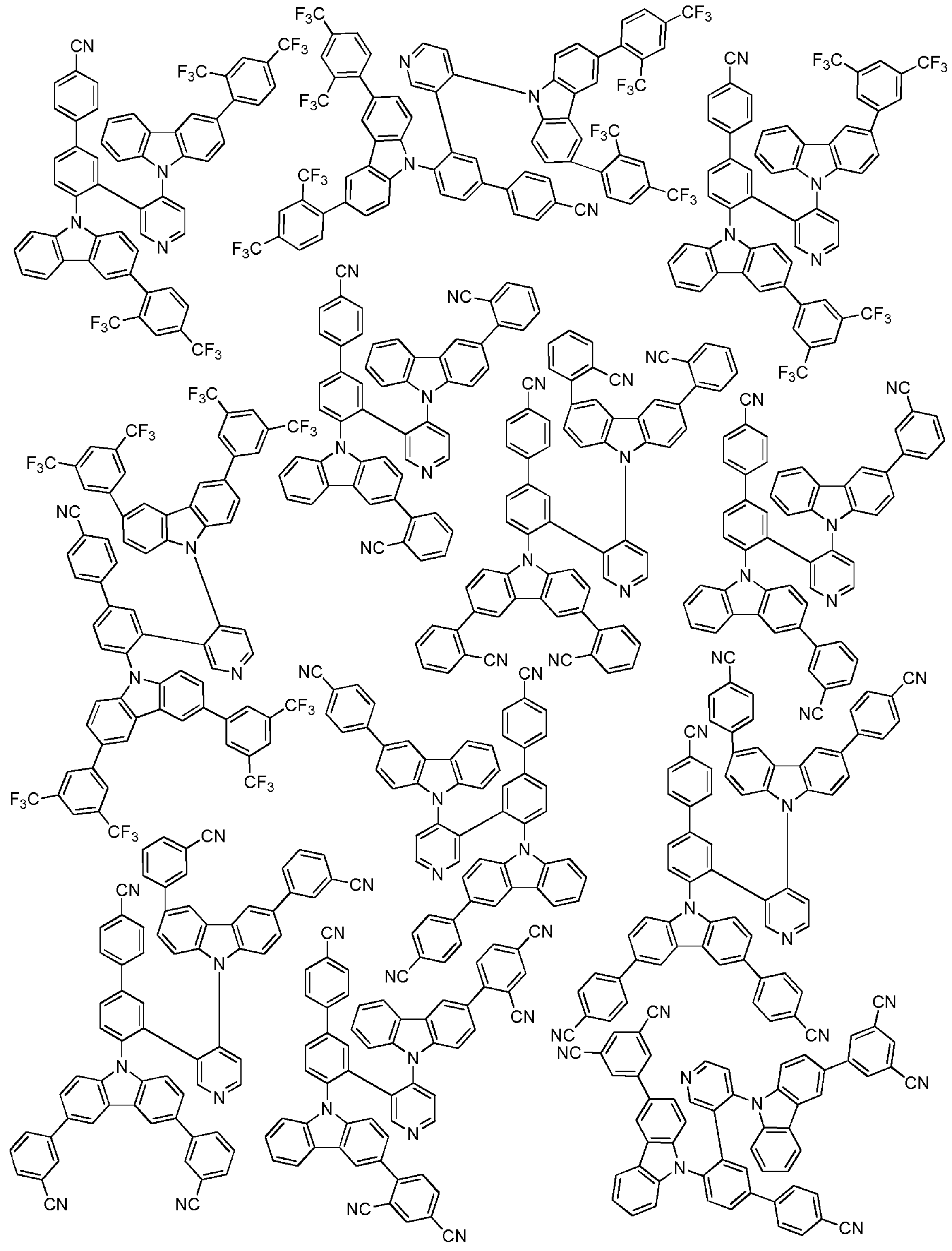

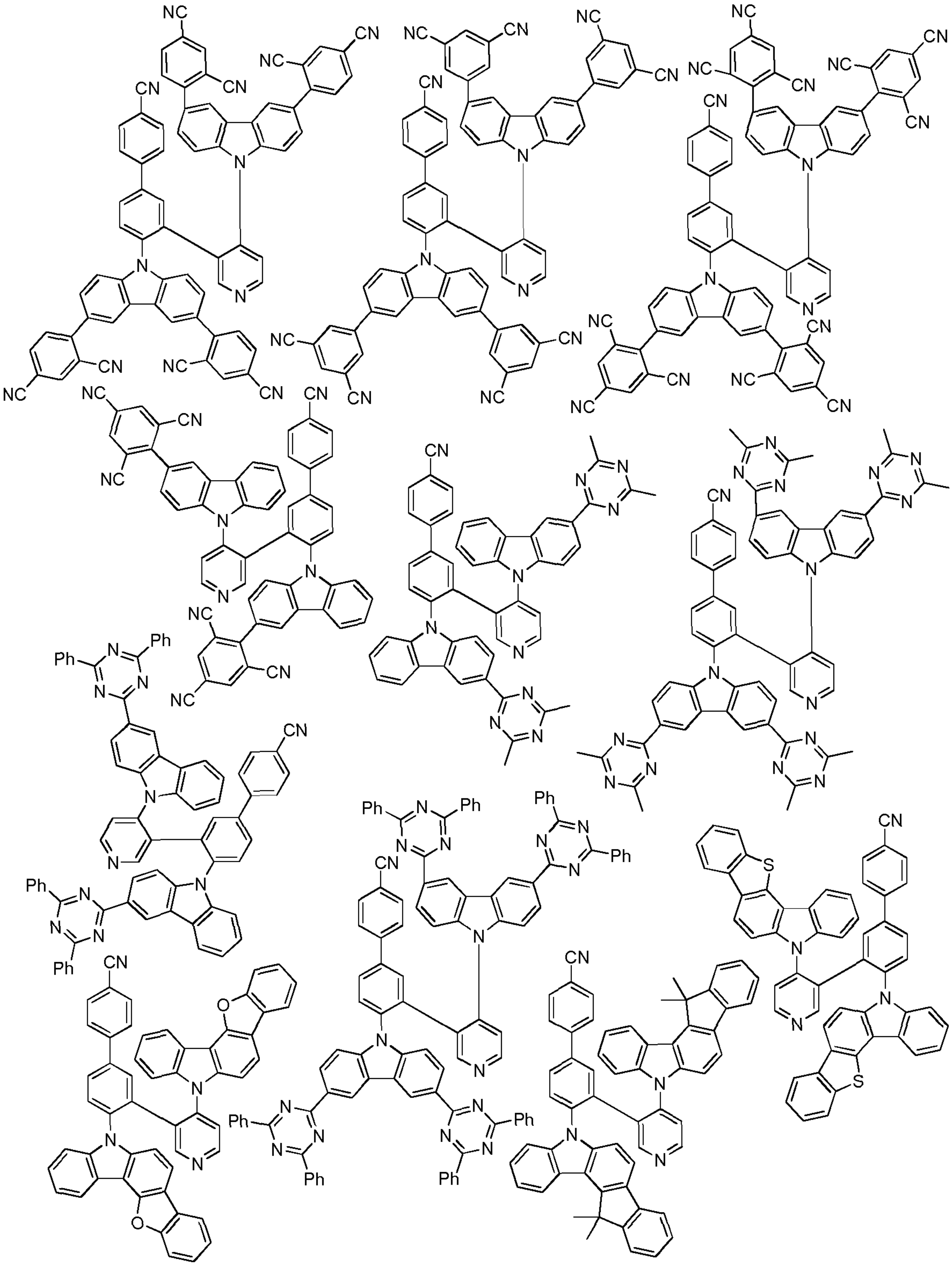

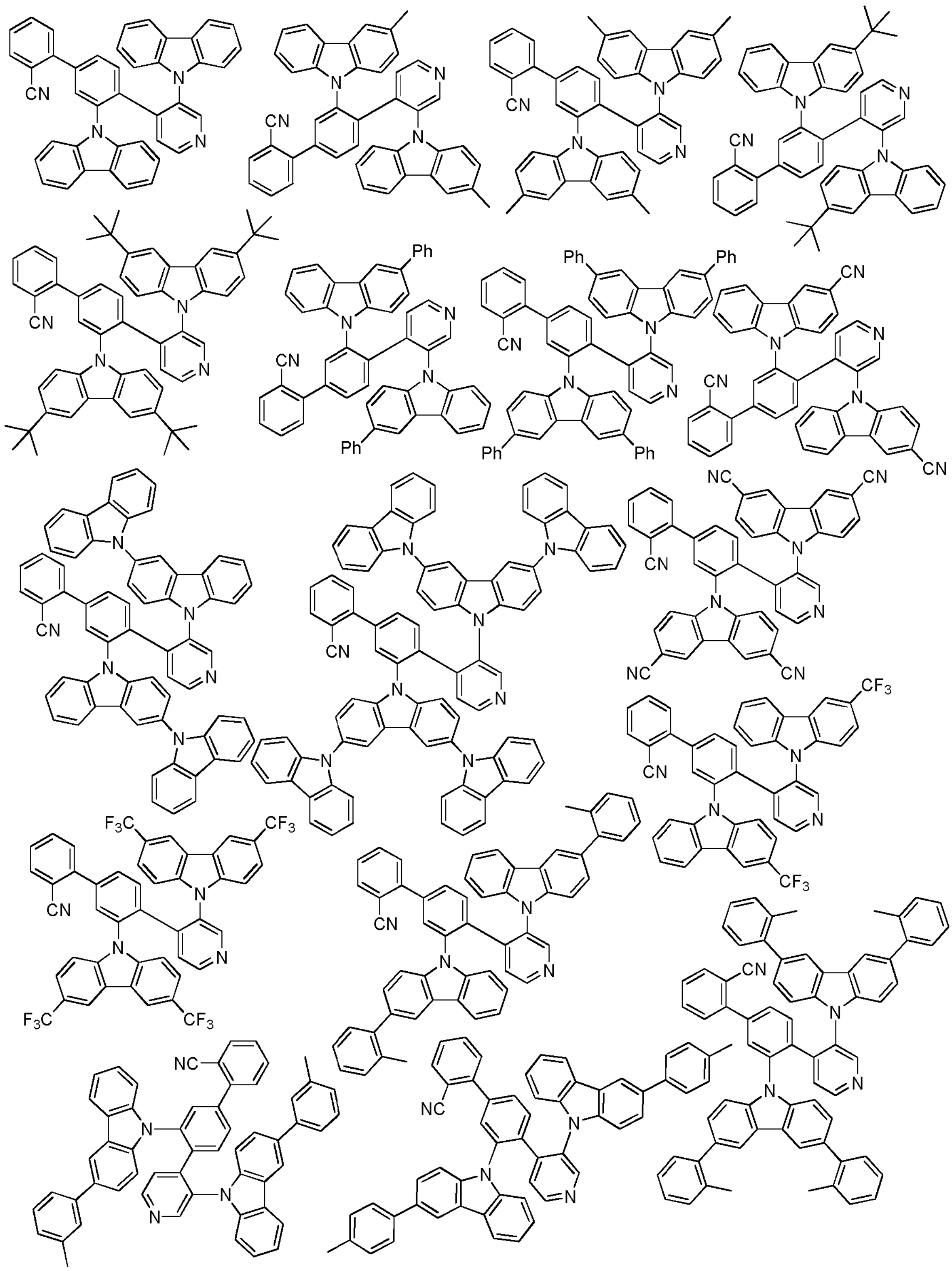

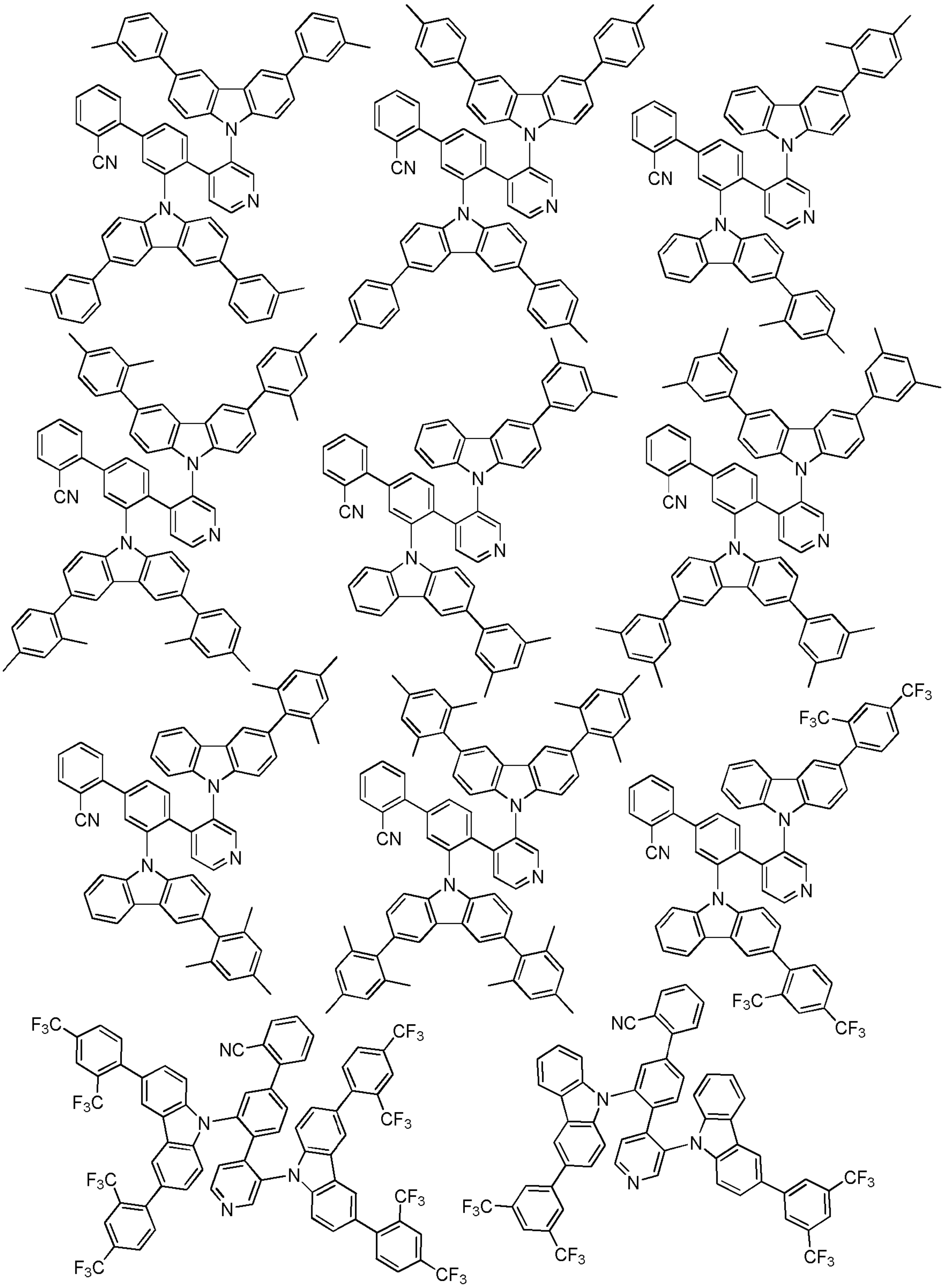

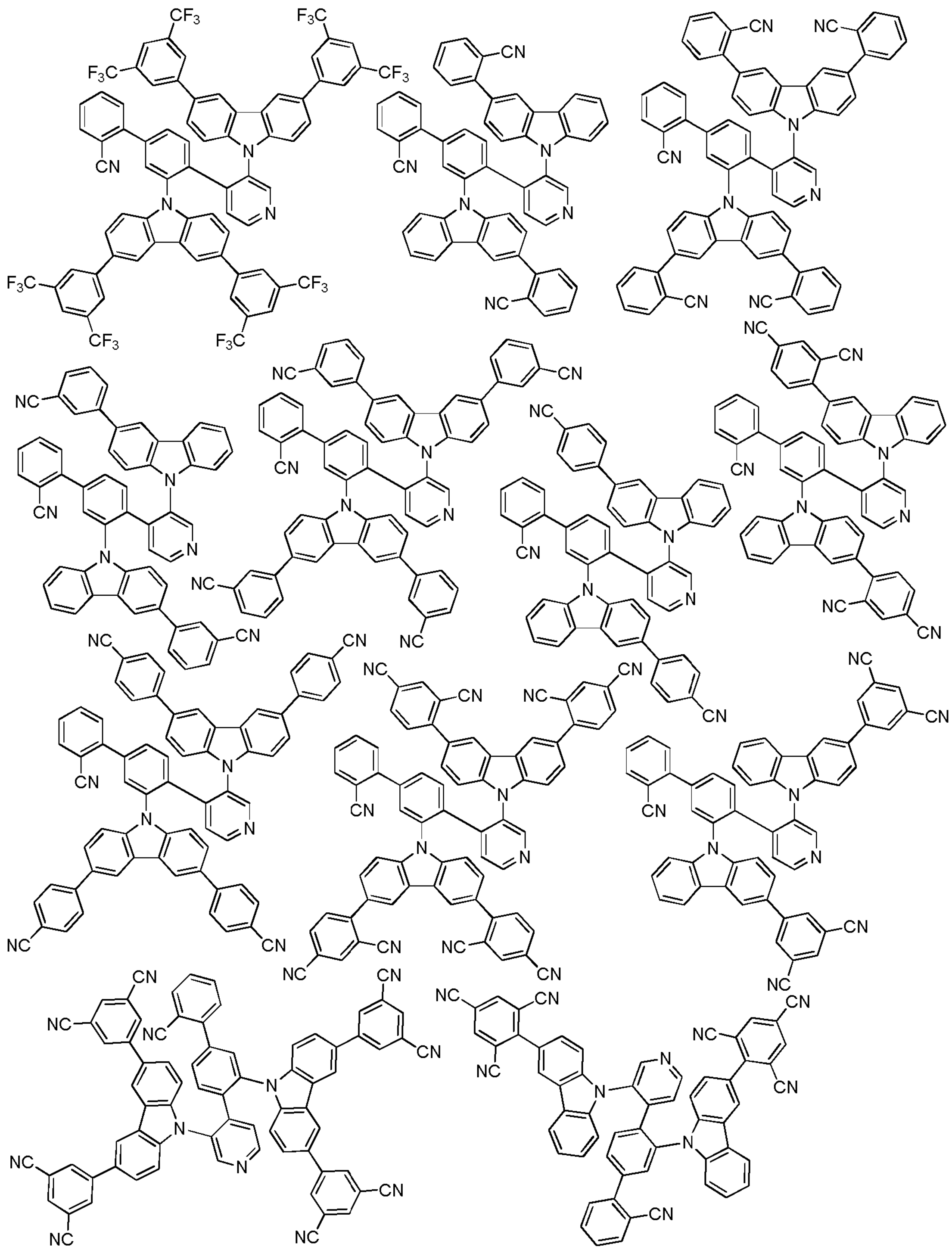

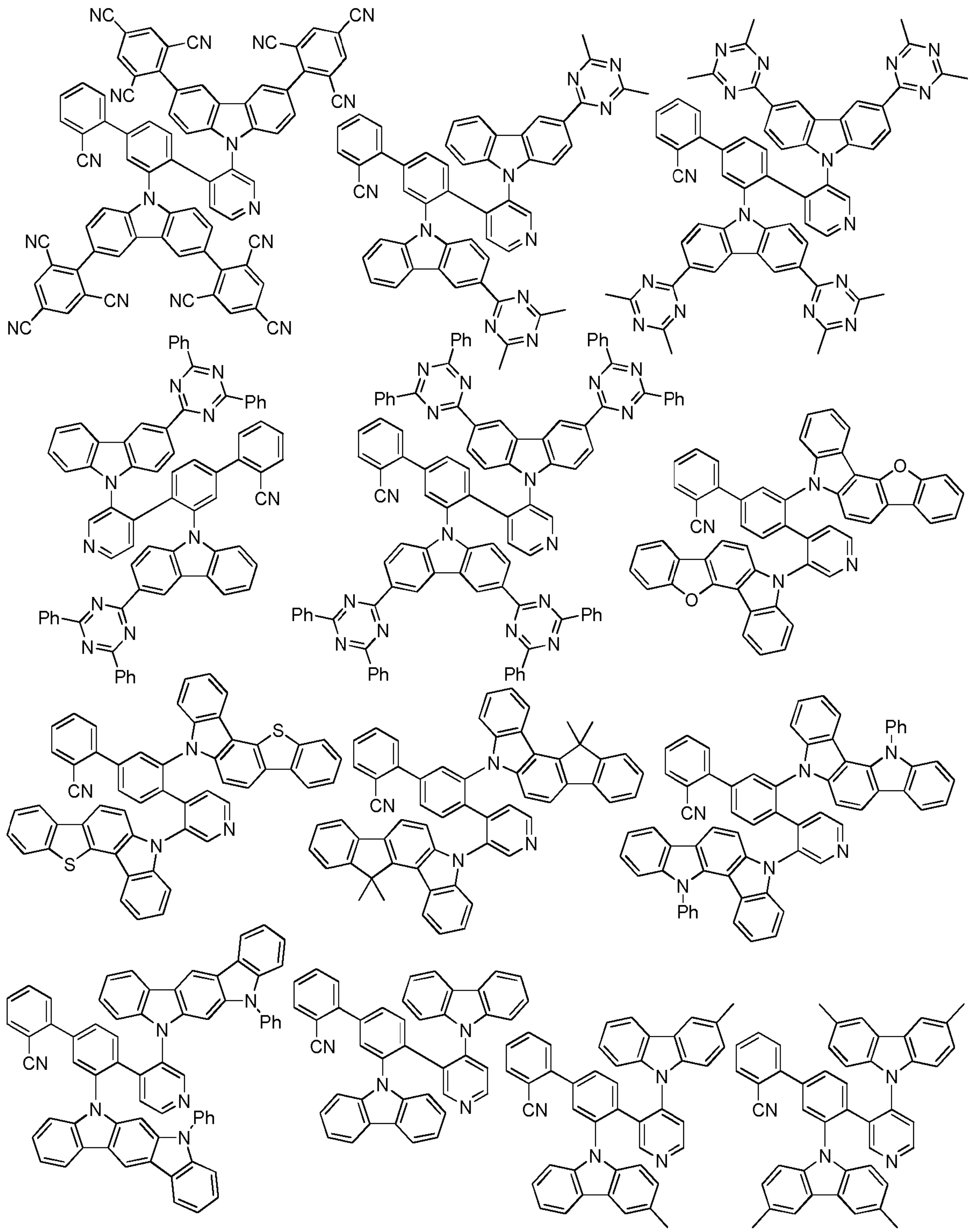

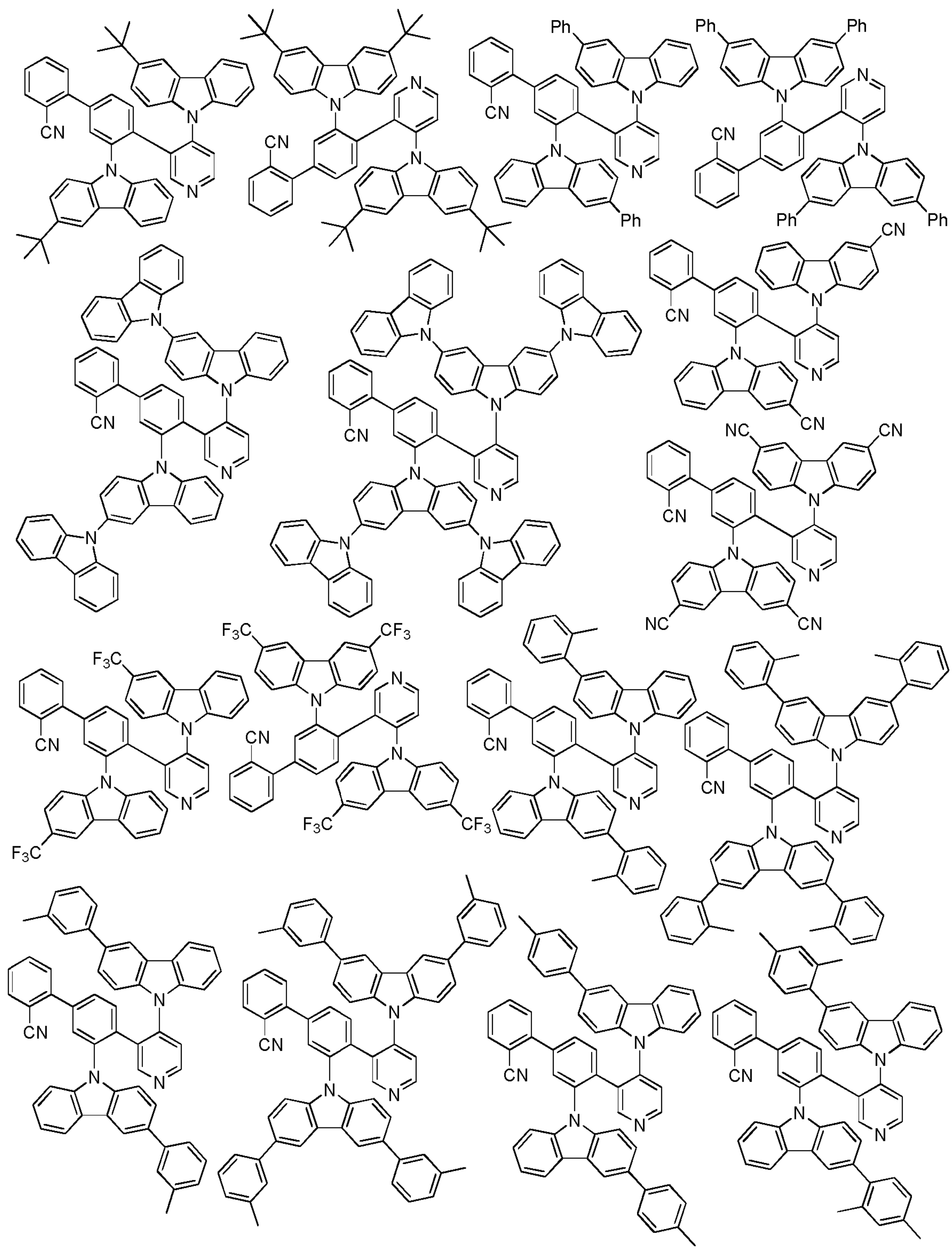

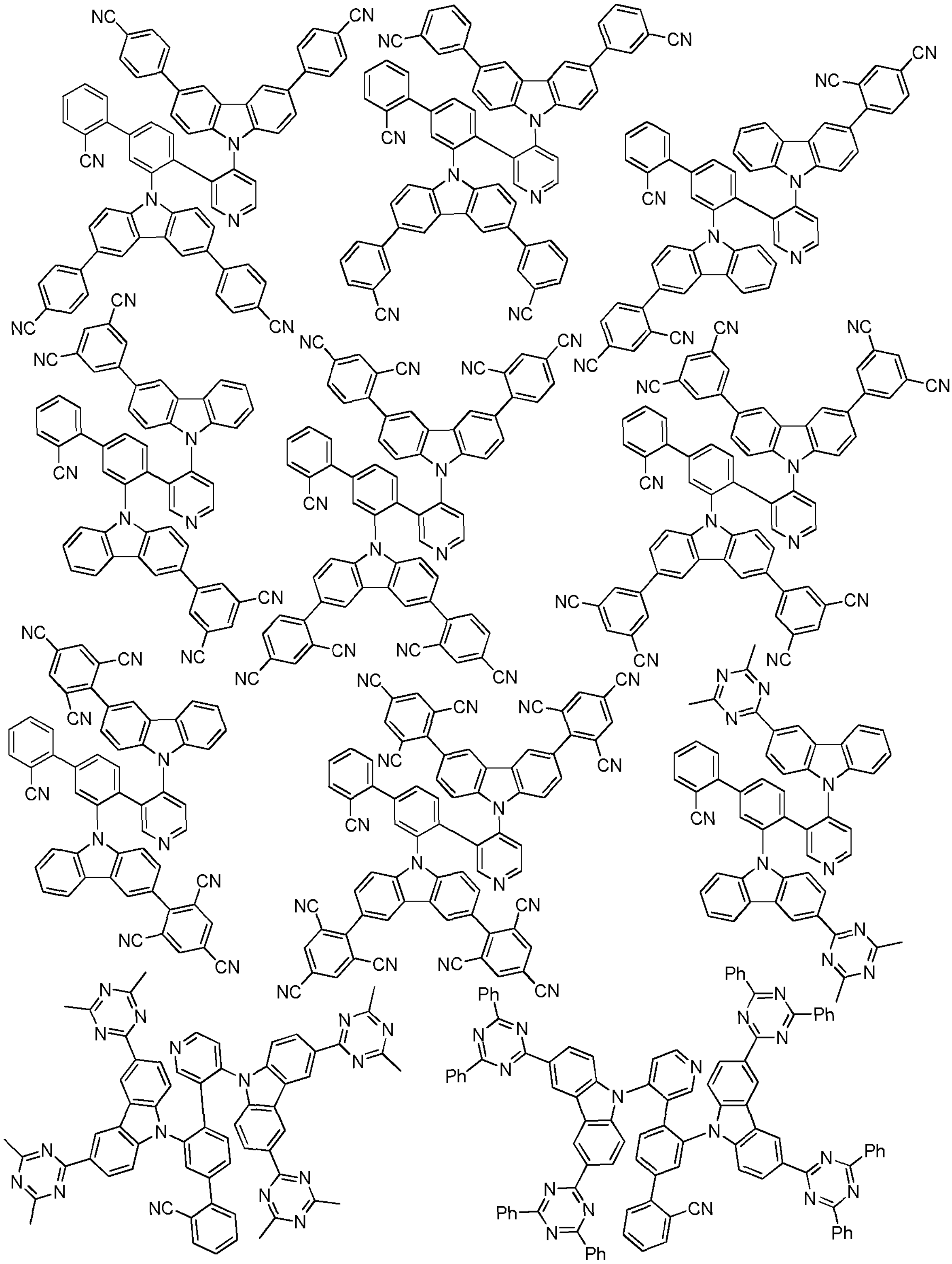

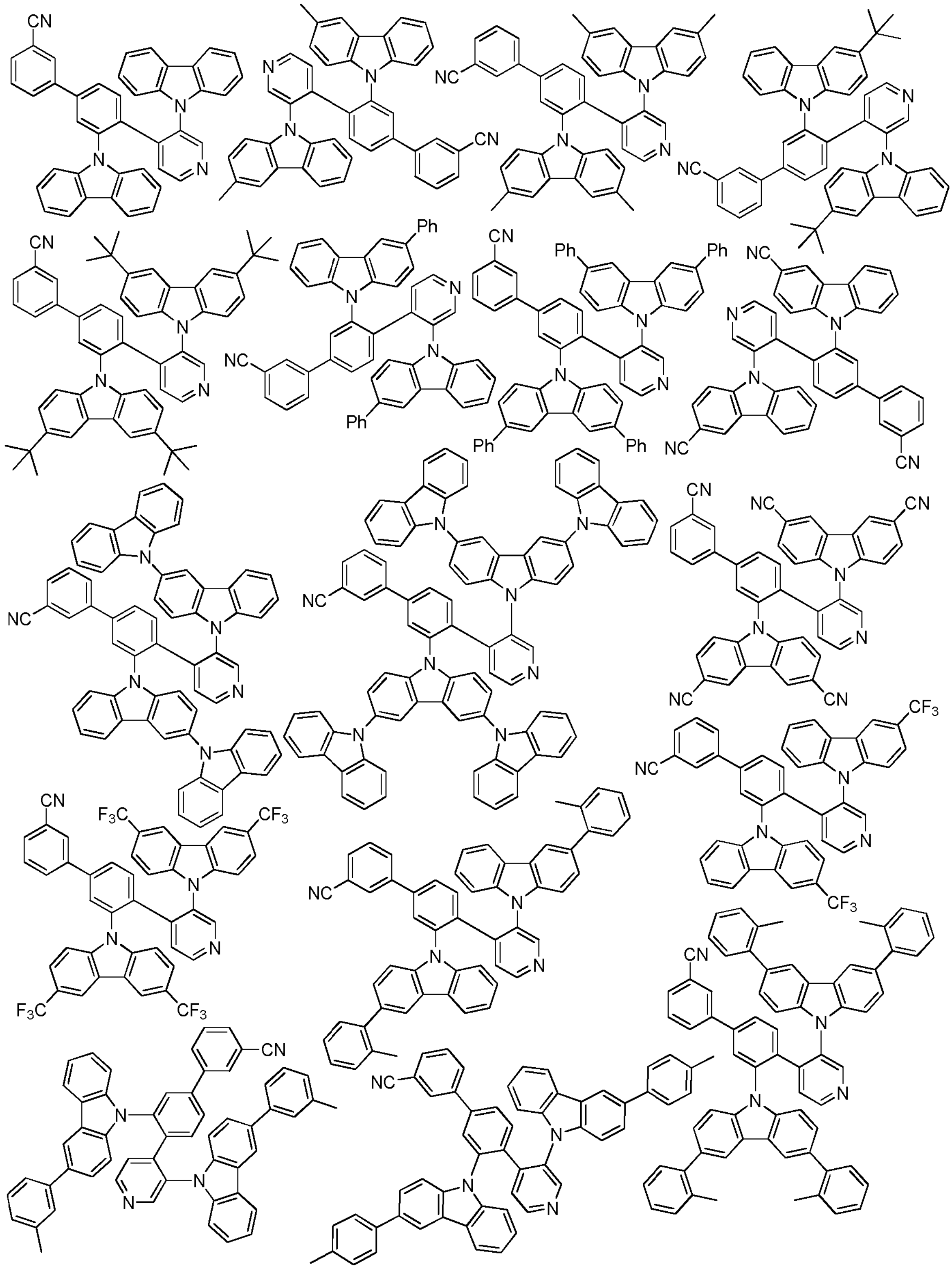

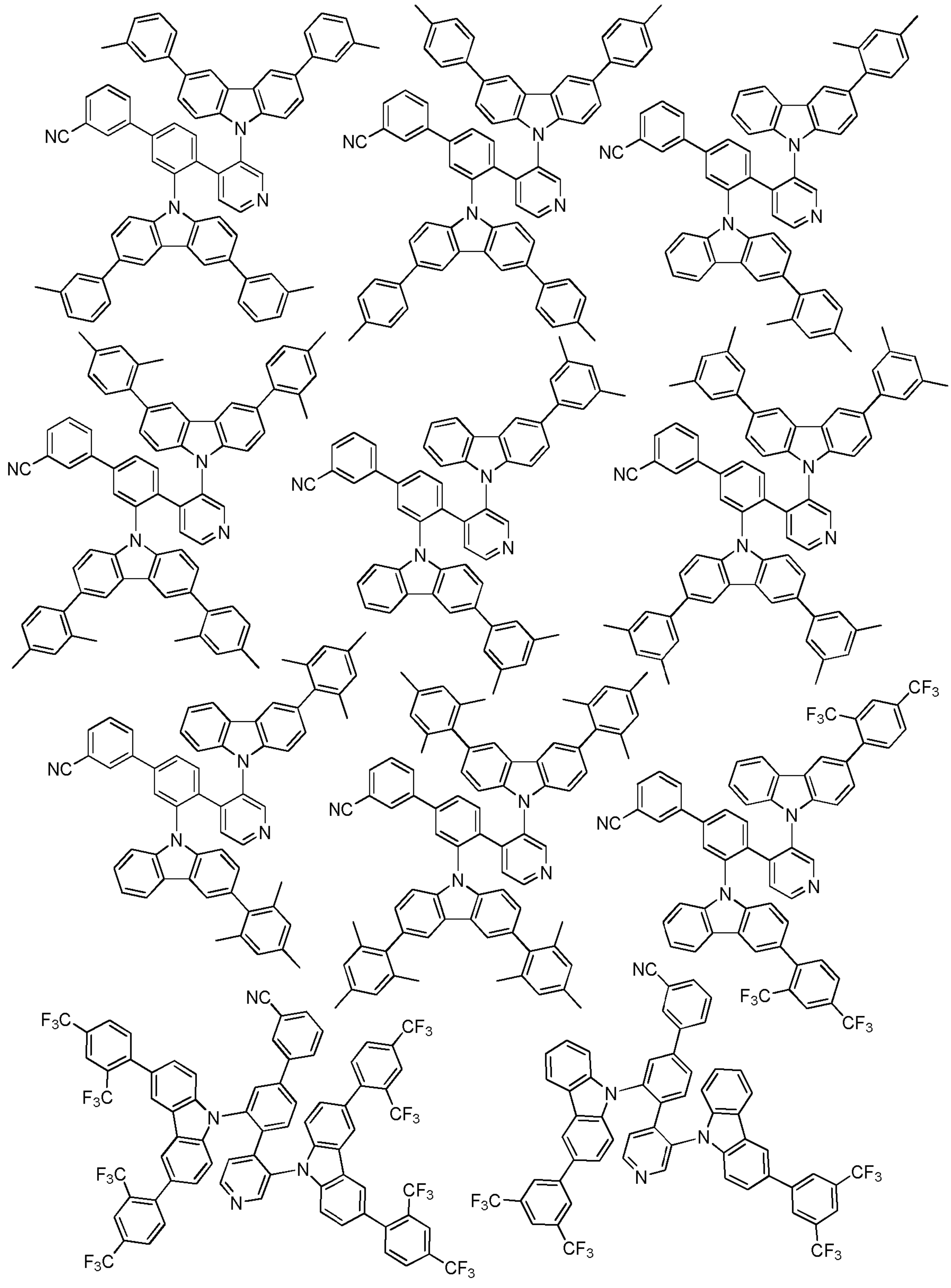

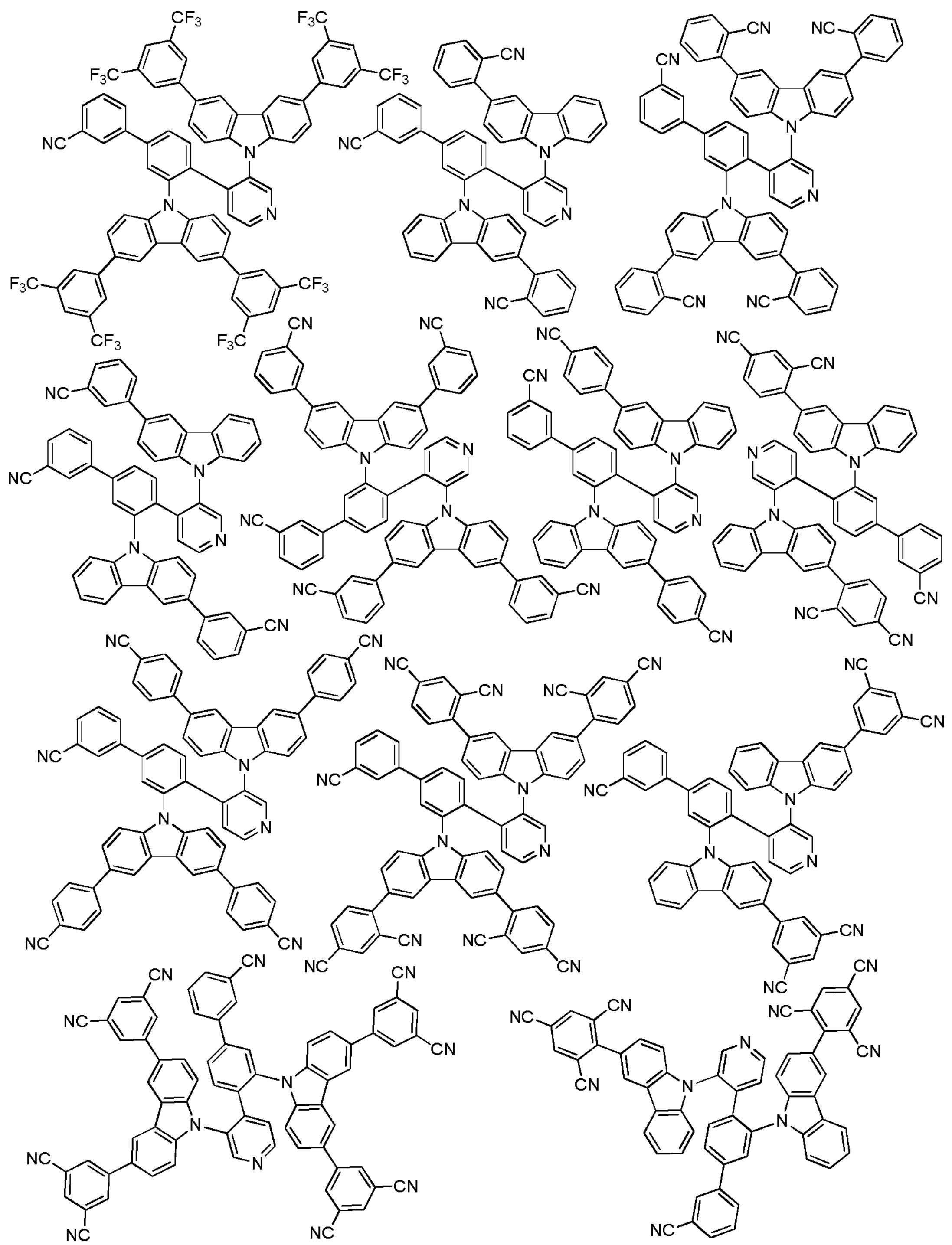

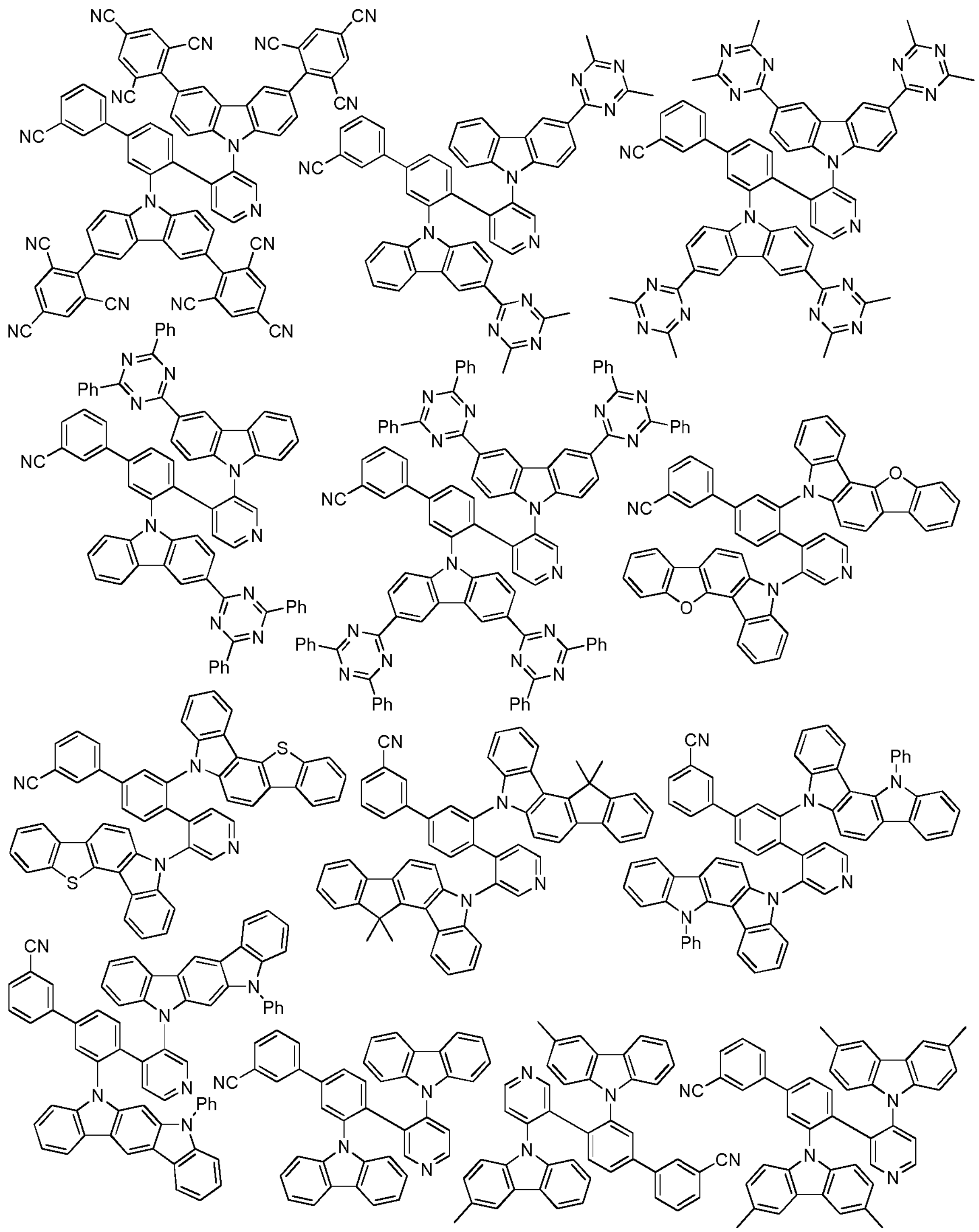

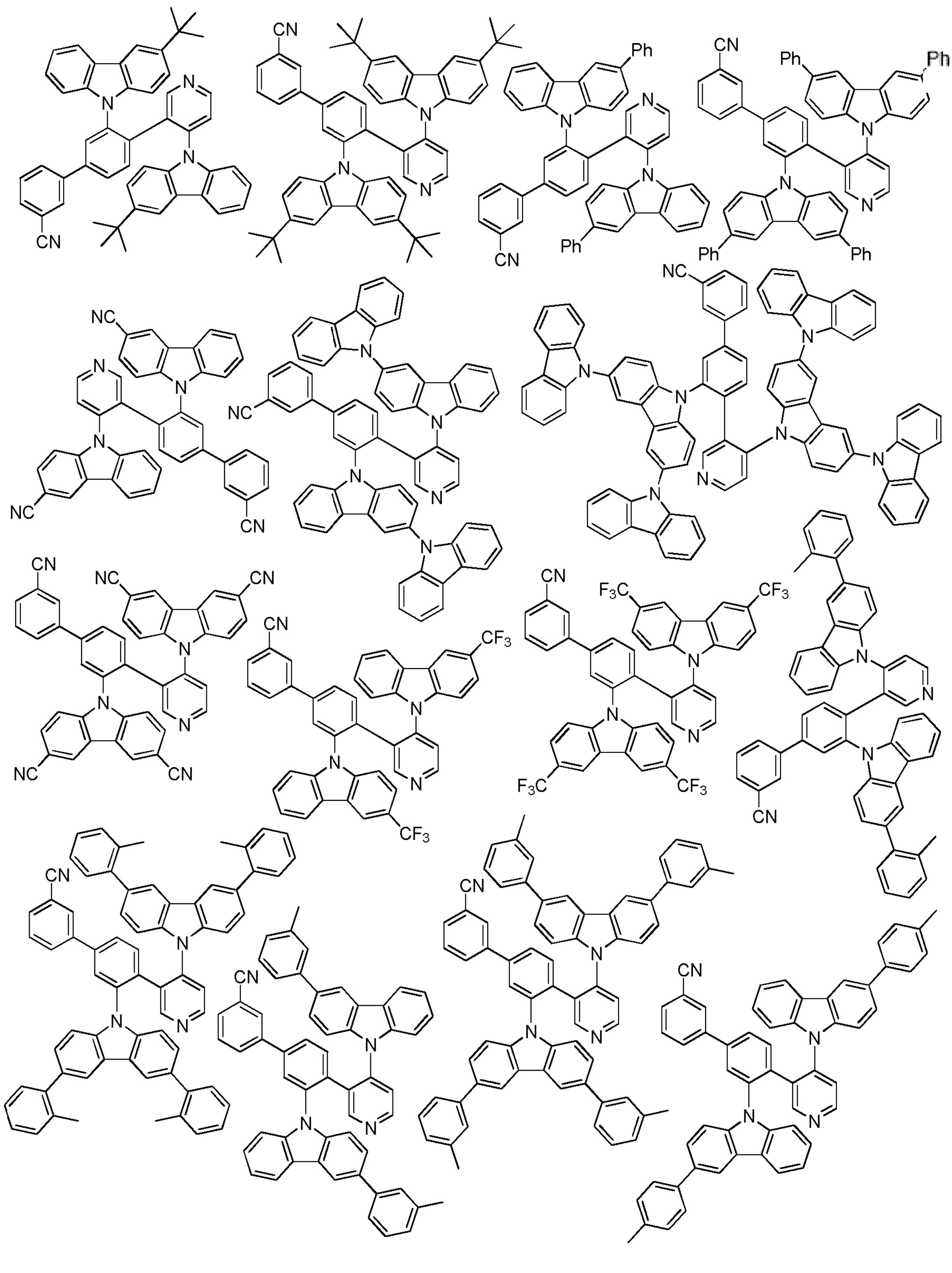

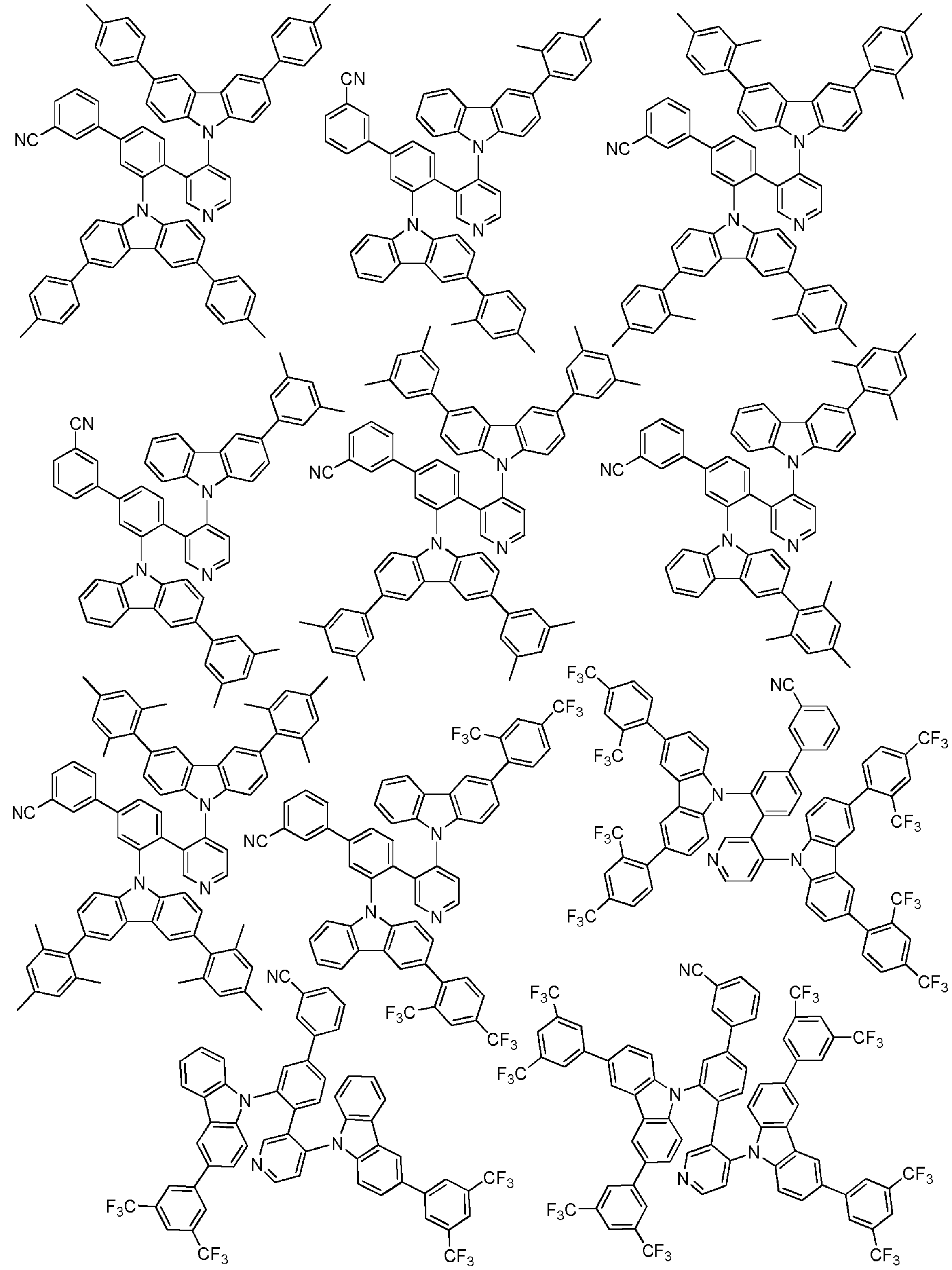

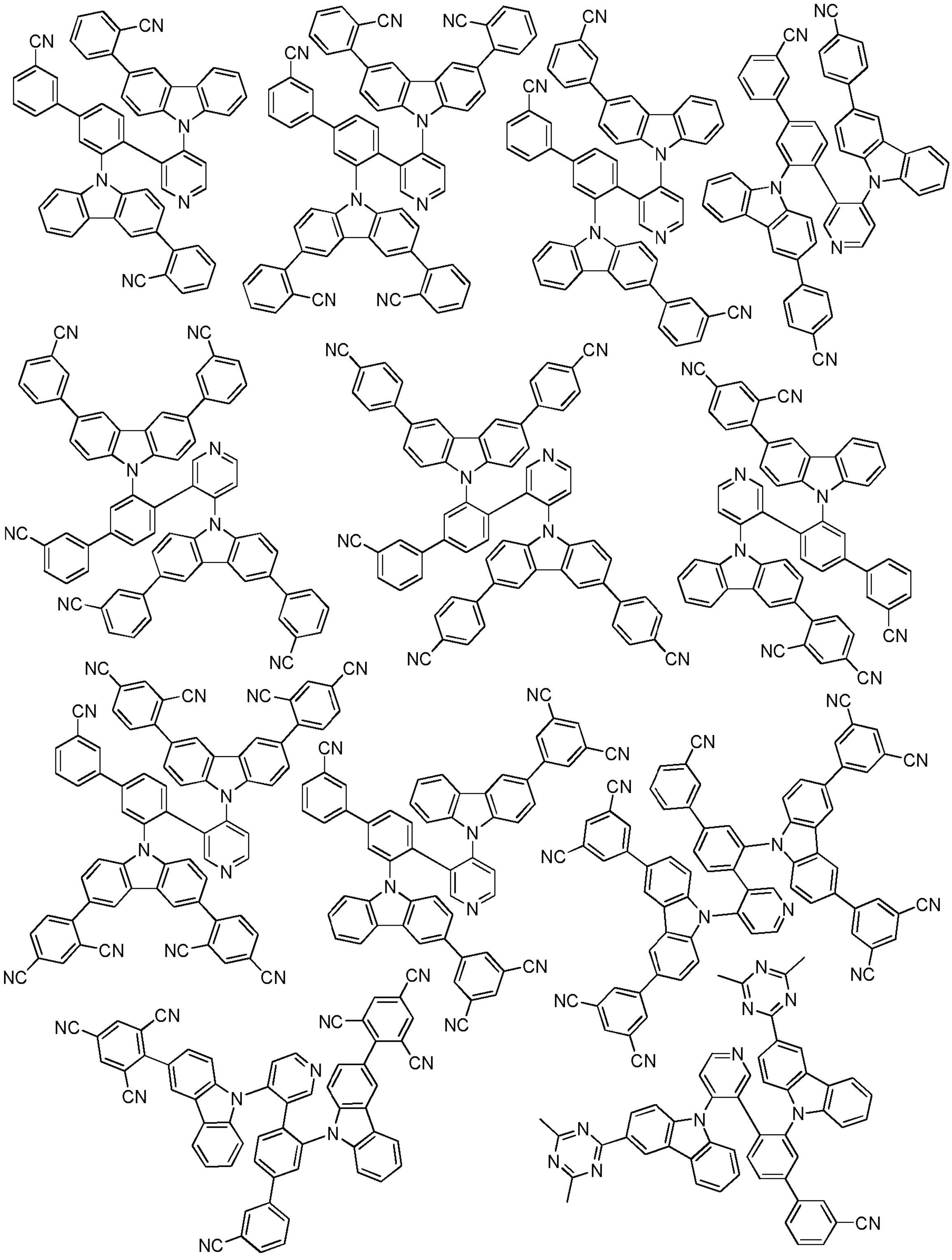

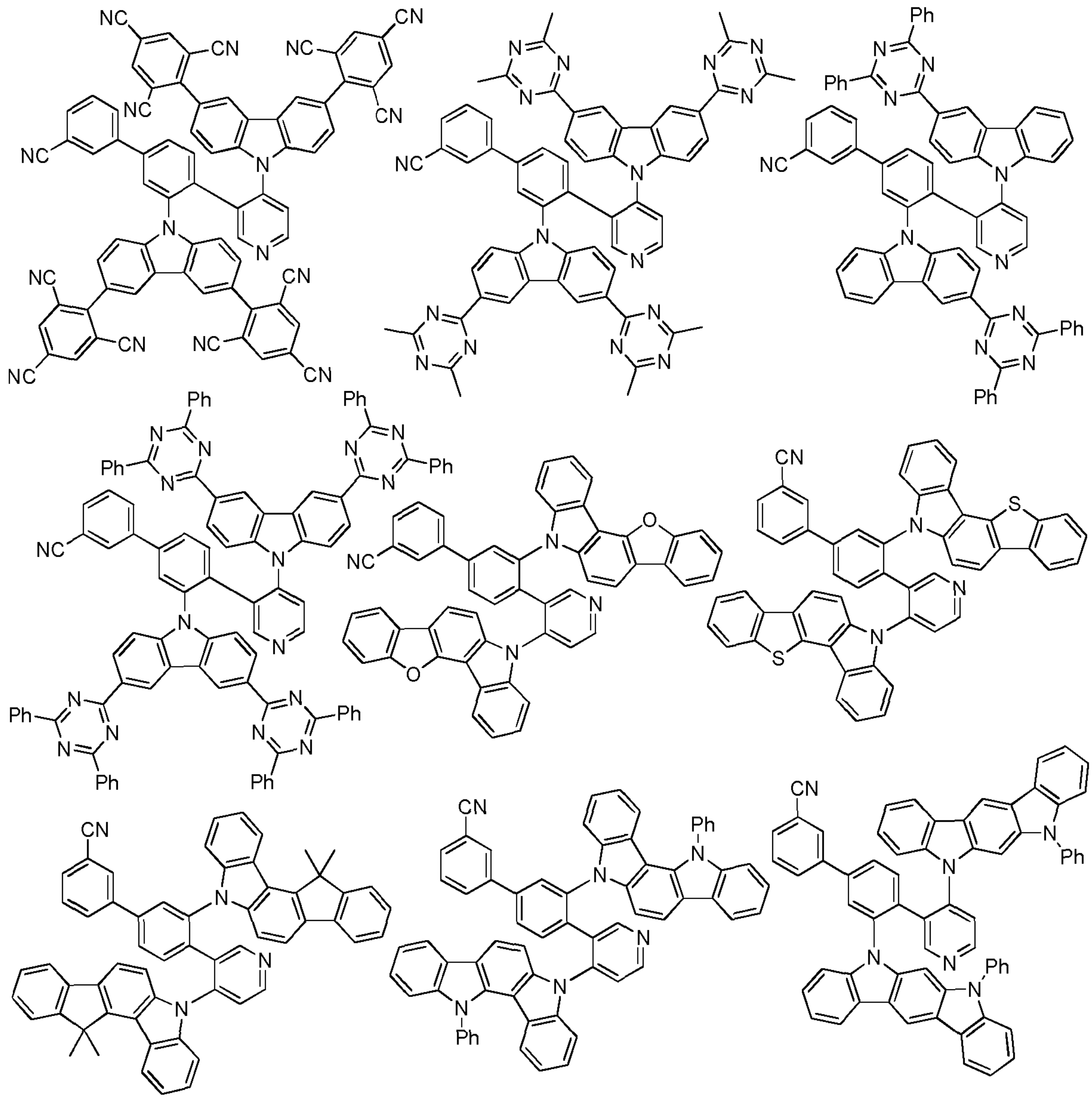

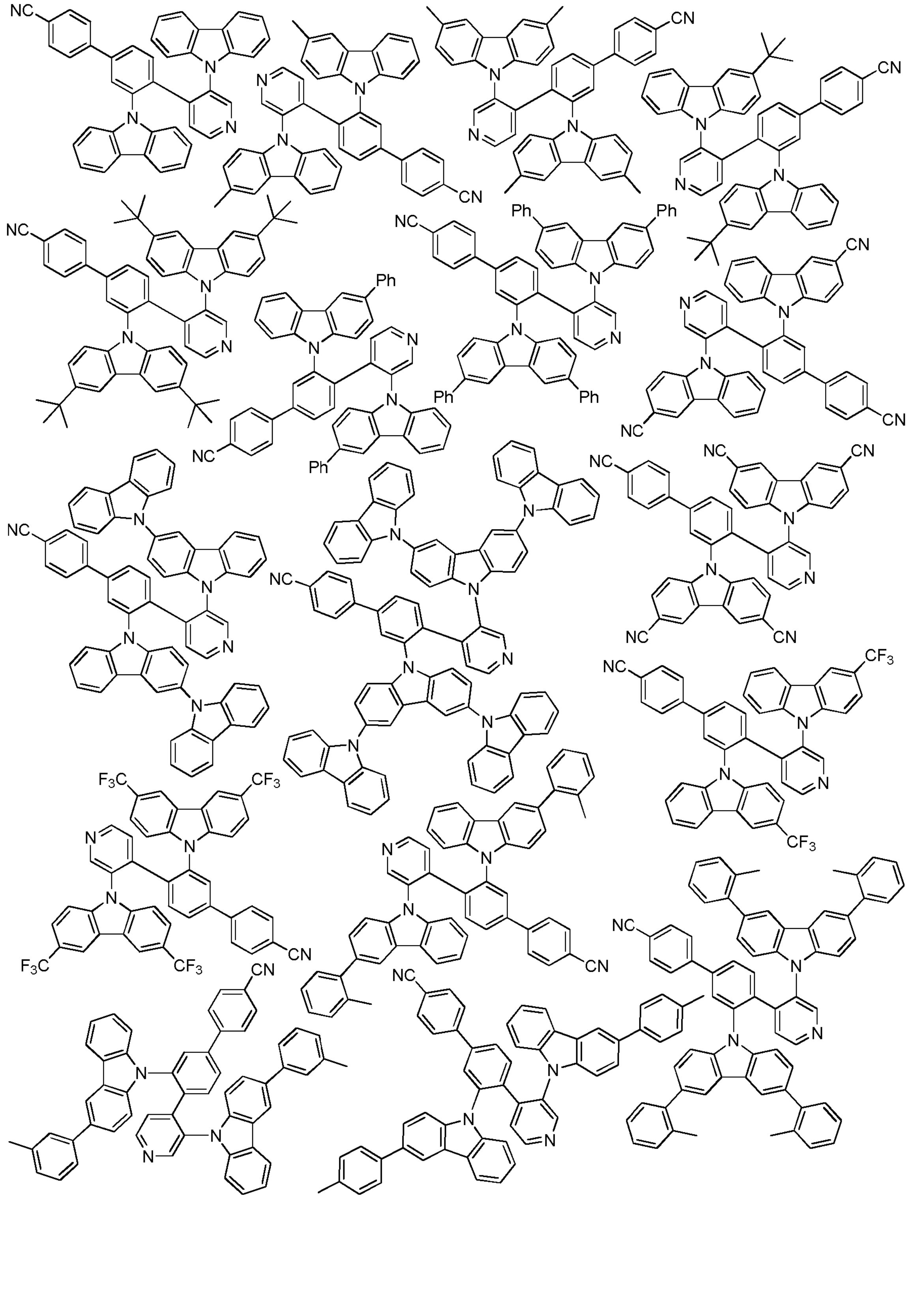

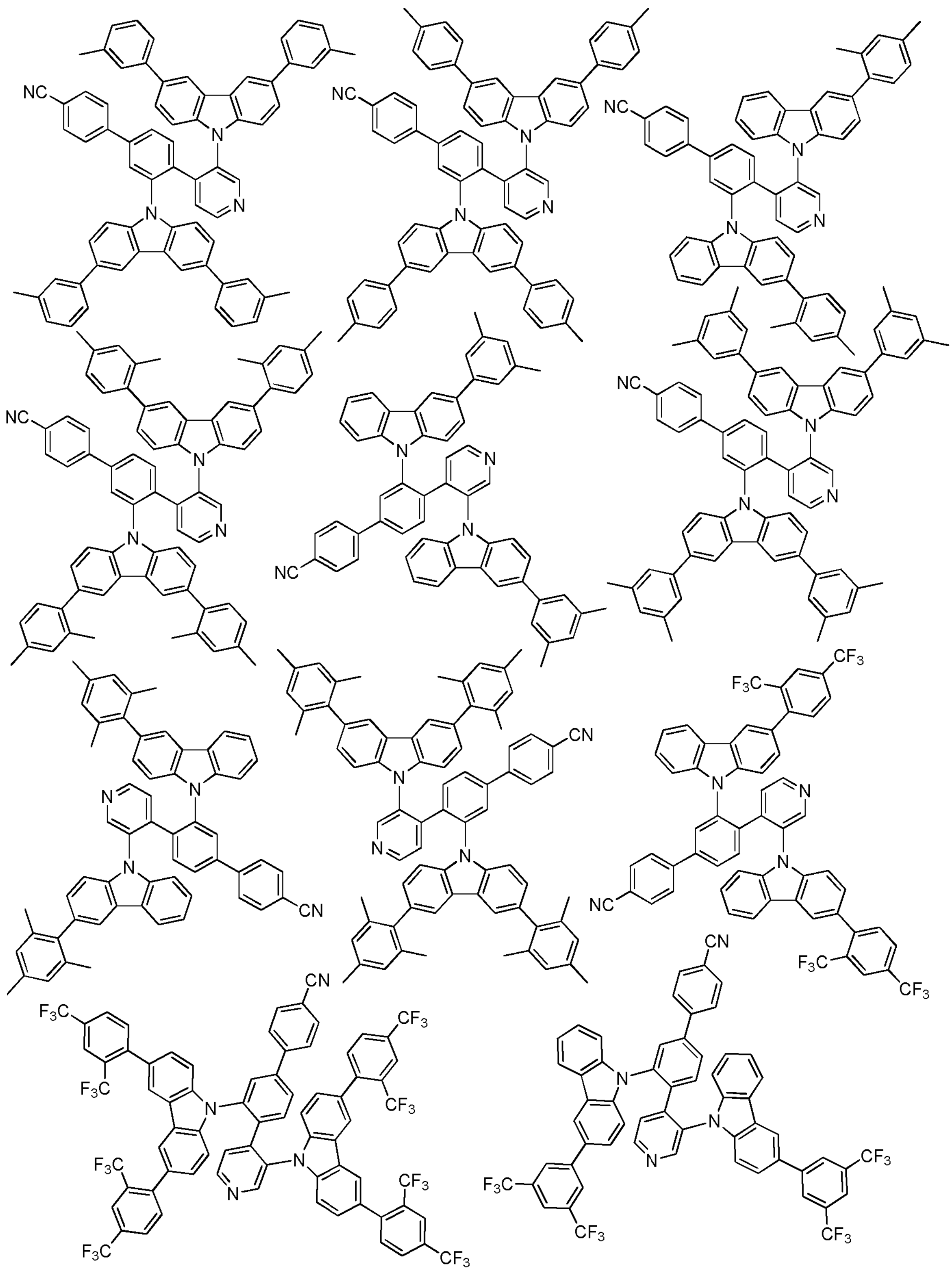

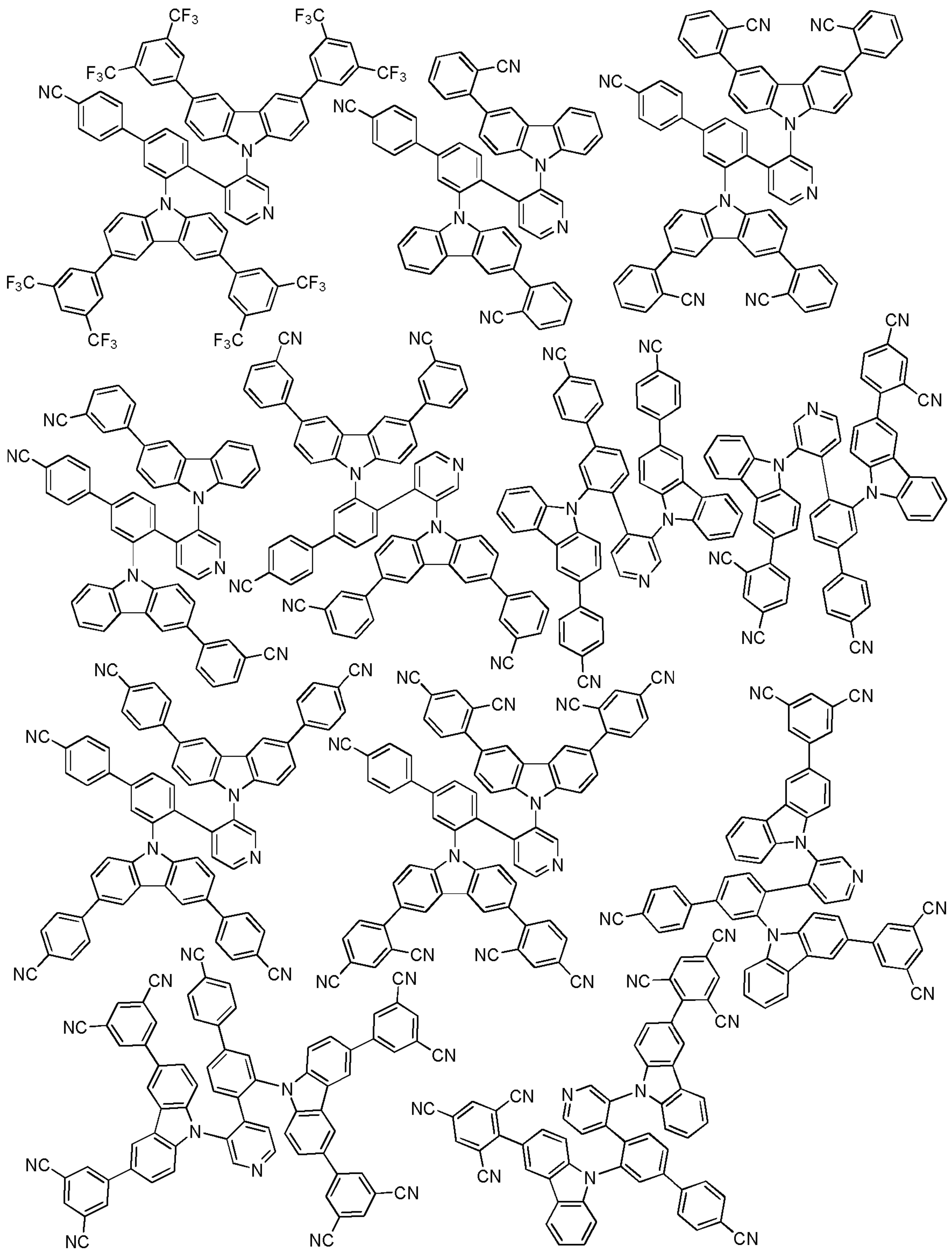

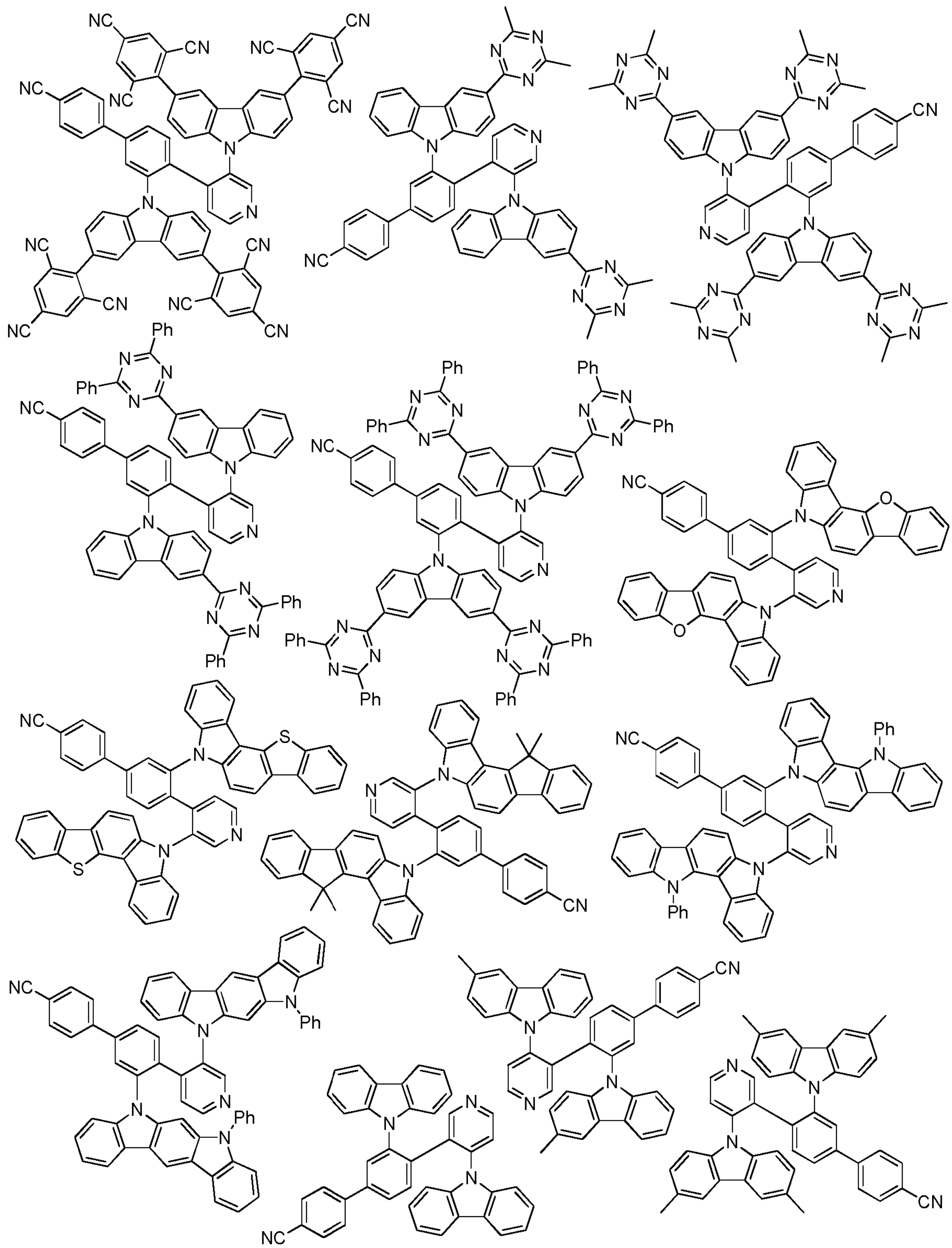

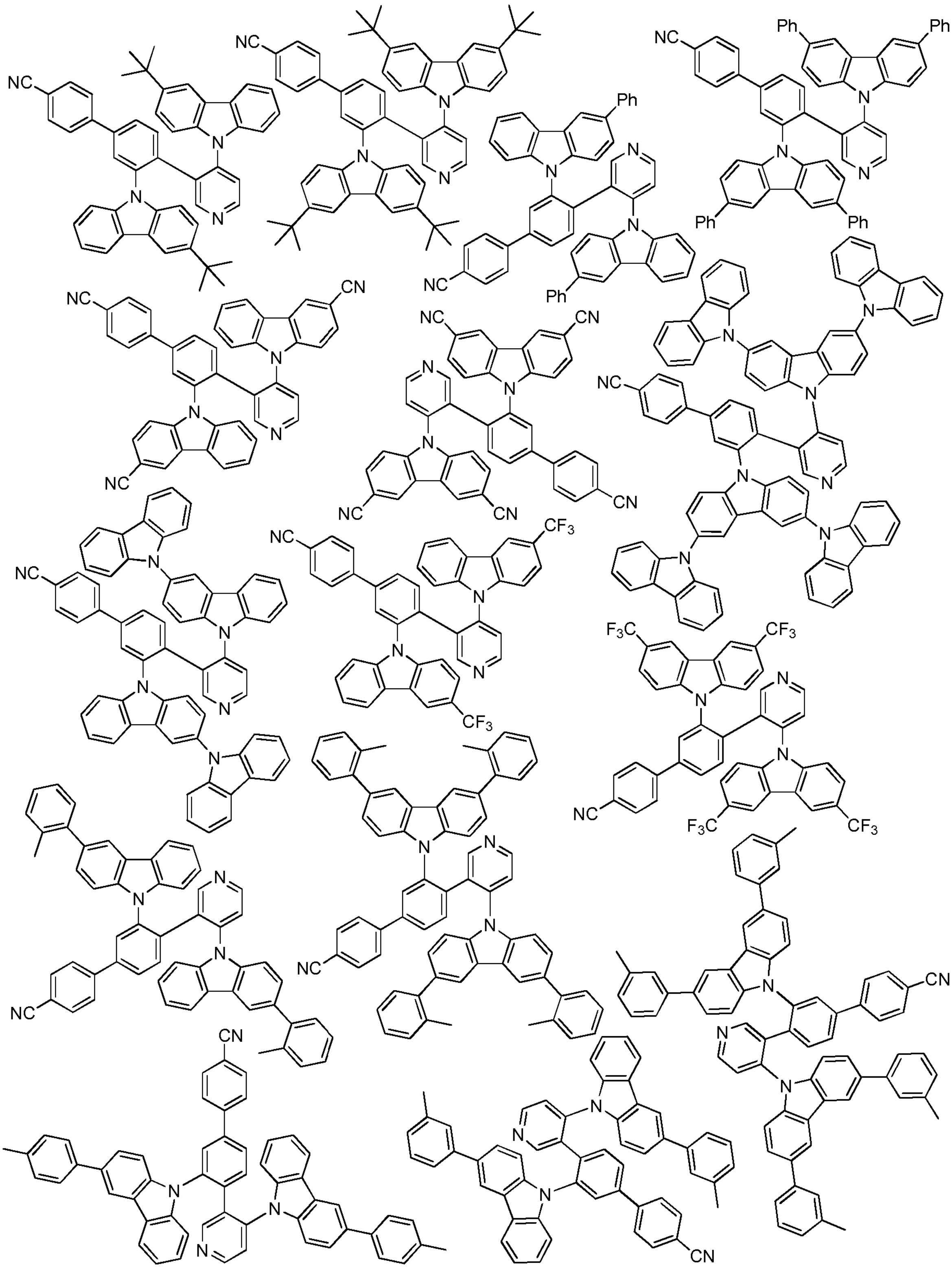

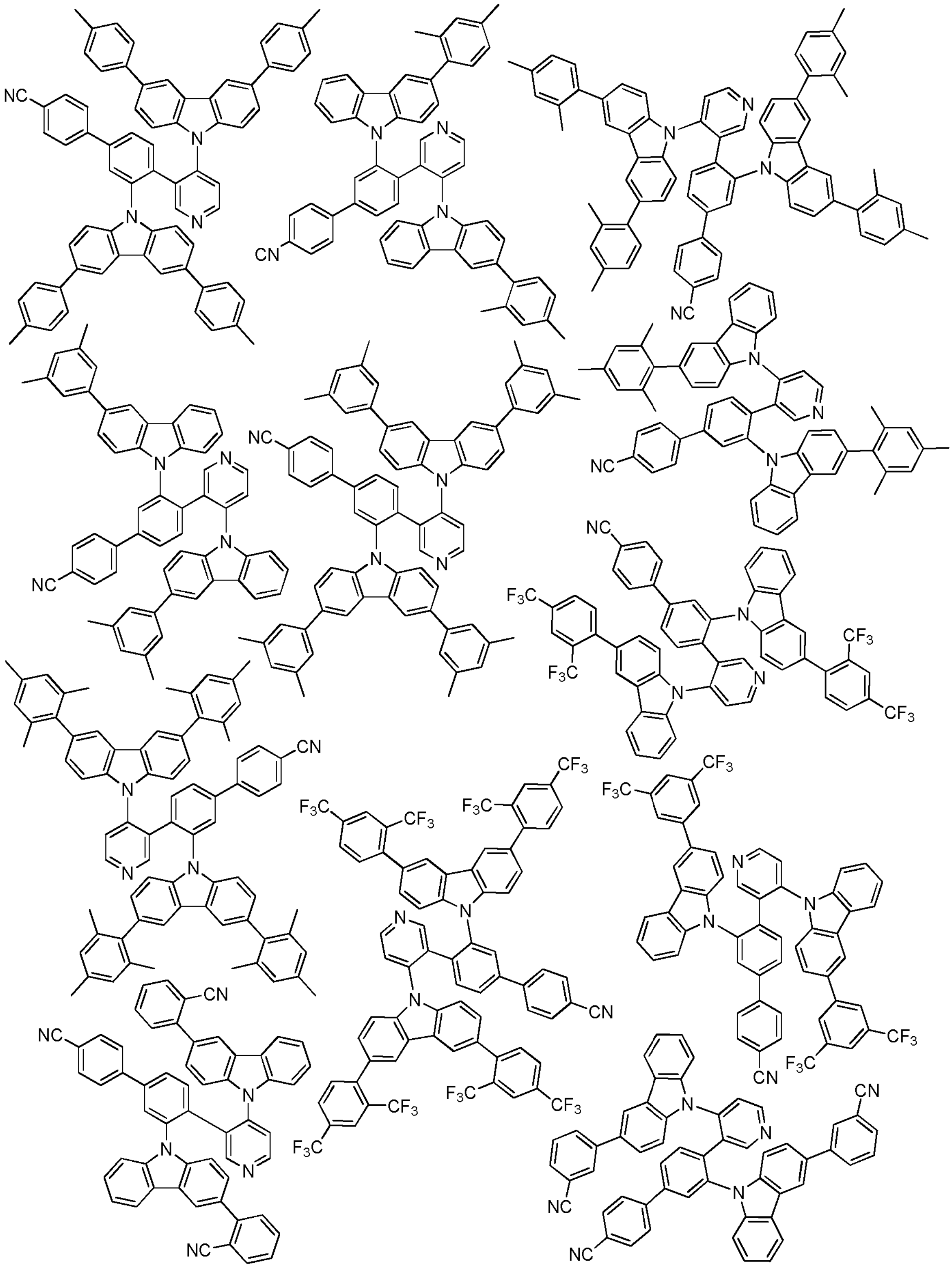

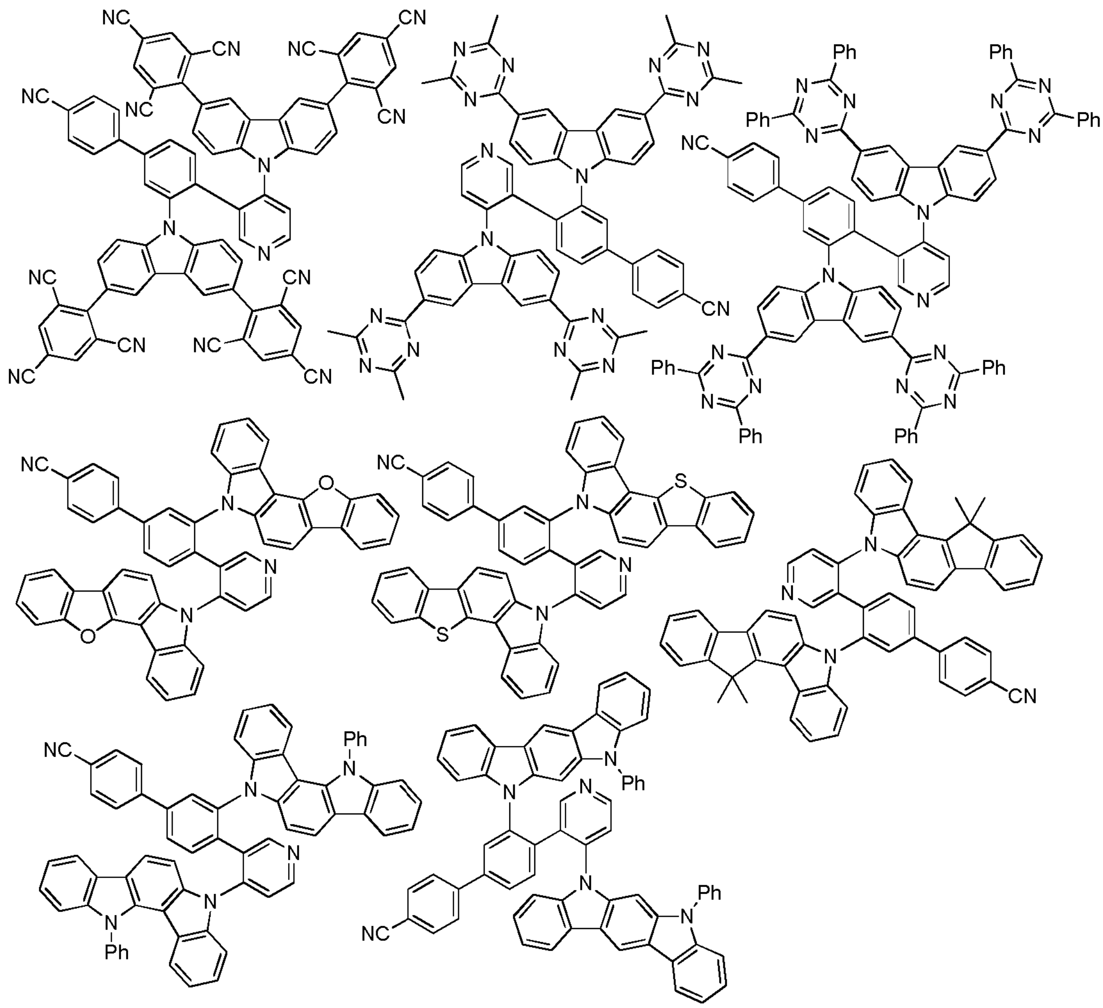

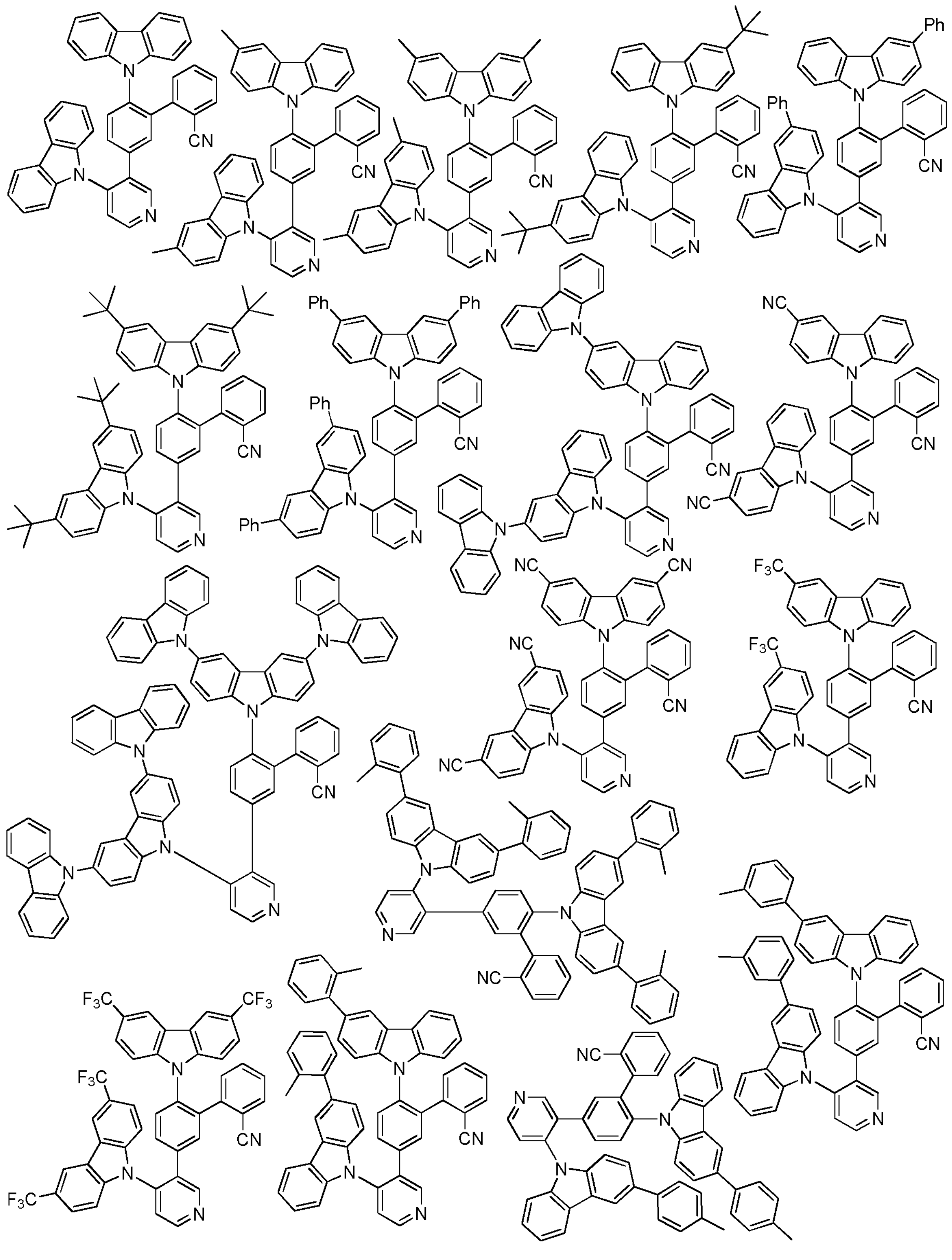

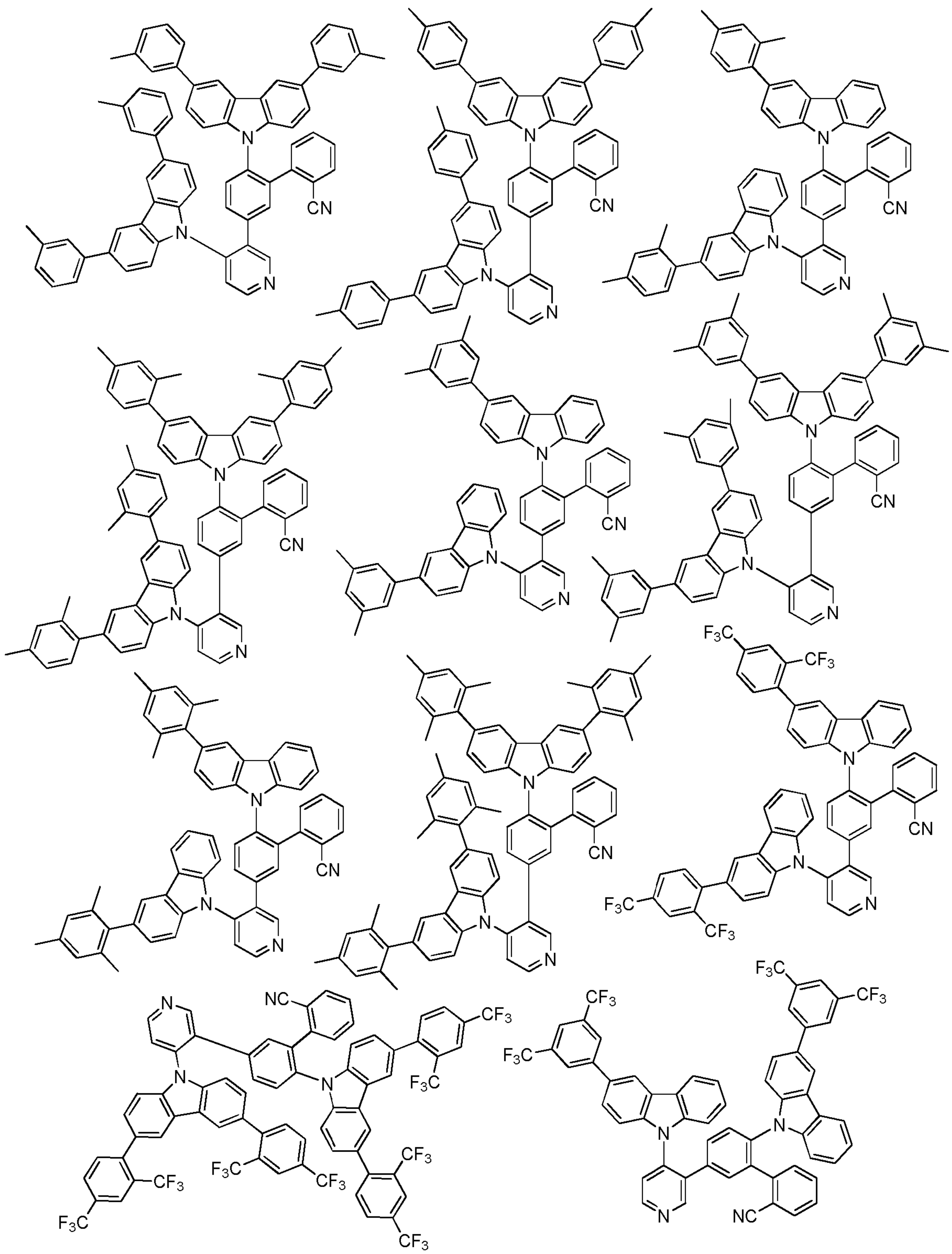

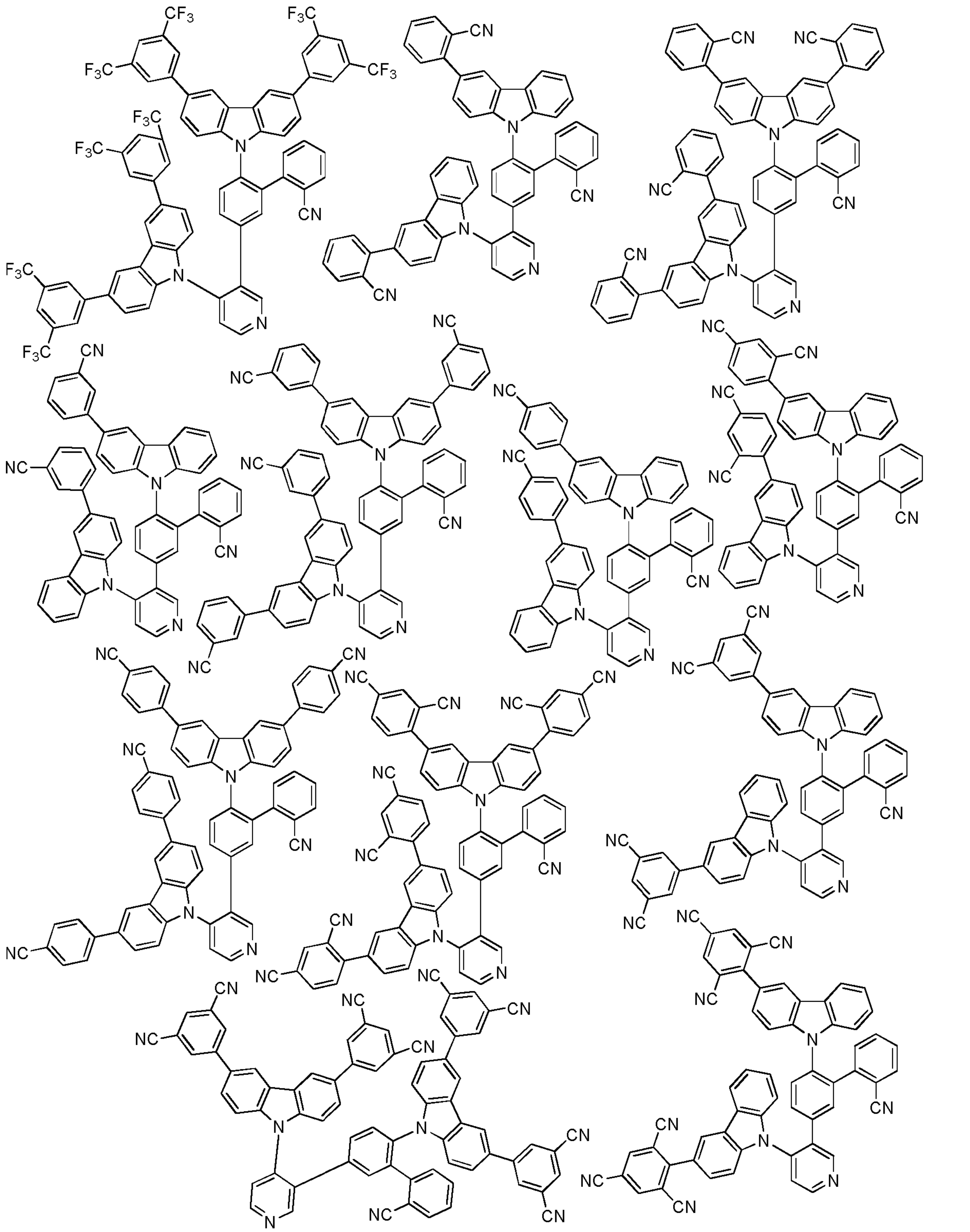

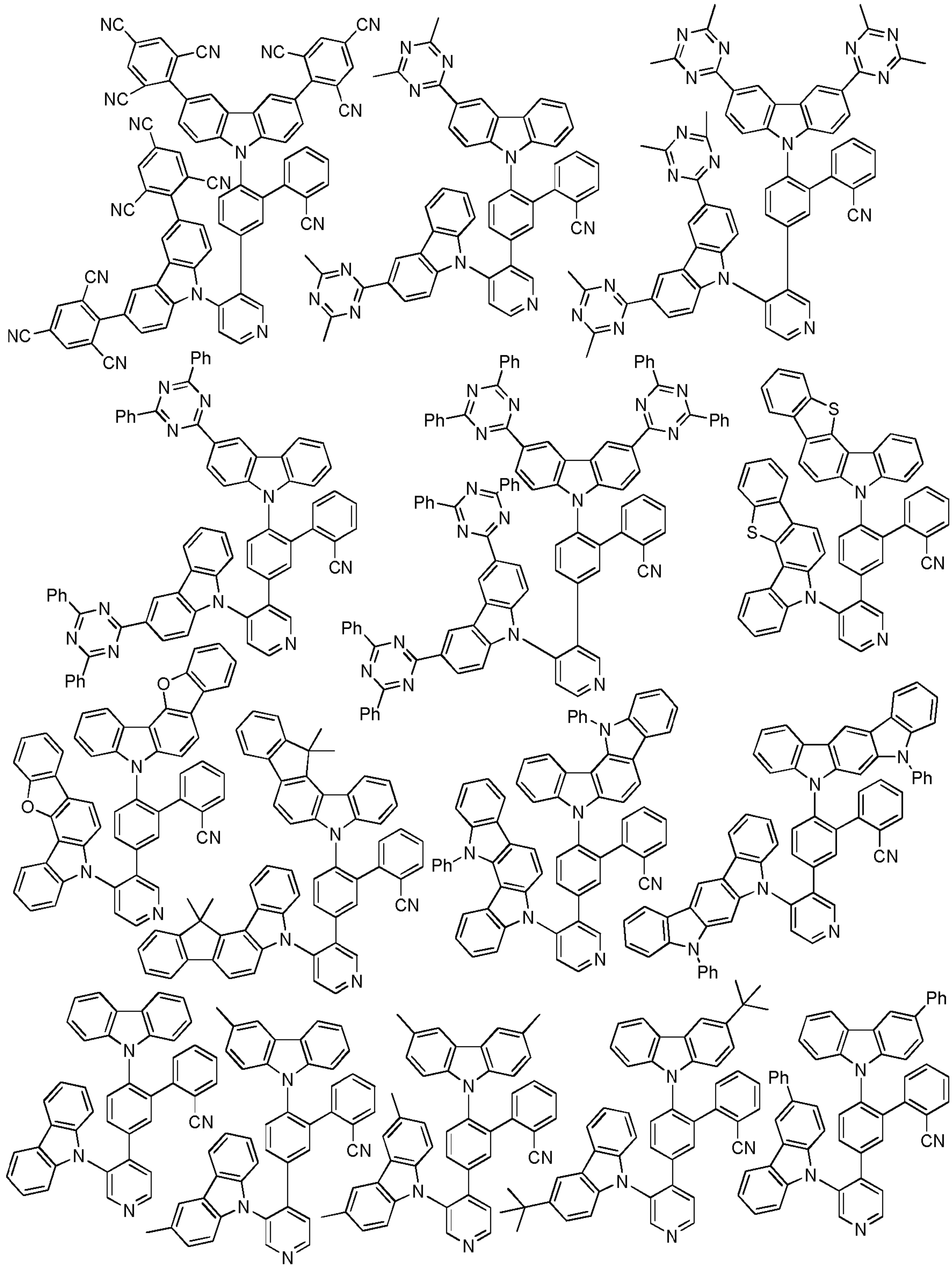

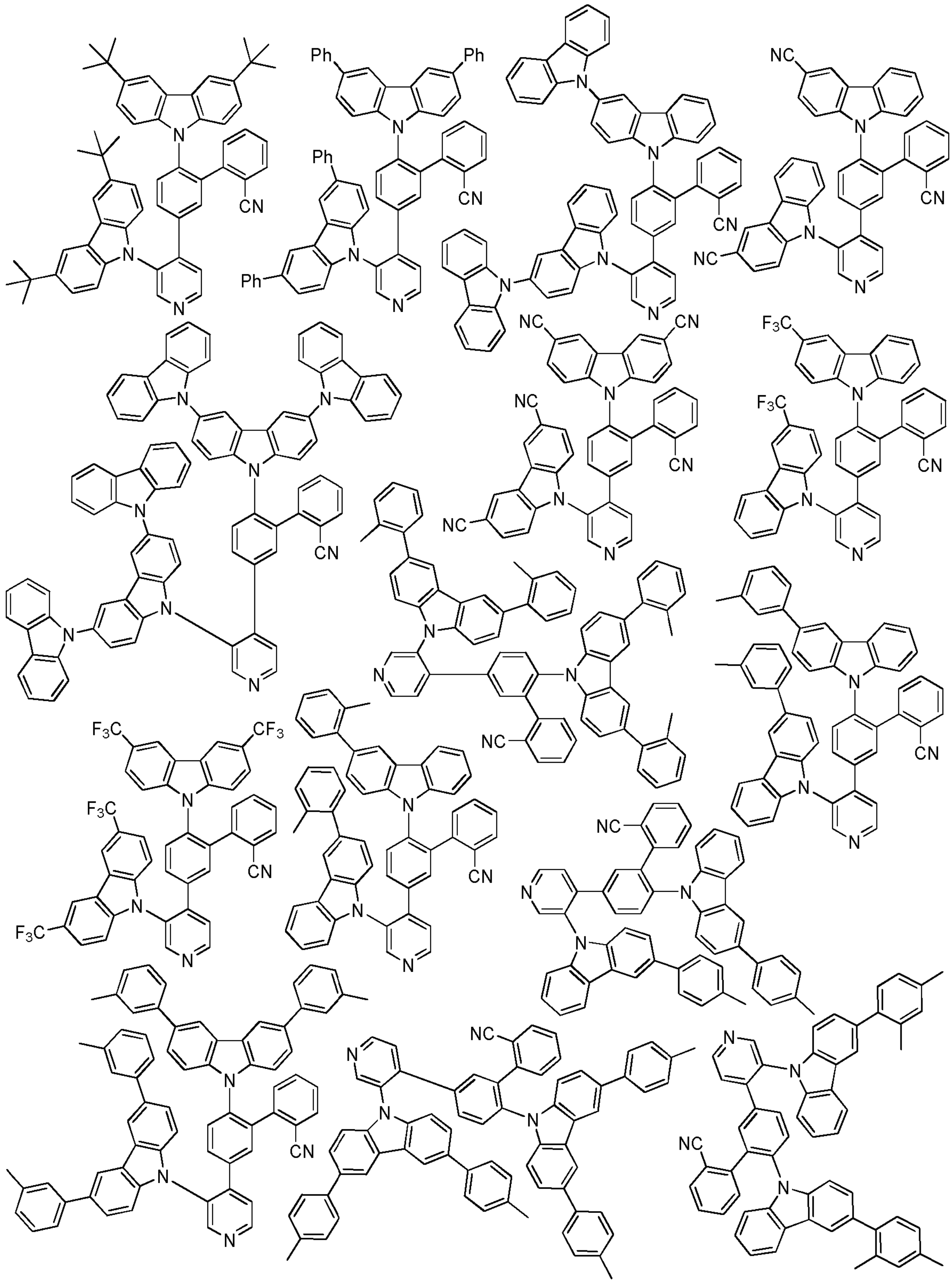

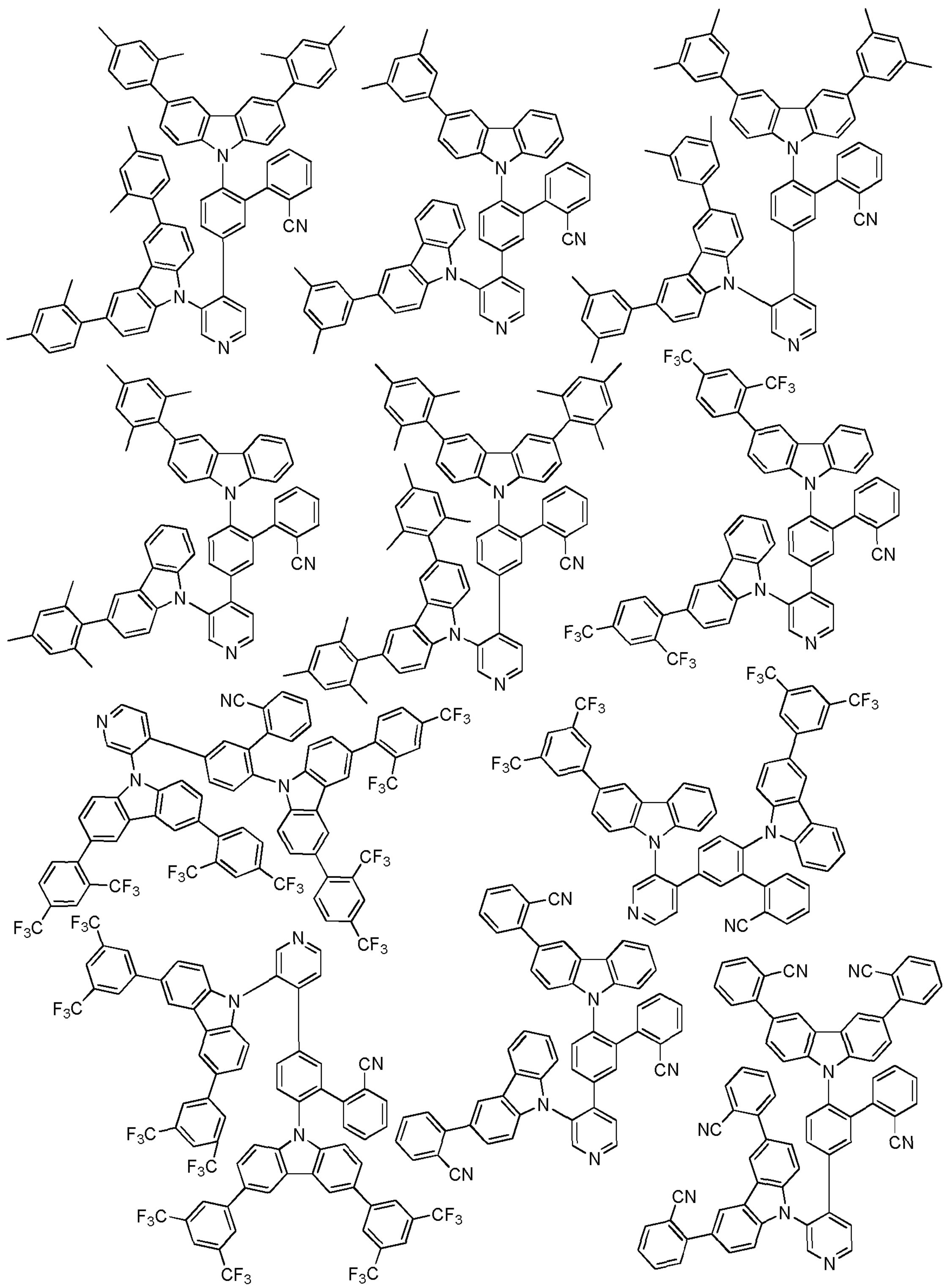

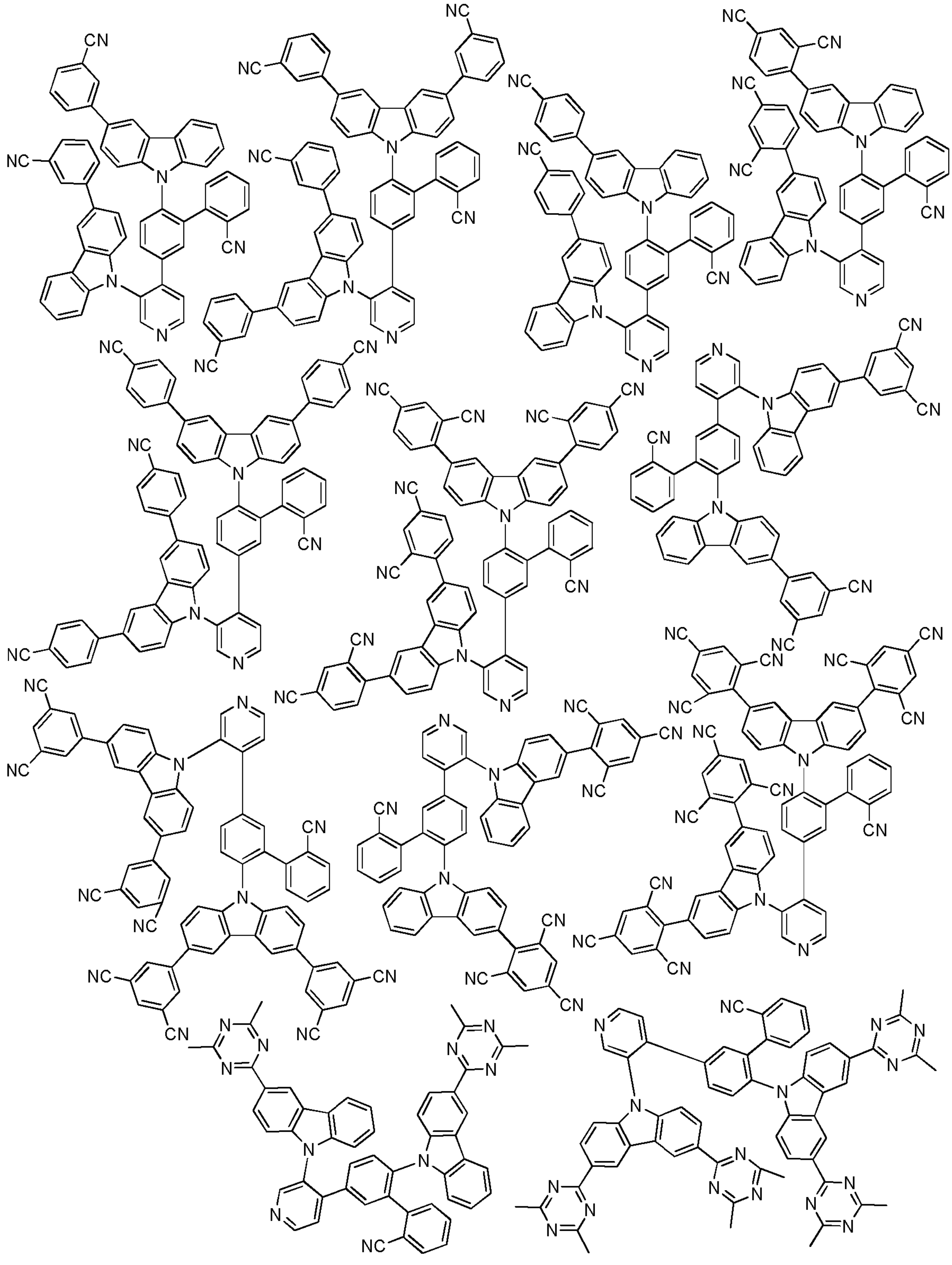

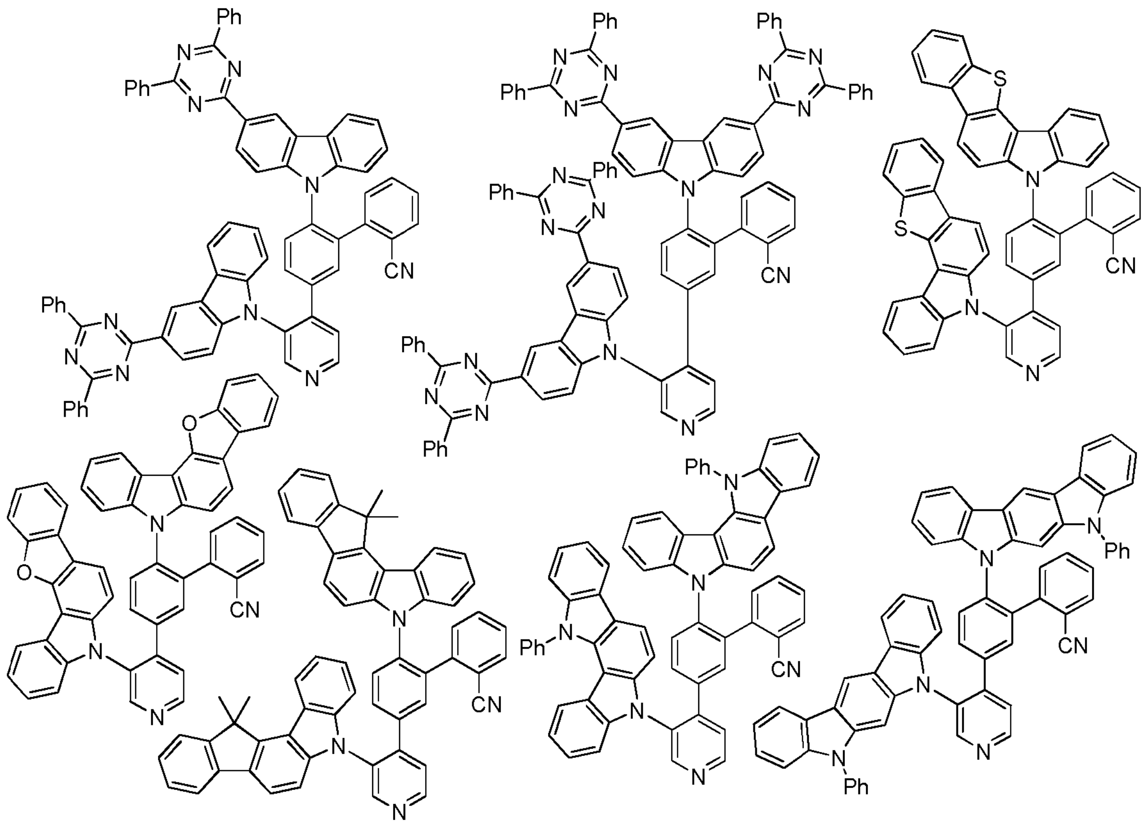

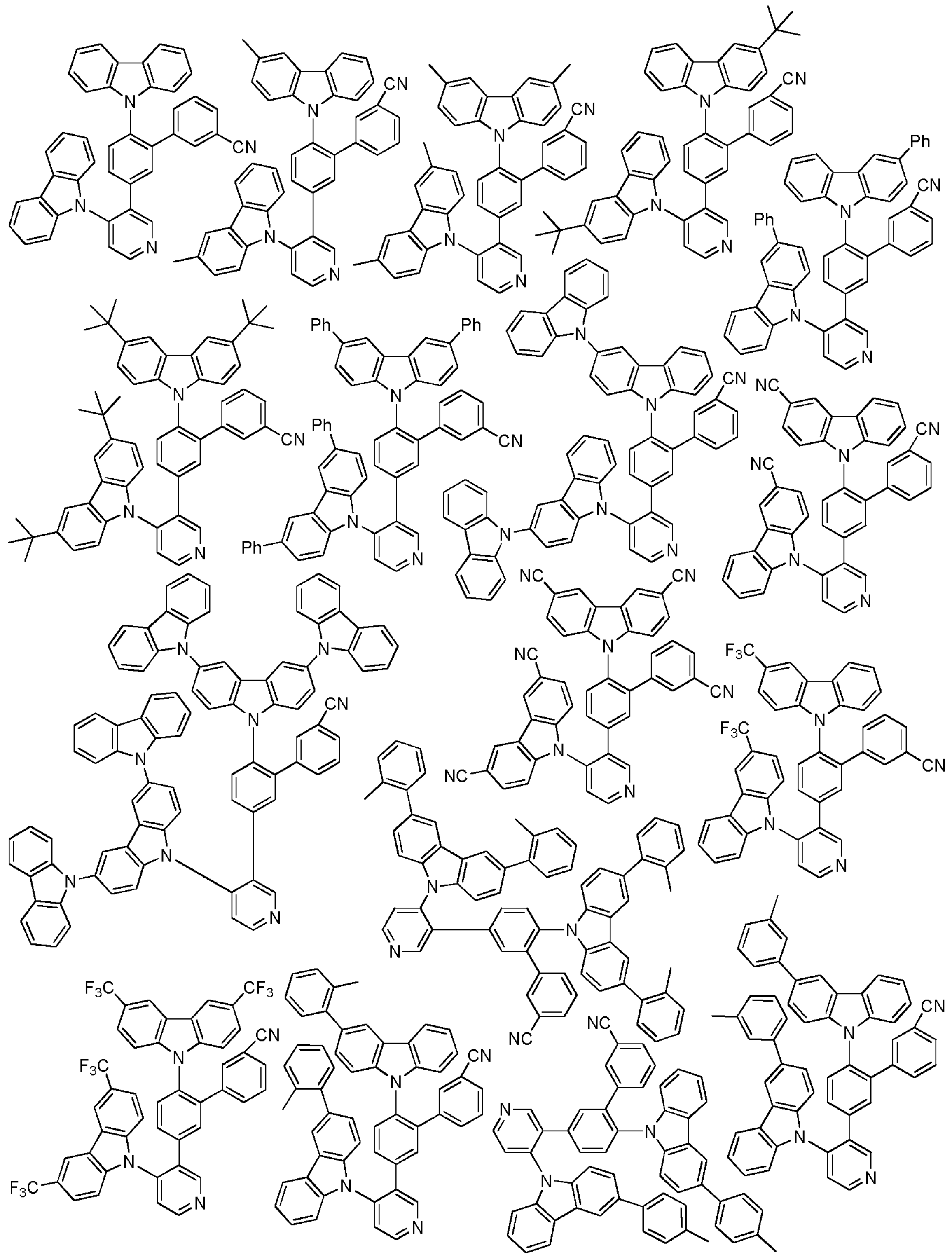

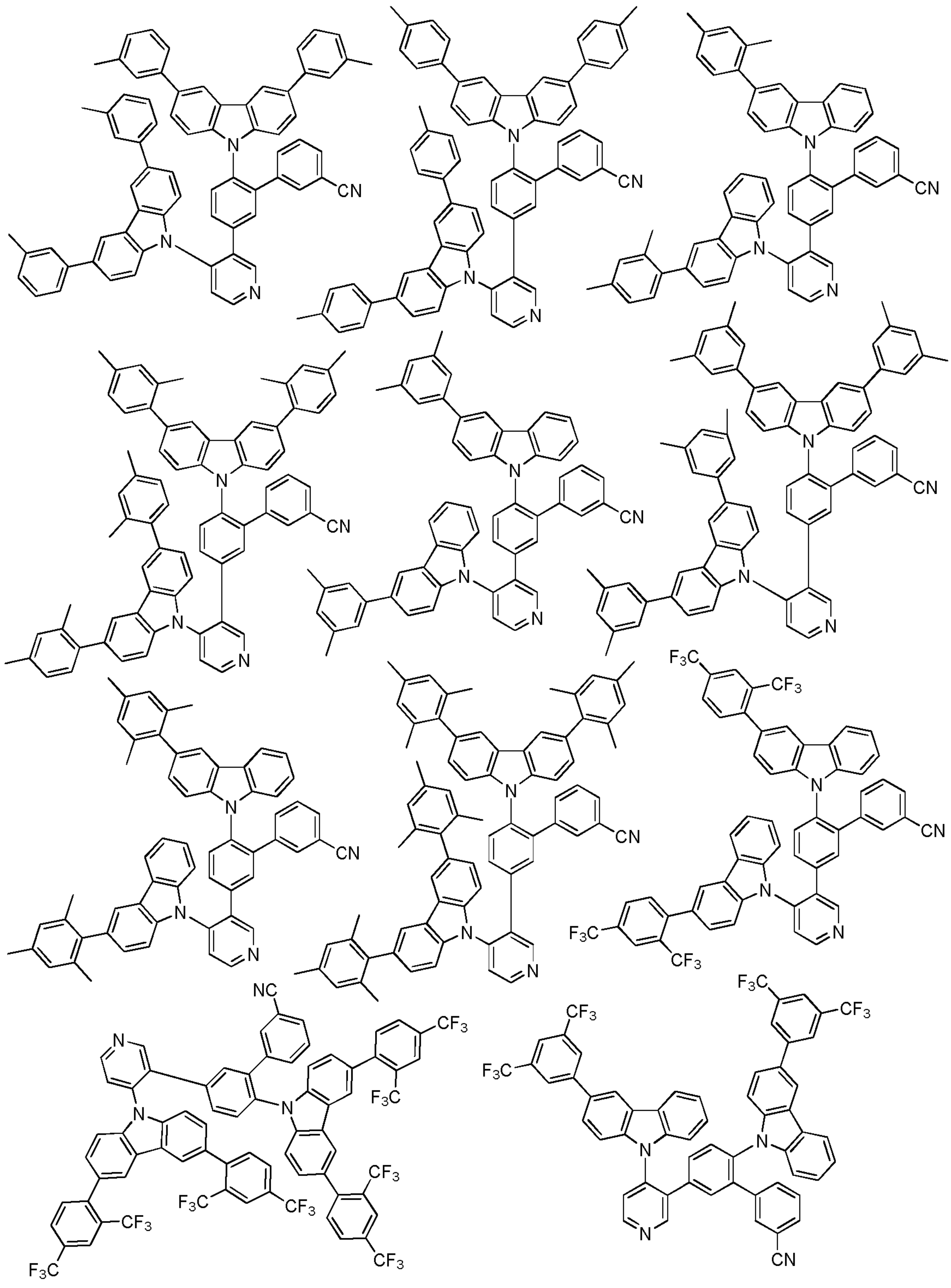

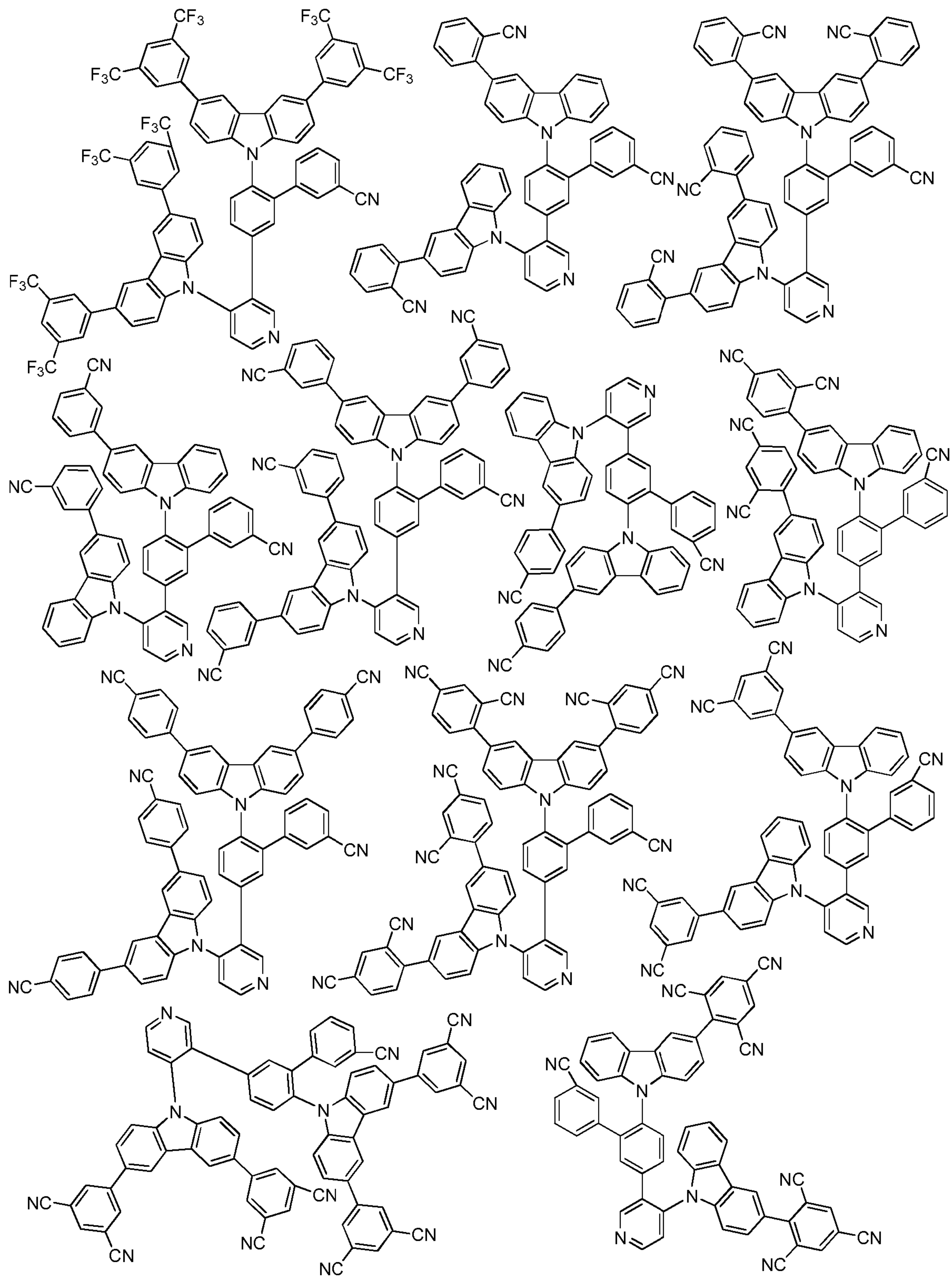

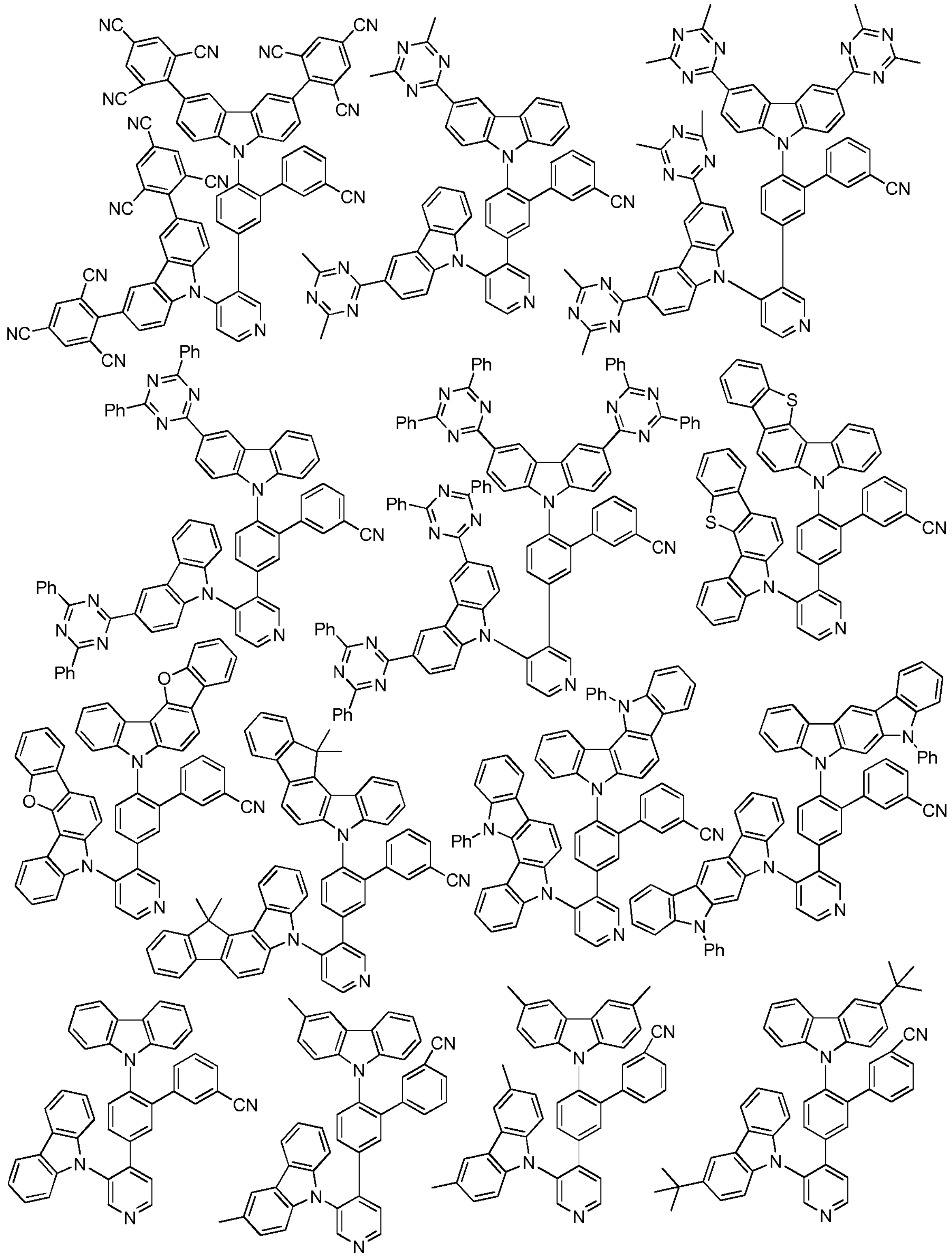

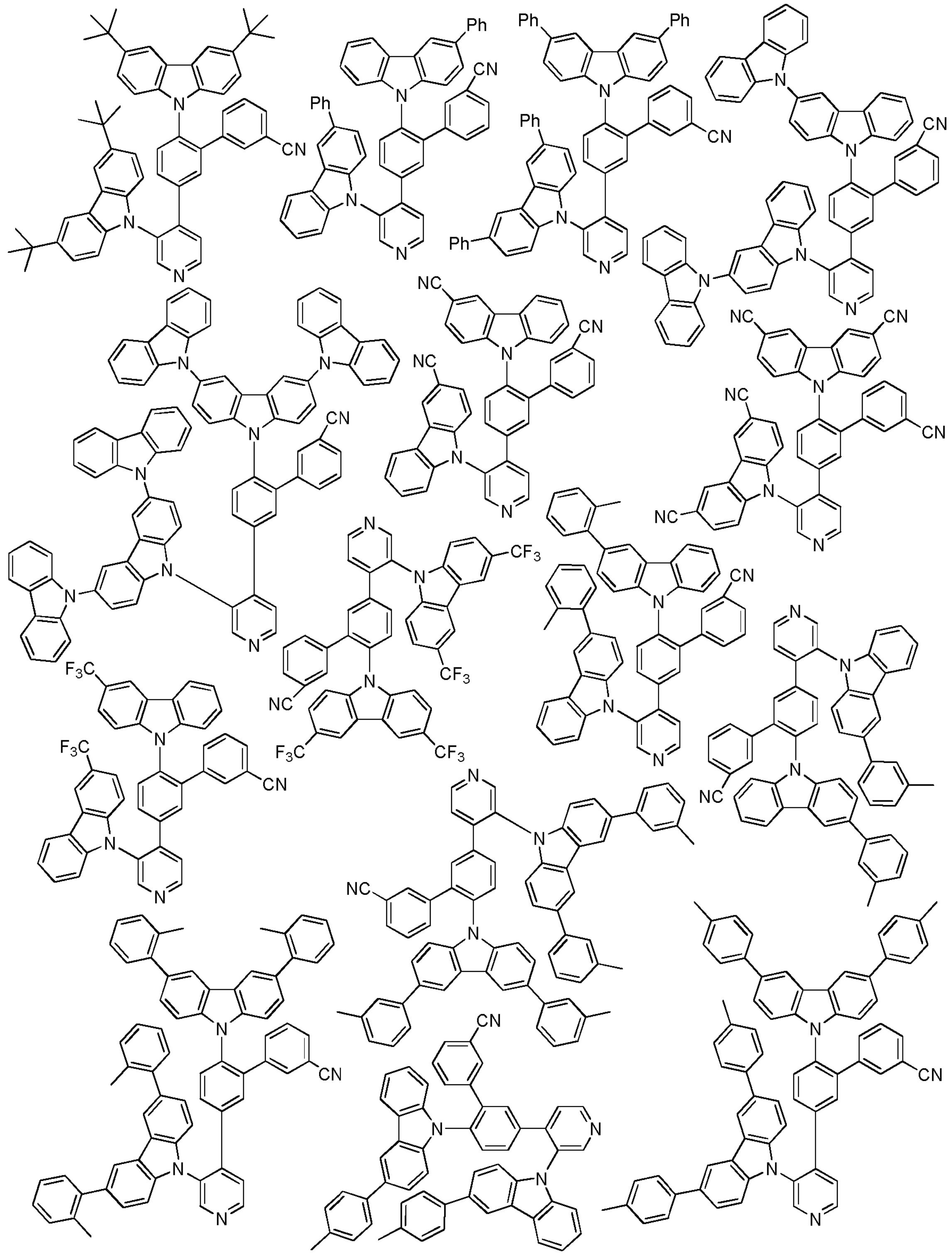

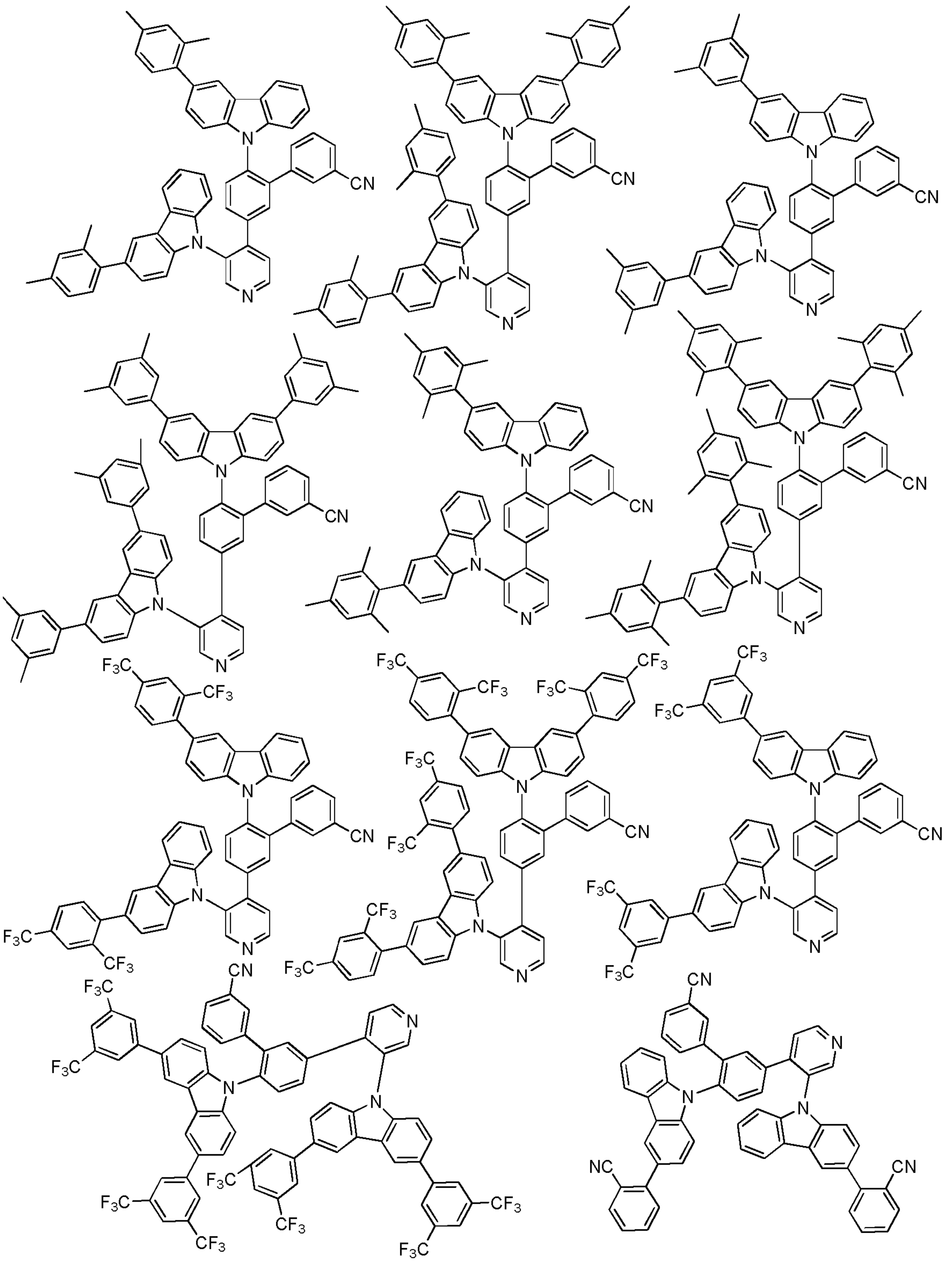

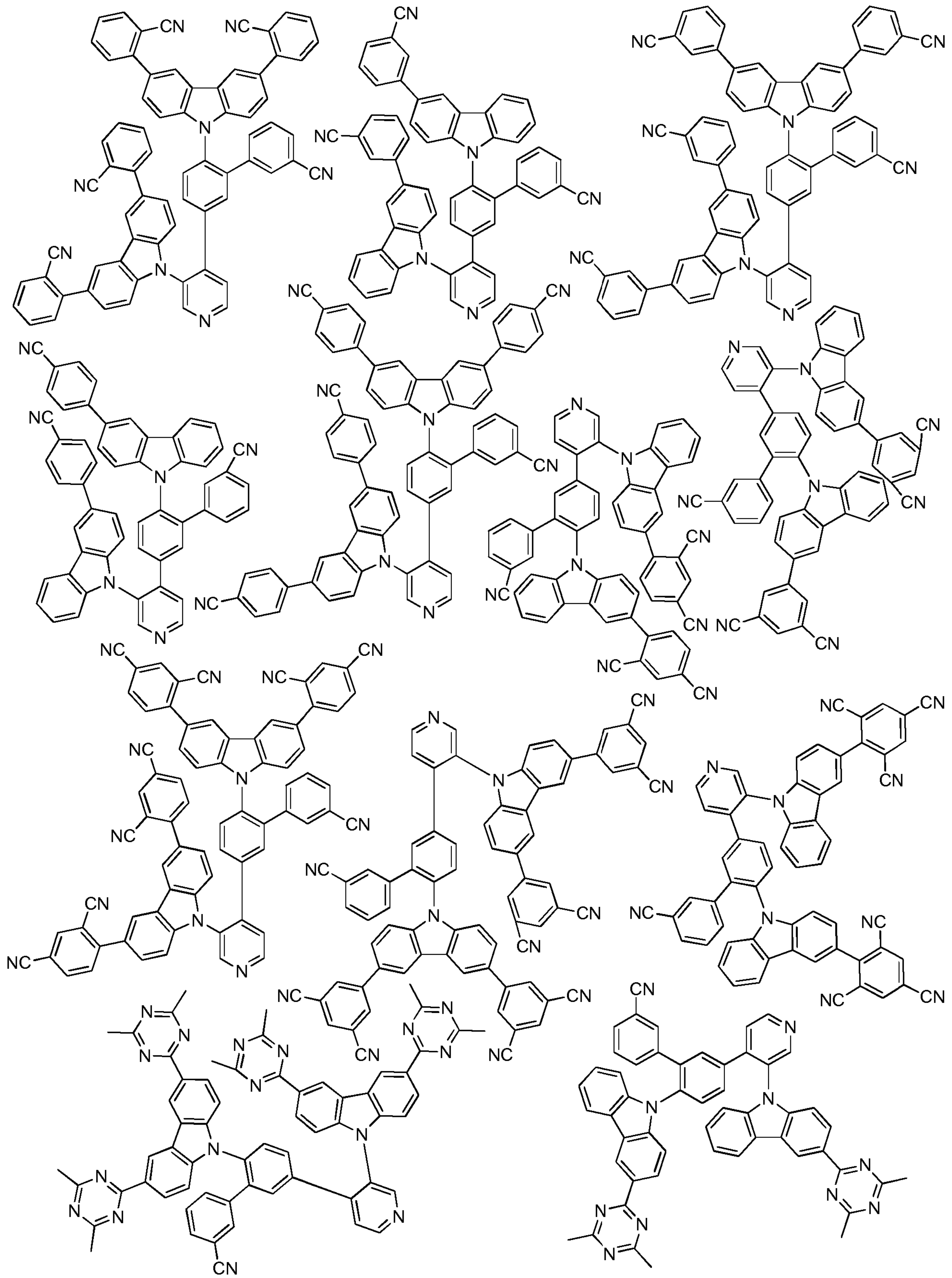

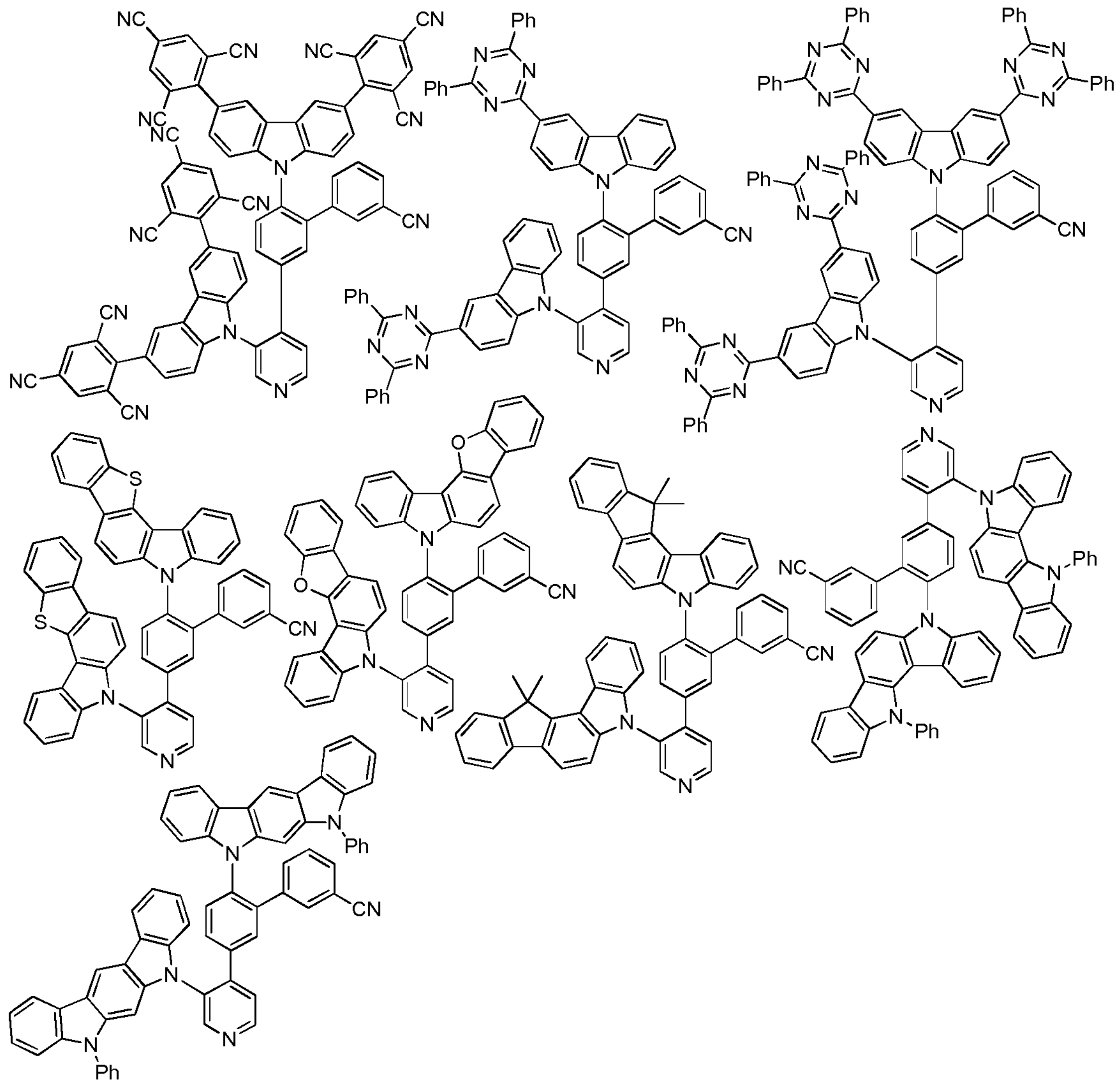

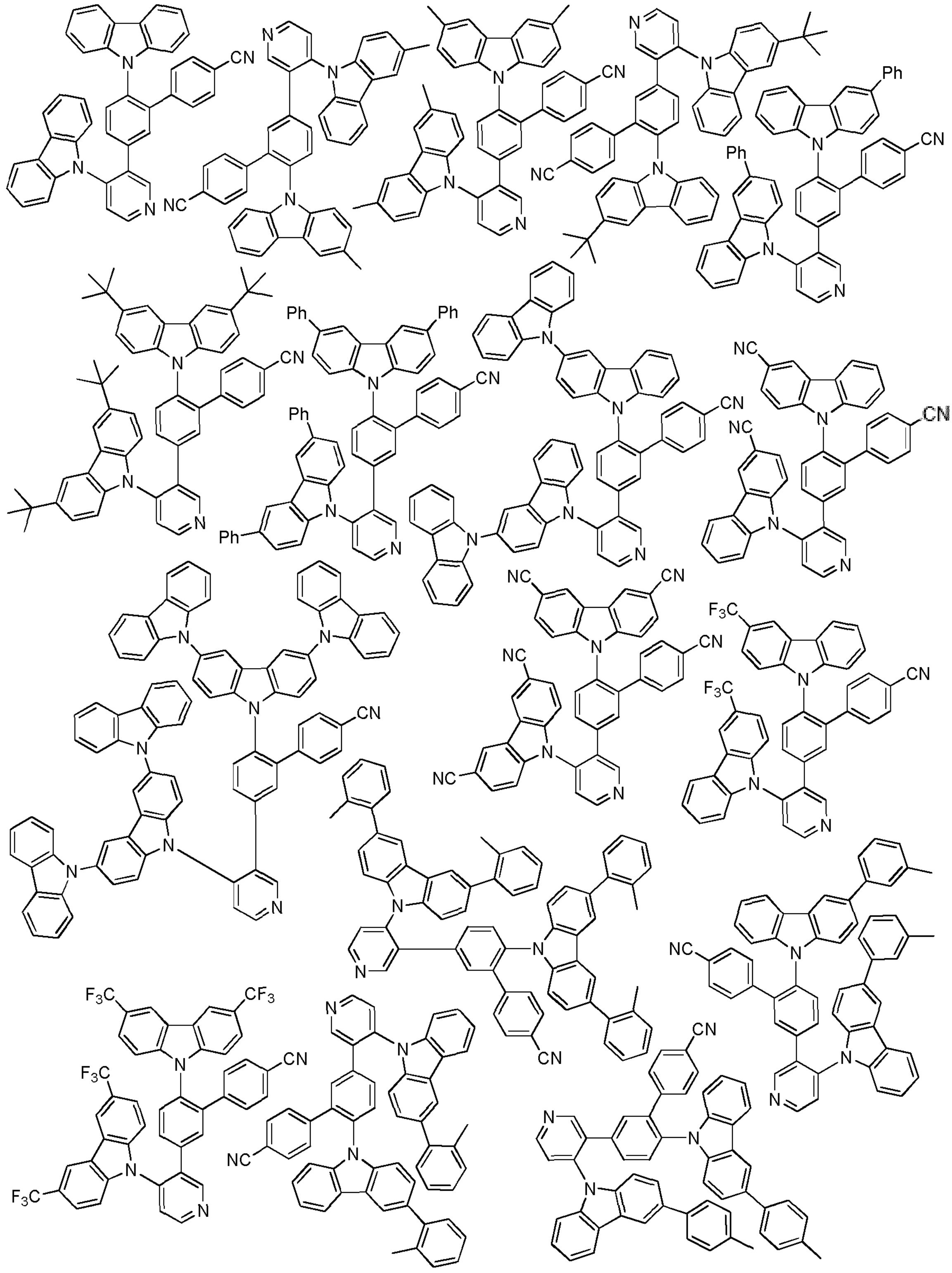

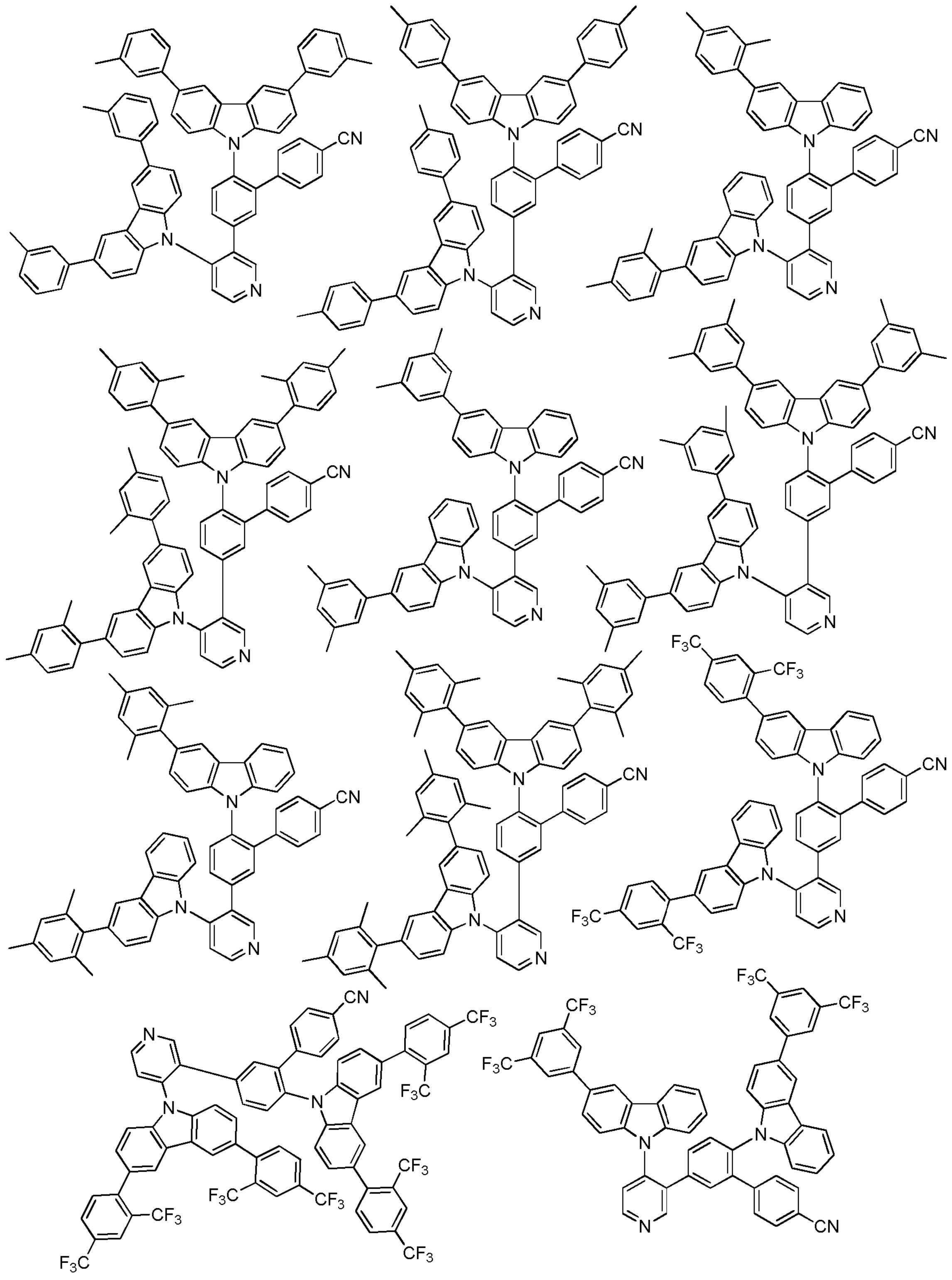

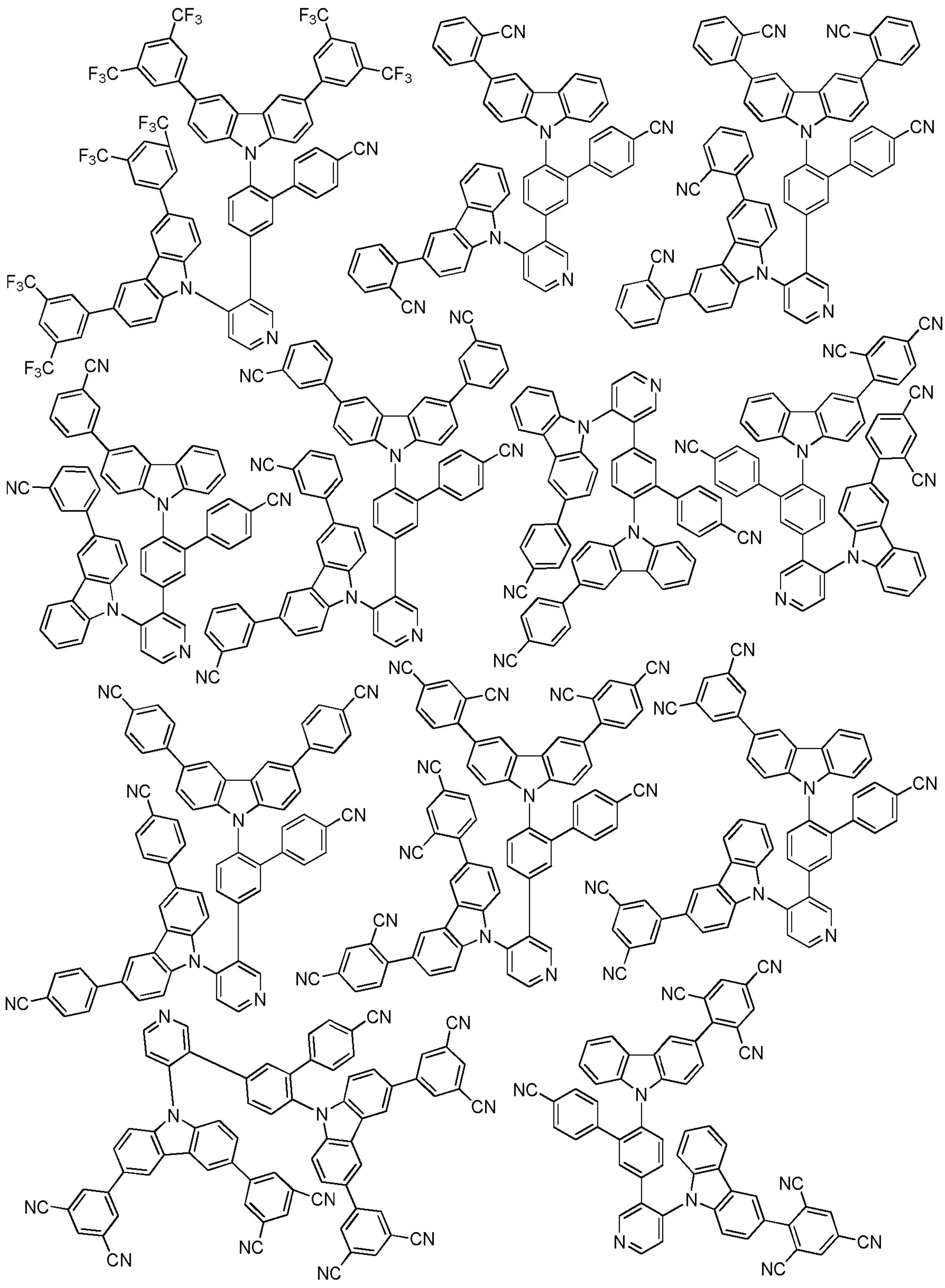

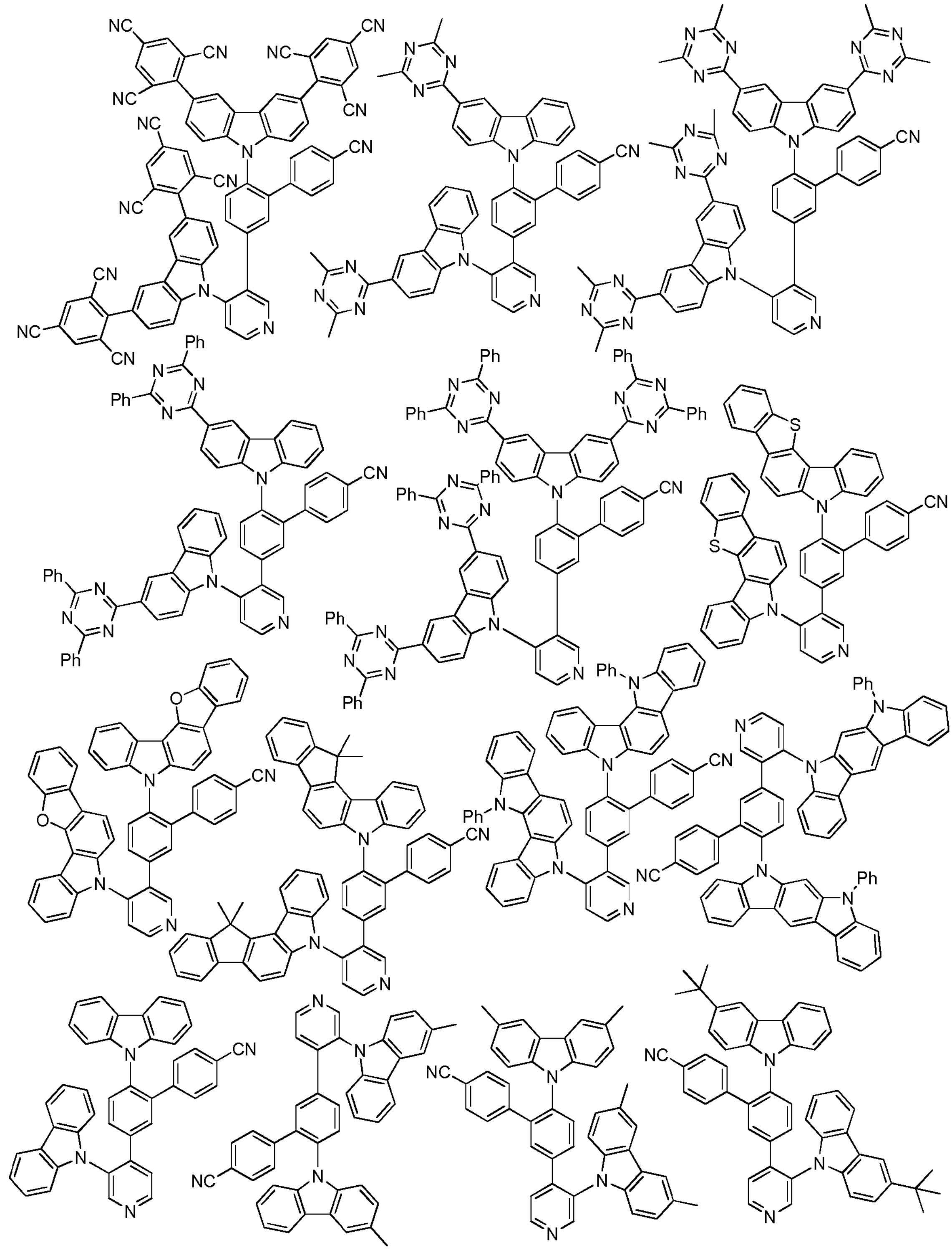

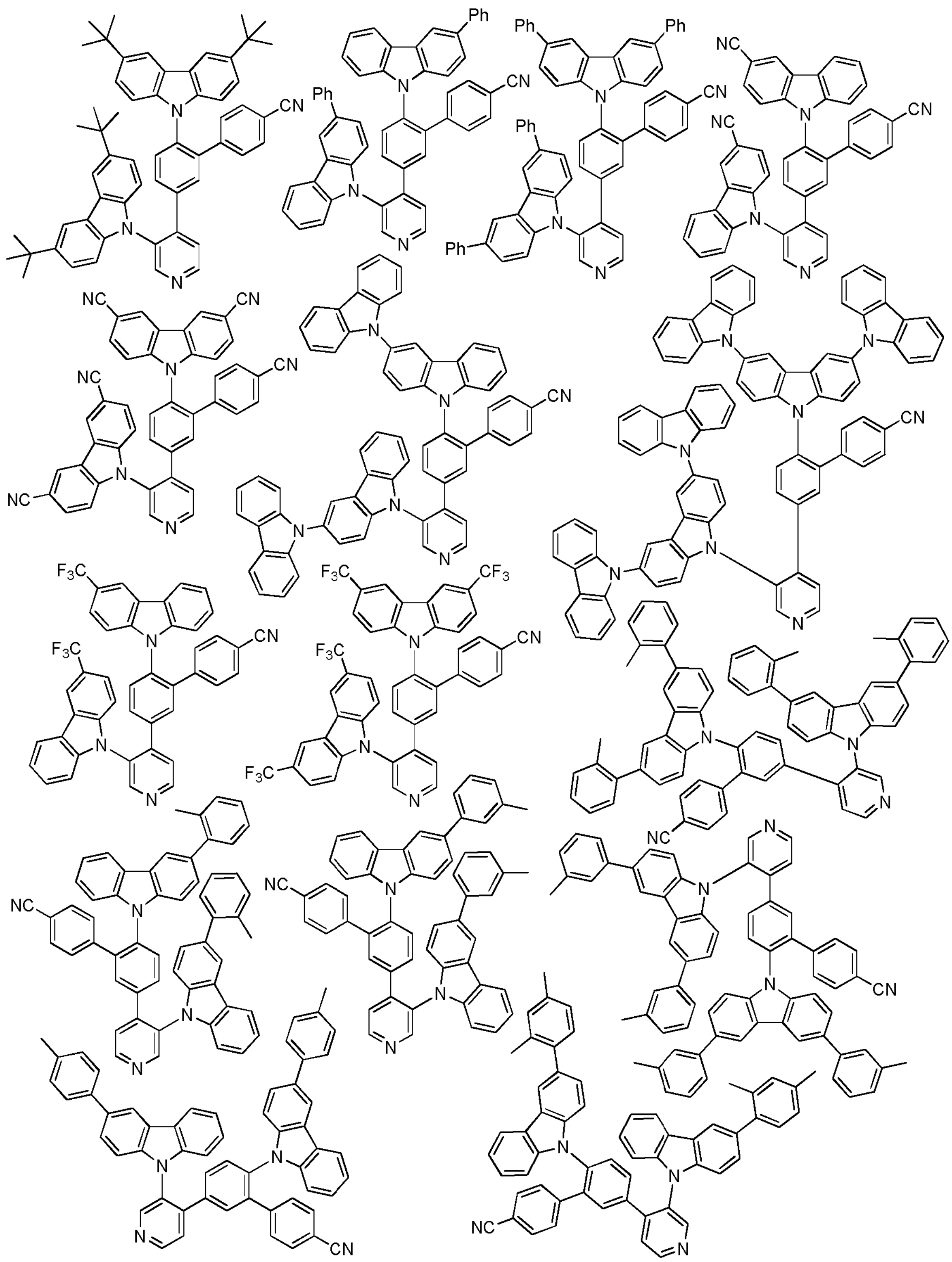

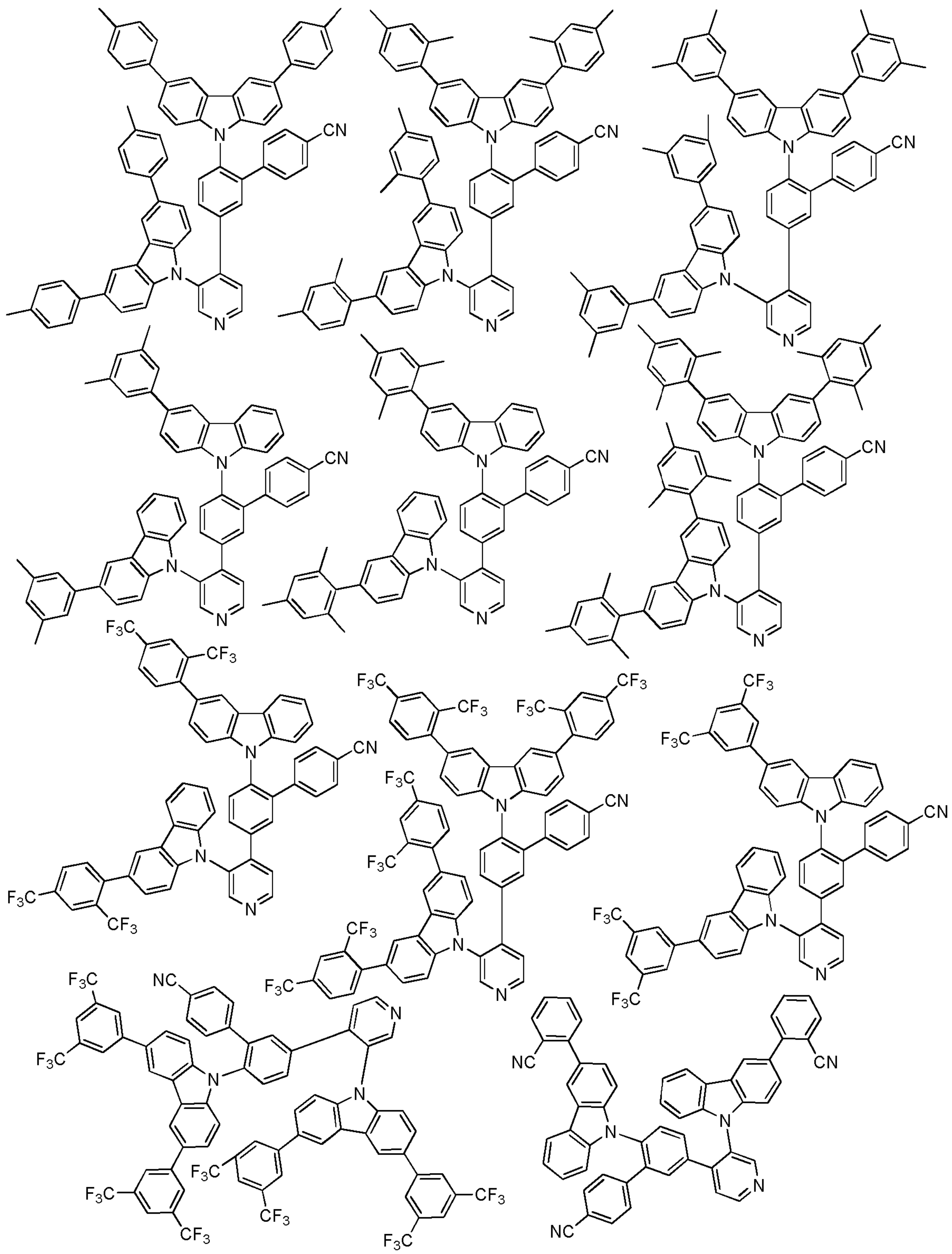

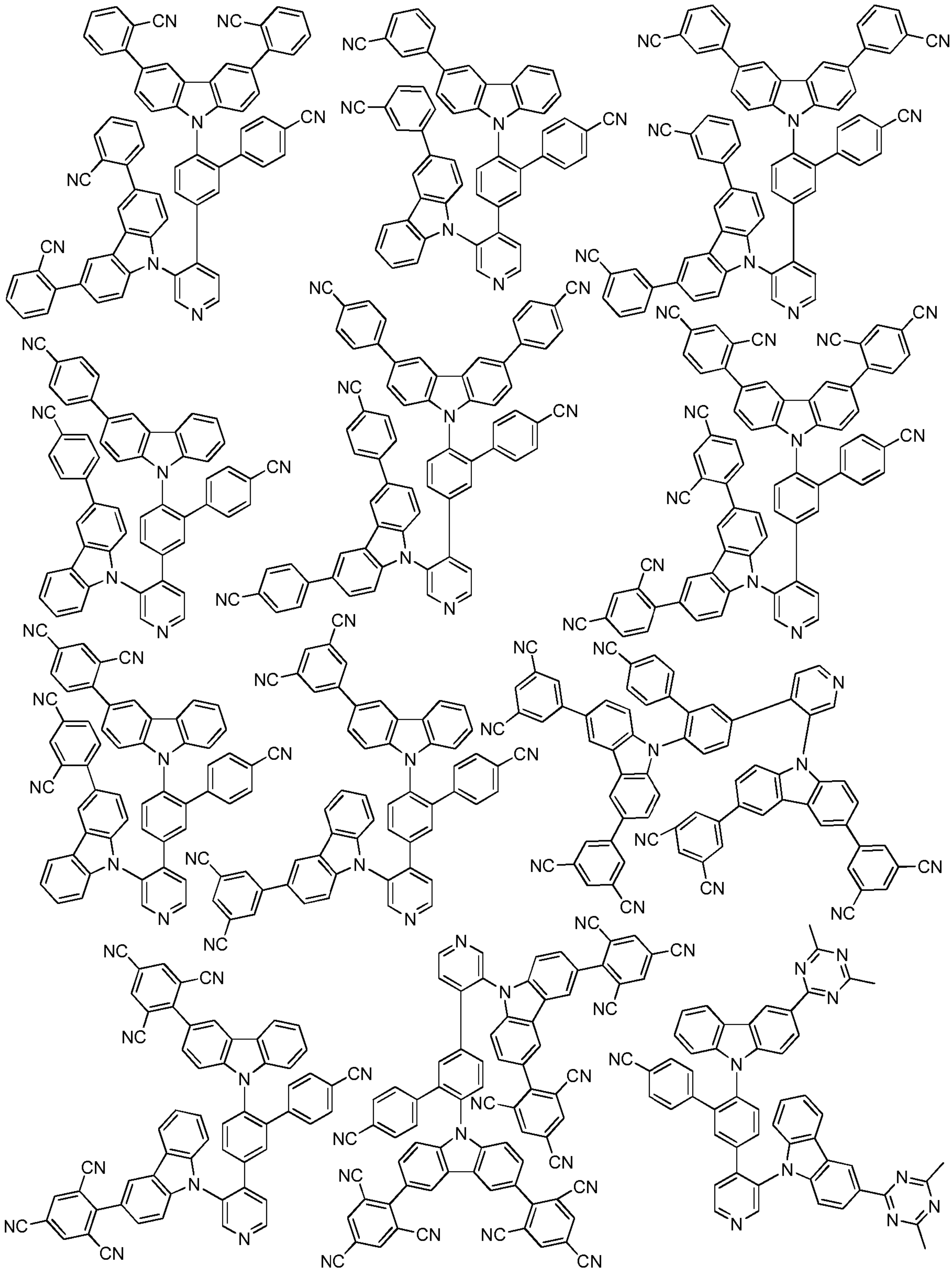

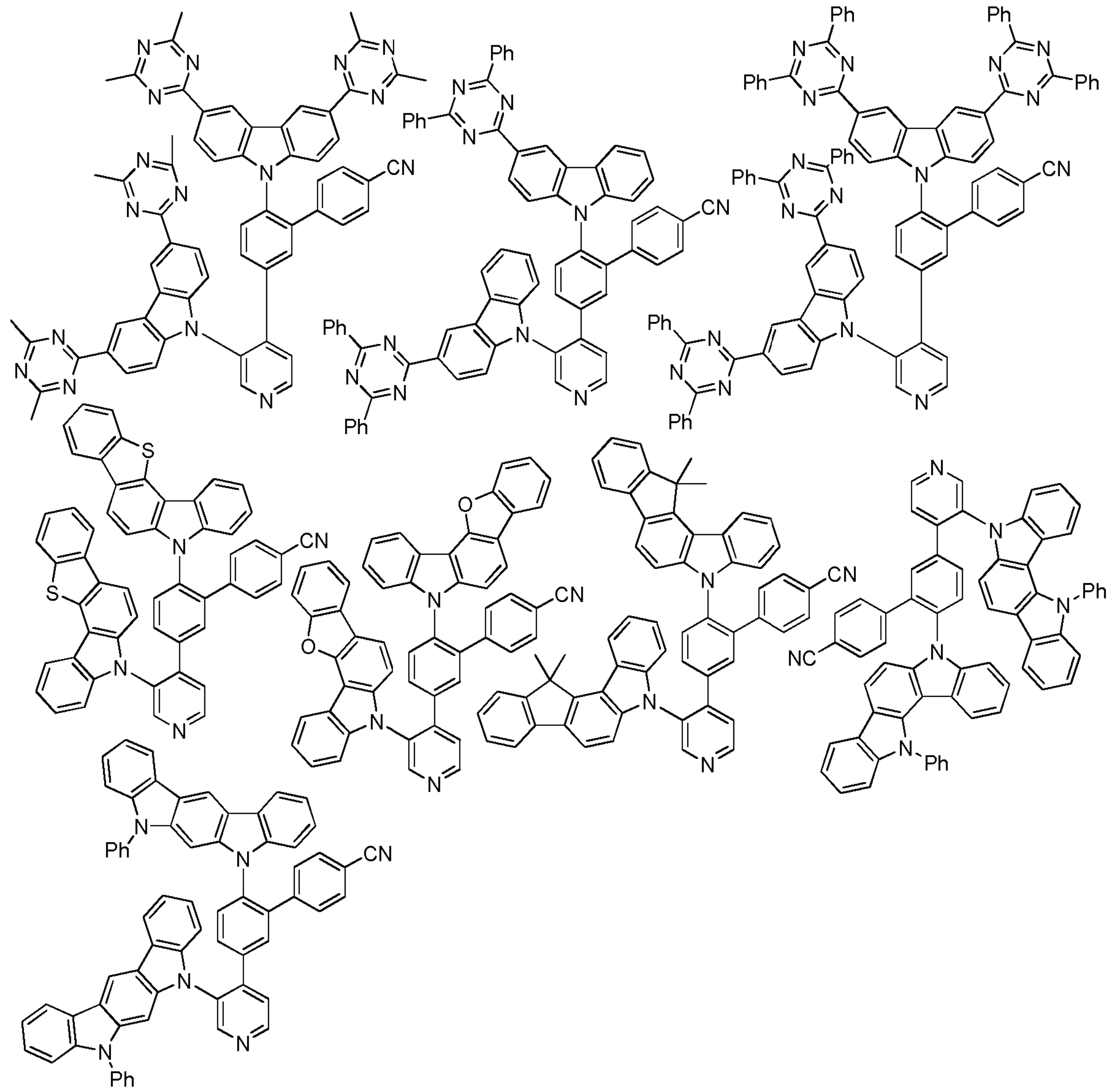

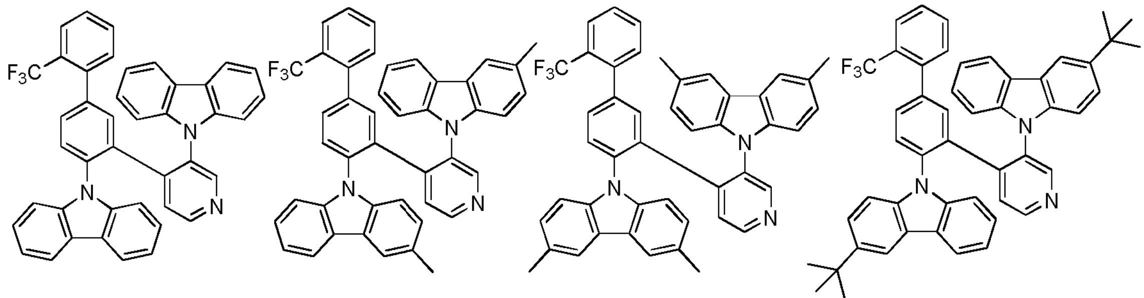

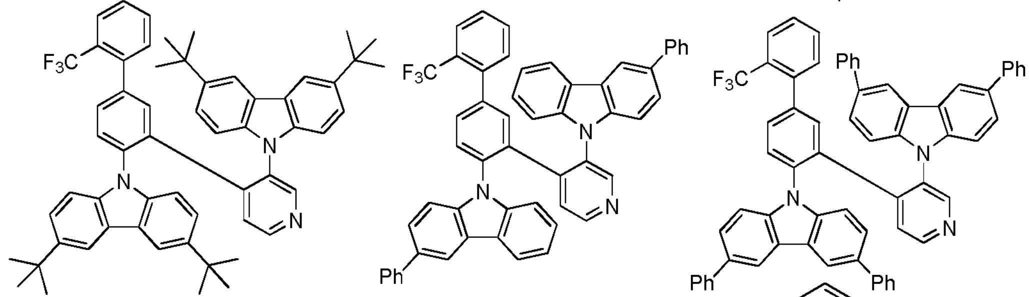

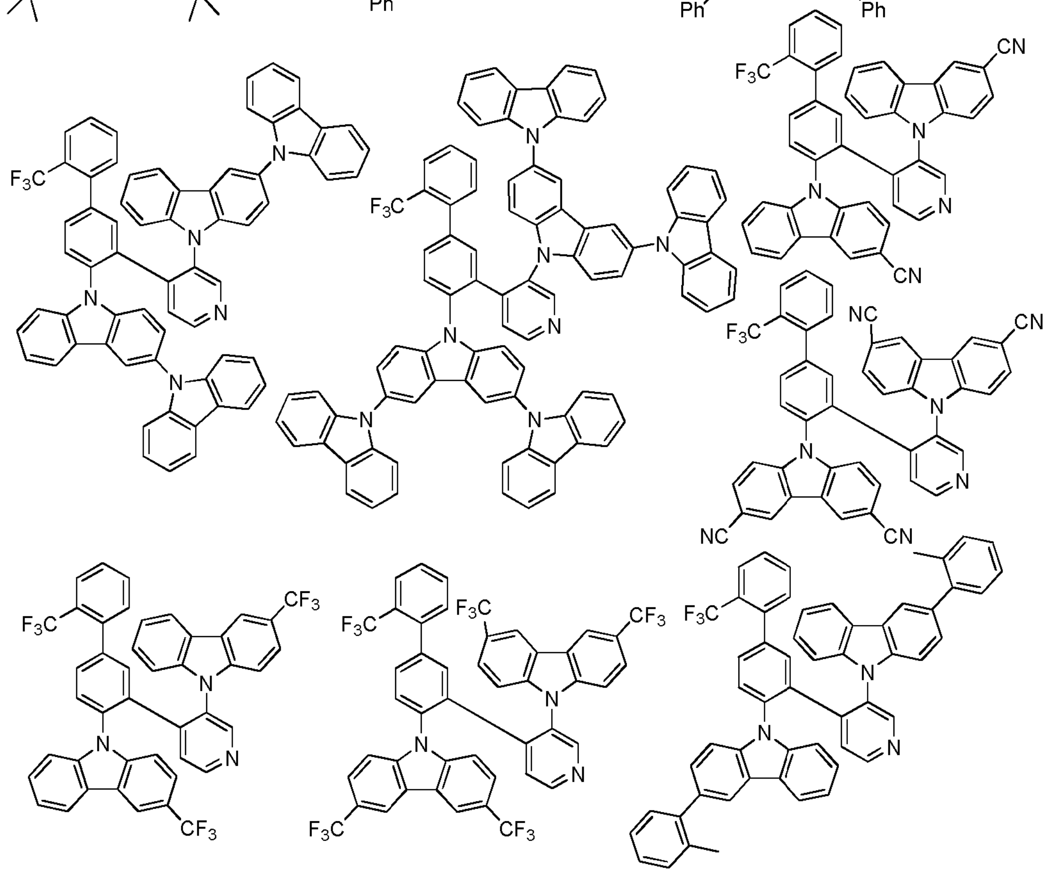

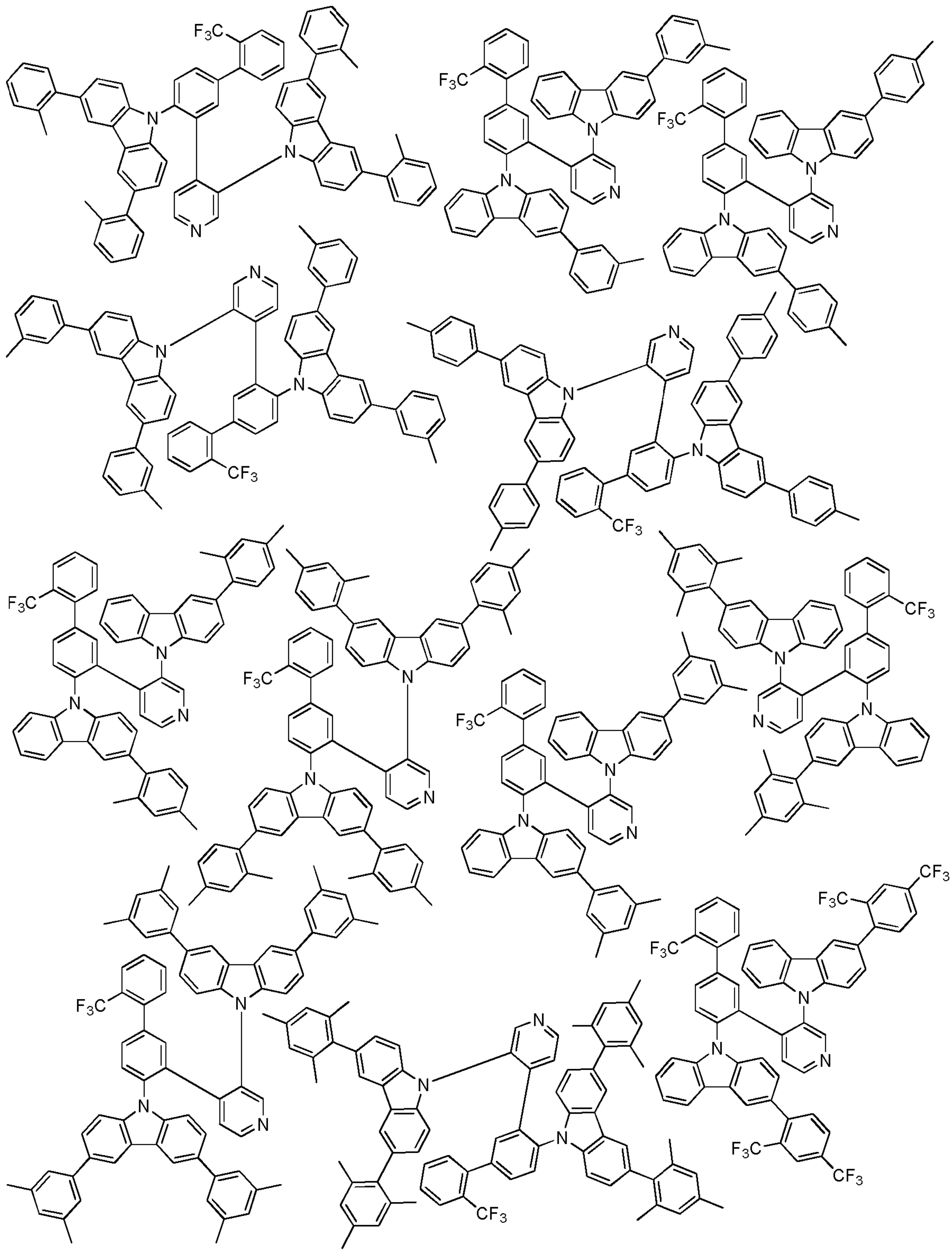

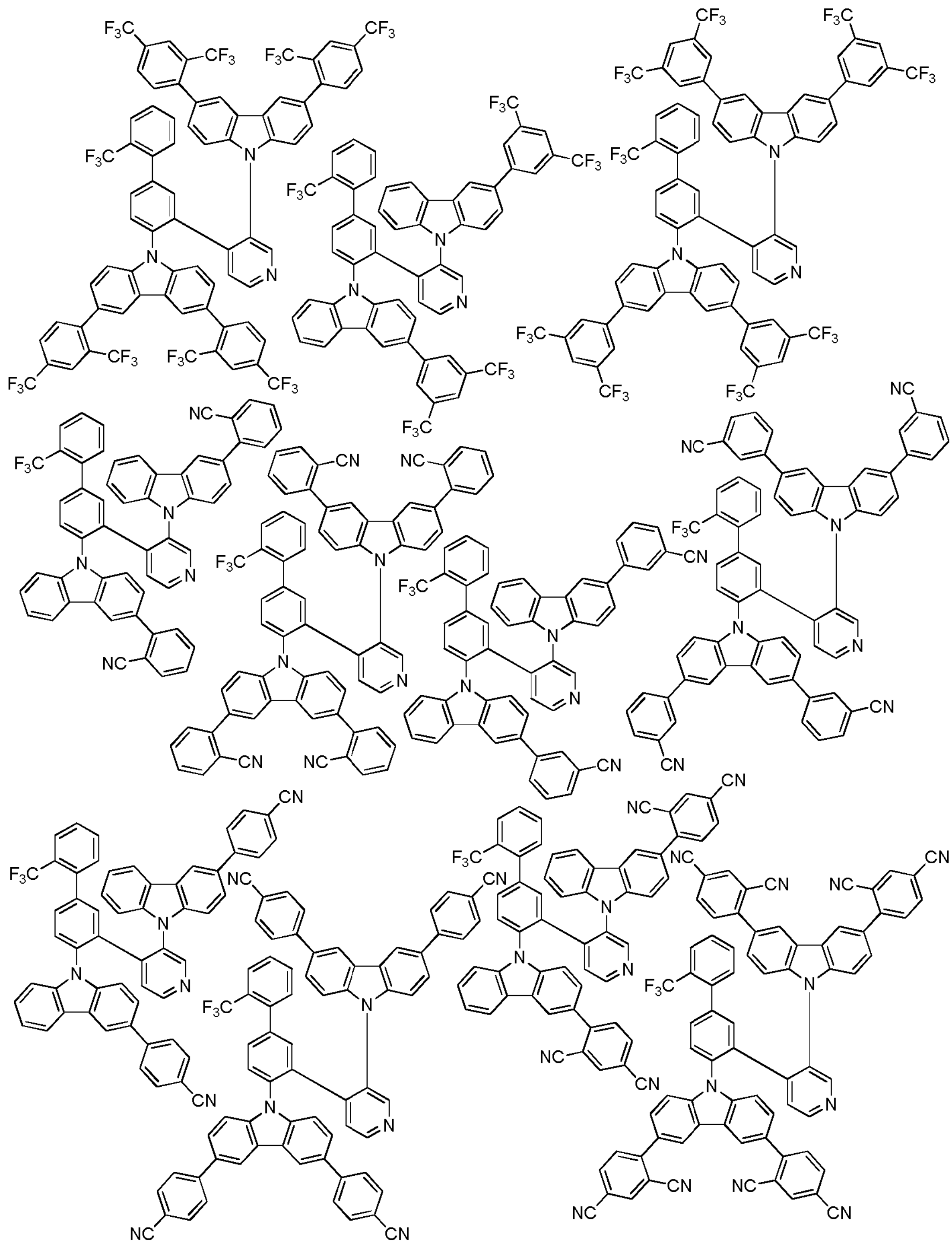

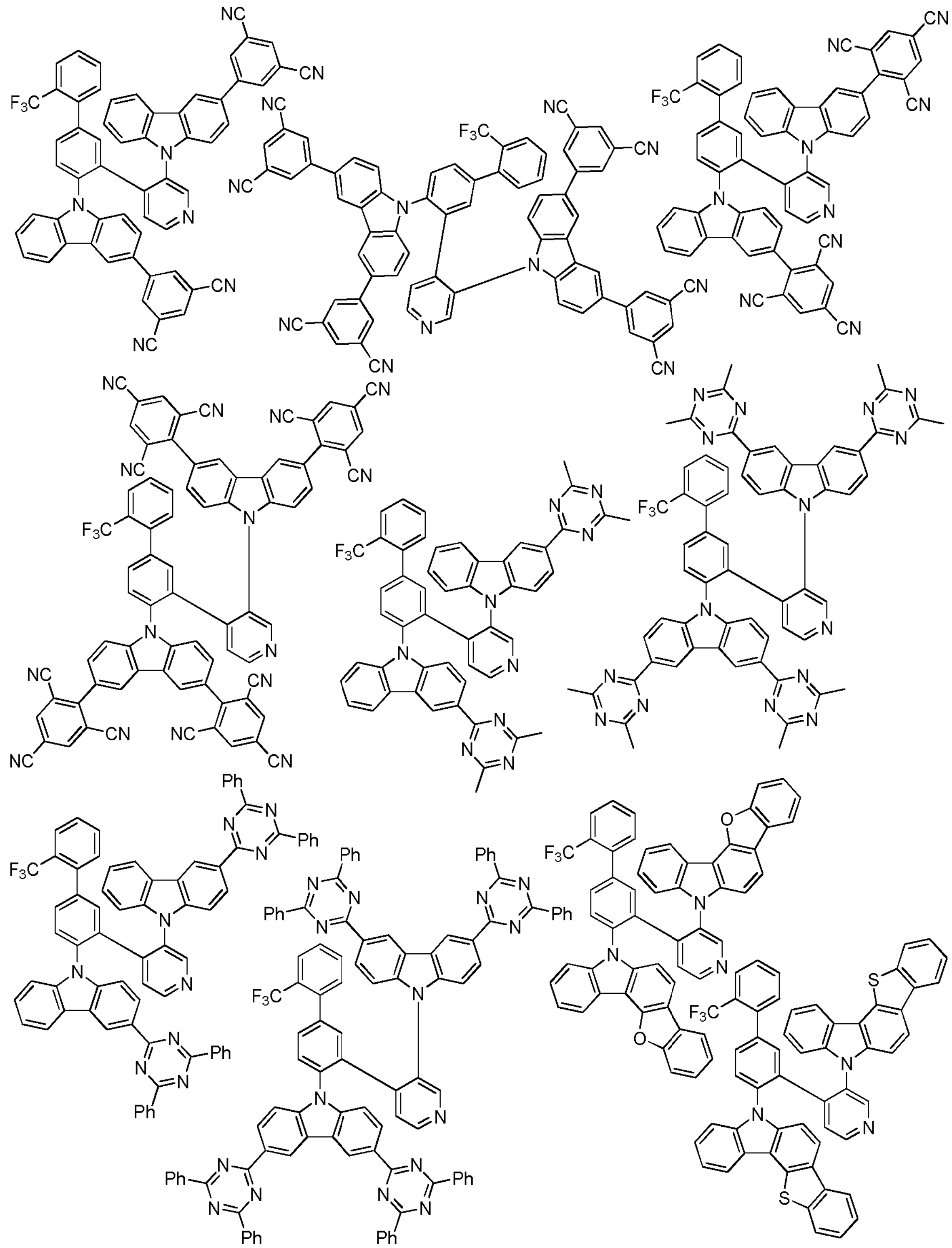

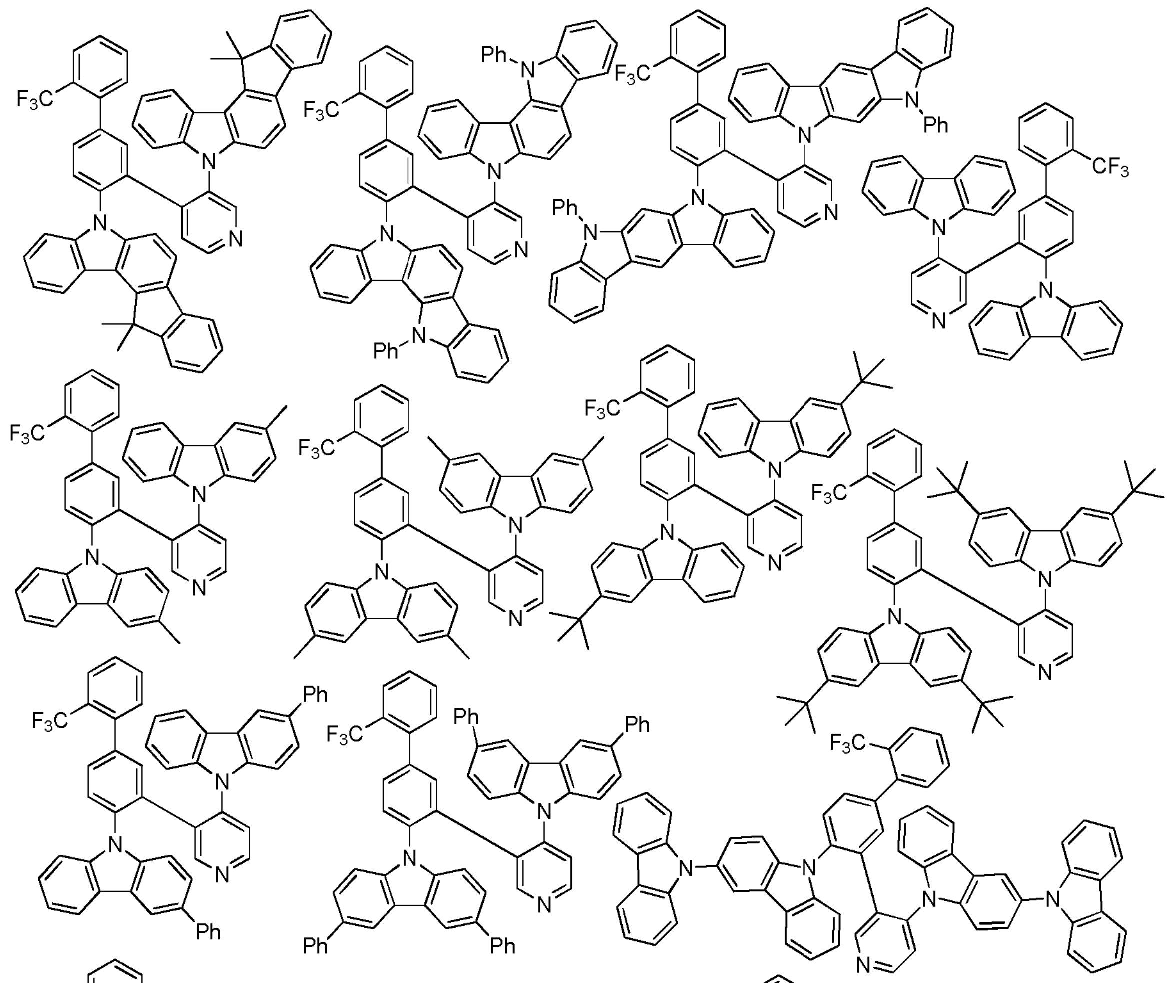

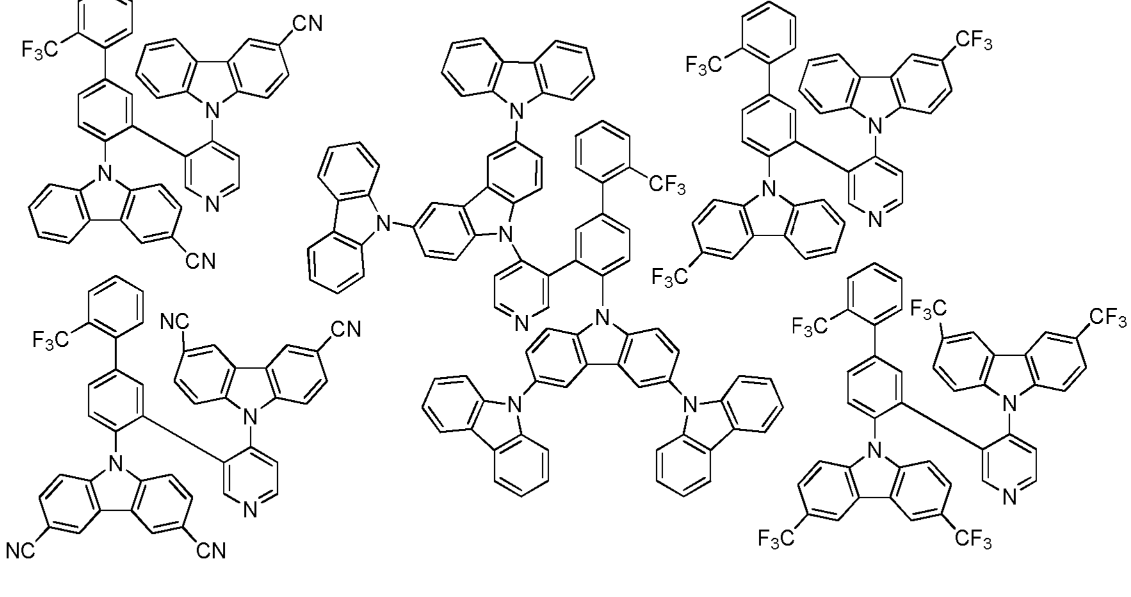

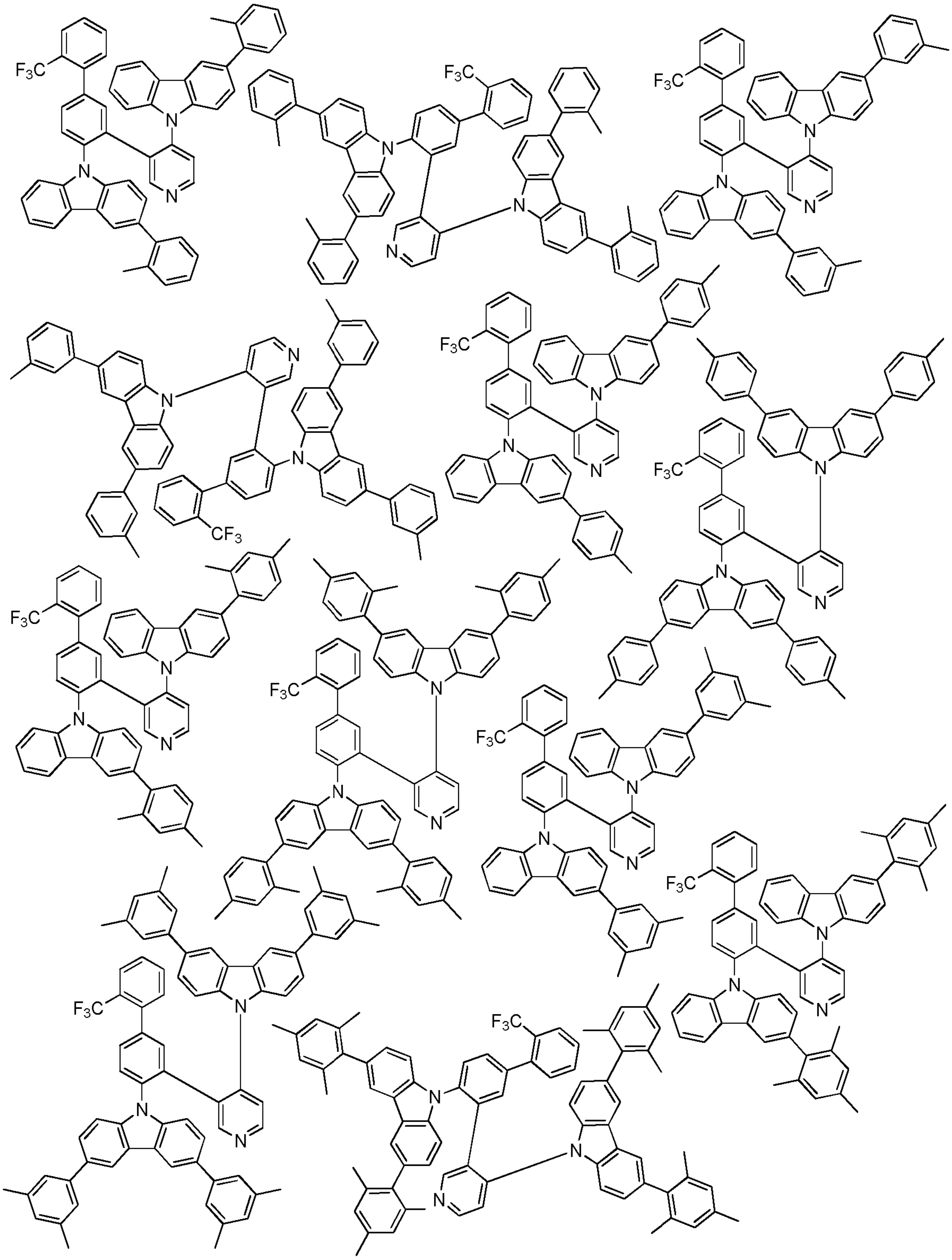

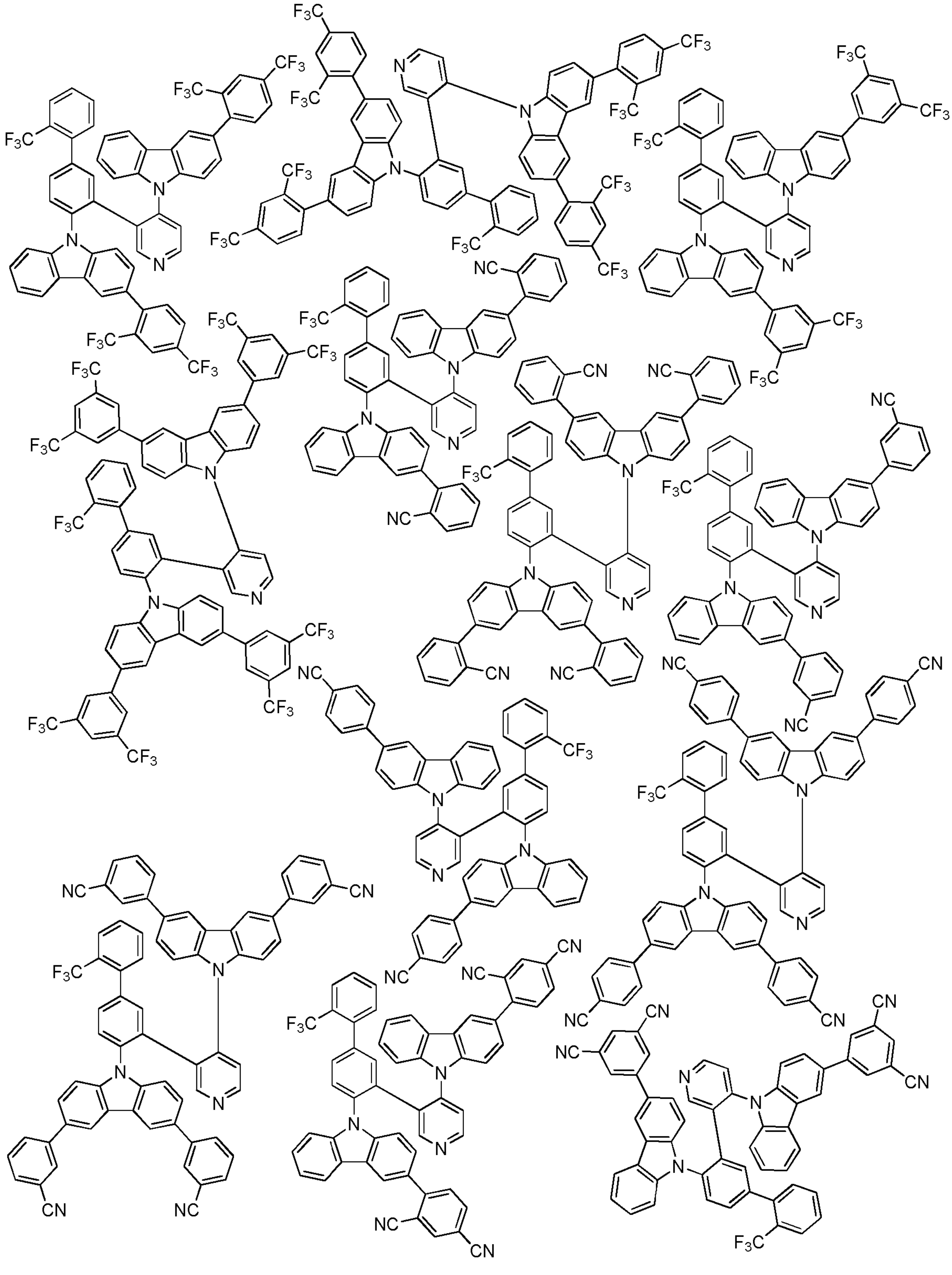

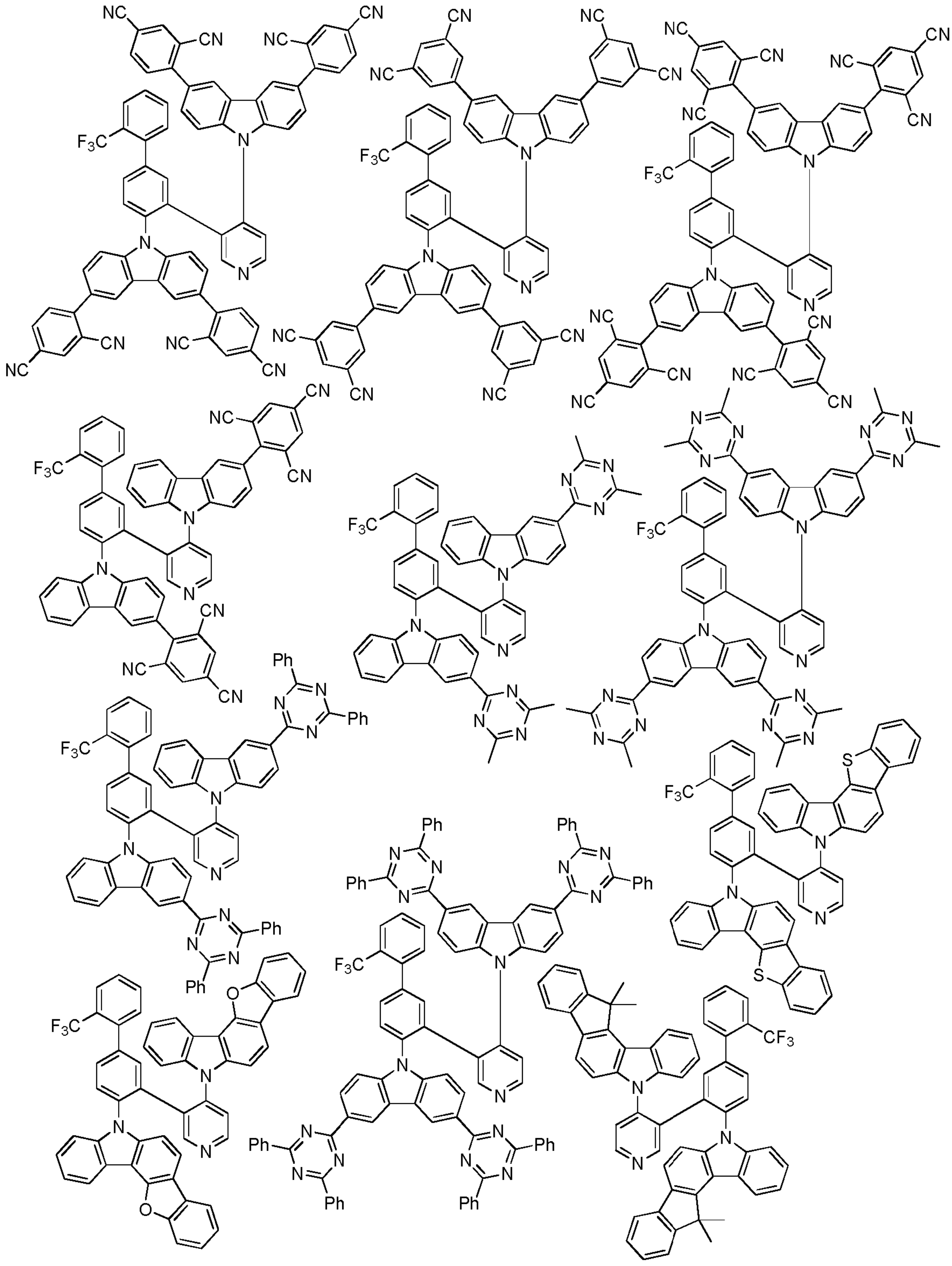

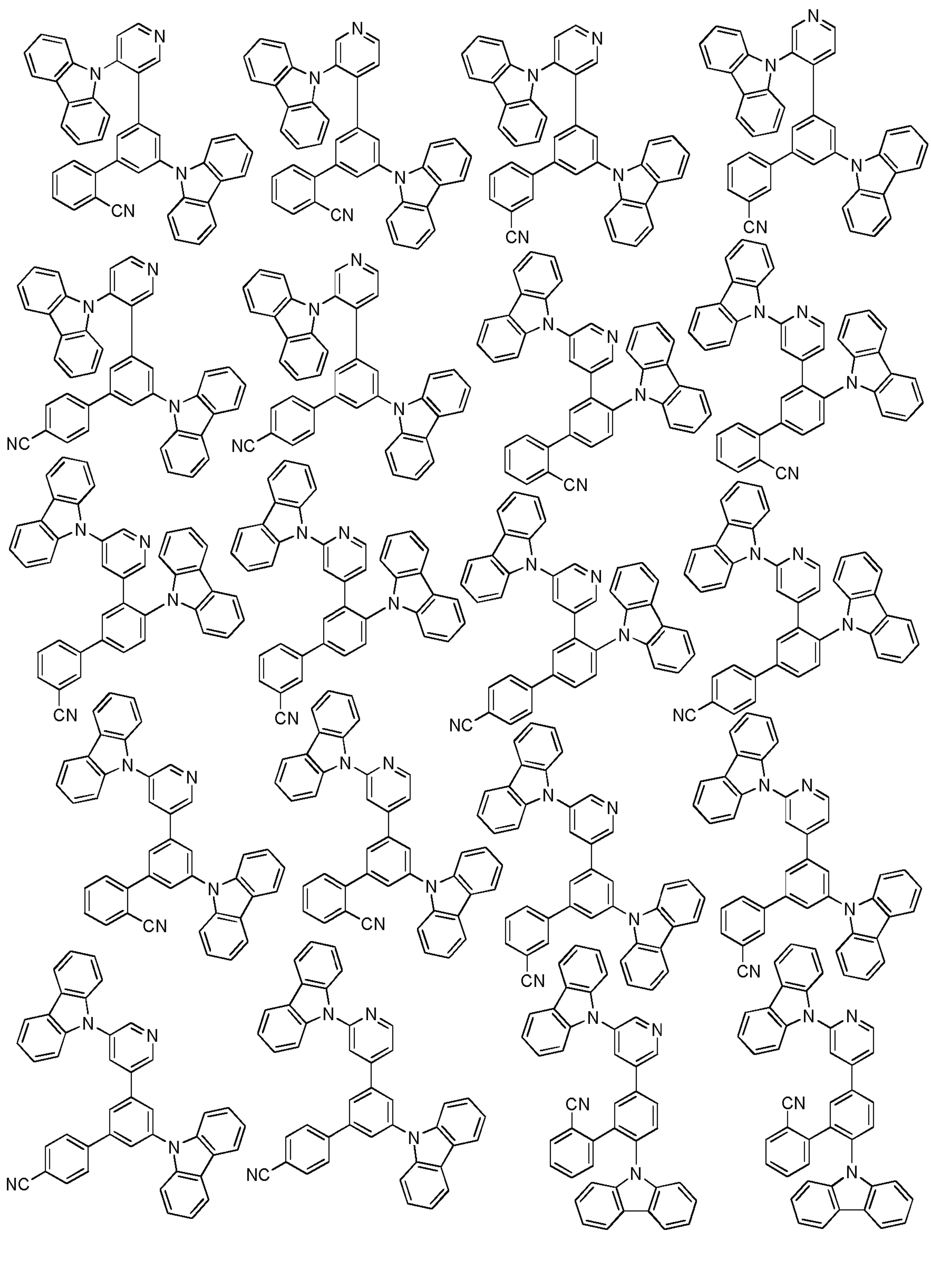

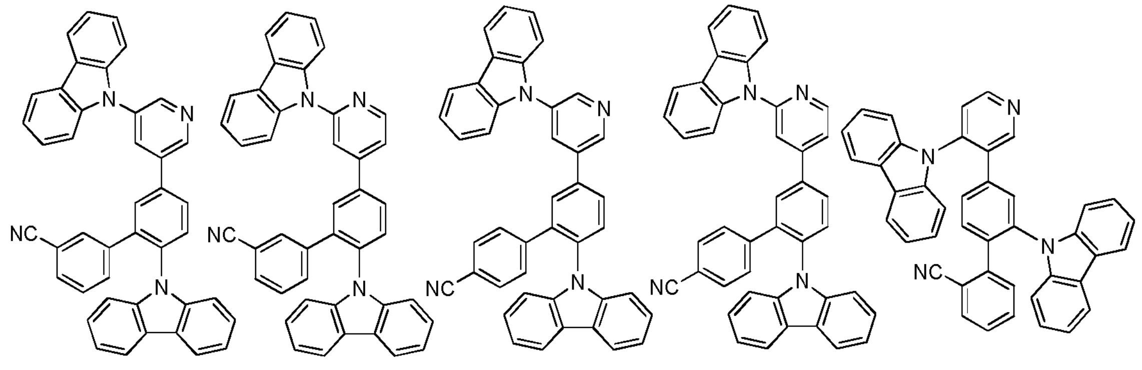

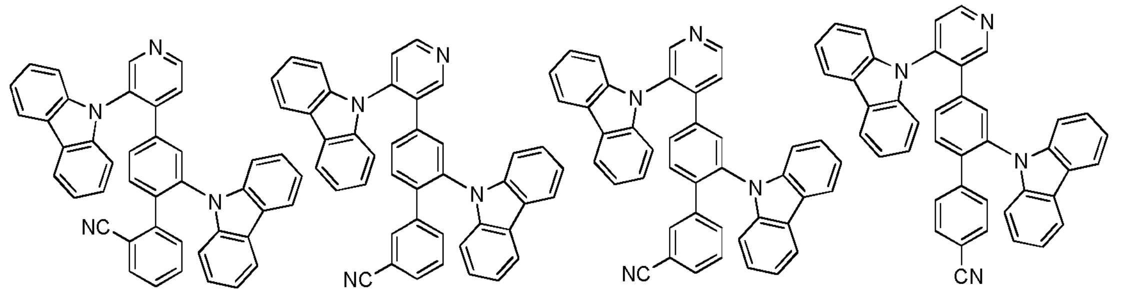

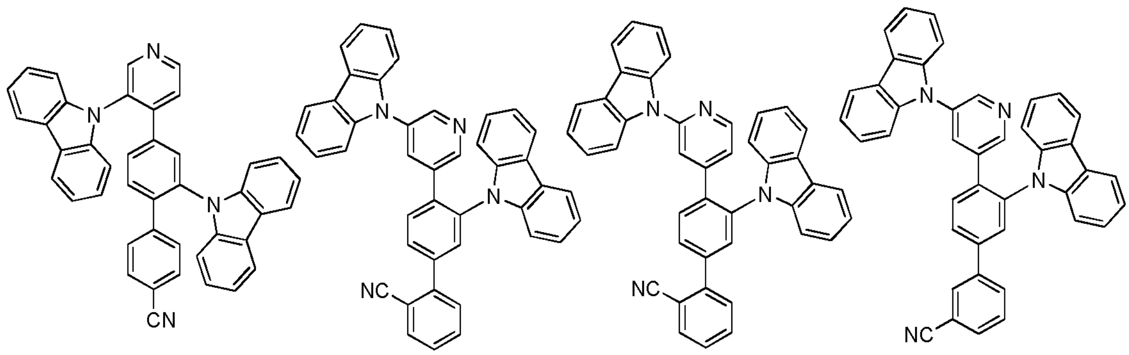

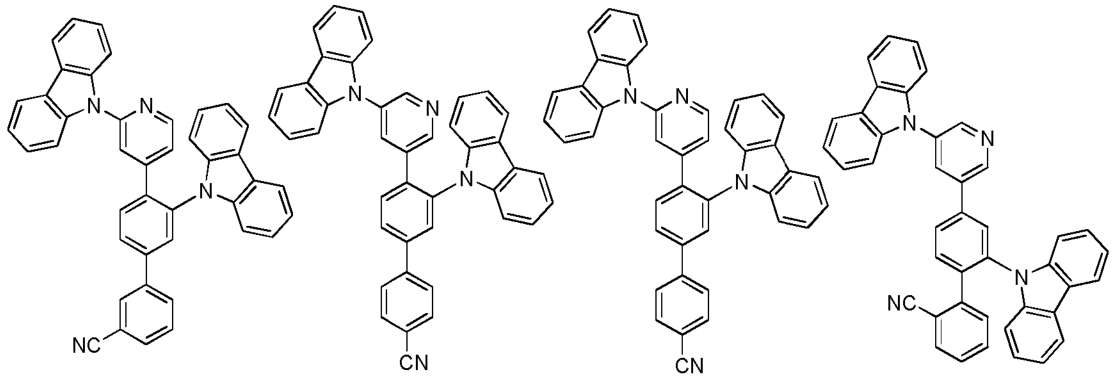

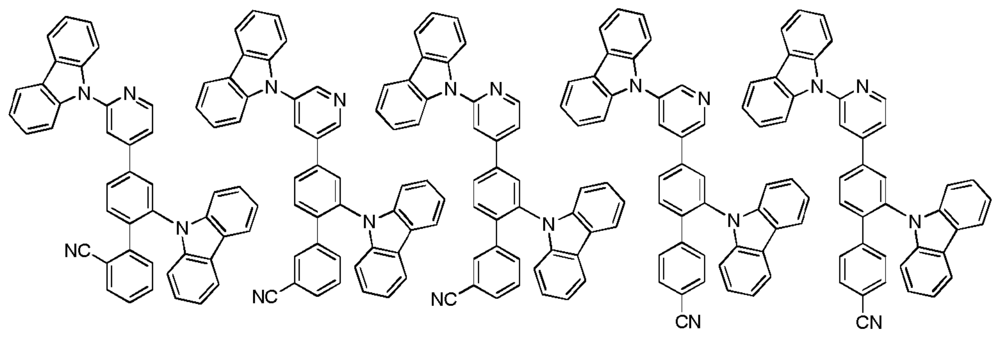

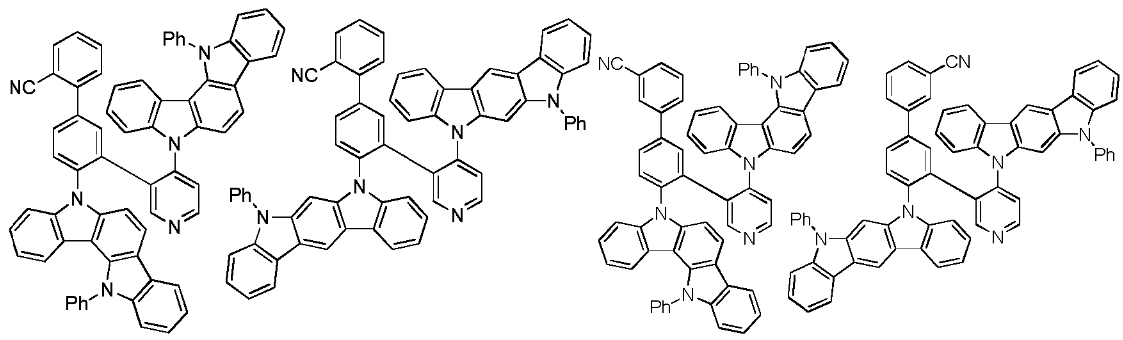

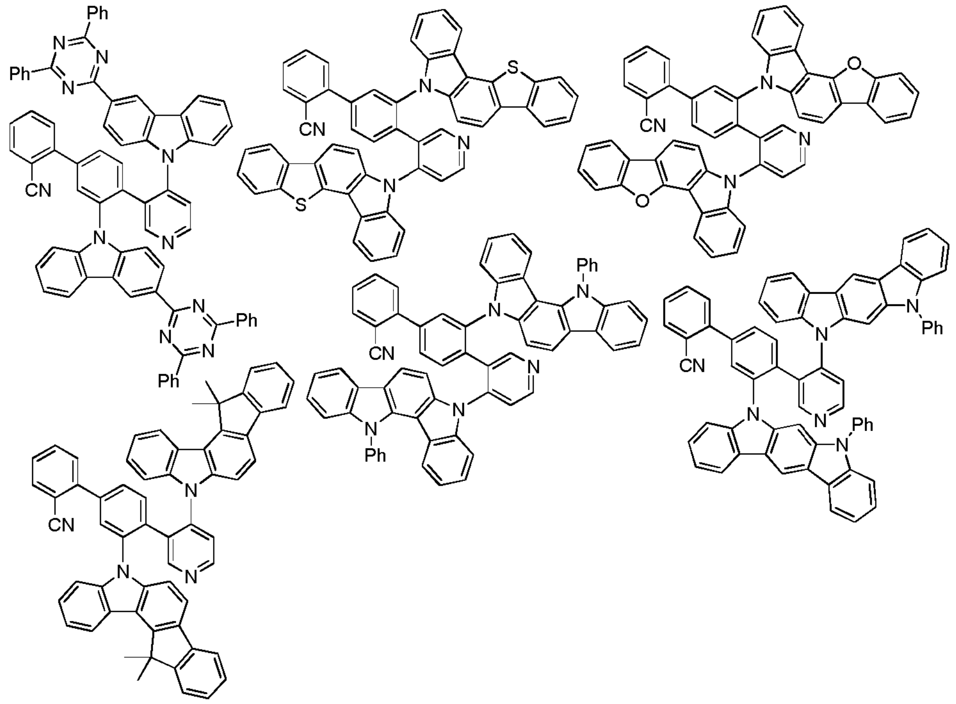

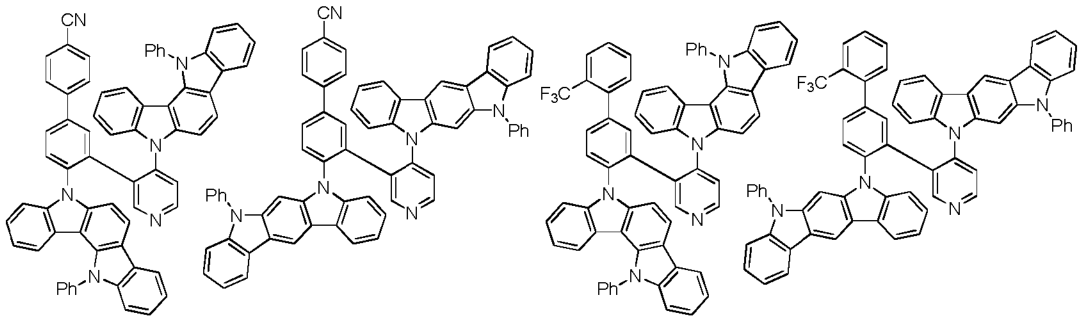

## Figures

The drawings of the figures show:

Figure 1 Emission spectrum of example 1 (10% by weight) in PMMA.
Figure 2 Emission spectrum of example 2 (10% by weight) in PMMA.
Figure 3 Emission spectrum of example 3 (10% by weight) in PMMA.
Figure 4 Emission spectrum of example 4 (10% by weight) in PMMA.
Figure 5 Emission spectrum of example 5 (10% by weight) in PMMA.
Figure 6 Emission spectrum of example 6 (10% by weight) in PMMA.
Figure 7 Emission spectrum of example 7 (10% by weight) in PMMA.

## Claims

**1.** Organic molecule, comprising

- one first chemical moiety comprising a structure of Formula I,

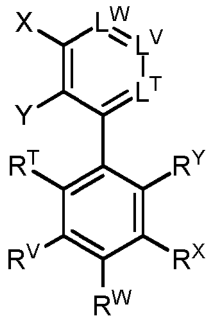

Formula I

and
- two second chemical moieties, each independently from another comprising a structure of Formula II,

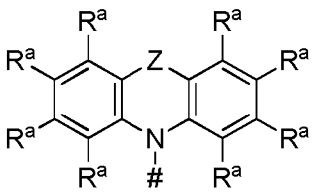

Formula II

wherein the first chemical moiety is linked to each of the two second chemical moieties via a single bond;
wherein
$L^T$ is N or C-$R^1$;
$L^V$ is N or C-$R^1$;
$L^W$ is N or C-W;
W is the binding site of a single bond linking the first chemical moiety to one of the two second chemical moieties or is $R^2$;
X is the binding site of a single bond linking the first chemical moiety to one of the two second chemical moieties or is $R^2$;
Y is the binding site of a single bond linking the first chemical moiety to one of the two second chemical moieties or is $R^2$;
$R^T$ is selected from the group consisting of $R^B$ and $R^I$;
$R^V$ is selected from the group consisting of $R^B$ and $R^I$;
$R^W$ is the binding site of a single bond linking the first chemical moiety to one of the two second chemical moieties or is selected from the group consisting of $R^B$ and $R^I$;
$R^X$ is the binding site of a single bond linking the first chemical moiety to one of the two second chemical moieties or is selected from the group consisting of $R^B$ and $R^I$;
$R^Y$ is the binding site of a single bond linking the first chemical moiety to one of the two second chemical moieties or is selected from the group consisting of $R^B$ and $R^I$;
$R^B$ comprises a structure of Formula BZN:

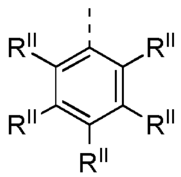

Formula BZN

wherein the dotted line represents the single bond linking the structure of formula BZN to the lower benzene ring of the structure shown in formula I.
# represents the binding site of a single bond linking the second chemical moieties to the first chemical moiety;
Z is at each occurrence independently from another selected from the group consisting of: a direct bond, $CR^3R^4$, $C=CR^3R^4$, C=O, $C=NR^3$, $NR^3$, O, $SiR^3R^4$, S, S(O) and $S(O)_2$;
$R^1$ is at each occurrence independently from another selected from the group consisting of: hydrogen, deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;
$R^2$ is at each occurrence independently from another selected from the group consisting of: hydrogen, deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;
$R^I$ is at each occurrence independently from another selected from the group consisting of:
hydrogen, deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;
$R^{II}$ is at each occurrence independently from another selected from the group consisting of $R^{III}$ and $R^{IV}$;
$R^{III}$ is selected from the group consisting of CN and $CF_3$;
$R^{IV}$ is at each occurrence independently from another selected from the group consisting of:

hydrogen, deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;

$R^a$, $R^3$ and $R^4$ is at each occurrence independently from another selected from the group consisting of: hydrogen, deuterium, $N(R^5)_2$, $OR^5$, $Si(R^5)_3$, $B(OR^5)_2$, $OSO_2R^5$, $CF_3$, CN, F, Br, I, $C_1$-$C_{40}$-alkyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, $C=O$, $C=S$, $C=Se$, $C=NR^5$, $P(=O)(R^5)$, $SO$, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, $C=O$, $C=S$, $C=Se$, $C=NR^5$, $P(=O)(R^5)$, $SO$, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, $C=O$, $C=S$, $C=Se$, $C=NR^5$, $P(=O)(R^5)$, $SO$, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, $C=O$, $C=S$, $C=Se$, $C=NR^5$, $P(=O)(R^5)$, $SO$, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, $C=O$, $C=S$, $C=Se$, $C=NR^5$, $P(=O)(R^5)$, $SO$, $SO_2$, $NR^5$, O, S or $CONR^5$;

$C_6$-$C_{60}$-aryl,
which is optionally substituted with one or more substituents $R^5$;
$C_3$-$C_{57}$-heteroaryl,
which is optionally substituted with one or more substituents $R^5$; and
a mono- or polycyclic, aliphatic, aromatic and/or benzo-fused ring system formed by ring-closure with one or more of the other substituents selected from the group consisting of $R^a$, $R^3$, $R^4$ and $R^5$;
$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I, $C_1$-$C_{40}$-alkyl,

which is optionally substituted with one or more substituents $R^6$ and
wherein one or more non-adjacent $CH_2$-groups are optionally substituted by $R^6C=CR^6$, $C\equiv C$, $Si(R^6)_2$, $Ge(R^6)_2$, $Sn(R^6)_2$, $C=O$, $C=S$, $C=Se$, $C=NR^6$, $P(=O)(R^6)$, $SO$, $SO_2$, $NR^6$, O, S or $CONR^6$;

$C_1$-$C_{40}$-alkoxy,

which is optionally substituted with one or more substituents $R^6$ and
wherein one or more non-adjacent $CH_2$-groups are optionally substituted by $R^6C=CR^6$, $C=C$, $Si(R^6)_2$, $Ge(R^6)_2$, $Sn(R^6)_2$, $C=O$, $C=S$, $C=Se$, $C=NR^6$, $P(=O)(R^6)$, $SO$, $SO_2$, $NR^6$, O, S or $CONR^6$;

$C_1$-$C_{40}$-thioalkoxy,

which is optionally substituted with one or more substituents $R^6$ and
wherein one or more non-adjacent $CH_2$-groups are optionally substituted by $R^6C=CR^6$, $C\equiv C$, $Si(R^6)_2$, $Ge(R^6)_2$, $Sn(R^6)_2$, $C=O$, $C=S$, $C=Se$, $C=NR^6$, $P(=O)(R^6)$, $SO$, $SO_2$, $NR^6$, O, S or $CONR^6$;

$C_2$-$C_{40}$-alkenyl,

which is optionally substituted with one or more substituents $R^6$ and
wherein one or more non-adjacent $CH_2$-groups are optionally substituted by $R^6C=CR^6$, $C\equiv C$, $Si(R^6)_2$,

$Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$; and
a mono- or polycyclic, aliphatic, aromatic and/or benzo-fused ring system formed by ring-closure with one or more of the other substituents selected from the group consisting of $R^a$, $R^3$, $R^4$ and $R^5$;
$R^6$ is at each occurrence independently from another selected from the group consisting of:

hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
C2-C5-alkenyl,
wherein one or more hydrogen atoms are optionally, independently from each other substituted by deuterium, CN, $CF_3$, or F;
C2-C5-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$,
and $N(C_3$-$C_{17}$-heteroaryl$)(C_6$-$C_{18}$-aryl$)$;

wherein optionally, the substituents $R^a$, $R^3$, $R^4$ or $R^5$ independently from each other form a mono- or polycyclic, aliphatic, aromatic and/or benzo-fused ring system with one or more substituents of the other substituents $R^a$, $R^3$, $R^4$ or $R^5$;
wherein the mono- or polycyclic, aliphatic, aromatic and/or benzo-fused ring system is optionally substituted with at least one substituent $R^5$, and
optionally forms a mono- or polycyclic, aliphatic, aromatic and/or benzo-fused ring system together with one or more of the other $R^a$, $R^3$, $R^4$ or $R^5$ within the organic molecule;
wherein the mono- or polycyclic, aliphatic, aromatic and/or benzo-fused ring system is optionally substituted with at least one substituent $R^6$;
wherein
exactly one ring member selected from the group consisting of $L^T$, $L^V$ and $L^W$ is N,
exactly one substituent selected from the group consisting of $R^T$, $R^V$, $R^X$, $R^Y$ and $R^W$ is $R^B$; exactly one substituent $R^{II}$ is $R^{III}$;
exactly one substituent selected from the group consisting of W, Y and X represents the binding site of a single bond linking the first chemical moiety and one of the two second chemical moieties; and
exactly one substituent selected from the group consisting of $R^W$, $R^Y$ and $R^X$ represents the binding site of a single bond linking the first chemical moiety and one of the two second chemical moieties.

**2.** Organic molecule according to claim 1, wherein the first chemical moiety comprises a structure of Formula Iaa:

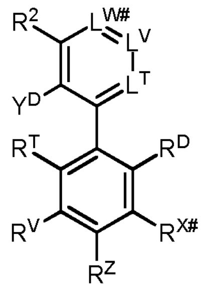

Formula Iaa

wherein

$L^{W\#}$ is N or C-$R^2$;
$R^Z$ is selected from the group consisting of $R^I$ and $R^B$;
$R^{X\#}$ is selected from the group consisting of $R^I$ and $R^B$;
$R^D$ is the binding site of a single bond linking the first chemical moiety to one of the two second chemical moieties;
$Y^D$ is the binding site of a single bond linking the first chemical moiety to one of the two second chemical moieties;
wherein exactly one substituent selected from the group consisting of $L^{W\#}$, $L^V$, $L^T$ is N;
and wherein exactly one substituent selected from the group consisting of $R^V$, $R^T$, $R^{X\#}$ and $R^Z$ is $R^B$.

**3.** Organic molecule according to claim 1 or 2, wherein $R^1$, $R^2$, $R^I$ and $R^{IV}$ is at each occurrence independently from each other selected from the group consisting of: H, methyl, mesityl, tolyl, and phenyl.

**4.** Organic molecule according to one or more of claims 1 to 3, wherein $R^1$, $R^2$, $R^I$ and $R^{IV}$ is H.

**5.** Organic molecule according to one or more of claims 1 to 4, wherein the two second chemical moieties, each at each occurrence independently from another, comprise a structure of Formula IIa:

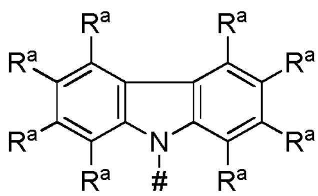

Formula IIa

**6.** Organic molecule according to one or more of claims 1 to 5, wherein the two second chemical moieties, at each occurrence independently from another, comprise a structure of Formula IIb:

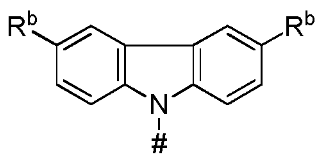

Formula IIb

wherein
$R^b$ is at each occurrence independently from another selected from the group consisting of:

deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.

**7.** Organic molecule according to one or more of claims 1 to 5, wherein the two second chemical moieties, each at each occurrence independently from another comprise a structure of Formula IIc:

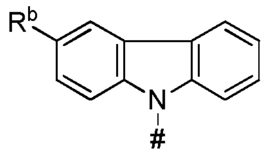

Formula IIc

wherein

$R^b$ is at each occurrence independently from another selected from the group consisting of deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and wherein one or more non-adjacent $CH_2$-groups are optionally substituted by $R^5C=CR^5$, $C\equiv C$, $Si(R^5)_2$,

$Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$.

**8.** Organic molecule according to claim 6 or 7, wherein $R^b$ is at each occurrence independently from another selected from the group consisting of:

- Me, $^i$Pr, $^t$Bu, CN, $CF_3$,
- Ph, which is optionally substituted with one or more substituents independently from each other selected from the group consisting of Me, $^i$Pr, $^t$Bu, CN, $CF_3$ and Ph;
- pyridinyl, which is optionally substituted with one or more substituents independently from each other selected from the group consisting of Me, $^i$Pr, $^t$Bu, CN, $CF_3$ and Ph;
- pyrimidyl, which is optionally substituted with one or more substituents independently from each other selected from the group consisting of Me, $^i$Pr, $^t$Bu, CN, $CF_3$ and Ph;
- carbazolyl, which is optionally substituted with one or more substituents independently from each other selected from the group consisting of Me, $^i$Pr, $^t$Bu, CN, $CF_3$ and Ph;
- triazinyl, which is optionally substituted with one or more substituents independently from each other selected from the group consisting of Me, $^i$Pr, $^t$Bu, CN, $CF_3$, and Ph;

and
- $N(Ph)_2$.

**9.** Method for preparing an organic molecule according to claims 1 to 8, comprising providing a $R^1$-, $R^2$-substituted $Hal^a$-fluoropyridine as a reactant, wherein $Hal^a$ is selected from the group consisting of Cl, Br and I.

**10.** Use of an organic molecule according to one or more of claims 1 to 8 as a luminescent emitter and/or as a host material and/or as an electron transport material and/or as a hole injection material and/or as a hole blocking material in an optoelectronic device.

**11.** Use according to claim 10, wherein the optoelectronic device is selected from the group consisting of:

• organic light-emitting diodes (OLEDs),
• light-emitting electrochemical cells,
• OLED-sensors,
• organic diodes,
• organic solar cells,
• organic transistors,
• organic field-effect transistors,

• organic lasers, and
• down-conversion elements.

**12.** Composition, comprising:

(a) an organic molecule according to one or more of claims 1 to 8, in particular in the form of an emitter and/or a host, and
(b) an emitter and/or host material, which differs from the organic molecule, and
(c) optionally, a dye and/or a solvent.

**13.** Optoelectronic device, comprising an organic molecule according to one or more of claims 1 to 8 or a composition according to claim 12, in particular in the form of a device selected from the group consisting of: organic light-emitting diode (OLED), light-emitting electrochemical cell, OLED-sensor, organic diode, organic solar cell, organic transistor, organic field-effect transistor, organic laser, and down-conversion element.

**14.** Optoelectronic device according to claim 13, comprising or consisting of:

- a substrate,
- an anode, and
- a cathode, wherein the anode or the cathode are disposed on the substrate, and
- at least a light-emitting layer, which is arranged between the anode and the cathode and which comprises the organic molecule or the composition.

**15.** Method for producing an optoelectronic device, wherein an organic molecule according to any one of claims 1 to 8 or a composition according to claim 12 is used, in particular comprising processing the organic molecule or the composition by a vacuum evaporation method or from a solution.

**Patentansprüche**

**1.** Organisches Molekül, umfassend

- eine erste chemische Komponente, die eine Struktur der Formel I umfasst,

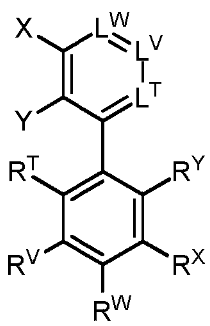

Formel I

und
- zwei zweite chemische Komponenten, die jeweils unabhängig voneinander eine Struktur der Formel II umfassen,

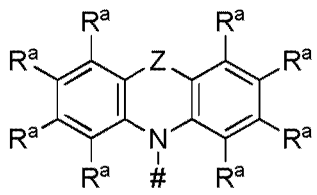

Formel II

wobei die erste chemische Komponente mit jeder der zwei zweiten chemischen Komponenten über eine Einfachbindung verbunden ist;
wobei
$L^T$ N oder C-$R^1$ ist;
$L^V$ N oder C-$R^1$ ist;
$L^W$ N oder C-W ist;
W die Bindungsstelle einer Einfachbindung ist, die die erste chemische Komponente mit einer der zwei zweiten chemischen Komponenten verbindet, oder $R^2$ ist;
X die Bindungsstelle einer Einfachbindung ist, die die erste chemische Komponente mit einer der zwei zweiten chemischen Komponenten verbindet, oder $R^2$ ist;
Y die Bindungsstelle einer Einfachbindung ist, die die erste chemische Komponente mit einer der zwei zweiten chemischen Komponenten verbindet, oder $R^2$ ist;
$R^T$ aus der Gruppe bestehend aus $R^B$ und $R^I$ ausgewählt ist;
$R^V$ aus der Gruppe bestehend aus $R^B$ und $R^I$ ausgewählt ist;
$R^W$ die Bindungsstelle einer Einfachbindung ist, die die erste chemische Komponente mit einer der zwei zweiten chemischen Komponenten verbindet, oder aus der Gruppe bestehend aus $R^B$ und $R^I$ ausgewählt ist;
$R^X$ die Bindungsstelle einer Einfachbindung ist, die die erste chemische Komponente mit einer der zwei zweiten chemischen Komponenten verbindet, oder aus der Gruppe bestehend aus $R^B$ und $R^I$ ausgewählt ist;
$R^Y$ die Bindungsstelle einer Einfachbindung ist, die die erste chemische Komponente mit einer der zwei zweiten chemischen Komponenten verbindet, oder aus der Gruppe bestehend aus $R^B$ und $R^I$ ausgewählt ist;
$R^B$ eine Struktur der Formel BZN umfasst:

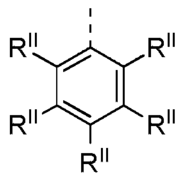

Formel BZN

wobei die gestrichelte Linie die Einfachbindung darstellt, die die Struktur der Formel BZN mit dem unteren Benzolring der in Formel I dargestellten Struktur verbindet;
# die Bindungsstelle einer Einfachbindung darstellt, die die zweiten chemischen Komponenten mit der ersten chemischen Komponente verbindet;
Z bei jedem Auftreten unabhängig voneinander ausgewählt ist aus der Gruppe bestehend aus: einer direkten Bindung, $CR^3R^4$, $C{=}CR^3R^4$, C=O, $C{=}NR^3$, $NR^3$, O, $SiR^3R^4$, S, S(O) und $S(O)_2$;
$R^1$ bei jedem Auftreten unabhängig voneinander ausgewählt ist aus der Gruppe bestehend aus:
Wasserstoff, Deuterium,
$C_1$-$C_5$-Alkyl,
wobei ein oder mehrere Wasserstoffatome optional substituiert sind durch Deuterium;
$C_2$-$C_8$-Alkenyl,
wobei ein oder mehrere Wasserstoffatome optional substituiert sind durch Deuterium;
$C_2$-$C_8$-Alkinyl,
wobei ein oder mehrere Wasserstoffatome optional substituiert sind durch Deuterium; und
$C_6$-$C_{18}$-Aryl,

das optional substituiert ist mit einem oder mehreren Substituenten $R^6$;
$R^2$ bei jedem Auftreten unabhängig voneinander ausgewählt ist aus der Gruppe bestehend aus:

Wasserstoff, Deuterium,
$C_1$-$C_5$-Alkyl,
wobei ein oder mehrere Wasserstoffatome optional substituiert sind durch Deuterium;
$C_2$-$C_8$-Alkenyl,
wobei ein oder mehrere Wasserstoffatome optional substituiert sind durch Deuterium;
$C_2$-$C_8$-Alkinyl,
wobei ein oder mehrere Wasserstoffatome optional substituiert sind durch Deuterium; und
$C_6$-$C_{18}$-Aryl,
das optional substituiert ist mit einem oder mehreren Substituenten $R^6$;

$R^I$ bei jedem Auftreten unabhängig voneinander ausgewählt ist aus der Gruppe bestehend aus:

Wasserstoff, Deuterium,
$C_1$-$C_5$-Alkyl,
wobei ein oder mehrere Wasserstoffatome optional substituiert sind durch Deuterium;
$C_2$-$C_8$-Alkenyl,
wobei ein oder mehrere Wasserstoffatome optional substituiert sind durch Deuterium;
$C_2$-$C_8$-Alkinyl,
wobei ein oder mehrere Wasserstoffatome optional substituiert sind durch Deuterium; und
$C_6$-$C_{18}$-Aryl,
das optional substituiert ist mit einem oder mehreren Substituenten $R^6$;
$R^{II}$ bei jedem Auftreten unabhängig voneinander ausgewählt ist aus der Gruppe bestehend aus $R^{III}$ und $R^{IV}$;
$R^{III}$ aus der Gruppe bestehend aus CN und $CF_3$ ausgewählt ist;

$R^{IV}$ bei jedem Auftreten unabhängig voneinander ausgewählt ist aus der Gruppe bestehend aus:

Wasserstoff, Deuterium,
$C_1$-$C_5$-Alkyl,
wobei ein oder mehrere Wasserstoffatome optional substituiert sind durch Deuterium;
$C_2$-$C_8$-Alkenyl,
wobei ein oder mehrere Wasserstoffatome optional substituiert sind durch Deuterium;
$C_2$-$C_8$-Alkinyl,
wobei ein oder mehrere Wasserstoffatome optional substituiert sind durch Deuterium; und
$C_6$-$C_{18}$-Aryl,
das optional substituiert ist mit einem oder mehreren Substituenten $R^6$;

$R^a$, $R^3$ und $R^4$ bei jedem Auftreten jeweils unabhängig voneinander ausgewählt ist aus der Gruppe bestehend aus: Wasserstoff, Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional substituiert ist mit einem oder mehreren Substituenten $R^5$ und wobei eine oder mehrere nicht benachbarte $CH_2$-Gruppen optional substituiert sind durch $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S oder $CONR^5$;
$C_1$-$C_{40}$-Alkoxy,
das optional substituiert ist mit einem oder mehreren Substituenten $R^5$ und
wobei eine oder mehrere nicht benachbarte $CH_2$-Gruppen optional substituiert sind durch $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S oder $CONR^5$;
$C_1$-$C_{40}$- Thioalkoxy,
das optional substituiert ist mit einem oder mehreren Substituenten $R^5$ und
wobei eine oder mehrere nicht benachbarte $CH_2$-Gruppen optional substituiert sind durch $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S oder $CONR^5$;
$C_2$-$C_{40}$-Alkenyl,
das optional substituiert ist mit einem oder mehreren Substituenten $R^5$ und
wobei eine oder mehrere nicht benachbarte $CH_2$-Gruppen optional substituiert sind durch $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S oder $CONR^5$;
$C_2$-$C_{40}$-Alkinyl,

das optional substituiert ist mit einem oder mehreren Substituenten $R^5$ und
wobei eine oder mehrere nicht benachbarte $CH_2$-Gruppen optional substituiert sind durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$;
$C_6$-$C_{60}$-Aryl,
das optional substituiert ist mit einem oder mehreren Substituenten $R^5$;
$C_3$-$C_{57}$-Heteroaryl,
das optional substituiert ist mit einem oder mehreren Substituenten $R^5$; und
einem mono- oder polycyclischen, aliphatischen, aromatischen und/oder benzokondensierten Ringsystem, gebildet durch Ringschluss mit einem oder mehreren der anderen Substituenten, ausgewählt aus der Gruppe bestehend aus $R^a$, $R^3$, $R^4$ und $R^5$;
$R^5$ bei jedem Auftreten unabhängig voneinander ausgewählt ist aus der Gruppe bestehend aus
Wasserstoff, Deuterium, $N(R^6)_2$, $OR^6$, $Si(R^6)_3$, $B(OR^6)_2$, $OSO_2R^6$, $CF_3$, CN, F, Br, I, $C_1$-$C_{40}$-Alkyl,
das optional substituiert ist mit einem oder mehreren Substituenten $R^6$ und
wobei eine oder mehrere nicht benachbarte $CH_2$-Gruppen optional substituiert sind durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;
$C_1$-$C_{40}$-Alkoxy,
das optional substituiert ist mit einem oder mehreren Substituenten $R^6$ und
wobei eine oder mehrere nicht benachbarte $CH_2$-Gruppen optional substituiert sind durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;
$C_1$-$C_{40}$- Thioalkoxy,
das optional substituiert ist mit einem oder mehreren Substituenten $R^6$ und
wobei eine oder mehrere nicht benachbarte $CH_2$-Gruppen optional substituiert sind durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;
$C_2$-$C_{40}$-Alkenyl,
das optional substituiert ist mit einem oder mehreren Substituenten $R^6$ und
wobei eine oder mehrere nicht benachbarte $CH_2$-Gruppen optional substituiert sind durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;
$C_2$-$C_{40}$-Alkinyl,
das optional substituiert ist mit einem oder mehreren Substituenten $R^6$ und
wobei eine oder mehrere nicht benachbarte $CH_2$-Gruppen optional substituiert sind durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;
$C_6$-$C_{60}$-Aryl,
das optional substituiert ist mit einem oder mehreren Substituenten $R^6$; und
$C_3$-$C_{57}$-Heteroaryl,
das optional substituiert ist mit einem oder mehreren Substituenten $R^6$; und
einem mono- oder polycyclischen, aliphatischen, aromatischen und/oder benzokondensierten Ringsystem, gebildet durch Ringschluss mit einem oder
mehreren der anderen Substituenten, ausgewählt aus der Gruppe bestehend aus $R^a$, $R^3$, $R^4$ und $R^5$;
$R^6$ bei jedem Auftreten unabhängig voneinander ausgewählt ist aus der Gruppe bestehend aus:

Wasserstoff, Deuterium, OPh, $CF_3$, CN, F,
$C_1$-$C_5$-Alkyl,
wobei optional ein oder mehrere Wasserstoffatome unabhängig voneinander substituiert sind durch Deuterium, CN, $CF_3$ oder F;
$C_1$-$C_5$-Alkoxy,
wobei optional ein oder mehrere Wasserstoffatome unabhängig voneinander substituiert sind durch Deuterium, CN, $CF_3$ oder F;
$C_1$-$C_5$- Thioalkoxy,
wobei optional ein oder mehrere Wasserstoffatome unabhängig voneinander substituiert sind durch Deuterium, CN, $CF_3$ oder F;
$C_2$-$C_5$-Alkenyl,
wobei optional ein oder mehrere Wasserstoffatome unabhängig voneinander substituiert sind durch Deuterium, CN, $CF_3$ oder F;
$C_2$-$C_5$-Alkinyl,
wobei optional ein oder mehrere Wasserstoffatome unabhängig voneinander substituiert sind durch Deuterium, CN, $CF_3$ oder F;
$C_6$-$C_{18}$-Aryl,
das optional substituiert ist mit einem oder mehreren $C_1$-$C_5$-Alkylsubstituenten;

$C_3$-$C_{17}$-Heteroaryl,
das optional substituiert ist mit einem oder mehreren $C_1$-$C_5$-Alkylsubstituenten;
$N(C_6$-$C_{18}$-$Aryl)_2$;
$N(C_3$-$C_{17}$- $Heteroaryl)_2$,
und $N(C_3$-$C_{17}$-Heteroaryl)($C_6$-$C_{18}$-aryl);
wobei optional die Substituenten $R^a$, $R^3$, $R^4$ oder $R^5$ unabhängig voneinander ein mono- oder polycyclisches, aliphatisches, aromatisches und/oder benzokondensiertes Ringsystem mit einem oder mehreren Substituenten der anderen Substituenten $R^a$, $R^3$, $R^4$ oder $R^5$ bilden.
wobei das mono- oder polycyclische, aliphatische, aromatische und/oder benzokondensierte Ringsystem optional mit mindestens einem Substituenten $R^5$ substituiert ist, und
optional ein mono- oder polycyclisches, aliphatisches, aromatisches und/oder benzokondensiertes Ringsystem zusammen mit einem oder mehreren der anderen $R^a$, $R^3$, $R^4$ oder $R^5$ innerhalb des organischen Moleküls bildet;
wobei das mono- oder polycyclische, aliphatische, aromatische und/oder benzokondensierte Ringsystem optional mit mindestens einem Substituenten $R^6$ substituiert ist;
wobei
genau ein Ringglied, ausgewählt aus der Gruppe bestehend aus $L^T$, $L^V$ und $L^W$, N ist,
genau ein Substituent, ausgewählt aus der Gruppe bestehend aus $R^T$, $R^V$, $R^X$, $R^Y$ und $R^W$, $R^B$ ist;
genau ein Substituent $R^{II}$ $R^{III}$ ist;
genau ein Substituent, ausgewählt aus der Gruppe bestehend aus W, Y und X, die Bindungsstelle einer Einfachbindung darstellt, die die erste chemische Komponente und eine der zwei zweiten chemischen Komponenten verbindet; und
genau ein Substituent, ausgewählt aus der Gruppe bestehend aus $R^W$, $R^Y$ und $R^X$, die Bindungsstelle einer Einfachbindung darstellt, die die erste chemische Komponente und eine der zwei zweiten chemischen Komponenten verbindet.

2. Organisches Molekül nach Anspruch 1, wobei die erste chemische Komponente eine Struktur der Formel Iaa umfasst:

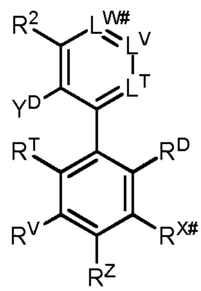

Formel Iaa

wobei

wobei $L^{W\#}$ N oder C-$R^2$ ist;
$R^Z$ aus der Gruppe bestehend aus $R^I$ und $R^B$ ausgewählt ist;
$R^{X\#}$ aus der Gruppe bestehend aus $R^I$ und $R^B$ ausgewählt ist;
$R^D$ die Bindungsstelle einer Einfachbindung ist, die die erste chemische Komponente mit einer der zwei zweiten chemischen Komponenten verbindet;
$Y^D$ die Bindungsstelle einer Einfachbindung ist, die die erste chemische Komponente mit einer der zwei zweiten chemischen Komponenten verbindet;
wobei genau ein Substituent, ausgewählt aus der Gruppe bestehend aus $L^{W\#}$, $L^V$ und $L^T$, N ist;
und wobei genau ein Substituent, ausgewählt aus der Gruppe bestehend aus $R^V$, $R^T$, $R^{X\#}$ und $R^Z$, $R^B$ ist.

3. Organisches Molekül nach Anspruch 1 oder 2, wobei $R^1$, $R^2$, $R^I$ und $R^{IV}$ bei jedem Auftreten unabhängig voneinander ausgewählt ist aus der Gruppe bestehend aus: H, Methyl, Mesityl, Tolyl, und Phenyl.

4. Organisches Molekül nach einem oder mehreren der Ansprüche 1 bis 3, wobei $R^1$, $R^2$, $R^I$ und $R^{IV}$ H ist.

**5.** Organisches Molekül nach einem oder mehreren der Ansprüche 1 bis 4, wobei die zwei zweiten chemischen Komponenten bei jedem Auftreten unabhängig voneinander jeweils eine Struktur der Formel IIa umfassen:

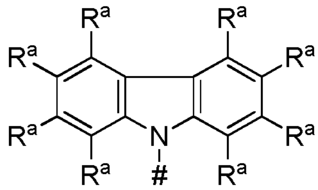

Formel IIa

**6.** Organisches Molekül nach einem oder mehreren der Ansprüche 1 bis 5, wobei die zwei zweiten chemischen Komponenten bei jedem Auftreten unabhängig voneinander eine Struktur der Formel IIb umfassen:

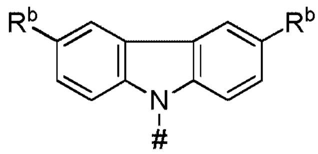

Formel IIb

wobei
$R^b$ bei jedem Auftreten unabhängig voneinander ausgewählt ist aus der Gruppe bestehend aus:

Deuterium, $N(R^5)_2$, $OR^5$, $Si(R^5)_3$, $B(OR^5)_2$, $OSO_2R^5$, $CF_3$, CN, F, Br, I, $C_1$-$C_{40}$-Alkyl,
das optional substituiert ist mit einem oder mehreren Substituenten $R^5$ und
wobei eine oder mehrere nicht benachbarte $CH_2$-Gruppen optional substituiert sind durch $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$;
$C_1$-$C_{40}$-Alkoxy,
das optional substituiert ist mit einem oder mehreren Substituenten $R^5$ und
wobei eine oder mehrere nicht benachbarte $CH_2$-Gruppen optional substituiert sind durch $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$;
$C_1$-$C_{40}$- Thioalkoxy,
das optional substituiert ist mit einem oder mehreren Substituenten $R^5$ und
wobei eine oder mehrere nicht benachbarte $CH_2$-Gruppen optional substituiert sind durch $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$;
$C_2$-$C_{40}$-Alkenyl,
das optional substituiert ist mit einem oder mehreren Substituenten $R^5$ und
wobei eine oder mehrere nicht benachbarte $CH_2$-Gruppen optional substituiert sind durch $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$;
$C_2$-$C_{40}$-Alkinyl,
das optional substituiert ist mit einem oder mehreren Substituenten $R^5$ und
wobei eine oder mehrere nicht benachbarte $CH_2$-Gruppen optional substituiert sind durch $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$;
$C_6$-$C_{60}$-Aryl,
das optional substituiert ist mit einem oder mehreren Substituenten $R^5$; und
$C_3$-$C_{57}$-Heteroaryl,
das optional substituiert ist mit einem oder mehreren Substituenten $R^5$.

**7.** Organisches Molekül nach einem oder mehreren der Ansprüche 1 bis 5, wobei die zwei zweiten chemischen Komponente bei jedem Auftreten unabhängig voneinander jeweils eine Struktur der Formel IIc umfassen:

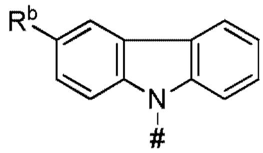

Formel IIc

wobei

$R^b$ bei jedem Auftreten unabhängig voneinander ausgewählt ist aus der Gruppe bestehend aus
Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional substituiert ist mit einem oder mehreren Substituenten $R^5$ und
wobei eine oder mehrere nicht benachbarte $CH_2$-Gruppen optional substituiert sind durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$;
$C_1$-$C_{40}$-Alkoxy,
das optional substituiert ist mit einem oder mehreren Substituenten $R^5$ und
wobei eine oder mehrere nicht benachbarte $CH_2$-Gruppen optional substituiert sind durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$;
$C_1$-$C_{40}$- Thioalkoxy,
das optional substituiert ist mit einem oder mehreren Substituenten $R^5$ und
wobei eine oder mehrere nicht benachbarte $CH_2$-Gruppen optional substituiert sind durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$;
$C_2$-$C_{40}$-Alkenyl,
das optional substituiert ist mit einem oder mehreren Substituenten $R^5$ und
wobei eine oder mehrere nicht benachbarte $CH_2$-Gruppen optional substituiert sind durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$;
$C_2$-$C_{40}$-Alkinyl,
das optional substituiert ist mit einem oder mehreren Substituenten $R^5$ und
wobei eine oder mehrere nicht benachbarte $CH_2$-Gruppen optional substituiert sind durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$;
$C_6$-$C_{60}$-Aryl,
das optional substituiert ist mit einem oder mehreren Substituenten $R^5$; und
$C_3$-$C_{57}$-Heteroaryl,
das optional substituiert ist mit einem oder mehreren Substituenten $R^5$.

**8.** Organisches Molekül nach Anspruch 6 oder 7, wobei $R^b$ bei jedem Auftreten unabhängig voneinander ausgewählt ist aus der Gruppe bestehend aus:

- Me, $^i$Pr, $^t$Bu, CN, $CF_3$,
- Ph, das optional substituiert ist mit einem oder mehreren Substituenten, unabhängig voneinander ausgewählt aus der Gruppe bestehend aus Me, $^i$Pr, $^t$Bu, CN, $CF_3$ und Ph;
- Pyridinyl, das optional substituiert ist mit einem oder mehreren Substituenten, unabhängig voneinander ausgewählt aus der Gruppe bestehend aus Me, $^i$Pr, $^t$Bu, CN, $CF_3$ und Ph;
- Pyrimidyl, das optional substituiert ist mit einem oder mehreren Substituenten, unabhängig voneinander ausgewählt aus der Gruppe bestehend aus Me, $^i$Pr, $^t$Bu, CN, $CF_3$ und Ph;
- Carbazolyl, das optional substituiert ist mit einem oder mehreren Substituenten, unabhängig voneinander ausgewählt aus der Gruppe bestehend aus Me, $^i$Pr, $^t$Bu, CN, $CF_3$ und Ph;
- Triazinyl, das optional substituiert ist mit einem oder mehreren Substituenten, unabhängig voneinander ausgewählt aus der Gruppe bestehend aus Me, $^i$Pr, $^t$Bu, CN, $CF_3$ und Ph;
und
- $N(Ph)_2$.

**9.** Verfahren zum Herstellen eines organischen Moleküls nach den Ansprüchen 1 bis 8, umfassend das Bereitstellen eines $R^1$-, $R^2$-substituierten $Hal^a$-Fluorpyridins als Reaktant, wobei $Hal^a$ aus der Gruppe bestehend aus Cl, Br und

I ausgewählt ist.

**10.** Verwendung eines organischen Moleküls nach einem oder mehreren der Ansprüche 1 bis 8 als ein Lumineszenzemitter und/oder als ein Wirtmaterial und/oder als ein Elektronentransportmaterial und/oder als ein Lochinjektionsmaterial und/oder als ein Lochblockiermaterial in einer optoelektronischen Vorrichtung.

**11.** Verwendung nach Anspruch 10, wobei die optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren,
- organischen Dioden,
- organischen Solarzellen,
- organischen Transistoren,
- organischen Feldeffekttransistoren,
- organischen Lasern und
- Abwärtswandlungselementen.

**12.** Zusammensetzung, umfassend:

(a) ein organisches Molekül nach einem oder mehreren der Ansprüche 1 bis 8, insbesondere in der Form eines Emitters und/oder eines Wirts, und
(b) ein Emitter- und/oder Wirtmaterial, das sich von dem organischen Molekül unterscheidet, und
(c) optional einen Farbstoff und/oder ein Lösungsmittel.

**13.** Optoelektronische Vorrichtung, umfassend ein organisches Molekül nach einem oder mehreren der Ansprüche 1 bis 8 oder eine Zusammensetzung nach Anspruch 12, insbesondere in der Form einer Vorrichtung, die ausgewählt ist aus der Gruppe bestehend aus: organischer lichtemittierender Diode (OLED), lichtemittierender elektrochemischer Zelle, OLED-Sensor, organischer Diode, organischer Solarzelle, organischem Transistor, organischem Feldeffekttransistor, organischem Laser und Abwärtswandlungselement.

**14.** Optoelektronische Vorrichtung nach Anspruch 13, mit oder bestehend aus:

- einem Substrat,
- einer Anode und
- einer Kathode, wobei die Anode oder die Kathode auf dem Substrat angeordnet sind, und
- mindestens einer lichtemittierenden Schicht, die zwischen der Anode und der Kathode angeordnet ist und die das organische Molekül oder die Zusammensetzung umfasst.

**15.** Verfahren zum Herstellen einer optoelektronischen Vorrichtung, wobei ein organisches Molekül nach einem der Ansprüche 1 bis 8 oder eine Zusammensetzung nach Anspruch 12 verwendet wird, insbesondere umfassend das Verarbeiten des organischen Moleküls oder der Zusammensetzung durch ein Vakuumverdampfungsverfahren oder aus einer Lösung.

## Revendications

**1.** Molécule organique, comprenant

- une première fraction chimique comprenant une structure de formule I,

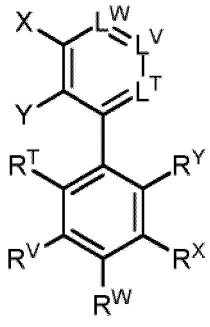

Formule I

et

- deux secondes fractions chimiques, comprenant chacune, indépendamment de l'autre, une structure de formule II,

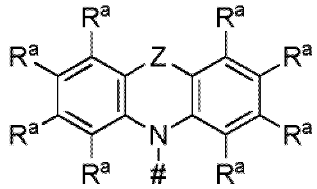

Formule II

dans laquelle la première fraction chimique est reliée à chacune des deux secondes fractions chimiques par une liaison simple ;
dans laquelle

$L^T$ est N ou C-$R^1$ ;
$L^V$ est N ou C-$R^1$ ;
$L^W$ est N ou C-W ;
W est le site de liaison d'une liaison simple reliant la première fraction chimique à l'une des deux secondes fractions chimiques, ou est $R^2$ ;
X est le site de liaison d'une liaison simple reliant la première fraction chimique à l'une des deux secondes fractions chimiques, ou est $R^2$ ;
Y est le site de liaison d'une liaison simple reliant la première fraction chimique à l'une des deux secondes fractions chimiques, ou est $R^2$ ;
$R^T$ est choisi dans le groupe consistant en $R^B$ et $R^I$ ;
$R^V$ est choisi dans le groupe consistant en $R^B$ et $R^I$ ;
$R^W$ est le site de liaison d'une liaison simple reliant la première fraction chimique à l'une des deux secondes fractions chimiques, ou est choisi dans le groupe consistant en $R^B$ et $R^I$ ;
$R^X$ est le site de liaison d'une liaison simple reliant la première fraction chimique à l'une des deux secondes fractions chimiques, ou est choisi dans le groupe consistant en $R^B$ et $R^I$ ;
$R^Y$ est le site de liaison d'une liaison simple reliant la première fraction chimique à l'une des deux secondes fractions chimiques, ou est choisi dans le groupe consistant en $R^B$ et $R^I$ ;
$R^B$ comprend une structure de formule BZN :

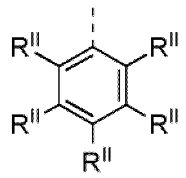

Formule BZN

dans laquelle la ligne en pointillé représente la liaison simple reliant la structure de formule BZN au cycle de benzène inférieur de la structure illustrée dans la formule I ;

# représente le site de liaison d'une liaison simple reliant les secondes fractions chimiques à la première fraction chimique ;
Z est, à chaque occurrence, indépendamment d'un autre, choisi dans le groupe consistant en : une liaison directe, $CR^3R^4$, $C=CR^3R^4$, C=O, $C=NR^3$, $NR^3$, O, $SiR^3R^4$, S, S(O) et $S(O)_2$ ;
$R^1$ est, à chaque occurrence, indépendamment d'un autre, choisi dans le groupe consistant en :
hydrogène, deutérium,
alkyle en $C_1$ à $C_5$,
dans lequel un ou plusieurs atomes d'hydrogène sont facultativement substitués par du deutérium ;
alcényle en $C_2$ à $C_8$,
dans lequel un ou plusieurs atomes d'hydrogène sont facultativement substitués par du deutérium ;
alcynyle en $C_2$ à $C_8$,
dans lequel un ou plusieurs atomes d'hydrogène sont facultativement substitués par du deutérium ; et
aryle en $C_6$ à $C_{18}$,
qui est facultativement substitué par un ou plusieurs substituants $R^6$ ;
$R^2$ est, à chaque occurrence, indépendamment d'un autre, choisi dans le groupe consistant en :

hydrogène, deutérium,
alkyle en $C_1$ à $C_5$,
dans lequel un ou plusieurs atomes d'hydrogène sont facultativement substitués par du deutérium ;
alcényle en $C_2$ à $C_8$,
dans lequel un ou plusieurs atomes d'hydrogène sont facultativement substitués par du deutérium ;
alcynyle en $C_2$ à $C_8$,
dans lequel un ou plusieurs atomes d'hydrogène sont facultativement substitués par du deutérium ; et
aryle en $C_6$ à $C_{18}$,
qui est facultativement substitué par un ou plusieurs substituants $R^6$ ;

$R^I$ est, à chaque occurrence, indépendamment d'un autre, choisi dans le groupe consistant en :

hydrogène, deutérium,
alkyle en $C_1$ à $C_5$,
dans lequel un ou plusieurs atomes d'hydrogène sont facultativement substitués par du deutérium ;
alcényle en $C_2$ à $C_8$,
dans lequel un ou plusieurs atomes d'hydrogène sont facultativement substitués par du deutérium ;
alcynyle en $C_2$ à $C_8$,
dans lequel un ou plusieurs atomes d'hydrogène sont facultativement substitués par du deutérium ; et
aryle en $C_6$ à $C_{18}$,
qui est facultativement substitué par un ou plusieurs substituants $R^6$ ;
$R^{II}$ est, à chaque occurrence, indépendamment d'un autre, choisi dans le groupe consistant en $R^{III}$ et $R^{IV}$ ;
$R^{III}$ est choisi dans le groupe consistant en CN et $CF_3$ ;

$R^{IV}$ est, à chaque occurrence, indépendamment d'un autre, choisi dans le groupe consistant en :

hydrogène, deutérium,
alkyle en $C_1$ à $C_5$,

dans lequel un ou plusieurs atomes d'hydrogène sont facultativement substitués par du deutérium ;
alcényle en $C_2$ à $C_8$,
dans lequel un ou plusieurs atomes d'hydrogène sont facultativement substitués par du deutérium ;
alcynyle en $C_2$ à $C_8$,
dans lequel un ou plusieurs atomes d'hydrogène sont facultativement substitués par du deutérium ; et
aryle en $C_6$ à $C_{18}$,
qui est facultativement substitué par un ou plusieurs substituants $R^6$ ;
$R^a$, $R^3$ et $R^4$ sont, à chaque occurrence, indépendamment d'un autre, choisi dans le groupe consistant en : hydrogène, deutérium, $N(R^5)_2$, $OR^5$, $Si(R^5)_3$, $B(OR^5)_2$, $OSO_2R^5$, $CF_3$, CN, F, Br, I,
alkyle en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ;
hétéroaryle en $C_3$ à $C_{57}$,
qui est facultativement substitué par un ou plusieurs substituants $R^5$ ; et
un système de cycle mono- ou polycyclique, aliphatique, aromatique et/ou benzo-fusionné formé par fermeture de cycle avec un ou plusieurs des autres substituants choisis dans le groupe consistant en $R^a$, $R^3$, $R^4$ et $R^5$ ;
$R^5$ est, à chaque occurrence, indépendamment d'un autre, choisi dans le groupe consistant en hydrogène, deutérium, $N(R^6)_2$, $OR^6$, $Si(R^6)_3$, $B(OR^6)_2$, $OSO_2R^6$, $CF_3$, CN, F, Br, I,
alkyle en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcoxy en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
thioalcoxy en $C_1$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcényle en $C_2$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcynyle en $C_2$ à $C_{40}$,
qui est facultativement substitué par un ou plusieurs substituants $R^6$ et
dans lequel un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;

aryle en $C_6$ à $C_{60}$,
qui est facultativement substitué par un ou plusieurs substituants $R^6$ ; et
hétéroaryle en $C_3$ à $C_{57}$,
qui est facultativement substitué par un ou plusieurs substituants $R^6$ ; et
un système de cycle mono- ou polycyclique, aliphatique, aromatique et/ou benzo-fusionné formé par fermeture de cycle avec un ou plusieurs des autres substituants choisis dans le groupe consistant en $R^a$, $R^3$, $R^4$ et $R^5$ ;

$R^6$ est, à chaque occurrence, indépendamment d'un autre, choisi dans le groupe consistant en :

hydrogène, deutérium, OPh, $CF_3$, CN, F,
alkyle en $C_1$ à $C_5$,
dans lequel un ou plusieurs atomes d'hydrogène sont facultativement, indépendamment les uns des autres, substitués par du deutérium, CN, $CF_3$ ou F;
alcoxy en $C_1$ à $C_5$,
dans lequel un ou plusieurs atomes d'hydrogène sont facultativement, indépendamment les uns des autres, substitués par du deutérium, CN, $CF_3$ ou F;
thioalcoxy en $C_1$ à $C_5$,
dans lequel un ou plusieurs atomes d'hydrogène sont facultativement, indépendamment les uns des autres, substitués par du deutérium, CN, $CF_3$ ou F;
alcényle en $C_2$ à $C_5$,
dans lequel un ou plusieurs atomes d'hydrogène sont facultativement, indépendamment les uns des autres, substitués par du deutérium, CN, $CF_3$ ou F;
alcynyle en $C_2$ à $C_5$,
dans lequel un ou plusieurs atomes d'hydrogène sont facultativement, indépendamment les uns des autres, substitués par du deutérium, CN, $CF_3$ ou F;
aryle en $C_6$ à $C_{18}$,
qui est facultativement substitué par un ou plusieurs substituants alkyle en $C_1$ à $C_5$ ;
hétéroaryle en $C_3$ à $C_{17}$,
qui est facultativement substitué par un ou plusieurs substituants alkyle en $C_1$ à $C_5$ ;
N(aryle en $C_6$ à $C_{18}$)$_2$ ;
N(hétéroaryle en $C_3$ à $C_{17}$)$_2$,
et N(hétéroaryl en $C_3$ à $C_{17}$)(aryle en $C_6$ à $C_{18}$) ;
dans laquelle facultativement, les substituants $R^a$, $R^3$, $R^4$ ou $R^5$, indépendamment les uns des autres, forment un système de cycle mono- ou polycyclique, aliphatique, aromatique et/ou benzo-fusionné avec un ou plusieurs substituants des autres substituants $R^a$, $R^3$, $R^4$ ou $R^5$ ;
dans laquelle le système de cycle mono- ou polycyclique, aliphatique, aromatique et/ou benzo-fusionné est facultativement substitué par au moins un substituant $R^5$, et
forme facultativement un système de cycle mono- ou polycyclique, aliphatique, aromatique et/ou benzo-fusionné conjointement avec les un ou plusieurs des autres $R^a$, $R^3$, $R^4$ ou $R^5$ au sein de la molécule organique ;
dans laquelle le système de cycle mono- ou polycyclique, aliphatique, aromatique et/ou benzo-fusionné est facultativement substitué par au moins un substituant $R^6$ ;
dans laquelle
exactement un chaînon de cycle choisi dans le groupe consistant en $L^T$, $L^V$ et $L^W$ est N ;
exactement un substituant choisi dans le groupe consistant en $R^T$, $R^V$, $R^X$, $R^Y$ et $R^W$ est $R^B$ ;
exactement un substituant $R^{II}$ est $R^{III}$ ;
exactement un substituant choisi dans le groupe consistant en W, Y et X représente le site de liaison d'une liaison simple reliant la première fraction chimique et l'une des deux secondes fractions chimiques ; et
exactement un substituant choisi dans le groupe consistant en $R^W$, $R^Y$ et $R^X$ représente le site de liaison d'une liaison simple reliant la première fraction chimique et l'une des deux secondes fractions chimiques.

**2.** Molécule organique selon la revendication 1, dans laquelle la première fraction chimique comprend une structure de formule Iaa :

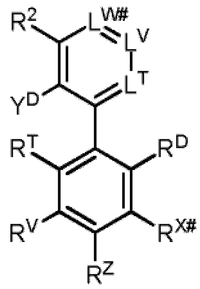

Formule Iaa

dans laquelle

$L^{W\#}$ est N ou C-$R^2$ ;
$R^Z$ est choisi dans le groupe consistant en $R^I$ et $R^B$ ;
$R^{X\#}$ est choisi dans le groupe consistant en $R^I$ et $R^B$ ;
$R^D$ est le site de liaison d'une liaison simple reliant la première fraction chimique à l'une des deux secondes fractions chimiques ;
$Y^D$ est le site de liaison d'une liaison simple reliant la première fraction chimique à l'une des deux secondes fractions chimiques ;
dans laquelle exactement un substituant choisi dans le groupe consistant en $L^{W\#}$ , $L^V$, $L^T$ est N ;
et dans laquelle exactement un substituant choisi dans le groupe consistant en $R^V$, $R^T$, $R^{X\#}$ et $R^Z$ est $R^B$.

**3.** Molécule organique selon la revendication 1 ou 2, dans laquelle $R^1$, $R^2$, $R^I$ et $R^{IV}$, à chaque occurrence, indépendamment les uns des autres, sont choisis dans le groupe consistant en : H, méthyle, mésityle, tolyle et phényle.

**4.** Molécule organique selon une ou plusieurs des revendications 1 à 3, dans laquelle $R^1$, $R^2$, $R^I$ et $R^{IV}$ est H.

**5.** Molécule organique selon une ou plusieurs des revendications 1 à 4, dans laquelle les deux secondes fractions chimiques comprennent chacune, à chaque occurrence, indépendamment d'une autre, une structure de formule IIa :

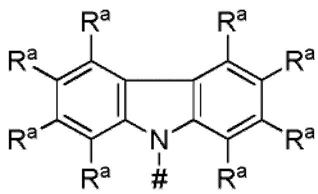

Formule IIa.

**6.** Molécule organique selon une ou plusieurs des revendications 1 à 5, dans laquelle les deux secondes fractions chimiques, à chaque occurrence indépendamment d'une autre, comprennent une structure de formule IIb :

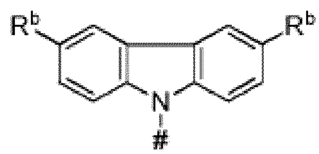

Formule IIb

dans laquelle

$R^b$ est, à chaque occurrence, indépendamment d'un autre, choisi dans le groupe consistant en : deutérium, $N(R^5)_2$, $OR^5$, $Si(R^5)_3$, $B(OR^5)_2$, $OSO_2R^5$, $CF_3$, CN, F, Br, I,
alkyle en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ; et
hétéroaryle en $C_3$ à $C_{57}$,
qui est facultativement substitué par un ou plusieurs substituants $R^5$.

**7.** Molécule organique selon une ou plusieurs des revendications 1 à 5, dans laquelle les deux secondes fractions chimiques comprennent, à chaque occurrence indépendamment d'une autre, une structure de formule IIc :

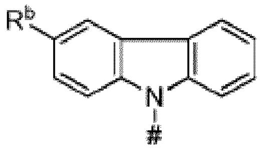

Formule IIc

dans laquelle

$R^b$ est, à chaque occurrence, indépendamment d'un autre, choisi dans le groupe consistant en deutérium, $N(R^5)_2$, $OR^5$, $Si(R^5)_3$, $B(OR^5)_2$, $OSO_2R^5$, $CF_3$, CN, F, Br, I,
alkyle en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C,

$Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans lequel un ou plusieurs groupes $CH_2$ non adjacents sont facultativement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle à $C_6$ à $C_{60}$,
qui est facultativement substitué par un ou plusieurs substituants $R^5$ ; et
hétéroaryle en $C_3$ à $C_{57}$,
qui est facultativement substitué par un ou plusieurs substituants $R^5$.

**8.** Molécule organique selon la revendication 6 ou 7, dans laquelle $R^b$ est à chaque occurrence, indépendamment d'un autre, choisi dans le groupe consistant en :

- Me, $^i$Pr, $^t$Bu, CN, $CF_3$,
- Ph, qui est facultativement substitué par un ou plusieurs substituants choisis indépendamment les uns des autres dans le groupe consistant en Me, $^i$Pr, $^t$Bu, CN, $CF_3$ et Ph ;
- pyridinyle, qui est facultativement substitué par un ou plusieurs substituants choisis indépendamment les uns des autres dans le groupe consistant en Me, $^i$Pr, $^t$Bu, CN, $CF_3$ et Ph ;
- pyrimidyle, qui est facultativement substitué par un ou plusieurs substituants choisis indépendamment les uns des autres dans le groupe consistant en Me, $^i$Pr, $^t$Bu, CN, $CF_3$ et Ph ;
- carbazolyle, qui est facultativement substitué par un ou plusieurs substituants choisis indépendamment les uns des autres dans le groupe consistant en Me, $^i$Pr, $^t$Bu, CN, $CF_3$ et Ph ;
- triazinyle, qui est facultativement substitué par un ou plusieurs substituants choisis indépendamment les uns des autres dans le groupe consistant en Me, $^i$Pr, $^t$Bu, CN, $CF_3$ et Ph ;

et
- $N(Ph)_2$.

**9.** Procédé de préparation d'une molécule organique selon les revendications 1 à 8, comprenant la fourniture d'une $R^1$-,$R^2$-$Hal^a$-fluoropyridine substituée comme réactif, dans lequel $Hal^a$ est choisi dans le groupe consistant en Cl, Br et I.

**10.** Utilisation d'une molécule selon une ou plusieurs des revendications 1 à 8, comme émetteur luminescent et/ou matériau hôte et/ou matériau de transport d'électrons et/ou matériau d'injection de trous et/ou matériau de blocage de trous dans un dispositif optoélectronique.

**11.** Utilisation selon la revendication 10, dans laquelle le dispositif optoélectronique est choisi dans le groupe consistant en :

• les diodes électroluminescentes organiques (DELO),
• les cellules électrochimiques électroluminescentes,
• les capteurs DELO,
• les diodes organiques,
• les cellules solaires organiques,
• les transistors organiques,
• les transistors à effet de champ organiques,
• les lasers organiques et
• les éléments de conversion vers le bas.

**12.** Composition, comprenant :

(a) une molécule organique selon une ou plusieurs des revendications 1 à 8, en particulier sous la forme d'un émetteur et/ou d'un hôte, et
(b) un matériau émetteur et/ou hôte, qui diffèrent de la molécule organique, et
(c) facultativement, une teinture et/ou un solvant.

**13.** Dispositif optoélectronique, comprenant une molécule organique selon une ou plusieurs des revendications 1 à 8 ou une composition selon la revendication 12, en particulier sous la forme d'un dispositif choisi dans le groupe consistant en : une diode électroluminescente organique (DELO), une cellule électrochimique électroluminescente,

un capteur DELO, une diode organique, une cellule solaire organique, un transistor organique, un transistor à effet de champ organique, un laser organique et un élément de conversion vers le bas.

**14.** Dispositif optoélectronique selon la revendication 13, comprenant ou consistant en :

- un substrat,
- une anode et
- une cathode, l'anode ou la cathode étant disposées sur le substrat, et
- au moins une couche électroluminescente, qui est disposée entre l'anode et la cathode et qui comprend la molécule organique ou la composition.

**15.** Procédé de fabrication d'un dispositif optoélectronique, dans lequel une molécule organique selon l'une quelconque des revendications 1 à 8 ou une composition selon la revendication 12 est utilisée, comprenant en particulier le traitement de la molécule organique ou de la composition par un procédé d'évaporation sous vide ou à partir d'une solution.

**Figure 1**

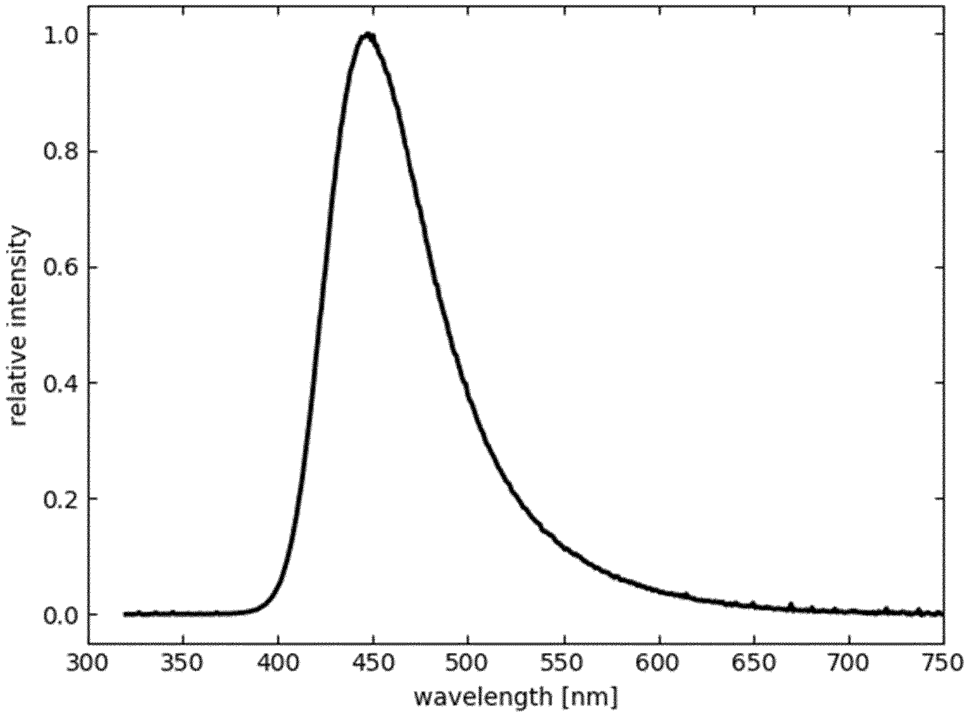


**Figure 2**

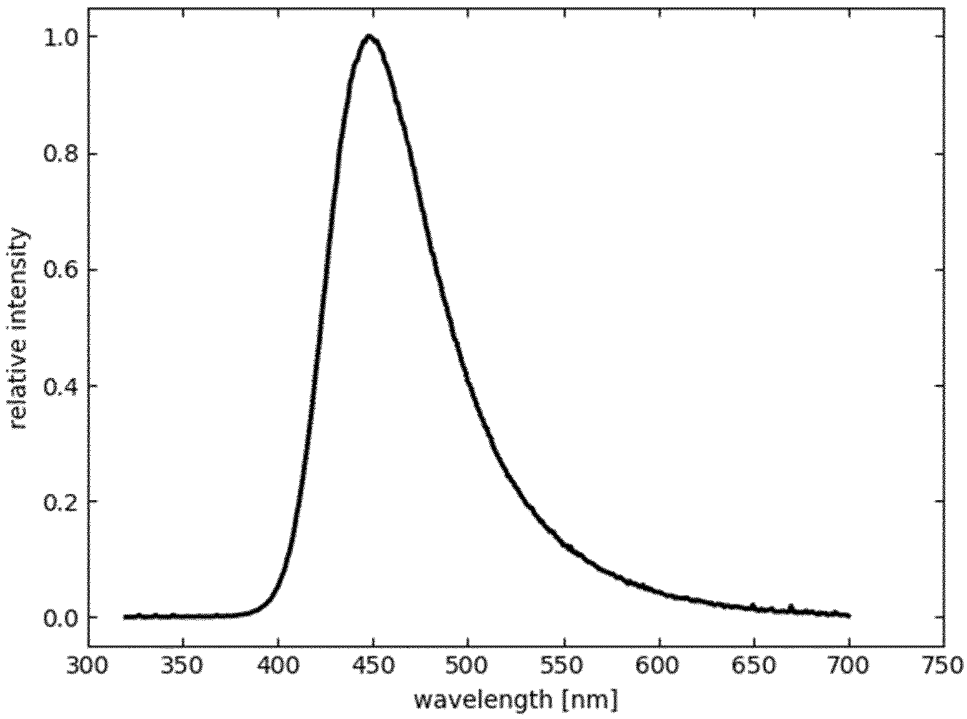

Figure 3

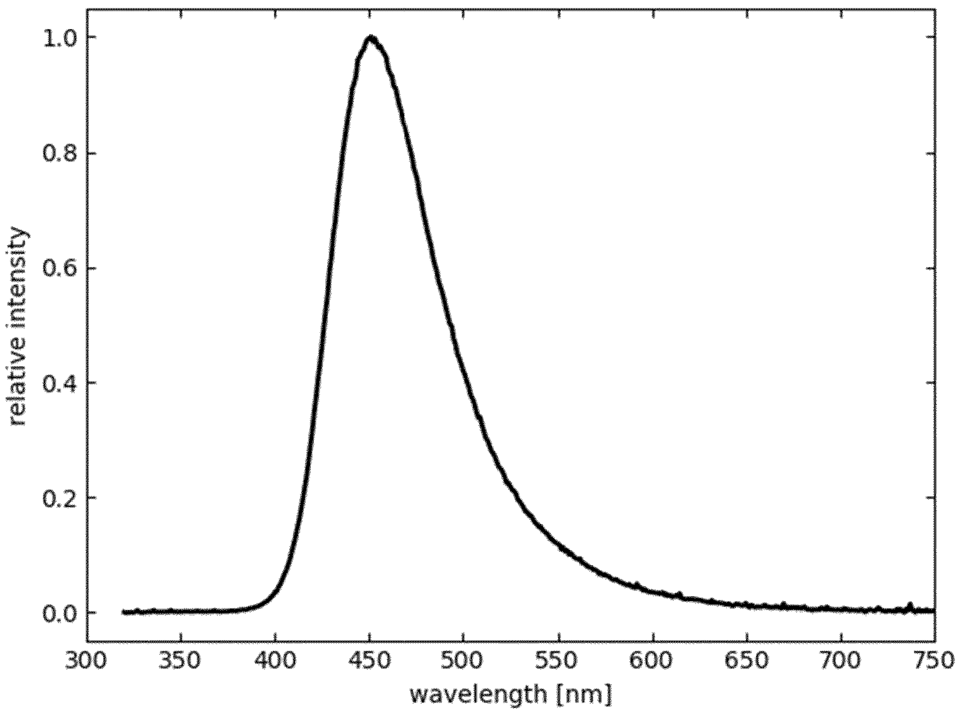


Figure 4

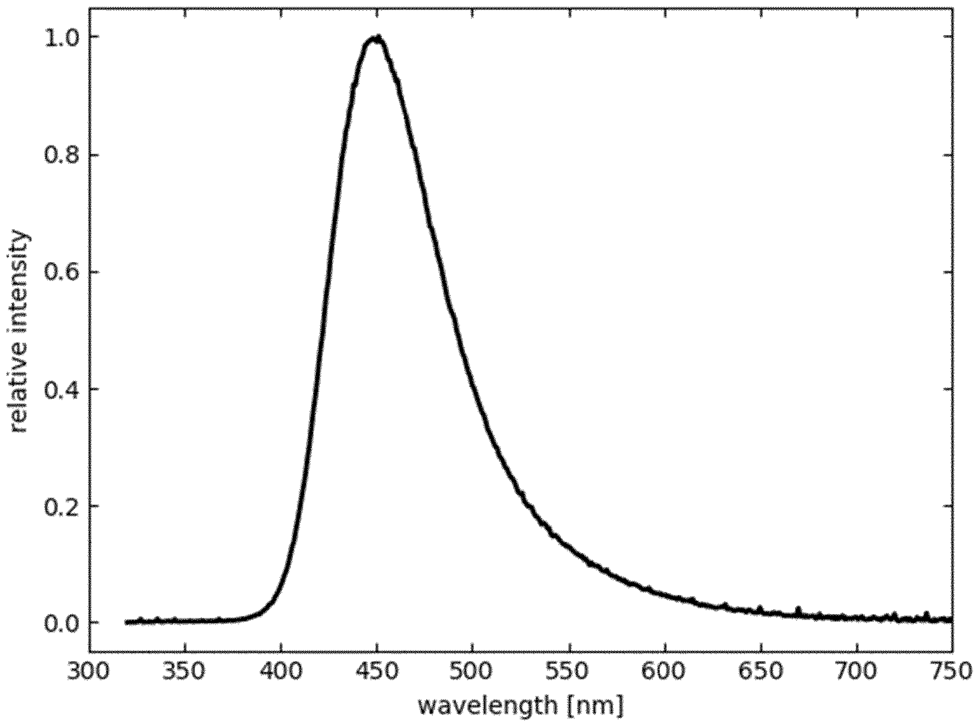

Figure 5

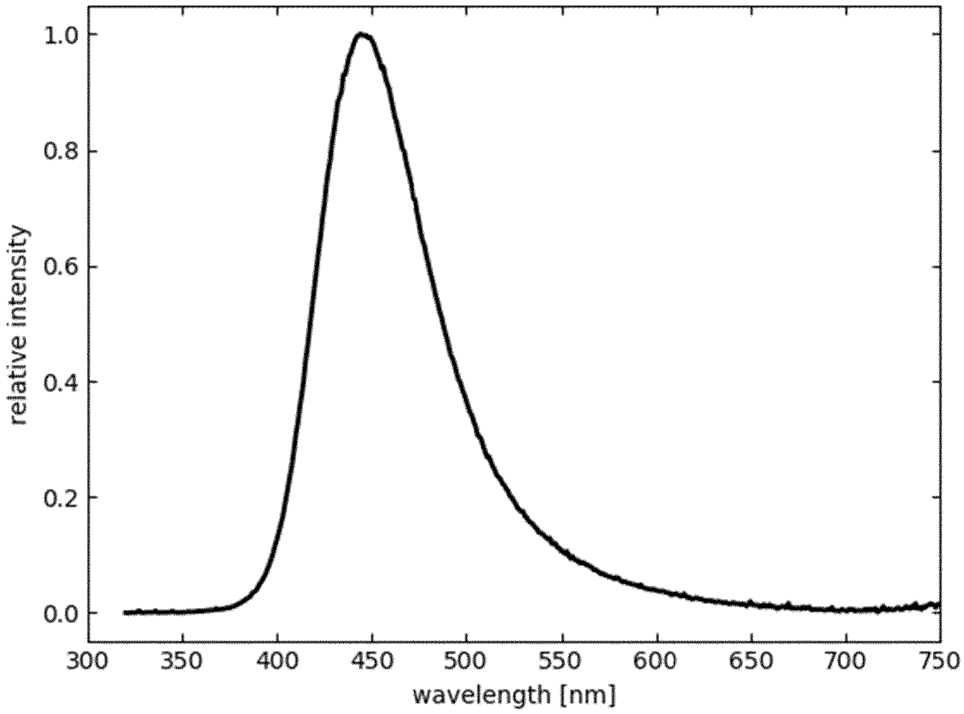


Figure 6

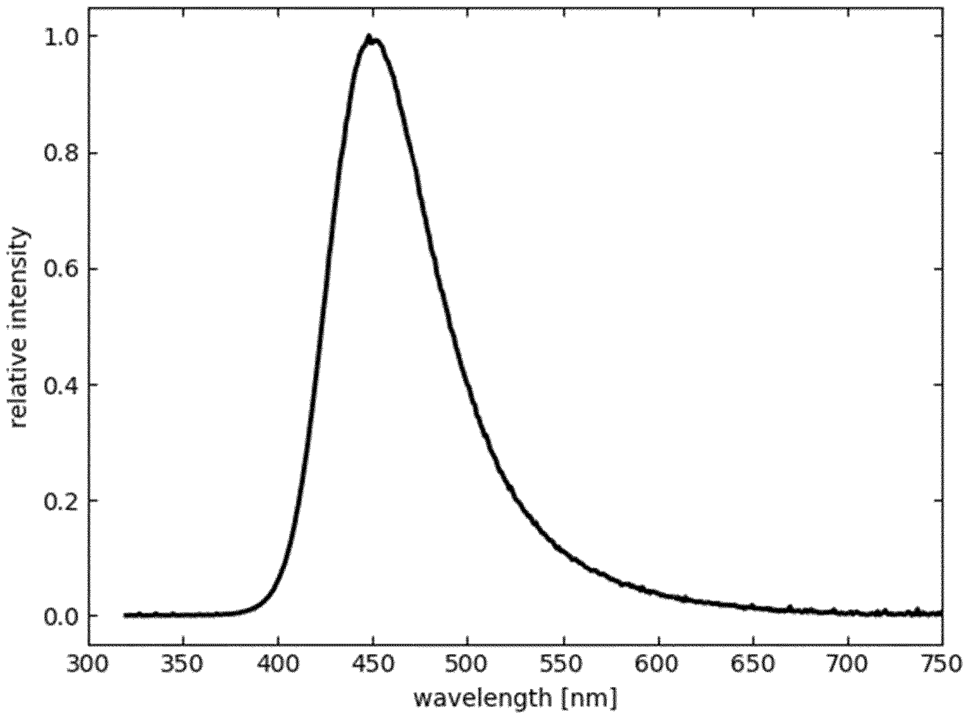

**Figure 7**

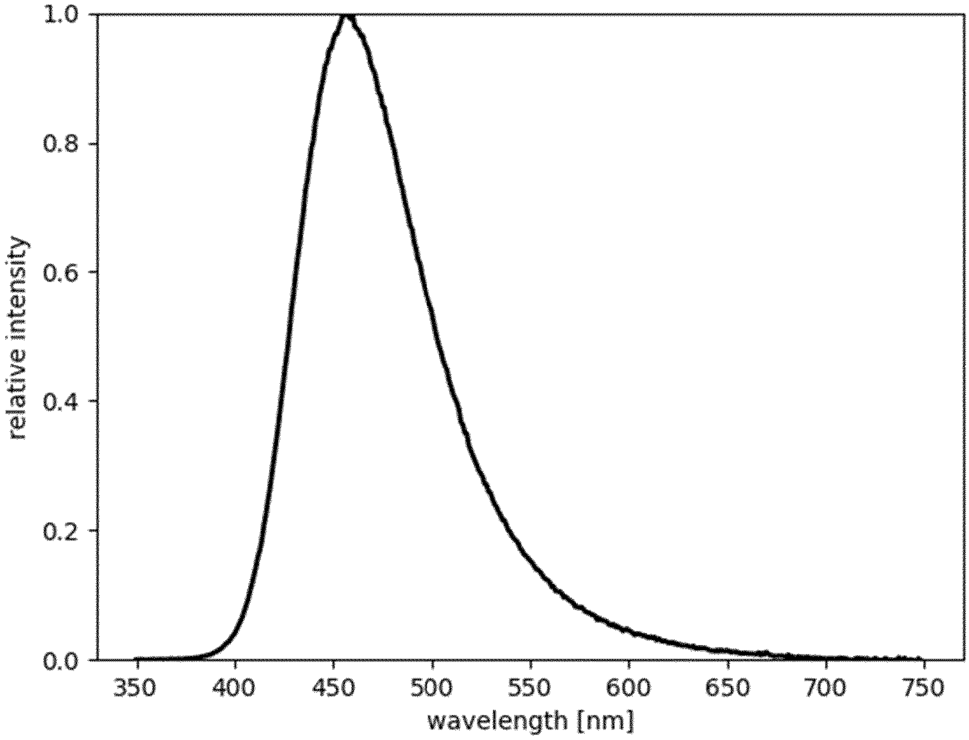
1.0
0.8
0.6
0.4
0.2
0.0
relative intensity
350
400
450
500
550
600
650
700
750
wavelength [nm]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3287451 A


### Non-patent literature cited in the description


- *CHEMICAL ABSTRACTS,* 73183-34-3
- *CHEMICAL ABSTRACTS,* 2546-56-7
- *CHEMICAL ABSTRACTS,* 352535-83-2
- *CHEMICAL ABSTRACTS,* 138642-62-3
- *CHEMICAL ABSTRACTS,* 150255-96-2
- *CHEMICAL ABSTRACTS,* 126747-14-6
- *CHEMICAL ABSTRACTS,* 1060735-14-9